# EUROPEAN PATENT APPLICATION

(11) **EP 4 538 270 A1**
(43) Date of publication of application: **16.04.2025**
(21) Application number: 23820162.8
(22) Date of filing: 12.06.2023
(51) Int. Cl.: C07D 403/12, A61K 31/498, A61K 31/506, A61K 31/4985, A61P 11/00, A61P 43/00, C07D 403/06, C07D 401/12, C07D 471/04, C07D 403/14

(54) **NOVEL COMPOUND, AND PHARMACEUTICAL COMPOSITION COMPRISING SAME AS ACTIVE INGREDIENT**

(30) Priority: 10.06.2022 KR 20220070989
(71) Applicant: Sapiensbio Inc., Seongnam-si, Gyeonggi-do 13207 (KR)
(72) Inventor: YOON, Dong-Oh, Seongnam-si Gyeonggi-do 13207 (KR); RYU, Incheol, Seongnam-si Gyeonggi-do 13207 (KR); RYOO, Kanghyun, Seongnam-si Gyeonggi-do 13207 (KR); LEE, Hyunseung, Seongnam-si Gyeonggi-do 13207 (KR); PARK, Yeonkyeong, Seongnam-si Gyeonggi-do 13207 (KR); AN, Sihyeon, Seongnam-si Gyeonggi-do 13207 (KR); LEE, Myongwoo, Seongnam-si Gyeonggi-do 13207 (KR)
(74) Representative: Dehns
(86) International application number: PCT/KR2023/008062
(87) International publication number: WO 2023/239227

(57) **Abstract**

The present invention relates to a novel compound that regulates the function and differentiation of the cytoskeleton, which is involved in pulmonary fibrosis, and thus is useful in the preparation of drugs that can treat associated diseases. It has been identified that a novel compound or a pharmaceutically acceptable salt thereof, according to the present invention, has the effect of regulating actin polymerization required for the change of cytoskeleton, and that the expression of α-SMA and F-actin, which are main factors causing fibrosis, are effectively inhibited in human lung tissue-derived fibroblasts (MRC5). In addition, it has been identified that when activated macrophages are treated with the compound, the level of inflammation of inflammatory cytokines, caused by macrophages, is reduced. Therefore, a novel compound according to the present invention is expected to be effectively used, as a preparation having the effect of inhibiting or alleviating fibrosis of the lungs, in the prevention and treatment of diseases associated with pulmonary fibrosis.

## Description

### Technical Field

The present invention relates to a novel compound, and a pharmaceutical composition including the same as active ingredients.

This application claims priority to and the benefit of Korean Patent Application No. 10-2022-0070989, filed on June 10, 2022, and Korean Patent Application No. 10-2023-0074884, filed on June 12, 2023, the disclosure of which is incorporated herein by reference in their entirety.

### Background Art

Idiopathic pulmonary fibrosis (IPF) is a disease in which the fibrosis of lung parenchyma chronically progresses and is characterized by the infiltration of immune cells into the alveolar wall, inducing fibrosis and causing serious structural changes in lung tissue, resulting in a gradual decline in lung function. If the fibrosis of the lung parenchyma continues to progress due to IPF, respiratory failure may occur and even lead to death. However, since there is no effective treatment, IPF is a highly fatal disease that has a 5-year survival rate of less than 40%, and a 10-year survival rate of less than 15%, after diagnosis. Pirfenidone and nintedanib that are currently used clinically as therapeutic drugs for pulmonary fibrosis were approved by the U.S. FDA, but exhibit a low clinical efficacy by showing a lung function improvement of only about 10%. In addition, both drugs show no significant difference in extending the patient's life expectancy after diagnosis. Accordingly, there is a need for the development of a treatment for pulmonary fibrosis through a new mechanism.

In damaged tissue, inflammatory cytokines such as TGF-β and TNF-α are produced and secreted out of cells, causing functional and structural changes in the cytoskeleton and a healing process. In normal conditions, after the healing process, inflammatory cytokines and the accumulated extracellular matrix (ECM) are reduced and a recovery process occurs, but in pathological conditions, the accumulation of the ECM continues without normal recovery proceeding. This is one of the main mechanisms causing fibrosis. Cells respond to external physical stimuli by causing changes in gene expression, cell morphology, and the cytoskeleton through changes in intracellular signal transmission (mechanotransduction) and cell-cell interactions. These cellular changes serve as an important driving force for cell movement and cell surface remodeling. When the damaged tissue cannot be restored to its original state through repeated abnormal healing processes due to the continuous influx of various external factors, the damaged tissue is converted into fibrotic tissue.

Epithelial-mesenchymal transition (EMT) and fibroblast-myofibroblast transition (FMT) are known as the key mechanisms causing pulmonary fibrosis. In the damaged tissue of the alveoli, the inflammatory cytokine, TGF-β, is secreted, and in the activated epithelial layer, EMT results from the breakdown of cell connective tissue caused by an inflammatory signal, and the epithelial cells are converted into fibroblasts. In addition, when fibroblasts present in the subepithelial layer receive mechanical and physical damage from external stimuli, stress fibers are expressed via the mechanotransduction pathway. In addition, it is effectively activated by cytokines causing fibrosis, and fibroblasts are converted into myofibroblasts by FMT. Changes in the cytoskeleton occur from the above process. The cytoskeleton consists of microtubules, actin microfilaments, and intermediate filaments and not only maintains the skeletal structure of cells but also performs various roles such as cell movement and interaction with an external environment. Among them, the actin microfilament is a microfilament consist of a filamentous actin (F-actin) as a polymeric filament, and is involved in the mobility of cells such as lamellipodia or filopodia, and is also very important in cell transformation (EMT and FMT) caused by external mechanical stimuli. Cell transformation is achieved by changes in the cytoskeleton, and one of the factors that cause changes in the cytoskeleton is actin polymerization.

The control of cytoskeletal changes is important in pulmonary fibrosis, and an abnormal pulmonary fibrosis mechanism may be improved by the control of actin polymerization. Cells control cell mobility through actin polymerization and form stress fibers to cause cytoskeletal remodeling, and the target proteins involved therein are actin-binding proteins. Arp2/3 complex, which is one of the target proteins, acts on nucleation among various steps that control the mobility of the cytoskeleton and serves to form filament branches, and consists of 7 subunits (Arp2, Arp3, ARPC1, ARPC2, ARPC3, ARPC4, and ARPC5). Here, the Arp2/3 complex affects the amplification of actin microfilaments through actin branching and is associated with the speed of actin network formation in fibroblasts. Accordingly, cell transformation may be controlled by controlling the function of the Arp2/3 complex to suppress changes in the actin network and cytoskeleton due to actin polymerization.

Pulmonary fibrosis occurs between tissue and blood vessels surrounding the alveoli, and particularly, IPF is a disease in which tissue is gradually hardened by the accumulation of ECM due to myofibroblasts. This disease causes the contraction of myofibroblasts induced by changes in the cytoskeleton of fibroblasts, resulting in the induction of tissue hardening and reduced respiratory function of the lungs. Change of the cytoskeleton is associated with the activity of myofibroblasts, and the key biomarker for the activity of myofibroblasts is known to be α-smooth muscle actin (α-SMA). Recent study results reveal that, when IPF-derived fibroblasts were treated with the cytokine TGF-β, α-SMA expression increased. Accordingly, a hallmark of the activity of myofibroblasts involved in fibrosis is α-SMA expression. From previous study results, the improvement of cell transformation by the control of the cytoskeleton is believed to be a mechanism for treating IPF. Therefore, it is intended to develop a drug for treating various fibrosis-related diseases including IPF by developing a material for controlling the function of the Arp2/3 complex involved in actin polymerization that controls cytoskeletal changes.

### Disclosure

### Technical Problem

The present invention was devised to solve the above problems, and the inventors developed a novel compound which can treat cytoskeleton-related diseases such as pulmonary fibrosis by inhibiting a pulmonary fibrosis factor and an inflammatory factor and regulating actin polymerization, thereby completing the present invention.

Accordingly, the present invention is directed to providing a compound represented by Chemical Formula 1 below, a stereoisomer thereof, or a pharmaceutically acceptable salt thereof:

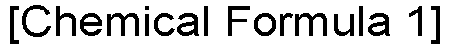

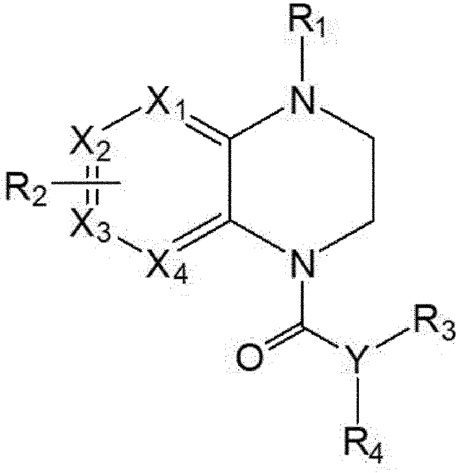

The present invention is also directed to providing a pharmaceutical composition for preventing or treating a pulmonary fibrosis-related disease, which includes the compound, a stereoisomer thereof, or a pharmaceutically acceptable salt thereof as an active ingredient.

The present invention is also directed to providing a kit for preventing or treating a pulmonary fibrosis-related disease, which includes the composition.

However, technical problems to be solved in the present invention are not limited to the above-described problems, and other problems which are not described herein will be fully understood by those of ordinary skill in the art from the following descriptions.

Related art documents of the present invention are as follows:
(Non-Patent Document 001) Cold Spring Harb. Perspect. Biol. 8, a018226 (2016)
(Non-Patent Document 002) J. Clin. Invest. 119, 1420-1428 (2009).
(Non-Patent Document 003) Front. Bioeng. Biotechnol. 8, 609653 (2020).
(Non-Patent Document 004) Cancers 13, 1882 (2021)
(Non-Patent Document 005) Mol. Biol. Cell 12, 2730 -2741 (2001)
(Non-Patent Document 006) Am J. Physiol. Lung. Cell. Mol. Physiol. 315, L59-L65 (2018)
(Non-Patent Document 007) Mol. Biol. Cell 24, 2861-2875 (2013)
(Non-Patent Document 008) Front. Pharmacol. 5, 00051 (2014)
(Non-Patent Document 009) Br. J. Pharmacol. 179, 125-140 (2022)

### Technical Solution

The present invention provides a compound represented by Chemical Formula 1 below, a stereoisomer thereof, or a pharmaceutically acceptable salt thereof: 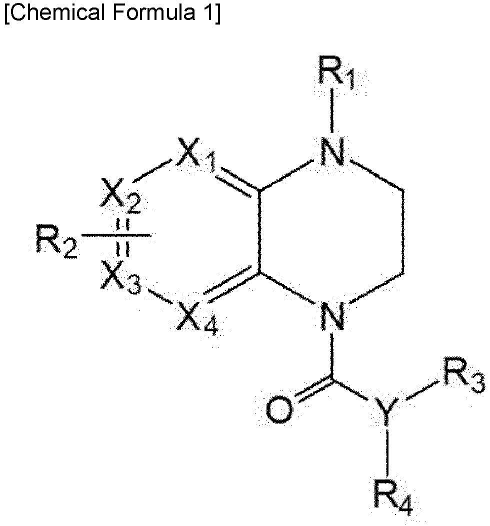 (In Chemical Formula 1,
X₁ to X₄ may each be independently N or C;
Y may be N, S, or O;
R₁ may be C₁-C₁₀ alkyl, C₃-C₂₀ cycloalkyl, C₂-C₂₀ heterocycloalkyl, a 3- to 10-membered cyclic aromatic group, a 3- to 10-membered heterocyclic aromatic group,-CO-(C₁-C₆ alkyl), or -CO-(halogen-substituted or unsubstituted C₆-C₁₂ aryl), and
at least one H of R₁ may be substituted with halogen, a cyano group, halogen-substituted or unsubstituted C₆-C₁₂ aryl, or halogen-substituted or unsubstituted C₅-C₁₂ heteroaryl;
when all of X₁ to X₄ are C, R₂ may be hydrogen, halogen, or C₁-C₅ alkyl, and when one or more of X₁ to X₄ are N, R₂ may be hydrogen;
R₃ and R₄ may be linked to each other to form a 5- or 6-membered ring, wherein at least one H in the ring may be substituted with -OH, -NH₂, dimethylamine, -NH-(C₁-C₅ alkyl), -NH-(C₁-C₅ alkoxy), -NH-COCH₃, -NH-SO₂CH₃, C₁-C₅ alkyl, or C₁-C₅ alkoxy, and
when R₃ and R₄ do not form a ring, R₄ may be hydrogen or C₁-C₅ alkyl, R₃ may be C₁-C₁₀ alkyl, C₃-C₁₀ cycloalkyl, C₂-C₁₀ heterocycloalkyl, -CH₂-(C₃-C₆ cycloalkyl), -CH₂-(C₂-C₆ heterocycloalkyl), or -CH₂-(3- to 6-membered heterocyclic aromatic group), at least one H of R₃ may be substituted with -OH, C₁-C₅ alkyl, acetyl, C₁-C₅ alkoxy, -COCF₃, or -SO₂CH₃; and
the heterocycloalkyl, heterocyclic group, and heteroaryl each independently include one or more hetero atoms selected from the group consisting of N, O, P, and S.)

In another embodiment of the present invention, R₃ and R₄ are linked to each other to form a 5- or 6-membered heterocycloalkyl, which includes one or two N atoms in the ring, wherein Y is N. At least one H in the 5- or 6-membered heterocycloalkyl formed by R₃ and R₄ may be substituted with -OH, -NH₂, dimethylamine, -NH-(C₁-C₅ alkyl), -NH-(C₁-C₅ alkoxy), -NH-COCH₃, -NH-SO₂CH₃, C₁-C₅ alkyl, or C₁-C₅ alkoxy, but the present invention is not limited thereto.

In still another embodiment of the present invention, R₁ may be a 6-membered cyclic aromatic group, 6-membered heterocyclic aromatic group, -CH₂-aryl, -CH₂-heteroaryl, 6-membered cycloalkyl, 6-membered heterocycloalkyl, -CO-aryl, or -CO-(C₄-C₅ alkyl), wherein the 6-membered heterocyclic aromatic group may include at least one N, and the 6-membered heterocycloalkyl may include at least one O. At least one H of R₁ may be substituted with halogen, a cyano group, halogen-substituted or unsubstituted C₆-C₁₂ aryl, or halogen-substituted or unsubstituted C₅-C₁₂ heteroaryl, but the present invention is not limited thereto.

In yet another embodiment of the present invention, the compound represented by Chemical Formula 1 may be any one selected from the group consisting of the following compounds, but the present invention is not limited thereto:
(1) (*R*)-6-fluoro-4-(4-fluorophenyl)-*N*-(pyrrolidin-3-yl)-3,4-dihydroquinoxaline-1 (2H)-carboxamide;
(2) (*R*)-6-fluoro-4-(4-fluorophenyl)-*N*-(1-methylpyrrolidin-3-yl)-3,4-dihydroquinoxaline-1(2H)-carboxamide;
(3) (*R*)-6-fluoro-4-(4-fluorophenyl)-*N*-(1-isopropylpyrrolidin-3-yl)-3,4-dihydroquinoxaline-1(2H)-carboxamide;
(4) (*R*)-6-fluoro-4-(4-fluorophenyl)-*N*-(1-isobutylpyrrolidin-3-yl)-3,4-dihydroquinoxaline-1 (2H)-carboxamide;
(5) (*R*)-6-fluoro-4-(4-fluorophenyl)-*N*-(1-(2,2,2-trifluoroacetyl)pyrrolidin-3-yl)-3,4-dihydroquinoxaline-1(2H)-carboxamide;
(6) (*R*)-*N*-(1-acetylpyrrolidin-3-yl)-6-fluoro-4-(4-fluorophenyl)-3,4-dihydroquinoxaline-1(2H)-carboxamide;
(7) (*R*)-6-fluoro-4-(4-fluorophenyl)-*N*-(1-(methylsulfonyl)pyrrolidin-3-yl)-3,4-dihydroquinoxaline-1(2*H*)-carboxamide;
(8) (*S*)-6-fluoro-4-(4-fluorophenyl)-*N*-(pyrrolidin-3-yl)-3,4-dihydroquinoxaline-1(2H)-carboxamide;
(9) (*S*)-6-fluoro-4-(4-fluorophenyl)-*N*-(1-methylpyrrolidin-3-yl)-3,4-dihydroquinoxaline-1(2H)-carboxamide;
(10) (*S*)-6-fluoro-4-(4-fluorophenyl)-*N*-(1-isopropylpyrrolidin-3-yl)-3,4-dihydroquinoxaline-1(2H)-carboxamide;
(11) (*S*)-(3-aminopyrrolidin-1-yl)(6-fluoro-4-(4-fluorophenyl)-3,4-dihydroquinoxalin-1(2H)-yl)methanone;
(12) (*S*)-(6-fluoro-4-(4-fluorophenyl)-3,4-dihydroquinoxalin-1(2H)-yl)(3-(methylamino)pyrrolidin-1-yl)methanone;
(13) (*S*)-(6-fluoro-4-(4-fluorophenyl)-3,4-dihydroquinoxalin-1(2H)-yl)(3-(isobutylamino)pyrrolidin-1-yl)methanone;
(14) (*S*)-(6-fluoro-4-(4-fluorophenyl)-3,4-dihydroquinoxalin-1(2H)-yl)(3-(isopropylamino)pyrrolidin-1-yl)methanone;
(15) (*S*)-*N*-(1-(6-fluoro-4-(4-fluorophenyl)-1,2,3,4-tetrahydroquinoxaline-1-carbonyl)pyrrolidin-3-yl)acetamide;
(16) (*S*)-*N*-(1-(6-fluoro-4-(4-fluorophenyl)-1,2,3,4-tetrahydroquinoxaline-1-carbonyl)pyrrolidin-3-yl)methanesulfonamide;
(17) (*S*)-6-fluoro-4-(4-fluorophenyl)-*N*-(pyrrolidin-3-ylmethyl)-3,4-dihydroquinoxaline-1(2H)-carboxamide;
(18) (*R*)-6-fluoro-4-(4-fluorophenyl)-*N*-((1-methylpyrrolidin-3-yl)methyl)-3,4-dihydroquinoxaline-1(2H)-carboxamide;
(19) (*R*)-6-fluoro-4-(4-fluorophenyl)-*N*-((1-isopropylpyrrolidin-3-yl)methyl)-3,4-dihydroquinoxaline-1(2H)-carboxamide;
(20) (*R*)-6-fluoro-4-(4-fluorophenyl)-*N*-((1-isobutylpyrrolidin-3-yl)methyl)-3,4-dihydroquinoxaline-1(2H)-carboxamide;
(21) (*R*)-6-fluoro-4-(4-fluorophenyl)-*N*-(pyrrolidin-2-ylmethyl)-3,4-dihydroquinoxaline-1(2H)-carboxamide;
(22) (*R*)-6-fluoro-4-(4-fluorophenyl)-*N*-((1-methylpyrrolidin-2-yl)methyl)-3,4-dihydroquinoxaline-1(2H)-carboxamide;
(23) (*R*)-6-fluoro-4-(4-fluorophenyl)-*N*-((1-isopropylpyrrolidin-2-yl)methyl)-3,4-dihydroquinoxaline-1(2H)-carboxamide;
(24) (*R*)-6-fluoro-4-(4-fluorophenyl)-*N*-((1-isobutylpyrrolidin-2-yl)methyl)-3,4-dihydroquinoxaline-1(2H)-carboxamide;
(25) (*S*)-6-fluoro-4-(4-fluorophenyl)-*N*-((1-methylpyrrolidin-3-yl)methyl)-3,4-dihydroquinoxaline-1(2H)-carboxamide;
(26) (*S*)-6-fluoro-4-(4-fluorophenyl)-*N*-((1-isopropylpyrrolidin-3-yl)methyl)-3,4-dihydroquinoxaline-1(2H)-carboxamide;
(27) (*S*)-6-fluoro-4-(4-fluorophenyl)-*N*-((1-isobutylpyrrolidin-3-yl)methyl)-3,4-dihydroquinoxaline-1(2H)-carboxamide;
(28) *N*-(cyclopropylmethyl)-6-fluoro-4-(4-fluorophenyl)-3,4-dihydroquinoxaline-1 (2H)-carboxamide;
(29) 6-fluoro-4-(4-fluorophenyl)-*N*-(piperidin-4-yl)-3,4-dihydroquinoxaline-1(2H)-carboxamide;
(30) 6-fluoro-4-(4-fluorophenyl)-*N*-(1-methylpiperidin-4-yl)-3,4-dihydroquinoxaline-1(2H)-carboxamide;
(31) 6-fluoro-4-(4-fluorophenyl)-*N*-(1-isopropylpiperidin-4-yl)-3,4-dihydroquinoxaline-1(2H)-carboxamide;
(32) 6-fluoro-4-(4-fluorophenyl)-*N*-(1-isobutylpiperidin-4-yl)-3,4-dihydroquinoxaline-1(2H)-carboxamide;
(33) 6-fluoro-4-(4-fluorophenyl)-*N*-(piperidin-4-ylmethyl)-3,4-dihydroquinoxaline-1 (2H)-carboxamide;
(34) 6-fluoro-4-(4-fluorophenyl)-*N*-(methylpiperidin-4-yl)methyl)-3,4-dihydroquinoxaline-1(2H)-carboxamide;
(35) 6-fluoro-4-(4-fluorophenyl)-*N*-((1-isobutylpiperidin-4-yl)methyl)-3,4-dihydroquinoxaline-1(2H)-carboxamide;
(36) 6-fluoro-4-(4-fluorophenyl)-*N*-((1-isopropylpiperidin-4-yl)methyl)-3,4-dihydroquinoxaline-1(2H)-carboxamide;
(37) *N*-((1H-imidazol-4-)methyl)-6-fluoro-4-(4-fluorophenyl)-3,4-dihydroquinoxaline-1(2H)-carboxamide;
(38) 6-fluoro-4-(4-fluorophenyl)-*N*-(pyrazin-2-ylmethyl)-3,4-dihydroquinoxaline-1(2H)-carboxamide;
(39) (*R*)-(6-fluoro-4-(4-fluorophenyl)-3,4-dihydroquinoxalin-1(2H)-yl)(3-hydroxypyrrolidin-1-yl)methanone;
(40) (6-fluoro-4-(4-fluorophenyl)-3,4-dihydroquinoxalin-1(2H)-yl)(3-methoxypyrrolidin-1-yl)methanone;
(41) *N*-(azetidin-3-yl)-6-fluoro-4-(4-fluorophenyl)-3,4-dihydroquinoxaline-1(2H)-carboxamide;
(42) 6-fluoro-4-(4-fluorophenyl)-*N*-(1-isopropylazetidin-3-yl)-3,4-dihydroquinoxaline-1(2H)-carboxamide;
(43) 6-fluoro-4-(4-fluorophenyl)-*N*-(1-isobutylazetidin-3-yl)-3,4-dihydroquinoxaline-1(2H)-carboxamide;
(44) *N*-(1-acetylazetidin-3-yl)-6-fluoro-4-(4-fluorophenyl)-3,4-dihydroquinoxaline-1(2H)-carboxamide;
(45) 6-fluoro-4-(4-fluorophenyl)-*N*-((1-methylazetidin-3-yl)methyl)-3,4-dihydroquinoxaline-1(2H)-carboxamide;
(46) 6-fluoro-4-(4-fluorophenyl)-*N*-((1-isopropylazetidin-3-yl)methyl)-3,4-dihydroquinoxaline-1(2H)-carboxamide;
(47) 6-fluoro-4-(4-fluorophenyl)-*N*-((1-isobutylazetidin-3-yl)methyl)-3,4-dihydroquinoxaline-1(2H)-carboxamide;
(48) 6-fluoro-4-(4-fluorophenyl)-*N*-((tetrahydrofuran-3-yl)methyl)-3,4-dihydroquinoxaline-1(2H)-carboxamide;
(49) (*S*)-4-(4-fluorophenyl)-*N*-(pyrrolidin-3-yl)-3,4-dihydroquinoxaline-1(2H)-carboxamide;
(50) 4-(4-fluorophenyl)-*N*-(piperidin-4-yl)-3,4-dihydroquinoxaline-1(2H)-carboxamide;
(51) (*S*)-4-(4-fluorophenyl)-*N*-(tetrahydrofuran-3-yl)-3,4-dihydroquinoxaline-1 (2H)-carboxamide;
(52) 4-(4-fluorophenyl)-*N*-((tetrahydro-2H-pyran-4-yl)methyl)-3,4-dihydroquinoxaline-1(2H)-carboxamide;
(53) (*S*)-4-(4-fluorophenyl)-*N*-(pyrrolidin-3-ylmethyl)-3,4-dihydroquinoxaline-1(2H)-carboxamide;
(54) (*R*)-4-(4-fluorophenyl)-*N*-((1-methylpyrrolidin-3-yl)methyl)-3,4-dihydroquinoxaline-1 (2H)-carboxamide;
(55) (*R*)-4-(4-fluorophenyl)-*N*-((1-isopropylpyrrolidin-3-yl)methyl)-3,4-dihydroquinoxaline-1 (2H)-carboxamide;
(56) (*R*)-4-(4-fluorophenyl)-*N*-((1-isobutylpyrrolidin-3-yl)methyl)-3,4-dihydroquinoxaline-1 (2H)-carboxamide;
(57) (*R*)-4-(4-fluorophenyl)-*N*-(pyrrolidin-2-ylmethyl)-3,4-dihydroquinoxaline-1 (2H)-carboxamide;
(58) (*S*)-4-(4-fluorophenyl)-*N*-((1-methylpyrrolidin-3-yl)methyl)-3,4-dihydroquinoxaline-1(2H)-carboxamide;
(59) (*S*)-4-(4-fluorophenyl)-*N*-((1-isopropylpyrrolidin-3-yl)methyl)-3,4-dihydroquinoxaline-1(2H)-carboxamide;
(60) (*S*)-4-(4-fluorophenyl)-*N*-((1-isobutylpyrrolidin-3-yl)methyl)-3,4-dihydroquinoxaline-1(2H)-carboxamide;
(61) 4-(4-fluorophenyl)-*N*-((1-isobutylazetidin-3-yl)methyl)-3,4-dihydroquinoxaline-1(2H)-carboxamide;
(62) 4-(4-fluorophenyl)-*N*-(piperidin-4-ylmethyl)-3,4-dihydroquinoxaline-1(2H)-carboxamide;
(63) 4-(4-fluorophenyl)-*N*-((1-isopropylpiperidin-4-yl)methyl)-3,4-dihydroquinoxaline-1(2H)-carboxamide;
(64) 4-(4-fluorophenyl)-*N*-((1-isobutylpiperidin-4-yl)methyl)-3,4-dihydroquinoxaline-1(2H)-carboxamide;
(65) (*R*)-6-fluoro-4-phenyl-*N*-(pyrrolidin-3-yl)-3,4-dihydroquinoxaline-1(2H)-carboxamide;
(66) (4-(2-chlorophenyl)-3,4-dihydroquinoxalin-1(2H)-yl)(piperidin-1-yl)methanone;
(67) 4-(2-chlorophenyl)-3,4-dihydroquinoxalin-1(2H)-yl)(pyrrolidin-1-yl)methanone;
(68) (4-(2-chlorophenyl)-3,4-dihydroquinoxalin-1(2H)-yl)(4-methylpiperazin-1-yl)methanone;
(69) (*S*)-*N*-(1-isopropylpyrrolidin-3-yl)-4-(pyridin-2-yl)-3,4-dihydroquinoxaline-1(2H)-carboxamide;
(70) (*R*)-*N*-(1-acetylpyrrolidin-3-yl)-4-(pyridin-2-yl)-3,4-dihydroquinoxaline-1(2H)-carboxamide;
(71) (*S*)-4-(pyridin-2-yl)-*N*-(pyrrolidin-3-ylmethyl)-3,4-dihydroquinoxaline-1(2H)-carboxamide;
(72) (*R*)-4-(pyridin-2-yl)-*N*-(pyrrolidin-2-ylmethyl)-3,4-dihydroquinoxaline-1(2H)-carboxamide;
(73) (*R*)-4-(pyridin-3-yl)-*N*-(tetrahydrofuran-3-yl)-3,4-dihydroquinoxaline-1(2H)-carboxamide;
(74) *N*-(cyclopropylmethyl)-4-(pyridin-4-yl)-3,4-dihydroquinoxaline-1(2H)-carboxamide;
(75) (*R*)-4-(5-fluoropyridin-2-yl)-*N*-(1-methylpyrrolidin-3-yl)-3,4-dihydroquinoxaline-1(2H)-carboxamide;
(76) (*R*)-4-(5-fluoropyridin-2-yl)-*N*-(1-methylpyrrolidin-3-yl)methyl)-3,4-dihydroquinoxaline-1(2H)-carboxamide;
(77) 4-(5-fluoropyridin-2-yl)-*N*-(1-methylpiperidin-4-yl)methyl)-3,4-dihydroquinoxaline-1(2H)-carboxamide;
(78) (*S*)-(3-aminopyrrolidin-1-yl)(4-(5-fluoropyridin-2-yl)-3,4-dihydroquinoxaline-1(2H)-methanone;
(79) (*S*)-4-(4-cyanophenyl)-*N*-(pyrrolidin-3-ylmethyl)-3,4-dihydroquinoxaline-1(2H)-carboxamide;
(80) (*S*)-4-(pyrazin-2-yl)-*N*-(pyrrolidin-3-yl)-3,4-dihydroquinoxaline-1(2H)-carboxamide;
(81) (*S*)-*N*-(1-isopropylpyrrolidin-3-yl)-4-(pyrazin-2-yl)-3,4-dihydroquinoxaline-1 (2H)-carboxamide;
(82) *N*-(1-isobutylpiperidin-4-yl)-4-(pyrazin-2-yl)-3,4-dihydroquinoxaline-1(2H)-carboxamide;
(83) *N*-(1-isopropylpiperidin-4-yl)-4-(pyrazin-2-yl)-3,4-dihydroquinoxaline-1(2H)-carboxamide;
(84) (*R*)-4-benzyl-*N*-(pyrrolidin-3-yl)-3,4-dihydroquinoxaline-1(2H)-carboxamide;
(85) (*S*)-(3-aminopyrrolidin-1-yl)(4-benzyl-3,4-dihydroquinoxalin-1(2H)-yl)methanone;
(86) (*S*)-(4-benzyl-3,4-dihydroquinoxalin-1(2H)-yl)(3-(methylamino)pyrrolidin-1-yl)methanone;
(87) (*R*)-4-(2-fluorobenzyl)-*N*-(1-methylpyrrolidin-3-yl)-3,4-dihydroquinoxaline-1(2H)-carboxamide;
(88) 4-(2-fluorobenzyl)-*N*-(1-methylpiperidin-4-yl)-3,4-dihydroquinoxaline-1(2H)-carboxamide;
(89) 4-(2-fluorobenzyl)-*N*-((1-methylpiperidin-4-yl)methyl)-3,4-dihydroquinoxaline-1(2H)-carboxamide;
(90) (*R*)-4-(2-fluorobenzyl)-*N*-((1-methylpyrrolidin-3-yl)methyl)-3,4-dihydroquinoxaline-1(2H)-carboxamide;
(91) (*R*)-4-(4-fluorobenzyl)-*N*-(pyrrolidin-3-yl)-3,4-dihydroquinoxaline-1(2H)-carboxamide;
(92) (*R*)-4-(4-fluorobenzyl)-*N*-(1-isopropylpyrrolidin-3-yl)-3,4-dihydroquinoxaline-1 (2H)-carboxamide;
(93) 4-(4-fluorobenzyl)-*N*-(piperidin-4-yl)-3,4-dihydroquinoxaline-1(2H)-carboxamide;
(94) 4-(4-fluorobenzyl)-*N*-(1-isopropylpiperidin-4-yl)-3,4-dihydroquinoxaline-1(2H)-carboxamide;
(95) (*R*)-4-(4-chlorobenzyl)-*N*-(pyrrolidin-3-yl)-3,4-dihydroquinoxaline-1(2H)-carboxamide;
(96) (*S*)-(3-(isobutylamino)pyrrolidin-1-yl)(4-(pyridin-2-ylmethyl)-3,4-dihydroquinoxalin-1(2H)-yl)methanone;
(97) (*S*)-(3-(dimethylamino)pyrrolidin-1-yl)(4-(pyridin-2-ylmethyl)-3,4-dihydroquinoxalin-1(2H)-yl)methanone;
(98) (*R*)-4-(pyridin-2-ylmethyl)-*N*-(tetrahydrofuran-3-yl)-3,4-dihydroquinoxaline-1(2H)-carboxamide;
(99) *N*-(oxetan-3-yl)-4-(pyridin-2-ylmethyl)-3,4-dihydroquinoxaline-1(2H)-carboxamide;
(100) 4-(pyridin-2-ylmethyl)-*N*-((tetrahydrofuran-3-yl)methyl)-3,4-dihydroquinoxaline-1(2H)-carboxamide;
(101) 4-(pyridin-2-ylmethyl)-*N*-((tetrahydro-2H-pyran-4-yl)methyl)-3,4-dihydroquinoxaline-1(2H)-carboxamide;
(102) *N*-(cyclopropylmethyl)-4-(pyridin-2-ylmethyl)-3,4-dihydroquinoxaline-1(2H)-carboxamide;
(103) (4-(4-fluorophenyl)-3,4-dihydropyrido[3,4-*b*]pyrazin-1(2H)-yl)(3-hydroxypyrrolidin-1-yl)methanone;
(104) (*R*)-4-cyclohexyl-*N*-(pyrrolidin-3-yl)-3,4-dihydroquinoxaline-1(2H)-carboxamide;
(105) (*R*)-4-cyclohexyl-*N*-(1-methylpyrrolidin-3-yl)-3,4-dihydroquinoxaline-1(2H)-carboxamide;
(106) (*R*)-4-cyclohexyl-*N*-(1-isobutylpyrrolidin-3-yl)-3,4-dihydroquinoxaline-1(2H)-carboxamide;
(107) (4-cyclohexyl-3,4-dihydroquinoxalin-1(2H)-yl)(4-methylpiperazin-1-yl)methanone;
(108) (4-cyclohexyl-3,4-dihydroquinoxalin-1(2H)-yl)(pyrrolidin-1-yl)methanone;
(109) (4-cyclohexyl-3,4-dihydroquinoxalin-1(2H)-yl)(piperidin-1-yl)methanone;
(110) (*R*)-*N*-(1-methylpyrrolidin-3-yl)-4-(tetrahydro-2H-pyran-4-yl)-3,4-dihydroquinoxaline-1(2H)-carboxamide;
(111) (*R*)-*N*-(1-isobutylpyrrolidin-3-yl)-4-(tetrahydro-2H-pyran-4-yl)-3,4-dihydroquinoxaline-1(2H)-carboxamide;
(112) (*S*)-(3-aminopyrrolidin-1-yl)(4-(tetrahydro-2H-pyran-4-yl)-3,4-dihydroquinoxalin-1(2H)-yl)methanone;
(113) (*S*)-(3-(dimethylamino)pyrrolidin-1-yl)(4-(tetrahydro-2H-pyran-4-yl)-3,4-dihydroquinoxalin-1(2H)-yl)methanone;
(114) 4-benzoyl-*N*-(piperidin-4-ylmethyl)-3,4-dihydroquinoxaline-1(2H)-carboxamide;
(115) (*S*)-4-benzoyl-*N*-(pyrrolidin-3-ylmethyl)-3,4-dihydroquinoxaline-1(2H)-carboxamide;
(116) (*R*)-4-benzoyl-*N*-((1-methylpyrrolidin-3-yl)methyl)-3,4-dihydroquinoxaline-1 (2H)-carboxamide;
(117) (*S*)-4-(2-fluorobenzoyl)-*N*-(pyrrolidin-3-ylmethyl)-3,4-dihydroquinoxaline-1 (2H)-carboxamide;
(118) (*R*)-4-(2-fluorobenzoyl)-*N*-((1-methylpyrrolidin-3-yl)methyl)-3,4-dihydroquinoxaline-1(2H)-carboxamide;
(119) (*R*)-4-(3-fluorobenzoyl)-*N*-(pyrrolidin-3-yl)-3,4-dihydroquinoxaline-1(2H)-carboxamide;
(120) (*S*)-4-(3-fluorobenzoyl)-*N*-(pyrrolidin-3-yl)methyl*)*-3,4-dihydroquinoxaline-1 (2H)-carboxamide;
(121) (*R*)-4-(3-fluorobenzoyl)-*N*-((1-methylpyrrolidin-3-yl)methyl)-3,4-dihydroquinoxaline-1(2H)-carboxamide;
(122) (*S*)-4-(4-fluorobenzoyl)-*N*-(pyrrolidin-3-ylmethyl)-3,4-dihydroquinoxaline-1(2H)-carboxamide;
(123) (*R*)-4-(4-fluorobenzoyl)-*N*-((1-methylpyrrolidin-3-yl)methyl)-3,4-dihydroquinoxaline-1(2H)-carboxamide;
(124) 4-(4-fluorobenzoyl-*N*-(piperidin-4-yl)-3,4-dihydroquinoxaline-1(2H)-carboxamide;
(125) 4-(3-methylbutanoyl)-*N*-(piperidin-4-ylmethyl)-3,4-dihydroquinoxaline-1 (2H)-carboxamide;
(126) 4-(3-methylbutanoyl)-*N*-((1-methylpiperidin-4-yl)methyl)-3,4-dihydroquinoxaline-1(2H)-carboxamide;
(127) *N*-((1-isobutylpiperidin-4-yl)methyl)-4-(3-methylbutanoyl)-3,4-dihydroquinoxaline-1(2H)-carboxamide;
(128) (*R*)-4-(3-methylbutanoyl)-*N*-((1-methylpyrrolidin-3-yl)methyl)-3,4-dihydroquinoxaline-1(2H)-carboxamide;
(129) (*R*)-4-(3,3-dimethylbutanoyl)-IN-(pyrrolidin-3-ylmethyl)-3,4-dihydroquinoxaline-1(2H)-carboxamide; and
(130) 4-(3,3-dimethylbutanoyl)-*N*-(piperidin-4-ylmethyl)-3,4-dihydroquinoxaline-1 (2H)-carboxamide.

In addition, the present invention provides a pharmaceutical composition for preventing or treating a pulmonary fibrosis-related disease, which includes the compound represented by Chemical Formula 1, a stereoisomer thereof, or a pharmaceutically acceptable salt thereof as an active ingredient.

In addition, the present invention provides a method of preventing or treating a pulmonary fibrosis-related disease, which includes administering the compound represented by Chemical Formula 1, a stereoisomer thereof, or a pharmaceutically acceptable salt thereof to a subject in need thereof.

In addition, the present invention provides a use of the compound represented by Chemical Formula 1, a stereoisomer thereof, or a pharmaceutically acceptable salt thereof for preventing or treating a pulmonary fibrosis-related disease.

In addition, the present invention provides a use of the compound represented by Chemical Formula 1, a stereoisomer thereof, or a pharmaceutically acceptable salt thereof for preparing a drug for preventing or treating a pulmonary fibrosis-related disease.

In one embodiment of the present invention, the composition may inhibit actin polymerization, but the present invention is not limited thereto.

In another embodiment of the present invention, the composition may inhibit the level or activity of an inflammatory cytokine, but the present invention is not limited thereto.

In still another embodiment of the present invention, the composition may reduce the level or activity of one or more proteins selected from the group consisting of α-SMA, COL1A1, COL4A1, fibronectin, F-actin, and IL-6, but the present invention is not limited thereto.

In yet another embodiment of the present invention, the pulmonary fibrosis-related disease may be one or more selected from the group consisting of pulmonary fibrosis, idiopathic pulmonary fibrosis, desquamative interstitial pneumonia, non-specific interstitial pneumonia, cryptogenic organizing pneumonia, respiratory bronchiolitis-associated interstitial lung disease, acute interstitial pneumonia, lymphatic interstitial pneumonia, idiopathic pleuroparenchymal fibroelastosis, and chronic obstructive pulmonary disease (COPD), but the present invention is not limited thereto.

In yet another embodiment of the present invention, the pulmonary fibrosis-related disease may be a pulmonary fibrosis-related disease accompanied by the overactivation of actin polymerization, but the present invention is not limited thereto.

In addition, the present invention provides a kit for preventing or treating pulmonary fibrosis, which includes the compound represented by Chemical Formula 1, a stereoisomer thereof, or a pharmaceutically acceptable salt thereof; or the composition.

In addition, the present invention provides a pharmaceutical composition for inhibiting pulmonary fibrosis and/or lung inflammation, which includes the compound represented by Chemical Formula 1, a stereoisomer thereof, or a pharmaceutically acceptable salt thereof as an active ingredient.

Moreover, the present invention provides a method of inhibiting or improving pulmonary fibrosis and/or lung inflammation, which includes administering the compound represented by Chemical Formula 1, a stereoisomer thereof, or a pharmaceutically acceptable salt thereof to a subject in need thereof.

Moreover, the present invention provides a use of the compound represented by Chemical Formula 1, a stereoisomer thereof, or a pharmaceutically acceptable salt thereof for inhibiting pulmonary fibrosis and/or lung inflammation.

Moreover, the present invention provides a use of the compound represented by Chemical Formula 1, a stereoisomer thereof, or a pharmaceutically acceptable salt thereof for preparing a drug for inhibiting or improving pulmonary fibrosis and/or lung inflammation.

### Advantageous Effects

The present invention relates to a novel compound useful for the preparation of a medicine capable of treating related diseases by regulating the function and differentiation of the cytoskeleton involved in the process of pulmonary fibrosis. It was confirmed that the novel compound according to the present invention or a pharmaceutically acceptable salt thereof is effective in controlling actin polymerization required for changing the cytoskeleton, and the expression of α-SMA and F-actin, which are the major causative factors of fibrosis in lung tissue-derived fibroblasts, was effectively suppressed. In addition, it was confirmed that when activated macrophages were treated with the compound, the level of inflammatory cytokine generated by macrophages significantly decreases. Accordingly, the novel compound according to the present invention is expected to be useful in the prevention and treatment of various pulmonary fibrosis-related diseases as an agent effective in inhibiting or improving pulmonary fibrosis.

### Description of Drawings

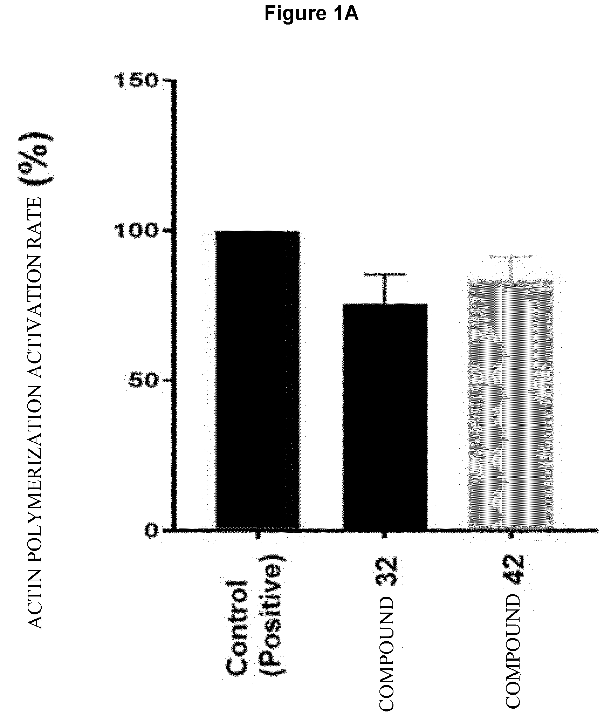

FIG. 1A shows the results of inducing actin polymerization after treating an actin monomer with a compound of the present invention and then measuring the activity of actin polymerization to confirm the actin polymerization inhibitory activity of the compound according to the present invention.

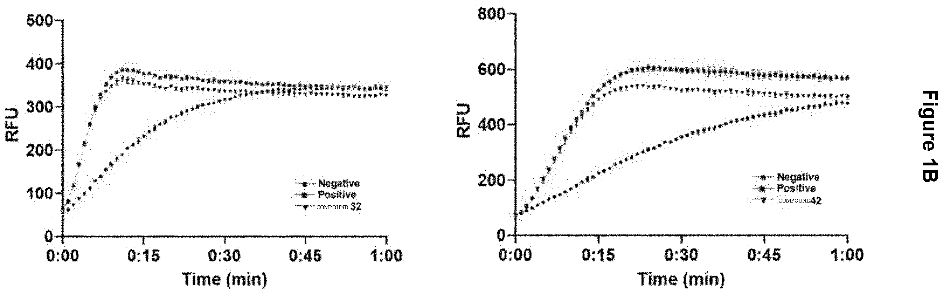

FIG. 1B shows the results of inducing actin polymerization after treating an actin monomer with a compound of the present invention and then measuring relative fluorescence units (RFU) to confirm the actin polymerization inhibitory activity of the compound according to the present invention.

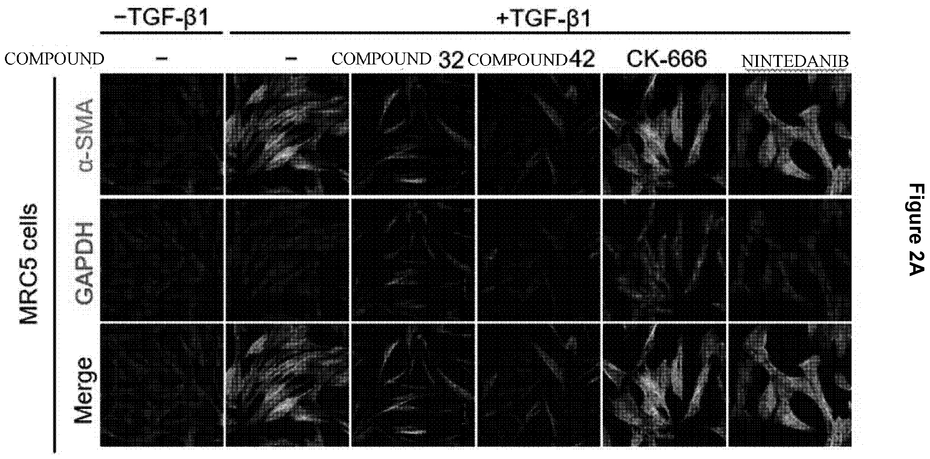

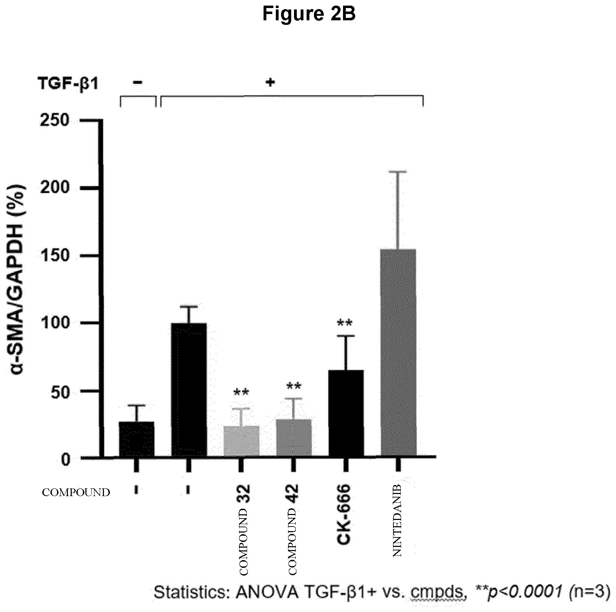

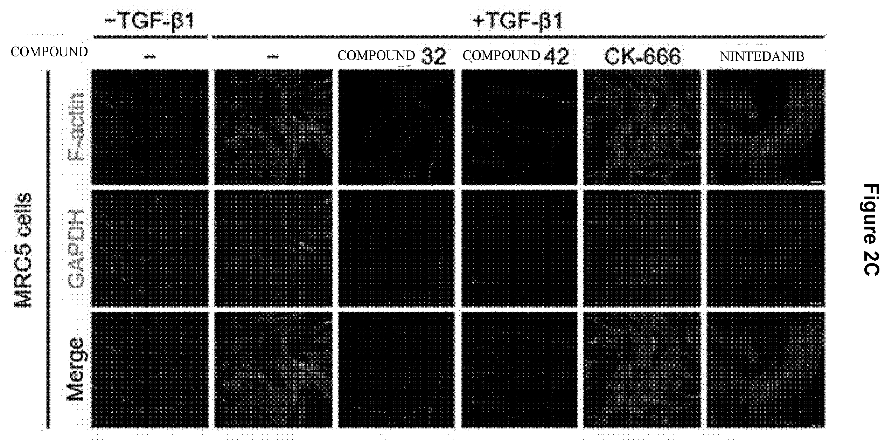

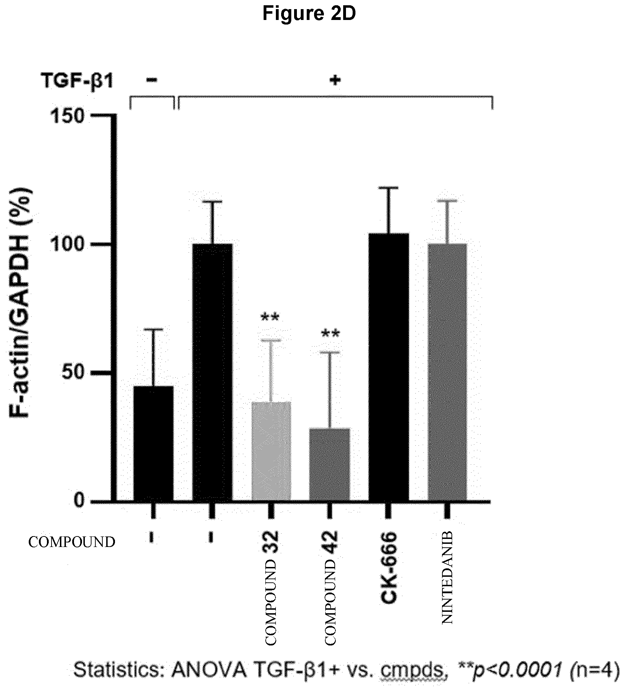

FIGS. 2A to 2D show the high resolution images that confirm the expression levels of pulmonary fibrosis biomarkers such as α-SMA and F-actin using a confocal microscope after inducing fibrosis markers by treating human lung tissue-derived fibroblasts (MRC5) with DMSO, a compound of the present invention, CK-666 (actin polymerization inhibitor), or nintedanib and then with TGF-β1, and quantification results to confirm the pulmonary fibrosis inhibitory effect of the compound according to the present invention (FIGS. 2A and 2B, the images of intracellular α-SMA and the quantification results of α-SMA levels; FIGS. 2C and 2D, the images of intracellular F-actin and the quantification results of F-actin levels).

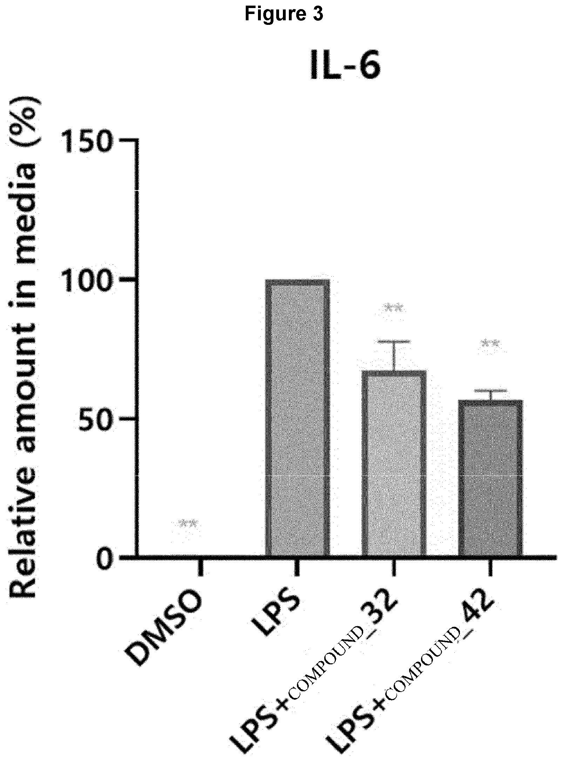

FIG. 3 shows the results of measuring the level of IL-6 protein secreted *ex vivo* after treatment with a compound of the present invention or not while inducing the differentiation of THP-1 into M1 macrophages to confirm the pulmonary fibrosis inhibitory effect caused by the anti-inflammatory effect of the compound according to the present invention.

### Modes of the Invention

The present invention relating to a novel compound was completed by confirming that the compound is effective in not only regulating the abnormal formation of cytoskeleton but also inhibiting the generation of a pulmonary fibrosis-related factor including α-SMA by inhibiting actin polymerization.

Specifically, in one embodiment of the present invention, a novel compound according to the present invention was prepared (Experimental Example 1).

In another embodiment of the present invention, as a result of inducing actin polymerization after treating an actin monomer with the compound according to the present invention, it was confirmed that the actin polymerization activity is significantly reduced by the above compound, indicating that the compound has an excellent actin polymerization inhibitory effect (Experimental Example 2).

In still another embodiment of the present invention, as a result of measuring the change in the levels of fibrosis-related factors such as α-SMA and F-actin by treating MRC5 with a compound according to the present invention, it was confirmed that the levels of the major biomarkers of pulmonary fibrosis, such as α-SMA and F-actin are significantly decreased by the treatment with the compound of the present invention (Experimental Example 3).

In yet another embodiment of the present invention, as a result of verifying the α-SMA inhibitory effect of compounds according to the present invention, it was confirmed that the compounds can effectively inhibit the protein expression of α-SMA in MRC5 (Experimental Example 4).

In yet another embodiment of the present invention, as a result of observing the generation and secretion levels of the major fibrosis-inducing factor IL-6 after treating activated macrophages with the compound according to the present invention, it was confirmed that the II-6 level is significantly decreased by the treatment with the compound of the present invention (Experimental Example 5).

The above results proved that the novel compound according to the present invention can effectively inhibit a pulmonary fibrosis-inducing factor, and the present invention is expected to be applied in the therapeutic fields of various pulmonary fibrosis-related diseases.

Hereinafter, the present invention will be described in detail.

The present invention provides a compound represented by Chemical Formula 1 below, a stereoisomer thereof, or a pharmaceutically acceptable salt thereof: 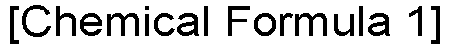 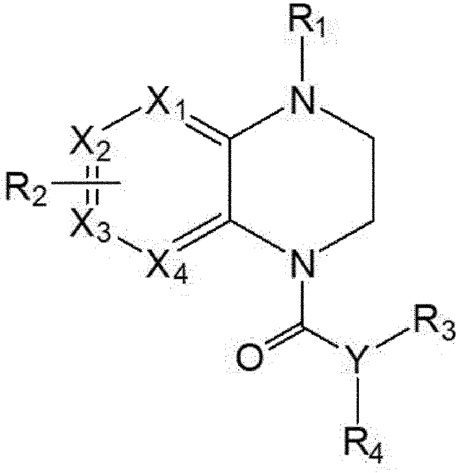 (In Chemical Formula 1,
X₁ to X₄ may each be independently N or C;
Y may be N, S, or O;
R₁ may be C₁-C₁₀ alkyl, C₃-C₂₀ cycloalkyl, C₂-C₂₀ heterocycloalkyl, a 3- to 10-membered cyclic aromatic group, a 3- to 10-membered heterocyclic aromatic group, - CO-(C₁-C₆ alkyl), or -CO-(halogen-substituted or unsubstituted C₆-C₁₂ aryl), and
at least one H of R₁ may be substituted with halogen, a cyano group, halogen-substituted or unsubstituted C₆-C₁₂ aryl, or halogen-substituted or unsubstituted C₅-C₁₂ heteroaryl;
when all of X₁ to X₄ are C, R₂ may be hydrogen, halogen, or C₁-C₅ alkyl, and when one or more of X₁ to X₄ are N, R₂ may be hydrogen (that is, when one or more of X₁ to X₄ are N, R₂ is hydrogen, which binds to Xₙ, other than N. In other words, when one or more of X₁ to X₄ are N, H of the ring formed by X₁ to X₄ is not substituted);
R₃ and R₄ may be linked to each other to form a 5- or 6-membered ring, wherein at least one H in the ring may be substituted with -OH, -NH₂, dimethylamine, -NH-(C₁-C₅ alkyl), -NH-(C₁-C₅ alkoxy), -NH-COCH₃, -NH-SO₂CH₃, C₁-C₅ alkyl, or C₁-C₅ alkoxy, and
when R₃ and R₄ do not form a ring, R₄ may be hydrogen or C₁-C₅ alkyl, R₃ may be C₁-C₁₀ alkyl, C₃-C₁₀ cycloalkyl, C₂-C₁₀ heterocycloalkyl, -CH₂-(C₃-C₆ cycloalkyl), -CH₂-(C₂-C₆ heterocycloalkyl), or -CH₂-(3- to 6-membered heterocyclic aromatic group), at least one H of R₃ may be substituted with -OH, C₁-C₅ alkyl, acetyl, C₁-C₅ alkoxy, -COCF₃, or -SO₂CH₃; and
the heterocycloalkyl, heterocyclic group, and heteroaryl each independently include one or more hetero atoms selected from the group consisting of N, O, P, and S.)

Here, the -CO-(C₁-C₆ alkyl) refers to C₁-C₆ alkyl linked to CO. For example, when R₁ is -CO-(C₁-C₆ alkyl), it means C₁-C₆ alkyl linked to a nitrogen (N) atom via CO.

In the present invention, when R₃ and R₄ are linked to each other to form a 5- or 6-membered ring, it means that R₃ and R₄ are linked to each other via another atom to form a ring structure. The ring may be formed of a single bond, a multiple bond (a double bond, a triple bond, etc.), or a combination thereof (that is, including both of cycloalkyl and cyclic aromatic), a ring composed of all C atoms, or a heterocyclic group having one or more hetero atoms. Preferably, when R₃ and R₄ are linked to each other to form a 5-or 6-membered ring, both of R₃ and R₄ are C.

In one embodiment of the present invention, R₃ and R₄ are linked to each other to form 5- or 6-membered heterocycloalkyl, which includes one or two N atoms in this ring. Here, Y may be N, but the present invention is not limited thereto. In addition, in the 5- or 6-membered heterocycloalkyl formed by R₃ and R₄, at least one H may be substituted with -OH, -NH₂, dimethylamine, -NH-(C₁-C₅ alkyl), -NH-(C₁-C₅ alkoxy), -NH-COCH₃, -NH-SO₂CH₃, C₁-C₅ alkyl, or C₁-C₅ alkoxy, but the present invention is not limited thereto.

In another embodiment of the present invention, R₁ may be a 6-membered cyclic aromatic group (e.g., aryl), 6-membered heterocyclic aromatic group (e.g., heteroaryl). -CH₂-aryl, -CH₂-heteroaryl, 6-membered cycloalkyl, 6-membered heterocycloalkyl, -CO-aryl, or -CO-(C₄-C₅ alkyl), wherein the 6-membered heterocyclic aromatic group includes at least one N, and the 6-membered heterocycloalkyl may include at least one O, but the present invention is not limited thereto. In addition, at least one H of R₁ may be substituted with halogen, a cyano group, halogen-substituted or unsubstituted C₆-C₁₂ aryl, or halogen-substituted or unsubstituted C₅-C₁₂ heteroaryl, but the present invention is not limited thereto.

In still another embodiment of the present invention, when R₃ and R₄ do not form a ring, R₄ may be hydrogen or C₁-C₅ alkyl, and R₃ may be -CH₂-(C₃-C₆ cycloalkyl), -CH₂-(C₂-C₆ heterocycloalkyl), or -CH₂-(3- to 6-membered heterocyclic aromatic group). Here, the heterocycloalkyl may be 4- to 6-membered heterocycloalkyl having one or two N or O atoms, and the heterocyclic aromatic group may be a 5- or 6-membered heterocyclic aromatic group having one or two N atoms, but the present invention is not limited thereto.

In one embodiment of the present invention, the compound may be represented by Chemical Formula 1-1 below, but the present invention is not limited thereto: 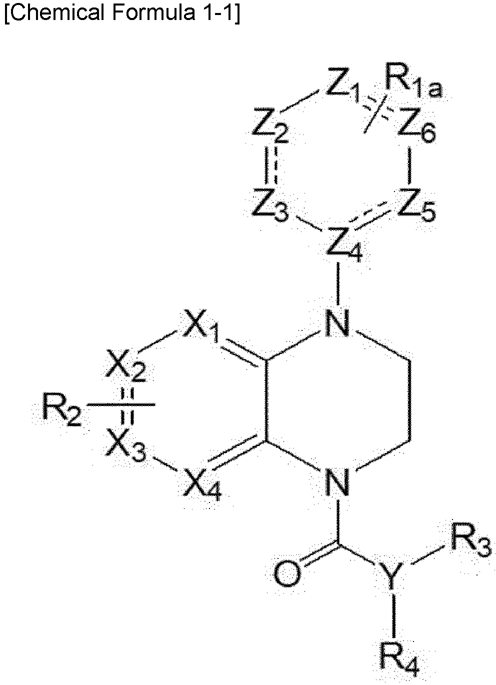 (In Chemical Formula 1-1,
X₁ to X₄ are each independently N or C;
Z₁ to Z₆ are each independently N, O, or C;
R₁ₐ is hydrogen, halogen, or a cyano group;
R ₂ is hydrogen, halogen, or C₁-C₅ alkyl;
R₃ is C₁-C₁₀ alkyl, C₃-C₁₀ cycloalkyl, C₂-C₁₀ heterocycloalkyl, -CH₂-(C₃-C₆ cycloalkyl), -CH₂-(C₂-C₆ heterocycloalkyl), or -CH₂-(3- to 6-membered heterocyclic aromatic group), wherein at least one H of R₃ may be substituted with -OH, C₁-C₅ alkyl, acetyl, C₁-C₅ alkoxy, -COCF₃, or -SO₂CH₃; and
R₄ is hydrogen or C₁-C₅ alkyl,
wherein the heterocycloalkyl and heterocyclic groups include each independently one or more hetero atoms selected from the group consisting of N, O, P, and S.)

In Chemical Formula 1-1, one solid line and one dotted line (i.e., 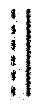 ) indicate that two atoms are linked by a single bond or a double bond.

Preferably, the compound may be any one of compounds shown in Table 1 below, but the present invention is not limited thereto.

Throughout the specification, a functional group may be written by omitting a "-group".

In the present invention, "halogen" includes F, CI, Br, or I.

In the present invention, "alkyl" refers to a fully saturated branched or unbranched (or straight-chain or linear) hydrocarbon. In addition, the alkyl according to the present invention includes linear or branched hydrocarbon including a hydrocarbon ring group at an end or in the middle. In the present invention, the alkyl may be C₁-C₂₀, C₁-C₁₅, C₁-C₁₂, C₁-C₁₀, C₁-C₈, C₁-C₆, C₁-C₈, or C₁-C₃ alkyl, but the present invention is not limited thereto. The alkyl is, for example, methyl, ethyl, n-propyl, isopropyl, n-butyl, isobutyl, sec-butyl, n-pentyl, isopentyl, neopentyl, iso-amyl, n-hexyl, 3-methylhexyl, 2,2-dimethylpentyl, 2,3-dimethylpentyl, or n-heptyl. In one embodiment of the present invention, "alkyl" may be aminoalkyl or haloalkyl. The "aminoalkyl" may refer to an alkyl group in which at least one H are substituted with N atoms (e.g., -NH-(C₁-C₅ alkyl)), and "haloalkyl" may refers to an alkyl group in which at least one H are substituted with halogen atoms.

The compound according to the present invention may include an alkoxy group. In the present invention, "alkoxy" refers to alkyl bonding to a single oxygen atom (-O-R). In the present invention, the alkoxy may be C₁-C₁₀, C₁-C₈, C₁-C₆, C₁-C₈, C₁-C₃, or C₁-C₂ alkoxy, but the present invention is not limited thereto. The alkoxy may refer to, for example, methoxy, ethoxy, phenoxy, or butoxy.

In the present invention, "acetyl" refers to a monovalent atomic group (CH₃CO-) consisting of a methyl group (-CH₃) and a carbonyl group (C=O), and "halogen-substituted acetyl" means that one or more hydrogen atoms of acetyl are substituted with halogen atoms. For example, in the present invention, "halogen-substituted acetyl" may be trifluoroacetyl (-COCF₃).

In the present invention, "sulfonyl" refers to a divalent atomic group (-SO₂-) consisting of two O atoms and one S atom. Preferably, the sulfonyl is C₁-C₁₀ alkyl-binding alkylsulfonyl. For example, the sulfonyl may be methyl-binding methylsulfonyl.

In the present invention, "amino" refers to a functional group (-NH₂) in which hydrogen binds to a nitrogen atom.

The compound according to the present invention may include a cyclic substituent (e.g., a cycloalkyl group, an aryl group, etc.) having a single bond, a multiple bond (a double bond, a triple bond, etc.), or a combination thereof. That is, the compound according to the present invention may include a (hetero)cyclic group having a single bond and/or a multiple bond.

In the present invention, "cycloalkyl" refers to a saturated or partially unsaturated cyclic non-aromatic hydrocarbon group. In the present invention, the cycloalkyl may be C₃-C₂₀, C₃-C₁₅, C₃-C₁₂, C₃-C₁₀, C₃-C₈, C₃-C₆, or C₃-C₅ cycloalkyl, but the present invention is not limited thereto. In other words, the cycloalkyl group may be, for example, cyclopropyl, cyclobutyl, cyclopentyl, cyclohexyl, cyclohexenyl, cycloheptyl, cycloheptenyl, cyclooctyl, or cyclooctenyl. In the present invention, "cycloalkyl" includes "bicycloalkyl" or "tricycloalkyl." Unless stated otherwise, the term "bicycloalkyl" or "tricycloalkyl" refers to a structure consisting of two or more cycloalkyl moieties having two or more atoms in common. In addition, the cycloalkyl group may be a polycyclic ring.

In the present invention, "heterocycloalkyl" refers to a cyclic hydrocarbon group that includes one or more hetero atoms in the ring in addition to carbon atoms. The hetero atom may be one or more selected from the group consisting of N, O, P, and S. In one embodiment of the present invention, the heterocycloalkyl may be cyclic amine or heterocyclic amine having an N atom, and preferably, 3- to 6-membered cyclic amine. For example, the heterocycloalkyl may be selected from the group consisting of piperidinyl, pyrrolidinyl, morphonyl, piperazinyl, pyrrolopyrazinyl, and azetidinyl. Alternatively, the heterocycloalkyl may be a 4- to 6-membered cyclic group having one or more O atoms. In the present invention, the heterocycloalkyl includes "biheterocycloalkyl.". The biheterocycloalkyl consists of two or more rings having two or more atoms in common, and one or more of the two or more rings are heterocycloalkyl.

In the present invention, "aryl" refers to an aromatic system (aromatic ring) including one or more rings, which is used alone or in combination, and also includes a group in which an aromatic ring is fused to one or more carbon rings. In the present invention, the aryl may be a C₃-C₂₀, C₃-C₁₅, C₃-C₁₂, C₃-C₁₀, C₃-C₈, C₃-C₆ aryl, but the present invention is not limited thereto. "Aryl" may be, for example, phenyl, benzyl, naphthyl, or tetrahydronaphthyl, but the present invention is not limited thereto. The aryl includes heteroaryl.

In the present invention, "heteroaryl" refers to a monocyclic or bicyclic organic compound, which includes one or more hetero atoms in the ring in addition to carbon atoms. The hetero atom may be one or more selected from the group consisting of N, O, P, and S. In one embodiment of the present invention, the heteroaryl may include at least one N (that is, at least one C constituting the ring is substituted with N). Alternatively, in the present invention, the heteroaryl may be aromatic amine. In another embodiment of the present invention, the heteroaryl may include one to three, one or two, or one N atom.

The aryl and heteroaryl (aromatic ring) according to the present invention may include, for example, phenyl, biphenyl, benzyl, benzoyl, benzidine, toluyl, thienyl, furyl, naphthyl, imidazolyl (imidazole), pyrrolyl, thiazolyl, oxazolyl, isoxazolyl, pyranyl, pyrazinyl, pyrolinyl, pyridinyl (pyridyl), piperazinyl, pyrimidinyl, pyrazinyl, pyridazinyl, pyrazolyl, thiadiazolyl, triazolyl, indolyl, azaindolyl, indazolyl, azaindazolyl, benzimidazolyl, benzofuranyl, benzothienyl, benzoisoxazolyl, benzoxazolyl, benzopyrazolyl, benzothiazolyl, benzothiadiazolyl, benzotriazolyl, adeninyl, quinolinyl, isoquinolinyl, naphthalenyl, tetrahydronaphthyl, and isomers thereof.

Alternatively, the compound according to the present invention may include a polycyclic ring consisting of multiple rings joined together. Specifically, the polycyclic ring refers to a substituent in which two or more rings are formed in a linear angled or closely packed structure. For example, the compound may include a polycyclic ring having 2 to 5 rings combined (i.e., a polycyclic ring with 2 to 5 rings), a polycyclic ring having 2 to 4 rings combined, or a polycyclic ring having 2 or 3 rings combined. Each ring constituting the polycyclic ring may be any ring known in the art, as well as the rings disclosed in the specification. For example, the ring may be a 3-, 4-, 5-, 6-, 10-membered ring, or a combination thereof, and preferably, 5- and/or6-membered ring(s). In addition, each ring constituting the polycyclic ring may be composed of only a single bond, or may include a multiple bond (a double bond, a triple bond, etc.). Each ring constituting the polycyclic ring may be the same as or different from each other. For example, the ring may each be independently cycloalkyl, heterocycloalkyl, a 3- to 10-membered cyclic aromatic group, or a 3- to 10-membered heterocyclic aromatic group.

Alternatively, the compound according to the present invention may include a heterocyclic group. The ring may include a single bond or a multiple bond (a double bond, a triple bond, etc.). In one embodiment of the present invention, the heterocyclic group may include one or more hetero atoms selected from the group consisting of N, O, and S.

In the present invention, "cyclic aromatic group" refers to a substituent in which carbon compounds are combined in a ring shape by an unsaturated bond (e.g., a double bond), a single electron pair, or an empty orbital. In addition, "heterocyclic aromatic group" refers to a heterocyclic group having two or more different types of ring-constituting atoms in the aromatic ring. In the present invention, the (hetero)cyclic aromatic group may be a 3- to 10-membred, 3- to 8-membered, 3- to 6-membered, 3-to 5-membered, 4- to 6-membered, 4- or 5-membered, or 5- or 6-membered (hetero)cyclic group, but the present invention is not limited thereto.

In the present invention, a substituent having hetero atoms (a heterocyclic group, a heterocycloalkyl group, a heteroaryl group, and other types of heterocyclic groups) may include one or more hetero atoms in its backbone structure. For example, the substituent having hetero atoms may include 1 to 5, 1 to 4, 1 to 3, 1 or 2, or 1 hetero atom, but the present invention is not limited thereto.

In the present invention, the term "substituted" in "substituted or unsubstituted" means that one or more hydrogen atoms in an organic compound are substituted by introducing another atomic group when forming a derivative, and a substituent refers to the introduced atomic group. That is, in the present invention, substituting an arbitrary group means that one or more hydrogen atoms of a corresponding functional group are replaced by other atomic groups. In the present invention, in each functional group (alkyl, haloalkyl, cycloalkyl, heterocycloalkyl, aryl, heteroaryl, alkoxy, or acetyl), one or more hydrogen atoms may each be independently substituted with different atomic groups. In the present invention, "substitution" includes single substitution, double substitution, triple substitution, or quadruple substitution.

In the present invention, unless particularly stated otherwise, "substituted or unsubstituted" may refer that at least one hydrogen (H) atom is substituted with a different functional group or unsubstituted. For example, "halogen-substituted or unsubstituted C₃-C₁₀ aryl" may refer that C₃-C₁₀ aryl is unsubstituted, or at least one H of the aryl is substituted with halogen.

In the present invention, the term "isomer" refers to a compound that has the same molecular formula but has different connection methods or spatial arrangements of the constituent atoms within the molecule. Isomers include, for example, structural isomers and stereoisomers. The stereoisomer may be a diastereomer or an enantiomer. The enantiomer refers to an isomer which does not overlap its mirror image like the relationship between the left hand and right hand and is also called an optical isomer. The enantiomers are classified into R (rectus: clockwise) and S (sinister: counterclockwise) when there are four or more different substituents at a chiral center carbon. The diastereomers refer to stereoisomers that are not mirror images and may be classified into cis-trans isomers due to different spatial arrangements of atoms.

In the present invention, the term "pharmaceutically acceptable salt" includes all salts that are derived from pharmaceutically acceptable inorganic acids, organic acids, or bases.

The term "pharmaceutically acceptable" used herein refers to a compound or composition that is suitable for use in contact with tissue of a subject (e.g., a human) due to a reasonable benefit/risk ratio without excessive toxicity, irritation, allergic reaction or other problems or complications and is included in the scope of sound medical judgment.

Examples of suitable acids may include hydrochloric acid, bromic acid, sulfuric acid, nitric acid, perchloric acid, hydroiodic acid, fumaric acid, maleic acid, phosphoric acid, glycolic acid, lactic acid, salicylic acid, succinic acid, toluene-p-sulfonic acid, tartaric acid, (+)-L-tartaric acid, di-L-tartaric acid, acetic acid, trichloroacetic acid or trifluoroacetic acid, 2,2-dichloroacetic acid, acylated amino acids, adipic acid, alginic acid, ascorbic acid, L-aspartic acid, 4-acetaminobenzoic acid, (+)-camphoric acid, camphorsulfonic acid, (+)-(1S)-camphorsulfonic acid, capric acid, caproic acid, caprylic acid, cinnamic acid, cyclomic acid, dodecylsulfonic acid, ethane-1,2-disulfonic acid, ethanesulfonic acid, 2-hydroxyethanesulfonic acid, galactaric acid, gentizic acid, glucoheptanoic acid, D-gluconic acid, D-glucuronic acid, L-glutamic acid, a-oxo-glutaric acid, hippuric acid, (+)-L-lactic acid, (+-)-DL-lactic acid, lactobionic acid, (-)-L-malic acid, (+-)-DL-mandelic acid, citric acid, methanesulfonic acid, formic acid, benzoic acid, malonic acid, gluconic acid, naphthalene-2-sulfonic acid, naphthalene-1,5-disulfonic acid, benzenesulfonic acid, 1-hydroxy-2-naphthoic acid, nicotinic acid, oleic acid, orotic acid, oxalic acid, palmitic acid, palmic acid, L-pyroglutamic acid, salicylic acid, 4-amino-salicylic acid, sebacic acid, stearic acid, tannic acid, thiocyanic acid, camsylic acid, and undecylic acid. An acid addition salt may be prepared by a conventional method, for example, by dissolving a compound in an excessive amount of acid aqueous solution and precipitating the salt using a water-miscible organic solvent such as methanol, ethanol, acetone or acetonitrile. Alternatively, the acid addition salt may be prepared by heating equimolar amounts of compound and an acid or alcohol in water, and evaporating or drying the mixture, or suction filtering the precipitated salt.

Salts derived from suitable bases may include, but are not limited to, alkali metals such as sodium, potassium, alkaline earth metals such as magnesium, and ammonium. An alkali metal or alkaline earth metal salt may be obtained, for example, by dissolving a compound in an excess of alkali metal hydroxide or alkaline earth metal hydroxide solution, filtering the undissolved compound salt, and then evaporating and drying the filtrate. Here, as a metal salt, particularly, it is pharmaceutically suitable to prepare a sodium, potassium, or calcium salt, and a silver salt corresponding thereto may be obtained by reacting an alkali metal or alkaline earth metal salt with a suitable silver salt (e.g., silver nitrate).

The range of the compound of the present invention may include not only pharmaceutically acceptable salts, but also all isomers, hydrates, and solvates, which can be prepared by conventional methods.

In addition, the present invention provides a pharmaceutical composition for preventing or treating a pulmonary fibrosis-related disease, which includes a compound represented by Chemical Formula 1, a stereoisomer thereof, or a pharmaceutically acceptable salt thereof as an active ingredient.

In the present invention, "pulmonary fibrosis-related disease" includes all diseases directly or indirectly caused by pulmonary fibrosis, which also includes pulmonary fibrosis. Fibrosis refers to a phenomenon in which connective tissue is formed within tissue as the extracellular matrix including collagen is secreted by activated fibroblasts. Fibrosis contributes to wound recovery by forming scar tissue, but if connective tissue accumulates excessively, it can paralyze the normal structure and function of a corresponding organ.

Specifically, the pulmonary fibrosis-related disease may be one or more selected from the group consisting of pulmonary fibrosis, idiopathic pulmonary fibrosis (IPF), desquamative interstitial pneumonia, non-specific interstitial pneumonia, cryptogenic organizing pneumonia, respiratory bronchiolitis-associated interstitial lung disease, acute interstitial pneumonia, lymphocytic interstitial pneumonia, idiopathic pleuroparenchymal fibroelastosis, and chronic obstructive pulmonary disease (COPD), but the present invention is not limited thereto. Any disease that has pulmonary fibrosis or can be directly or indirectly caused by pulmonary fibrosis is included without limitation. Preferably, in the present invention, the pulmonary fibrosis-related disease is accompanied by hyperactivation of actin polymerization. In another embodiment of the present invention, the pulmonary fibrosis-related disease may involve the overactivation or overexpression of α-SMA, F-actin, and/or IL-6.

In the present invention, "pulmonary fibrosis" is a respiratory disease that causes breathing difficulties due to hardening of lung tissue and specifically refers to a condition in which a normal lung structure is destroyed and hardened due to excessive accumulation of fibrous connective tissue in the lungs. As fibrosis progresses in the lungs, the lung wall thickens and the amount of oxygen supplied to the blood decreases, resulting in shortness of breath. The most common type of pulmonary fibrosis is idiopathic pulmonary fibrosis (IPF), and the main symptom is shortness of breath during exercise. For IPF treatment, nintedanib, which is an anti-fibrotic drug, is used, but the therapeutic effect is known to be only 10%.

In the present invention, the composition may inhibit actin polymerization. The actin includes F-actin. The inhibition of the actin polymerization may be performed by functional inhibition of an actin-binding protein, such as Arp2/3 complex. Actin filaments are assembled from actin monomers through actin polymerization, which hydrolyzes ATP to ADP. While actin polymerization is necessary for controlling cell movement, excessive actin polymerization promotes the expression of alpha-smooth muscle actin (α-SMA) protein and induces the activation of myofibroblasts, a heterogeneous cell population consisting of fibrosis-promoting cells, resulting in tissue fibrosis. The inventors confirmed through specific experiments that the compound according to the present invention can effectively inhibit actin polymerization, and thus pulmonary fibrosis can be fundamentally treated and alleviated.

In addition, in the present invention, the composition may inhibit the level or activity of an inflammatory cytokine. The inflammatory cytokine may be generated and secreted by macrophages. The inflammatory cytokine may be IL-6, but the present invention is not limited thereto. IL-6 is a cytokine that is involved in B cell differentiation, T cell activation, and inflammatory response and particularly induces pulmonary fibrosis. When pulmonary fibrosis occurs, an acute or chronic inflammatory response is preceded. During the inflammatory response, inflammatory cells including macrophages are activated and secrete inflammatory cytokines such as IL-6, IL-1β, and TNF-α, and these cytokines damage tissue, proliferate fibroblasts, accumulate ECM, and promote pulmonary fibrosis.

In addition, in the present invention, the composition is characterized by reducing the levels of one or more proteins selected from the group consisting of α-SMA, COL1A1, COL4A1, fibronectin, F-actin, and IL-6. The proteins are biomarkers for pulmonary fibrosis, and particularly, an "α-SMA" protein is a marker for activated myofibroblasts, which are the main effector cells of pulmonary fibrosis. The inventors confirmed through specific examples that the compounds according to the present invention exhibit an excellent effect of inhibiting α-SMA expression in fibroblasts compared to a conventional pulmonary fibrosis treatment, nintedanib, showing that the compounds according to the present invention have an excellent effect of inhibiting pulmonary fibrosis compared to nintedanib.

The content of the compound in the composition of the present invention can be appropriately adjusted according to the symptoms of a disease, the degree of the progression of symptoms, and a patient's condition, and may be, for example, 0.0001 to 99.9 wt%, or 0.001 to 50 wt% based on the total weight of the composition, but the present invention is not limited thereto. The content ratio is a value based on a dry content from which a solvent is removed.

The pharmaceutical composition according to the present invention may further include appropriate carriers, excipients, and diluents, which are generally used in the preparation of pharmaceutical compositions. The excipient may be, for example, one or more selected from the group consisting of a diluent, a binder, a disintegrant, a lubricant, an adsorbent, a humectant, a film-coating material, and a controlled-release additive.

The pharmaceutical composition according to the present invention may be used by being formulated as tablets, sustained-release tablets, enteric tablets, sublingual tablets, troches, pills, capsules, hard capsules, soft capsules, sustained-release capsules, enteric capsules, granules, sustained-release granules, enteric granules, powder, dry extracts, liquids, suspensions, soft extracts, fluid extracts, lemonades, or external preparations such as aromatic water, oils, spirits, tinctures, inhalants, elixirs, injections, perfusates, sterile injectable solutions, eye drops, plasters, lotions, pastes, sprays, patches, or aerosols according to conventional methods, and the external preparations may be formulated as a cream, a gel, patches, a spray, an ointment, a plaster, a lotion, a liniment, a paste, or a cataplasma.

Carriers, excipients, and diluents, which can be included in the pharmaceutical composition according to the present invention, may include lactose, dextrose, sucrose, an oligosaccharide, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, and mineral oil.

In the case of formulation, the composition according to the present invention may be prepared using a diluent or an excipient such as a filler, a thickening agent, a binder, a wetting agent, a disintegrant, and a surfactant, which are commonly used.

The pharmaceutical composition according to the present invention is administered at a pharmaceutically effective amount. The "pharmaceutically effective amount" used herein refers to an amount sufficient for treating a disease at a reasonable benefit/risk ratio applicable to medical treatment, and an effective dosage may be determined by parameters including the type and severity of a patient's disease, drug activity, sensitivity to a drug, administration time, an administration route and an excretion rate, the duration of treatment and drugs simultaneously used, and other parameters well known in the medical field.

The pharmaceutical composition of the present invention may be administered separately or in combination with other therapeutic agents, sequentially or simultaneously administered with a conventional therapeutic agent, or administered in a single or multiple dose(s). In consideration of all of the above-mentioned parameters, it is important to achieve the maximum effect with the minimum dose without side effects, and such a dose may be easily determined by one of ordinary skill in the art.

The pharmaceutical composition of the present invention may be administered to a subject via various routes. All administration routes may be considered, and the pharmaceutical composition of the present invention may be administered by, for example, oral administration, subcutaneous injection, intraperitoneal administration, intravenous, intramuscular or intrathecal injection, sublingual administration, buccal administration, rectal insertion, vaginal insertion, ocular administration, ear administration, nasal administration, inhalation, spraying through the mouth or nose, skin administration, or transdermal administration.

The pharmaceutical composition of the present invention is determined according to the type of drug as an active ingredient as well as various related parameters such as a disease to be treated, an administration route, a patient's age, sex, body weight, and the severity of a disease.

In the present invention, "individual" refers to a subject in need of treatment for a disease, and more specifically, a mammal such as a human or a non-human primate, a mouse, a rat, a dog, a cat, a horse, or a cow.

In the present invention, "administration" refers to providing a given composition of the present invention to a subject by any appropriate method.

In the present invention, "prevention" refers to all actions involved in inhibiting or delaying the onset of a desired disease, "treatment" refers to all actions involved in improving or beneficially changing a desired disease and its metabolic abnormalities by administering the pharmaceutical composition according to the present invention, and "alleviation" refers to all actions involved in reducing parameters related to a desired disease, e.g., the severity of symptoms, by administering the composition according to the present invention.

In addition, the present invention provides a kit for preventing or treating pulmonary fibrosis, which includes the composition according to the present invention. In the present invention, "kit" refers to a combination of materials and devices for preventing or treating pulmonary fibrosis using the compound according to the present invention, and there is no limitation on the specific form. The kit according to the present invention may not only include the compound according to the present invention for preventing and/or treating pulmonary fibrosis, but also one or more types of component compositions, solutions, or devices, which are suitable for preventing, alleviating, or treating the disease. Also, the kit may further include instructions that describe information related to the compound of the present invention.

Throughout the specification, when one part "includes" a component, it means that it may also include other components rather than excluding components unless specifically stated otherwise. The term "approximately" or "substantially" used herein are used at, or in proximity to, numerical values when allowable manufacturing and material tolerances, which are inherent in the meanings, are presented. These terms are used to prevent the unfair use of the disclosures in which correct or absolute values are cited to help in understanding the present invention by unscrupulous infringers.

Throughout the specification, the term "combination thereof" included in the Markush-type expression refers to a mixture or combination of one or more selected from the group consisting of constituents described in the Markush-type expression, that is, one or more selected from the group consisting of the components.

Hereinafter, preferred examples are presented to allow the present invention to be better understood. However, the following examples are merely provided to more easily understand the present invention, and the content of the present invention is not limited by the following examples.

### Experimental Examples

### Experimental Example 1. Preparation of compounds

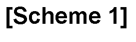

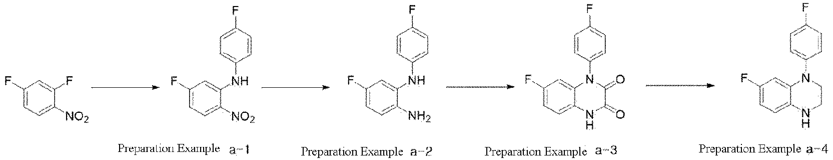

### <Preparation Process a> Preparation of 7-fluoro-1-(4-fluorophenyl)-1,2,3,4-tetrahydroquinoxaline

7-Fluoro-1-(4-fluorophenyl)-1,2,3,4-tetrahydroquinoxaline according to the present invention was prepared according to Preparation Examples a-1 to a-4 below.

### <Preparation Example a-1> Preparation of 5-fluoro-N-(4-fluorophenyl)-2-nitroaniline

A mixture of 2,4-difluoro-1-nitrobenzene (4.0 g, 25.14 mmol) and 4-fluoroaniline (2.8 g, 25. 14 mmol) was stirred at 130 °C for 24 h. After cooling the reaction mixture was quenched with water and extracted with dichloromethane (DCM). The organic layer was washed with brine, dried with MgSO₄, filtered, and then concentrated under reduced pressure. The residue was purified by silica column chromatography (with *n-*hexane/EtOAc) to give 5-fluoro-*N*-(4-fluorophenyl)-2-nitroaniline.

Yellow solid (yield: 63%); ¹H NMR (400 MHz, CDCl₃) δ 9.54 (s, 1H), 8.26 (dd, *J* = 9.4, 6.3 Hz, 1H), 7.27-7.23 (m, 2H), 7.18-7.13 (m, 2H), 6.63 (dd, *J* = 11.3, 2.7 Hz, 1H), 6.48 (ddd, 1H, *J* = 9.5, 6.9, 2.7 Hz).

### <Preparation Example a-2> Preparation of 5-fluoro-N¹-(4-fluorophenyl)benzene-1,2-diamine

Tin (II) chloride dihydrate (10.8 g, 47.96 mmol) was added to a solution of **Preparation Example a-1** (4.0 g, 15.98 mmol) in EtOAc (32 mL). The reaction mixture was stirred at 90 °C for 5 h. After cooling the reaction mixture was diluted with water, adjusted to pH 9 with 10M NaOH (aq.) and extracted with EtOAc. The combined organic layer dried with MgSO₄, filtered, and then concentrated under reduced pressure. The residue was purified by silica column chromatography (with *n*-hexane/EtOAc) to give 5-fluoro-*N¹*-(4-fluorophenyl)benzene-1,2-diamine.

Orange solid (yield: 56%); ¹H NMR (400 MHz, CDCl₃) δ 7.00-6.94 (m, 2H), 6.86-6.82 (m, 2H), 6.79 (dd, *J* = 10.0, 2.9 Hz, 1H), 6.70 (dd, *J* = 8.6, 5.9 Hz, 1H), 6.64-6.59 (m, 1H), 5.27 (s, 1H), 3.49 (s, 2H); LC/MS ESI (+): 220.6 (M+1).

### <Preparation Example a-3> Preparation of 7-fluoro-1-(4-fluorophenyl)quinoxalin-2,3(1H,4H)-dione

A mixture of **Preparation Example a-2** (3.0 g, 14.83 mmol) in diethyl oxalate (130 mL, 89.00 mmol) was heated to160 °C for 24 h and then cooled to room temperature. The precipitate formed in the mixture was collected by filtration, washed with EtOAc and then dried vacuo to give 7-fluoro-1-(4-fluorophenyl)quinoxalin-2,3(*1H,4H*)-dione.

Ivory solid (yield: 68%); ¹H NMR (400 MHz, DMSO-*d₆*) δ 12.13 (s, 1H), 7.47 (d, *J* = 6.7 Hz, 4H), 7.23 (dd, *J* = 8.8, 5.3 Hz, 1H), 7.05-7.00 (m, 1H), 6.08 (dd, *J =* 10.4, 2.5 Hz, 1H); LC/MS ESI (+): 274.9 (M+1).

### <Preparation Example a-4> Preparation of 7-fluoro-1-(4-fluorophenyl)-1,2,3,4-tetrahydroquinoxaline

To a solution of **Preparation Example a-3** (2.0 g, 7.29 mmol) in THF (14 mL) was added dropwise 1M borane-THF solution (22 mL, 21.88 mmol) and stirred at room temperature. The reaction mixture was heated at 65°C for 6 h. After cooling the reaction mixture was quenched with water, and the pH was adjusted to 9 with Conc. NaHCO₃ (aq.). The mixture was extracted with EtOAc, and the organic layer was dried with MgSO₄, filtered, and then concentrated under reduced pressure. The residue was purified by silica column chromatography (with n-hexane/EtOAc) to give 7-fluoro-1-(4-fluorophenyl)-1,2,3,4-tetrahydroquinoxaline.

Light purple solid (yield: 72%); ¹H NMR (400 MHz, CDCl₃) δ 7.21-7.16 (m, 2H), 7.09-7.03 (m, 2H), 6.49 (dd, *J* = 8.6, 5.5 Hz, 1H), 6.36-6.27 (m, 2H), 3.66-3.63 (m, 2H), 3.46-3.44 (m, 2H); LC/MS ESI (+): 246.8 (M+1).

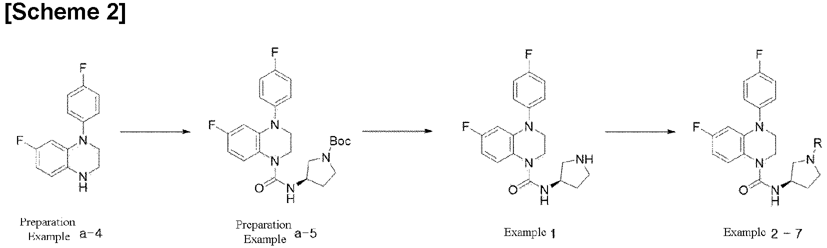

### <Preparation Example a-5> Preparation of tert-butyl (R)-3-(6-fluoro-4-(4-fluorophenyl)-1,2,3,4-tetrahydroquinoxaline-1-carboxamido)pyrrolidine-1-carboxylate

To a solution of **Preparation Example a-4** (200 mg, 0.81 mmol), TEA (0.23 mL, 1.62 mmol) and DCM (1.6 mL) cooled to 0 °C under a nitrogen atmosphere was added dropwise triphosgene (120 mg, 0.41 mmol). The reaction mixture was stirred for 1 h then TEA (0.23 mL, 1.62 mmol) and (*R*)-(+)-1-Boc-3-aminopyrrolidine (0.16 mL, 0.97 mmol) were added slowly. The mixture warmed to room temperature and stirred for 3 h. The resulting mixture was partitioned between DCM and water. The organic layer was washed with brine, dried with MgSO₄, filtered and then concentrated under reduced pressure. The residue was purified by silica column chromatography (with *n-*hexane/EtOAc) to give *tert*-butyl (*R*)-3-(6-fluoro-4-(4-fluorophenyl)-1,2,3,4-tetrahydroquinoxaline-1-carboxamido)pyrrolidine-1-carboxylate.

Light yellow solid (yield: 96%); ¹H NMR (400 MHz, CDCl₃) δ 7.24-7.20 (m, 2H), 7.16-7.10 (m, 2H), 7.05 (dd, *J* = 8.9, 5.6 Hz, 1H), 6.41-6.39 (m, 1H), 6.25 (dd, *J* = 11.3, 2.7 Hz, 1H), 5.25 (dd, *J* = 6.7 Hz, 1H), 4.41 (brs, 1H), 3.95-3.88 (m, 2H), 3.68-3.60 (m, 3H), 3.45-3.40 (m, 2H), 3.18 (brs, 1H), 2.20-2.14 (m, 1H), 1.85-1.75 (m, 1H), 1.46 (s, 9H).

### <Example 1> Synthesis of (R)-6-fluoro-4-(4-fluorophenyl)-N-(pyrrolidin-3-yl)-3,4-dihydroquinoxaline-1(2H)-carboxamide

To a solution of **Preparation Example a-5** (300 mg, 0.65 mmol) in DCM (1.3 mL) was added TFA (0.5 mL, 6.54 mmol). The reaction mixture was stirred at room temperature for 24 h. The reaction mixture was diluted with DCM, and the pH was adjusted to 9 with 10M NaOH (aq.). The mixture was extracted with DCM, the organic layer dried with MgSO₄, filtered, and then concentrated under reduced pressure. The residue was purified by silica column chromatography (with *n*-hexane/EtOAc) to give (*R*)-6-fluoro-4-(4-fluorophenyl)-*N*-(pyrrolidin-3-yl)-3,4-dihydroquinoxaline-1(*2H*)-carboxamide.

Yellow solid (yield: 47%); ¹H NMR (400 MHz, CD₃OD) δ 7.34-7.29 (m, 2H), 7.24-7.16 (m, 3H), 6.45-6.40 (m, 1H), 6.17 (dd, *J* = 11.3, 2.7 Hz, 1H), 4.29-4.23 (m, 1H), 3.88-3.82 (m, 2H), 3.68-3.60 (m, 2H), 3.14-3.08 (m, 1H), 3.06-2.99 (m, 1H), 2.91-2.85 (m, 1H), 2.81-2.73 (m, 1H), 2.19-2.10 (m, 1H), 1.74-1.66 (m, 1H); LC/MS ESI (+): 359.1 (M+1).

### <Example 2> Synthesis of (R)-6-fluoro-4-(4-fluorophenyl)-N-(1-methylpyrrolidin-3-yl)-3,4-dihydroquinoxaline-1(2H)-carboxamide

A solution of **Example 1** (50 mg, 0.14 mmol), sodium triacetoxyborohydride (59 mg, 0.27 mmol) and formaldehyde (20.0 µL, 0.56 mmol) in methanol (1.0 mL) was stirred at room temperature for 1 h. The reaction mixture was quenched with water and extracted with DCM. The organic layer was washed with brine, dried with MgSO₄, filtered, and then concentrated under reduced pressure. The residue was purified by silica column chromatography (with DCM/MeOH) to give (*R*)-6-fluoro-4-(4-fluorophenyl)-*N*-(1-methylpyrrolidin-3-yl)-3,4-dihydroquinoxaline-1(*2H*)-carboxamide.

Yellow oil (yield: 73%); ¹H NMR (400 MHz, CDCl₃) δ 7.25-7.20 (m, 2H), 7.16-7.09 (m, 3H), 6.42-6.38 (m, 1H), 6.24 (dd, *J* = 11.5, 2.9 Hz, 1H), 5.55 (d, *J* = 6.7 Hz, 1H), 4.47-4.38 (m, 1H), 4.01-3.95 (m, 1H), 3.87-3.81 (m, 1H), 3.60 (t, *J =* 5.1 Hz, 2H), 2.91-2.82 (m, 1H), 2.71-2.69 (m, 1H), 2.60-2.56 (m, 1H), 2.41-2.30 (m, 4H), 2.28-2.21 (m, 1H), 1.66-1.57 (m, 1H); LC/MS ESI (+): 373.1 (M+1).

### <Example 3> Synthesis of (R)-6-fluoro-4-(4-fluorophenyl)-N-(1-isopropylpyrrolidin-3-yl)-3,4-dihydroquinoxaline-1(2H)-carboxamide

The title compound was synthesized in the same manner as Example 2, using acetone in place of formaldehyde to give (*R*)-6-fluoro-4-(4-fluorophenyl)-*N*-(1-isopropylpyrrolidin-3-yl)-3,4-dihydroquinoxaline-1(*2H*)-carboxamide.

Yellow oil (yield: 32%); ¹H NMR (400 MHz, CDCl₃) δ 7.25-7.20 (m, 2H), 7.16-7.09 (m, 3H), 6.43-6.39 (m, 1H), 6.25 (dd, *J =* 11.3, 2.7 Hz, 1H), 5.85-5.74 (m, 1H), 4.49-4.38 (m, 1H), 4.00-3.94 (m, 1H), 3.89-3.83 (m, 1H), 3.61 (t, *J* = 5.1 Hz, 2H), 3.05-2.95 (m, 1H), 2.86-2.73 (m, 2H), 2.54-2.26 (m, 3H), 1.74-1.63 (m, 1H), 1.13 (t, *J =* 6.5 Hz, 6H); LC/MS ESI (+): 401.1 (M+1).

### <Example 4> Synthesis of (R)-6-fluoro-4-(4-fluorophenyl)-N-(1-isobutylpyrrolidin-3-yl)-3,4-dihydroquinoxaline-1(2H)-carboxamide

The title compound was synthesized in the same manner as Example 2, using isobutyraldehyde in place of formaldehyde to give (*R*)-6-fluoro-4-(4-fluorophenyl)-*N*-(1-isobutylpyrrolidin-3-yl)-3,4-dihydroquinoxaline-1(2H)-carboxamide.

Yellow oil (yield: 72%); ¹H NMR (400 MHz, CDCl₃) δ 7.25-7.20 (m, 2H), 7.15-7.09 (m, 3H), 6.41-6.37 (m, 1H), 6.25 (dd, *J* = 11.3, 2.7 Hz, 1H), 5.73-5.57 (m, 1H), 4.42-4.32 (m, 1H), 3.97-3.86 (m, 2H), 3.60 (t, *J* = 5.1 Hz, 2H), 2.92-2.80 (m, 1H), 2.67-2.56 (m, 1H), 2.53-2.42 (m, 1H), 2.29-2.14 (m, 4H), 1.76-1.58 (m, 2H), 0.90 (dd, *J* = 6.3, 2.7 Hz, 6H); LC/MS ESI (+): 414.8 (M+1).

### <Example 5> Synthesis of (R)-6-fluoro-4-(4-fluorophenyl)-N-(1-(2,2,2-trifluoroacetyl)pyrrolidin-3-yl)-3,4-dihydroquinoxaline-1(2H)-carboxamide

To a solution of **Example 1** (50 mg, 0.14 mmol), TEA (30.0 µL, 0.21 mmol) and DCM (1.0 mL) cooled to 0 °C under a nitrogen atmosphere was added dropwise trifluoroacetic anhydride (30.0 µL, 0.21 mmol). The resulting mixture was stirred at 0 °C for 30 min. The reaction mixture was quenched with water, and the pH was adjusted to 8 with Conc. NaHCO₃ (aq.). The mixture was extracted with EtOAc, the organic layer dried with MgSO₄, filtered, and then concentrated under reduced pressure. The residue was purified by silica column chromatography (with *n*-hexane/EtOAc) to give (*R*)-6-fluoro-4-(4-fluorophenyl)-*N*-(1-(2,2,2-trifluoroacetyl)pyrrolidin-3-yl)-3,4-dihydroquinoxaline-1(2H)-carboxamide.

White solid (yield: 45%); ¹H NMR (400 MHz, CDCl₃) δ 7.24-7.21 (m, 2H), 7.17-7.11 (m, 2H), 7.03 (dd, *J* = 8.6, 5.9 Hz, 1H), 6.43-6.37 (m, 1H), 6.26 (dd, *J* = 11.3, 2.7 Hz, 1H), 5.30-5.21 (m, 1H), 4.54-4.45 (m, 1H), 4.01-3.86 (m, 3H), 3.79-3.72 (m, 1H), 3.71-3.61 (m, 3H), 3.58-3.43 (m, 1H), 2.40-2.22 (m, 1H), 2.06-1.82 (m, 1H); LC/MS ESI (+): 454.9 (M+1).

### <Example 6> Synthesis of (R)-N-(1-acetylpyrrolidin-3-yl)-6-fluoro-4-(4-fluorophenyl)-3,4-dihydroquinoxaline-1(2H)-carboxamide

Following the procedure as described for Example 5, reaction of **Example 1** (50 mg, 0.14 mmol) in THF (1.4 mL) with TEA (20.0 µL, 0.14 mmol) and acetyl chloride (10.0 µL, 0.15 mmol) gave (*R*)-*N*-(1-acetylpyrrolidin-3-yl)-6-fluoro-4-(4-fluorophenyl)-3,4-dihydroquinoxaline-1(2*H*)-carboxamide.

Yellow solid (yield: 50%); ¹H NMR (400 MHz, CDCl₃) δ 7.25-7.19 (m, 2H), 7.16-7.10 (m, 2H), 7.07-7.01 (m, 2H), 6.43-6.36 (m, 1H), 6.28-6.23 (m, 1H), 5.29-5.22 (m, 1H), 4.49-4.41 (m, 1H), 4.01-3.93 (m, 1H), 3.89-3.75 (m, 2H), 3.64-3.60 (m, 2H), 3.57-3.49 (m, 2H), 3.36-3.26 (m, 1H), 2.34-2.15 (m, 1H), 2.05 (d, *J* = 3.1 Hz, 3H), 2.01-1.74 (m, 1H); LC/MS ESI (+): 401.0 (M+1).

### <Example 7> Synthesis of (R)-6-fluoro-4-(4-fluorophenyl)-N-(1-(methylsulfonyl)pyrrolidin-3-yl)-3,4-dihydroquinoxaline-1(2H)-carboxamide

The title compound was synthesized in the same manner as Example 6, using methanesulfonyl chloride in place of acetyl chloride to give (*R*)-6-fluoro-4-(4-fluorophenyl)-*N*-(1-(methylsulfonyl)pyrrolidin-3-yl)-3,4-dihydroquinoxaline-1(*2H*)-carboxamide.

Ivory solid (yield: 49%); ¹H NMR (400 MHz, CDCl₃) δ 7.24-7.20 (m, 2H), 7.16-7.06 (m, 3H), 6.44-6.39 (m, 1H), 6.25 (dd, *J =* 11.3, 2.7 Hz, 1H), 5.37 (d, *J =* 7.0 Hz, 1H), 4.51-4.43 (m, 1H), 3.97-3.85 (m, 2H), 3.62 (t, *J =* 5.3 Hz, 2H), 3.57-3.49 (m, 2H), 3.37-3.30 (m, 2H), 2.86 (s, 3H), 2.33-2.23 (m, 1H), 1.98-1.87 (m, 1H); LC/MS ESI (+): 436.9 (M+1).

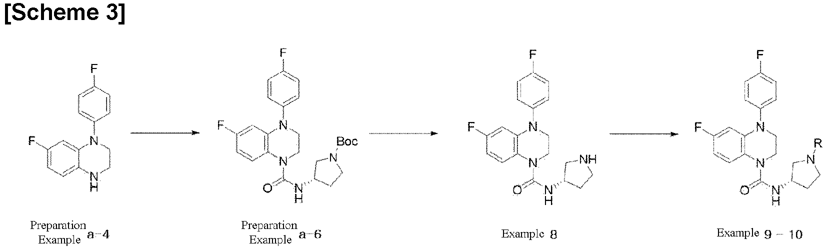

### <Preparation Example a-6> Preparation of tert-butyl (S)-3-(6-fluoro-4-(4-fluorophenyl)-1,2,3,4-tetrahydroquinoxaline-1-carboxamido)pyrrolidine-1-carboxylate

The title compound was prepared in the same manner as Preparation Example a-5, using (*S*)-(-)-1-Boc-3-aminopyrrolidine in place of (*R*)-(+)-1-Boc-3-aminopyrrolidine to give *tert*-butyl (S)-3-(6-fluoro-4-(4-fluorophenyl)-1,2,3,4-tetrahydroquinoxaline-1-carboxamido)pyrrolidine-1-carboxylate.

White solid (yield: 71%); ¹H NMR (400 MHz, CDCl₃) δ 7.25-7.20 (m, 2H), 7.17-7.10 (m, 2H), 7.08-7.02 (m, 1H), 6.44-6.35 (m, 1H), 6.26 (dd, *J =* 11.3, 2.7 Hz, 1H), 5.25 (d, *J* = 7.6 Hz, 1H), 4.48-4.35 (m, 1H), 4.02-3.81 (m, 2H), 3.70-3.59 (m, 3H), 3.49-3.35 (m, 2H), 3.23-3.12 (m, 1H), 2.25-2.12 (m, 1H), 1.89-1.72 (m, 1H), 1.46 (s, 9H).

### <Example 8> Synthesis of (S)-6-fluoro-4-(4-fluorophenyl)-N-(pyrrolidin-3-yl)-3,4-dihydroquinoxaline-1(2H)-carboxamide

Following the procedure as described for Example 1, reaction of **Preparation Example a-6** (1.0 equiv.) in DCM (0.5 M) with TFA (10.0 equiv.) gave (*S*)-6-fluoro-4-(4-fluorophenyl)-*N*-(pyrrolidin-3-yl)-3,4-dihydroquinoxaline-1(2H)-carboxamide.

White solid (yield: 65%); ¹H NMR (400 MHz, CDCl₃) δ 7.25-7.19 (m, 2H), 7.16-7.08 (m, 3H), 6.43-6.38 (m, 1H), 6.25 (dd, *J =* 11.3, 2.7 Hz, 1H), 5.35 (d, *J* = 6.7 Hz, 1H), 4.36-4.29 (m, 1H), 3.97-3.86 (m, 2H), 3.63-3.59 (m, 2H), 3.22-3.17 (m, 1H), 3.07-3.01 (m, 1H), 2.97-2.90 (m, 1H), 2.82-2.78 (m, 1H), 2.22-2.13 (m, 2H), 1.63-1.55 (m, 1H); LC/MS ESI (+): 359.0 (M+1).

### <Example 9> Synthesis of (S)-6-fluoro-4-(4-fluorophenyl)-N-(1-methylpyrrolidin-3-yl)-3,4-dihydroquinoxaline-1(2H)-carboxamide

Following the procedure as described for Example 2, reaction of **Example 8** (1.0 equiv.) in DCM (0.3 M) with sodium triacetoxyborohydride (1.5 equiv.) and formaldehyde (2.0 equiv.) gave (S)-6-fluoro-4-(4-fluorophenyl)-N-(1-methylpyrrolidin-3-yl)-3,4-dihydroquinoxaline-1(2*H*)-carboxamide.

Yellow oil (yield: 32%); ¹H NMR (400 MHz, CDCl₃) δ 7.25-7.20 (m, 2H), 7.16-7.09 (m, 3H), 6.42-6.38 (m, 1H), 6.24 (dd, *J* = 11.3, 2.7 Hz, 1H), 5.48 (d, *J* = 7.4 Hz, 1H), 4.42-4.35 (m, 1H), 4.02-3.96 (m, 1H), 3.87-3.81 (m, 1H), 3.62-3.59 (m, 2H), 2.85-2.80 (m, 1H), 2.66-2.61 (m, 1H), 2.58-2.54 (m, 1H), 2.39-2.31 (m, 4H), 2.25-2.19 (m, 1H), 1.63-1.56 (m, 1H); LC/MS ESI (+): 373.0 (M+1).

### <Example 10> Synthesis of (S)-6-fluoro-4-(4-fluorophenyl)-N-(1-isopropylpyrrolidin-3-yl)-3,4-dihydroquinoxaline-1(2H)-carboxamide

The title compound was synthesized in the same manner as Example 9, using acetone in place of formaldehyde to give (*S*)-6-fluoro-4-(4-fluorophenyl)-*N*-(1-isopropylpyrrolidin-3-yl)-3,4-dihydroquinoxaline-1(*2H*)-carboxamide.

Yellow oil (yield: 64%); ¹H NMR (400 MHz, CDCl₃) δ 7.25-7.20 (m, 2H), 7.16-7.08 (m, 3H), 6.42-6.37 (m, 1H), 6.25 (dd, *J* = 11.3, 2.7 Hz, 1H), 5.48 (d, *J* = 7.4 Hz, 1H), 4.41-4.34 (m, 1H), 4.00-3.94 (m, 1H), 3.89-3.83 (m, 1H), 3.62-3.59 (m, 2H), 2.91-2.84 (m, 1H), 2.73-2.64 (m, 2H), 2.37-2.26 (m, 3H), 1.62-1.54 (m, 1H), 1.08 (t, *J* = 6.5 Hz, 6H); LC/MS ESI (+): 401.0 (M+1).

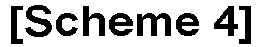

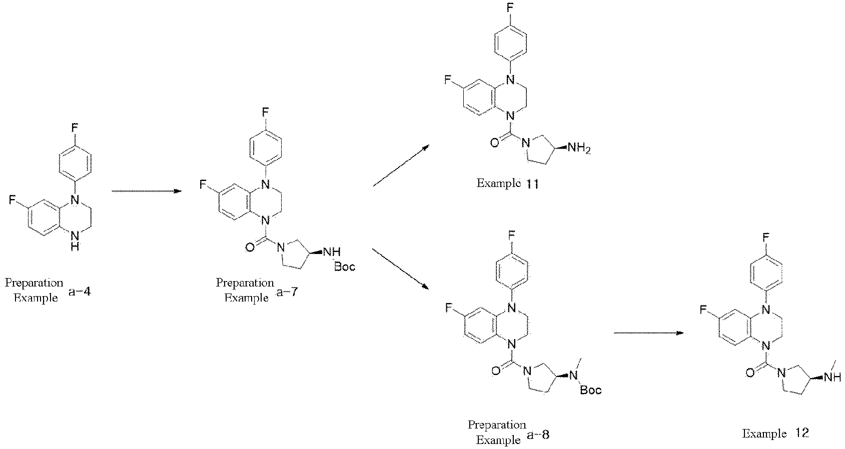

### <Preparation Example a-7> Preparation of tert-butyl (S)-(1-(6-fluoro-4-(4-fluorophenyl)-1,2,3,4-tetrahydroquinoxaline-1-carbonyl)pyrrolidin-3-yl)carbamate

The title compound was prepared in the same manner as Preparation Example a-5, using (*S*)-(-)-3-(Boc-amino)pyrrolidine in place of (*R*)-(+)-1-Boc-3-aminopyrrolidine to give *tert*-butyl (*S*)-(1-(6-fluoro-4-(4-fluorophenyl)-1,2,3,4-tetrahydroquinoxaline-1-carbonyl)pyrrolidin-3-yl)carbamate.

White solid (yield: 93%); ¹H NMR (400 MHz, CD₃OD) δ 7.36-7.27 (m, 2H), 7.22-7.13 (m, 2H), 6.93-6.85 (m, 1H), 6.44-6.34 (m, 1H), 6.20-6.12 (m, 1H), 4.06-3.99 (m, 1H), 3.86-3.67 (m, 4H), 3.57-3.37 (m, 3H), 3.25-3.16 (m, 1H), 2.14-1.99 (m, 1H), 1.91-1.78 (m, 1H), 1.42 (s, 9H).

### <Example 11> Synthesis of (S)-(3-aminopyrrolidin-1-yl)(6-fluoro-4-(4-fluorophenyl)-3,4-dihydroquinoxalin-1(2H)-yl)methanone

Following the procedure as described for Example 1, reaction of **Preparation Example a-7** (1.0 equiv.) in DCM (0.5 M) with TFA (10.0 equiv.) gave (*S*)-(3-aminopyrrolidin-1-yl)(6-fluoro-4-(4-fluorophenyl)-3,4-dihydroquinoxalin-1(2*H*)-yl)methanone.

White solid (yield: 49%); ¹H NMR (400 MHz, CDCl₃) δ 7.25-7.17 (m, 2H), 7.14-7.06 (m, 2H), 6.92-6.84 (m, 1H), 6.43-6.33 (m, 1H), 6.30-6.22 (m, 1H), 3.93-3.76 (m, 2H), 3.76-3.68 (m, 2H), 3.61-3.47 (m, 3H), 3.47-3.36 (m, 2H), 3.07-3.03 (m, 1H), 2.11-1.99 (m, 1H), 1.72-1.59 (m, 1H); LC/MS ESI (+): 358.6 (M+1).

### <Preparation Example a-8> Preparation of tert-butyl (S)-(1-(6-fluoro-4-(4-fluorophenyl)-1,2,3,4-tetrahydroquinoxaline-1-carbonyl)pyrrolidin-3-yl)(methyl)carbamate

To a solution of **Preparation Example a-7** (60.0 mg, 0.13 mmol) in THF (0.5 mL) were added sequentially NaH (6.3 mg, 0.26 mmol) and iodomethane (22.2 mg, 0.16 mmol). The reaction mixture was stirred at room temperature for 5 h. The reaction mixture was quenched with water and extracted with DCM. The organic layer was washed with brine, dried with MgSO₄, filtered, and then concentrated under reduced pressure. The residue was purified by silica column chromatography (with a *n-*hexane/EtOAc) to give *tert*-butyl (*S*)-(1-(6-fluoro-4-(4-fluorophenyl)-1,2,3,4-tetrahydroquinoxaline-1-carbonyl)pyrrolidin-3-yl)(methyl)carbamate.

White solid (yield: 81%); ¹H NMR (400 MHz, CDCl₃) δ 7.28-7.18 (m, 2H), 7.15-7.06 (m, 2H), 6.92-6.84 (m, 1H), 6.42-6.33 (m, 1H), 6.30-6.22 (m, 1H), 4.10-4.03 (m, 1H), 3.78-3.66 (m, 2H), 3.66-3.57 (m, 1H), 3.53-3.34 (m, 3H), 3.28-3.16 (m, 1H), 2.76 (s, 3H), 2.04-1.86 (m, 2H), 1.45 (s, 9H).

### <Example 12> Synthesis of (S)-(6-fluoro-4-(4-fluorophenyl)-3,4-dihydroquinoxalin-1(2H)-yl)(3-methylamino)pyrrolidin-1-yl)methanone

Following the procedure as described for Example 1, reaction of **Preparation Example a-8** (1.0 equiv.) in DCM (0.5 M) with TFA (10.0 equiv.) gave (*S*)-(6-fluoro-4-(4-fluorophenyl)-3,4-dihydroquinoxalin-1(*2H*)-yl)(3-methylamino)pyrrolidin-1-yl)methanone.

Ivory solid (yield: 80%); ¹H NMR (400 MHz, CD₃OD) δ 7.35-7.27 (m, 2H), 7.23-7.14 (m, 2H), 6.98-6.90 (m, 1H), 6.44-6.35 (m, 1H), 6.22-6.14 (m, 1H), 3.94-3.80 (m, 1H), 3.78-3.66 (m, 5H), 3.59-3.41 (m, 3H), 2.68 (s, 3H), 2.41-2.25 (m, 1H), 2.08-1.93 (m, 1H); LC/MS ESI (+): 372.7 (M+1).

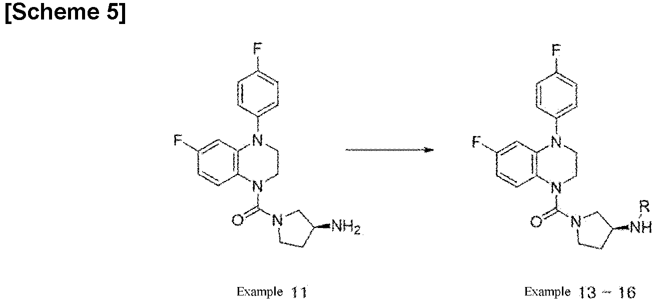

### <Example 13> Synthesis of (S)-(6-fluoro-4-(4-fluorophenyl)-3,4-dihydroquinoxalin-1(2H)-yl)(3-(isobutylamino)pyrrolidin-1-yl)methanone

Following the procedure as described for Example 2, reaction of **Example 11** (1.0 equiv.) in DCM (0.3 M) with sodium triacetoxyborohydride (1.5 equiv.) and isobutyraldehyde (2.0 equiv.) gave (*S*)-(6-fluoro-4-(4-fluorophenyl)-3,4-dihydroquinoxalin-1(2*H*)-yl)(3-(isobutylamino)pyrrolidin-1-yl)methanone.

Colorless oil (yield: 50%); ¹H NMR (400 MHz, CDCl₃) δ 7.25-7.17 (m, 2H), 7.15-7.06 (m, 2H), 6.91- 6.83 (m, 1H), 6.41-6.32 (m, 1H), 6.30-6.22 (m, 1H), 3.92-3.82 (m, 1H), 3.82-3.75 (m, 1H), 3.74-3.67 (m, 2H), 3.58-3.44 (m, 2H), 3.44-3.33 (m, 1H), 3.33-3.24 (m, 1H), 3.17-3.04 (m, 1H), 2.47-2.33 (m, 2H), 2.10-1.98 (m, 1H), 1.76-1.62 (m, 2H), 1.25 (s, 1H), 0.90 (d, *J* = 6.7 Hz, 6H); LC/MS ESI (+): 414.7 (M+1).

### <Example 14> Synthesis of (S)-(6-fluoro-4-(4-fluorophenyl)-3,4-dihydroquinoxalin-1(2H)-yl)(3-(isopropylamino)pyrrolidin-1-yl)methanone

The title compound was synthesized in the same manner as Example 13, using acetone in place of isobutyraldehyde to give (*S*)-(6-fluoro-4-(4-fluorophenyl)-3,4-dihydroquinoxalin-1(2*H*)-yl)(3-(isopropylamino)pyrrolidin-1-yl)methanone.

Light yellow oil (yield: 46%); ¹H NMR (400 MHz, CDCl₃) δ 7.24-7.17 (m, 2H), 7.15-7.05 (m, 2H), 6.91-6.83 (m, 1H), 6.42-6.32 (m, 1H), 6.29-6.21 (m, 1H), 4.00-3.88 (m, 1H), 3.77-3.65 (m, 3H), 3.61-3.46 (m, 2H), 3.46-3.34 (m, 2H), 3.10-3.01 (m, 1H), 2.93-2.81 (m, 1H), 2.17-2.06 (m, 1H), 1.74-1.60 (m, 1H), 1.25 (s, 1H), 1.06 (dd, J= 8.3, 6.2 Hz, 6H); LC/MS ESI (+): 400.7 (M+1).

### <Example 15> Synthesis of (S)-N-(1-(6-fluoro-4-(4-fluorophenyl)-1,2,3,4-tetrahydroquinoxaline-1-carbonyl)pyrrolidin-3-yl)acetamide

To a solution of **Example 11** (1.0 equiv.), TEA (2.5 equiv.) and THF (0.3 M) were added dropwise acetyl chloride (1.8 equiv.). The resulting mixture was stirred at room temperature for 4.5 h. The reaction mixture was quenched with water, and the pH was adjusted to 8 with Conc. NaHCO₃ (aq.). The mixture was extracted with DCM, the organic layer dried with MgSO₄, filtered, and then concentrated under reduced pressure. The residue was purified by silica column chromatography (with DCM/MeOH) to give (S)-N-(1-(6-fluoro-4-(4-fluorophenyl)-1,2,3,4-tetrahydroquinoxaline-1-carbonyl)pyrrolidin-3-yl)acetamide.

White solid (yield: 68%); ¹H NMR (400 MHz, CD₃OD) δ 7.25-7.06 (m, 4H), 6.91-6.83 (m, 1H), 6.41-6.32 (m, 1H), 6.28-6.20 (m, 1H), 5.48 (d, *J =* 7.2 Hz, 1H), 4.49-4.37 (m, 1H), 3.94-3.84 (m, 1H), 3.84-3.75 (m, 1H), 3.74-3.66 (m, 2H), 3.61-3.52 (m, 1H), 3.52-3.41 (m, 2H), 3.13 (dd, *J* = 11.3, 5.2 Hz, 1H), 2.23-2.11 (m, 1H), 1.97 (s, 3H), 1.87-1.74 (m, 1H); LC/MS ESI (+): 400.7 (M+1).

### <Example 16> Synthesis of (S)-N-(1-(6-fluoro-4-(4-fluorophenyl)-1,2,3,4-tetrahydroquinoxaline-1-carbonyl)pyrrolidin-3-yl)methanesulfonamide

The title compound was synthesized in the same manner as Example 15, using methanesulfonyl chloride in place of acetyl chloride to give (S)-N-(1-(6-fluoro-4-(4-fluorophenyl)-1,2,3,4-tetrahydroquinoxaline-1-carbonyl)pyrrolidin-3-yl)methanesulfonamide.

White solid (yield: 66%); ¹H NMR (400 MHz, CDCl₃) δ 7.25-7.18 (m, 2H), 7.16-7.07 (m, 2H), 6.91-6.83 (m, 1H), 6.42-6.33 (m, 1H), 6.29-6.21 (m, 1H), 4.40 (d, *J* = 7.3 Hz, 1H), 4.09-3.98 (m, 1H), 3.93-3.77 (m, 2H), 3.76-3.69 (m, 2H), 3.65-3.56 (m, 1H), 3.56-3.36 (m, 2H), 3.35-3.26 (m, 1H), 2.99 (s, 3H), 2.27-2.15 (m, 1H), 1.97-1.84 (m, 1H); LC/MS ESI (+): 436.6 (M+1).

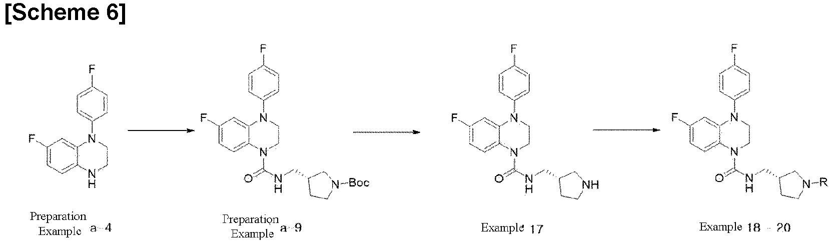

### <Preparation Example a-9> Preparation of tert-butyl (R)-3-((6-fluoro-4-(4-fluorophenyl)-1,2,3,4-tetrahydroquinoxaline-1-carboxamido)methyl)pyrrolidine-1-carboxylate

The title compound was prepared in the same manner as Preparation Example a-5, using (R)-1-Boc-3-(aminomethyl)pyrrolidine in place of (*R*)-(+)-1-Boc-3-aminopyrrolidine to give *tert*-butyl (*R*)-3-((6-fluoro-4-(4-fluorophenyl)-1,2,3,4-tetrahydroquinoxaline-1-carboxamido)methyl)pyrrolidine-1-carboxylate.

White solid (yield: 90%); ¹H NMR (400 MHz, CDCl₃) δ 7.25-7.03 (m, 5H), 6.47-6.34 (m, 1H), 6.30-6.19 (m, 1H), 5.40-5.28 (m, 1H), 3.98-3.83 (m, 2H), 3.66-3.57 (m, 2H), 3.56-3.35 (m, 3H), 3.34-3.23 (m, 2H), 3.10-2.94 (m, 1H), 2.53-2.30 (m, 1H), 2.06-1.87 (m, 1H), 1.71-1.52 (m, 1H), 1.44 (s, 9H).

### <Example 17> Synthesis of (S)-6-fluoro-4-(4-fluorophenyl)-N-(pyrrolidin-3-ylmethyl)-3,4-dihydroquinoxaline-1(2H)-carboxamide

Following the procedure as described for Example 1, reaction of **Preparation Example a-9** (1.0 equiv.) in DCM (0.5 M) with TFA (10.0 equiv.) gave (*S*)-6-fluoro-4-(4-fluorophenyl)-*N*-(pyrrolidin-3-ylmethyl)-3,4-dihydroquinoxaline-1(*2H*)-carboxamide.

White solid (yield: 46%); ¹H NMR (400 MHz, CDCl₃) δ 7.25-5-7.05 (m, 5H), 6.47-6.36 (m, 1H), 6.28-6.20 (m, 1H), 5.74-5.66 (m, 1H), 4.03-3.82 (m, 2H), 3.68-3.56 (m, 2H), 3.47-3.24 (m, 5H), 3.22-3.08 (m, 1H), 2.75-2.60 (m, 1H), 2.29-1.99 (m, 2H), 1.86-1.71 (m, 1H); LC/MS ESI (+): 373.2 (M+1).

### <Example 18> Synthesis of (R)-6-fluoro-4-(4-fluorophenyl)-N-((1-methylpyrrolidin-3-yl)methyl)-3,4-dihydroquinoxaline-1(2H)-carboxamide

Following the procedure as described for Example 2, reaction of **Example 17** (1.0 equiv.) in DCM (0.3 M) with sodium triacetoxyborohydride (1.5 equiv.) and formaldehyde (2.0 equiv.) gave (*R*)-6-fluoro-4-(4-fluorophenyl)-*N*-((1-methylpyrrolidin-3-yl)methyl)-3,4-dihydroquinoxaline-1(2*H*)-carboxamide.

White solid (yield: 40%); ¹H NMR (400 MHz, CDCl₃) δ 7.25-7.7.06 (m, 5H), 6.47-6.37 (m, 1H), 6.29-6.21 (m, 1H), 5.91 (brs, 1H), 3.99-3.83 (m, 2H), 3.69-3.52 (m, 2H), 3.42-3.26 (m, 2H), 3.06-2.83 (m, 2H), 2.82-2.58 (m, 3H), 2.50 (s, 3H), 2.23-2.06 (m, 1H), 1.80-1.63 (m, 1H); LC/MS ESI (+): 387.2 (M+1).

### <Example 19> Synthesis of (R)-6-fluoro-4-(4-fluorophenyl)-N-((1-isopropylpyrrolidin-3-yl)methyl)-3,4-dihydroquinoxaline-1(2H)-carboxamide

The title compound was synthesized in the same manner as Example 18, using acetone in place of formaldehyde to give (*R*)-6-fluoro-4-(4-fluorophenyl)-*N*-((1-isopropylpyrrolidin-3-yl)methyl)-3,4-dihydroquinoxaline-1(*2H*)-carboxamide.

Yellow solid (yield: 64%); ¹H NMR (400 MHz, CDCl₃) δ 7.25-7.07 (m, 5H), 6.46-6.37 (m, 1H), 6.31-6.19 (m, 1H), 5.83 (brs, 1H), 4.03-3.92 (m, 1H), 3.90-3.79 (m, 1H), 3.70-3.53 (m, 2H), 3.42-3.27 (m, 2H), 3.15-2.81 (m, 3H), 2.81-2.48 (m, 3H), 2.19-2.01 (m, 1H), 1.78-1.62 (m, 1H), 1.24-1.11 (m, 6H); LC/MS ESI (+): 415.2 (M+1).

### <Example 20> Synthesis of (R)-6-fluoro-4-(4-fluorophenyl)-N-((1-isobutylpyrrolidin-3-yl)methyl)-3,4-dihydroquinoxaline-1(2H)-carboxamide

The title compound was synthesized in the same manner as Example 18, using isobutyraldehyde in place of formaldehyde to give (*R*)-6-fluoro-4-(4-fluorophenyl)-*N*-((1-isobutylpyrrolidin-3-yl)methyl)-3,4-dihydroquinoxaline-1(*2H*)-carboxamide.

White solid (yield: 64%); ¹H NMR (400 MHz, CD₃OD) δ 7.37-7.14 (m, 5H), 6.50-6.40 (m, 1H), 6.26-6.16 (m, 1H), 3.96-3.77 (m, 2H), 3.70-3.59 (m, 2H), 3.28 (dd, *J =* 6.8, 1.7 Hz, 2H), 3.20-2.96 (m, 3H), 2.89-2.65 (m, 3H), 2.64-2.52 (m, 1H), 2.19-2.04 (m, 1H), 1.99-1.84 (m, 1H), 1.80-1.66 (m, 1H), 0.97 (dd, *J* = 6.6, 4.6 Hz, 6H); LC/MS ESI (+): 429.2 (M+1).

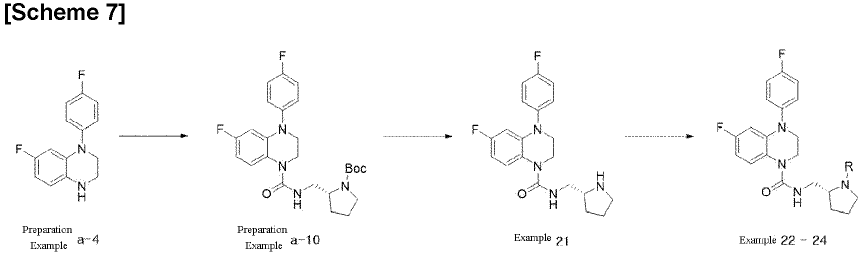

### <Preparation Example a-10> Preparation of tert-butyl (R)-2-((6-fluoro-4-(4-fluorophenyl)-1,2,3,4-tetrahydroquinoxaline-1-carboxamido)methyl)pyrrolidine-1-carboxylate

The title compound was prepared in the same manner as Preparation Example a-5, using (*R*)-1-Boc-2-(aminomethyl)pyrrolidine in place of (*R*)-(+)-1-Boc-3-aminopyrrolidine to give *tert-butyl* (*R*)-2-((6-fluoro-4-(4-fluorophenyl)-1,2,3,4-tetrahydroquinoxaline-1-carboxamido)methyl)pyrrolidine-1-carboxylate.

White solid (yield: 90%); ¹H NMR (400 MHz, CD₃OD) δ 7.37-7.10 (m, 5H), 6.51-6.37 (m, 1H), 6.25-6.11 (m, 1H), 4.02-3.91 (m, 1H), 3.90-3.77 (m, 2H), 3.69-3.59 (m, 2H), 3.51-3.33 (m, 3H), 3.29-3.14 (m, 1H), 2.01-1.71 (m, 4H), 1.55-1.34 (m, 9H).

### <Example 21> Synthesis of (R)-6-fluoro-4-(4-fluorophenyl)-N-(pyrrolidin-2-ylmethyl)-3,4-dihydroquinoxaline-1(2H)-carboxamide

Following the procedure as described for Example 1, reaction of **Preparation Example a-10** (1.0 equiv.) in DCM (0.5 M) with TFA (10.0 equiv.) gave (*R*)-6-fluoro-4-(4-fluorophenyl)-*N*-(pyrrolidin-2-ylmethyl)-3,4-dihydroquinoxaline-1(*2H*)-carboxamide.

Light yellow solid (yield: 53%); ¹H NMR (400 MHz, CD₃OD) δ 7.38-7.13 (m, 5H), 6.53-6.37 (m, 1H), 6.26-6.14 (m, 1H), 3.98-3.82 (m, 2H), 3.78-3.70 (m, 1H), 3.70-3.64 (m, 2H), 3.58-3.41 (m, 2H), 3.39-3.33 (m, 1H), 3.29-3.23 (m, 1H), 2.23-1.93 (m, 3H), 1.87-1.72 (m, 1H); LC/MS ESI (+): 373.2 (M+1).

### <Example 22> Synthesis of (R)-6-fluoro-4-(4-fluorophenyl)-N-((1-methylpyrrolidin-2-yl)methyl)-3,4-dihydroquinoxaline-1(2H)-carboxamide

Following the procedure as described for Example 2, reaction of **Example 21** (1.0 equiv.) in DCM (0.3 M) with sodium triacetoxyborohydride (1.5 equiv.) and formaldehyde (2.0 equiv.) gave (*R*)-6-fluoro-4-(4-fluorophenyl)-*N*-((1-methylpyrrolidin-2-yl)methyl)-3,4-dihydroquinoxaline-1(2*H*)-carboxamide.

Light yellow solid (yield: 23%); ¹H NMR (400 MHz, CD₃OD) δ 7.38-7.16 (m, 5H), 6.46 (ddd, *J* = 8.8, 8.0, 2.8 Hz, 1H), 6.20 (dd, *J* = 11.4, 2.8 Hz, 1H), 3.94-3.85 (m, 2H), 3.72-3.64 (m, 2H), 3.50-3.42 (m, 2H), 3.41-3.34 (m, 1H), 2.99 (brs, 1H), 2.77-2.59 (m, 4H), 2.17-2.05 (m, 1H), 2.02-1.89 (m, 1H), 1.88-1.69 (m, 2H); LC/MS ESI (+): 387.2 (M+1).

### <Example 23> Synthesis of (R)-6-fluoro-4-(4-fluorophenyl)-N-((1-isopropylpyrrolidin-2-yl)methyl)-3,4-dihydroquinoxaline-1(2H)-carboxamide

The title compound was synthesized in the same manner as Example 22, using acetone in place of formaldehyde to give (*R*)-6-fluoro-4-(4-fluorophenyl)-*N*-((1-isopropylpyrrolidin-2-yl)methyl)-3,4-dihydroquinoxaline-1(*2H*)-carboxamide.

White solid (yield: 51%); ¹H NMR (400 MHz, CD₃OD) δ 7.37-7.16 (m, 5H), 6.47 (ddd, *J* = 8.8, 8.0, 2.8 Hz, 1H), 6.21 (dd, *J =* 11.4, 2.8 Hz, 1H), 4.04-3.79 (m, 2H), 3.73-3.63 (m, 2H), 3.62-3.36 (m, 4H), 3.31-3.19 (m, 1H), 3.01 (brs, 1H), 2.20-1.77 (m, 4H), 1.39-1.18 (m, 6H); LC/MS ESI (+): 415.2 (M+1).

### <Example 24> Synthesis of (R)-6-fluoro-4-(4-fluorophenyl)-N-((1-isobutylpyrrolidin-2-yl)methyl)-3,4-dihydroquinoxaline-1(2H)-carboxamide

The title compound was synthesized in the same manner as Example 22, using isobutyraldehyde in place of formaldehyde to give (*R*)-6-fluoro-4-(4-fluorophenyl)-*N*-((1-isobutylpyrrolidin-2-yl)methyl)-3,4-dihydroquinoxaline-1(*2H*)-carboxamide.

Light yellow oil (yield: 49%); ¹H NMR (400 MHz, CD₃OD) δ 7.35-7.14 (m, 5H), 6.48-6.39 (m, 1H), 6.23-6.15 (m, 1H), 4.10-4.00 (m, 1H), 3.77-3.66 (m, 1H), 3.66-3.56 (m, 2H), 3.46-3.38 (m, 1H), 3.29-3.02 (m, 2H), 2.66 (brs, 1H), 2.38 (brs, 1H), 2.29-2.06 (m, 2H), 2.03-1.88 (m, 1H), 1.86-1.53 (m, 4H), 0.98-0.83 (m, 3H), 0.82-0.65 (m, 3H); LC/MS ESI (+): 429.2 (M+1).

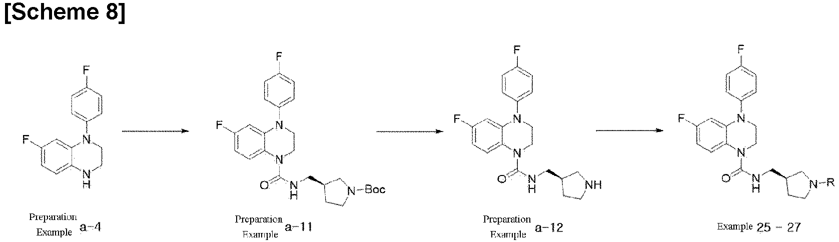

### <Preparation Example a-11> Preparation of tert-butyl (S)-3-((6-fluoro-4-(4-fluorophenyl)-1,2,3,4-tetrahydroquinoxaline-1-carboxamido)methyl)pyrrolidine-1-carboxylate

The title compound was prepared in the same manner as Preparation Example a-5, using (*S*)-1-Boc-3-(aminomethyl)pyrrolidine in place of (*R*)-(+)-1-Boc-3-aminopyrrolidine to give *tert*-butyl (*S*)-3-((6-fluoro-4-(4-fluorophenyl)-1,2,3,4-tetrahydroquinoxaline-1-carboxamido)methyl)pyrrolidine-1-carboxylate.

White solid (yield: 91%); ¹H NMR (400 MHz, CDCl₃) δ 7.25-7.02 (m, 5H), 6.45-6.36 (m, 1H), 6.29-6.21 (m, 1H), 5.41-5.26 (m, 1H), 3.98-3.84 (m, 2H), 3.60 (t, *J = 5.2* Hz, 2H), 3.55-3.35 (m, 3H), 3.35-3.25 (m, 2H), 3.09-2.93 (m, 1H), 2.49-2.37 (m, 1H), 2.02-1.92 (m, 1H), 1.68-1.54 (m, 1H), 1.45 (s, 9H).

### <Preparation Example a-12> Preparation of (R)-6-fluoro-4-(4-fluorophenyl)-N-(pyrrolidin-3-ylmethyl)-3,4-dihydroquinoxaline-1(2H)-carboxamide

Following the procedure as described for Example 1, reaction of **Preparation Example a-11** (1.0 equiv.) in DCM (0.5 M) with TFA (10.0 equiv.) gave (*R*)-6-fluoro-4-(4-fluorophenyl)-*N*-(pyrrolidin-3-ylmethyl)-3,4-dihydroquinoxaline-1(*2H*)-carboxamide.

Light yellow solid (yield: 28%); ¹H NMR (400 MHz, CDCl₃) δ 7.25-7.04 (m, 5H), 6.45-6.36 (m, 1H), 6.28-6.20 (m, 1H), 5.67 (t, *J =* 6.0 Hz, 1H), 4.01-3.82 (m, 2H), 3.67-3.53 (m, 2H), 3.47-3.24 (m, 6H), 3.20-3.08 (m, 1H), 2.73-2.61 (m, 1H), 2.20-2.07 (m, 1H), 1.85-1.72 (m, 1H); LC/MS ESI (+): 373.2 (M+1).

### <Example 25> Synthesis of (S)-6-fluoro-4-(4-fluorophenyl)-N-((1-methylpyrrolidin-3-yl)methyl)-3,4-dihydroquinoxaline-1(2H)-carboxamide

Following the procedure as described for Example 2, reaction of **Preparation Example a-12** (1.0 equiv.) in DCM (0.3 M) with sodium triacetoxyborohydride (1.5 equiv.) and formaldehyde (2.0 equiv.) gave (*S*)-6-fluoro-4-(4-fluorophenyl)-*N*-((1-methylpyrrolidin-3-yl)methyl)-3,4-dihydroquinoxaline-1(*2H*)-carboxamide.

Yellow solid (yield: 33%); ¹H NMR (400 MHz, CD₃OD) δ 7.38-7.15 (m, 5H), 6.51-6.39 (m, 1H), 6.20 (dd, *J =* 11.4, 2.8 Hz, 1H), 3.96-3.80 (m, 2H), 3.74-3.57 (m, 2H), 3.42-3.33 (m, 1H), 3.31-3.21 (m, 3H), 3.09-2.99 (m, 1H), 2.84 (s, 3H), 2.78-2.62 (m, 1H), 2.30-2.13 (m, 1H), 1.89-1.76 (m, 1H), 1.37-1.31 (m, 1H); LC/MS ESI (+): 387.2 (M+1).

### <Example 26> Synthesis of (S)-6-fluoro-4-(4-fluorophenyl)-N-((1-isopropylpyrrolidin-3-yl)methyl)-3,4-dihydroquinoxaline-1(2H)-carboxamide

The title compound was synthesized in the same manner as Example 25, using acetone in place of formaldehyde to give (*S*)-6-fluoro-4-(4-fluorophenyl)-*N*-((1-isopropylpyrrolidin-3-yl)methyl)-3,4-dihydroquinoxaline-1(*2H*)-carboxamide.

White solid (yield: 41%); ¹H NMR (400 MHz, CD₃OD) δ 7.38-7.14 (m, 5H), 6.51-6.40 (m, 1H), 6.27-6.15 (m, 1H), 3.94-3.81 (m, 2H), 3.72-3.60 (m, 2H), 3.57-3.46 (m, 1H), 3.46-3.33 (m, 4H), 3.14 (brs, 1H), 2.76-2.60 (m, 1H), 2.27-2.14 (m, 1H), 1.90-1.76 (m, 1H), 1.38 (d, *J =* 6.5 Hz, 6H), 1.35-1.25 (m, 1H); LC/MS ESI (+): 415.2 (M+1).

### <Example 27> Synthesis of (S)-6-fluoro-4-(4-fluorophenyl)-N-((1-isobutylpyrrolidin-3-yl)methyl)-3,4-dihydroquinoxaline-1(2H)-carboxamide

The title compound was synthesized in the same manner as Example 25, using isobutyraldehyde in place of formaldehyde to give (*S*)-6-fluoro-4-(4-fluorophenyl)-*N*-((1-isobutylpyrrolidin-3-yl)methyl)-3,4-dihydroquinoxaline-1(*2H*)-carboxamide.

White solid (yield: 54%); ¹H NMR (400 MHz, CD₃OD) δ 7.38-7.14 (m, 5H), 6.50-6.41 (m, 1H), 6.20 (dd, *J* = 11.4, 2.8 Hz, 1H), 3.97-3.77 (m, 2H), 3.74-3.56 (m, 2H), 3.30-3.24 (m, 2H), 3.18 (brs, 2H), 3.04-2.87 (m, 1H), 2.86-2.72 (m, 2H), 2.71-2.57 (m, 1H), 2.22-2.08 (m, 1H), 2.05-1.88 (m, 1H), 1.85-1.71 (m, 1H), 1.39-1.22 (m, 1H), 1.00 (dd, *J* = 6.6, 2.6 Hz, 6H); LC/MS ESI (+): 429.2 (M+1).

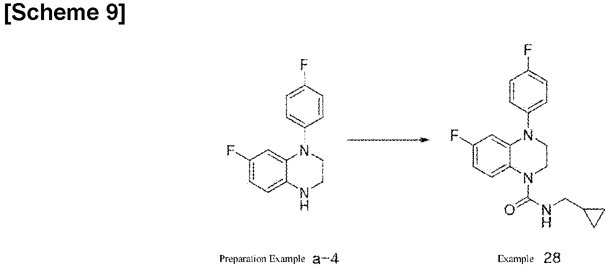

### <Example 28> Synthesis of N-(cyclopropylmethyl)-6-fluoro-4-(4-fluorophenyl)-3,4-dihydroquinoxaline-1(2H)-carboxamide

The title compound was synthesized in the same manner as Example a-5, using cyclopropylmethylamine in place of (*R*)-(+)-1-Boc-3-aminopyrrolidine to give *N-*(cyclopropylmethyl)-6-fluoro-4-(4-fluorophenyl)-3,4-dihydroquinoxaline-1(*2H*)-carboxamide.

White solid (yield: 57%); ¹H NMR (400 MHz, CDCl₃) δ 7.25-7.08 (m, 5H), 6.49-6.35 (m, 1H), 6.26 (dd, *J* = 11.4, 2.8 Hz, 1H), 5.37-5.30 (m, 1H), 3.96-3.89 (m, 2H), 3.65-3.58 (m, 2H), 3.21-3.10 (m, 2H), 1.06-0.91 (m, 1H), 0.56-0.44 (m, 2H), 0.27-0.15 (m, 2H); LC/MS ESI (+): 344.2 (M+1).

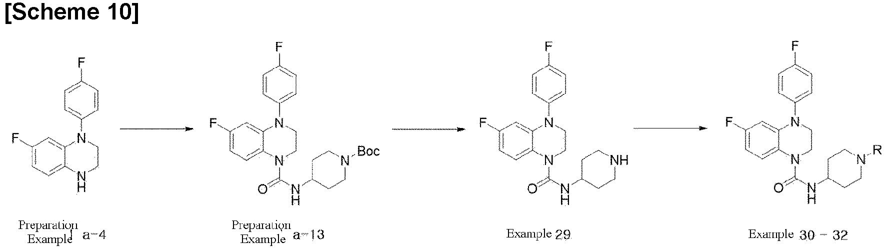

### <Preparation Example a-13> Preparation of tert-butyl 4-(6-fluoro-4-(4-fluorophenyl)-1,2,3,4-tetrahydroquinoxaline-1-carboxamido)piperidine-1-carboxylate

The title compound was prepared in the same manner as Preparation Example a-5, using 1-Boc-4-aminopiperidine in place of (*R*)-(+)-1-Boc-3-aminopyrrolidine to give *tert*-butyl 4-(6-fluoro-4-(4-fluorophenyl)-1,2,3,4-tetrahydroquinoxaline-1-carboxamido)piperidine-1-carboxylate.

White solid (yield: 73%); ¹H NMR (400 MHz, CDCl₃) δ 7.24-7.20 (m, 2H), 7.16-7.09 (m, 2H), 7.07 (dd, *J =* 8.6, 5.9 Hz, 1H), 6.40 (d, *J =* 2.7 Hz, 1H), 6.26 (dd, *J =* 11.3, 2.7 Hz, 1H), 5.11 (d, *J =* 7.8 Hz, 1H), 4.08-3.96 (m, 2H), 3.61 (t, *J =* 5.1 Hz, 2H), 2.96-2.84 (m,2H), 1.99-1.91 (m, 2H), 1.45 (s, 9H), 1.35-1.26 (m, 2H).

### <Example 29> Synthesis of 6-fluoro-4-(4-fluorophenyl)-N-(piperidin-4-yl)-3,4-dihydroquinoxaline-1(2H)-carboxamide

Following the procedure as described for Example 1, reaction of **Preparation Example a-13** (1.0 equiv.) in DCM (0.5 M) with TFA (10.0 equiv.) gave 6-fluoro-4-(4-fluorophenyl)-*N*-(piperidin-4-yl)-3,4-dihydroquinoxaline-1(*2H*)-carboxamide.

White solid (yield: 90%); ¹H NMR (400 MHz, CDCl₃) δ 7.25-7.18 (m, 2H), 7.16-7.08 (m, 3H), 6.43-6.38 (m, 1H), 6.26 (dd, *J =* 11.3, 2.7 Hz, 1H), 5.14 (d, *J =* 7.8 Hz, 1H), 3.92 (t, *J = 5.1* Hz, 2H), 3.87-3.77 (m, 1H), 3.62 (t, *J* = 5.1 Hz, 2H), 3.09-3.04 (m, 2H), 2.75-2.69 (m, 2H), 2.01-1.91 (m, 3H), 1.36-1.27 (m, 2H); LC/MS ESI (+): 373.0 (M+1).

### <Example 30> Synthesis of 6-fluoro-4-(4-fluorophenyl)-N-(1-methylpiperidin-4-yl)-3,4-dihydroquinoxaline-1(2H)-carboxamide

Following the procedure as described for Example 2, reaction of **Example 29** (1.0 equiv.) in DCM (0.3 M) with sodium triacetoxyborohydride (1.5 equiv.) and formaldehyde (2.0 equiv.) gave 6-fluoro-4-(4-fluorophenyl)-*N*-(1-methylpiperidin-4-yl)-3,4-dihydroquinoxaline-1(2*H*)-carboxamide.

White solid (yield: 48%); ¹H NMR (400 MHz, CDCl₃) δ 7.24-7.21 (m, 2H), 7.15-7.06 (m, 3H), 6.43-6.38 (m, 1H), 6.26 (dd, *J* = 11.3, 2.7 Hz, 1H), 5.11 (d, *J* = 7.4 Hz, 1H), 3.91 (t, *J = 5.1* Hz, 1H), 3.80-3.72 (m, 1H), 3.61 (t, *J =* 5.1 Hz, 1H), 2.90-2.77 (m, 2H), 2.33 (s, 3H), 2.26-2.14 (m, 2H), 2.05-1.95 (m, 2H), 1.59-1.48 (m, 2H); LC/MS ESI (+): 387.0 (M+1).

### <Example 31> Synthesis of 6-fluoro-4-(4-fluorophenyl)-N-(1-isopropylpiperidin-4-yl)-3,4-dihydroquinoxaline-1(2H)-carboxamide

The title compound was synthesized in the same manner as Example 30, using acetone in place of formaldehyde to give 6-fluoro-4-(4-fluorophenyl)-*N*-(1-isopropylpiperidin-4-yl)-3,4-dihydroquinoxaline-1(*2H*)-carboxamide.

White solid (yield: 45%); ¹H NMR (400 MHz, CDCl₃) δ 7.24-7.21 (m, 2H), 7.16-7.06 (m, 3H), 6.40-6.35 (m, 1H), 6.26 (dd, *J* = 11.3, 2.7 Hz, 1H), 5.15 (d, *J* = 7.8 Hz, 1H), 3.91 (t, *J = 5.1* Hz, 2H), 3.81-3.70 (m, 1H), 3.60 (t, *J* = 5.1 Hz, 2H), 2.97-2.83 (m, 3H), 2.41-2.32 (m, 2H), 2.05-2.00 (m, 2H), 1.62-1.45 (m, 2H), 1.09 (d, J= 6.7 Hz, 6H); LC/MS ESI (+): 415.0 (M+1).

### <Example 32> Synthesis of 6-fluoro-4-(4-fluorophenyl)-N-(1-isobutylpiperidin-4-yl)-3,4-dihydroquinoxaline-1(2H)-carboxamide

The title compound was synthesized in the same manner as Example 30, using isobutyraldehyde in place of formaldehyde to give 6-fluoro-4-(4-fluorophenyl)-*N*-(1-isobutylpiperidin-4-yl)-3,4-dihydroquinoxaline-1(2H)-carboxamide.

White solid (yield: 40%); ¹H-NMR (400 MHz, CDCl₃) δ 7.24-7.21 (m, 2H), 7.16-7.06 (m, 3H), 6.43-6.38 (m, 1H), 6.26 (dd, *J =* 11.3, 2.7 Hz, 1H), 5.21 (d, *J =* 7.8 Hz, 1H), 3.91 (t, *J = 5.1* Hz, 2H), 3.85-3.75 (m, 1H), 3.61 (t, *J =* 5.1 Hz, 2H), 3.09-2.96 (m, 1H), 2.35-2.20 (m, 4H), 2.07-1.95 (m, 5H), 1.94-1.81 (m, 1H), 0.94 (d, *J =* 6.7 Hz, 6H); LC/MS ESI (+): 428.9 (M+1).

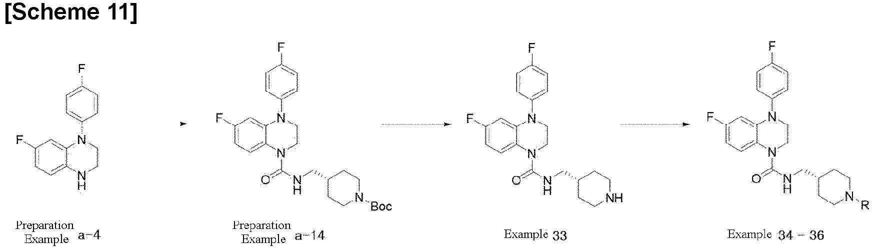

### <Preparation Example a-14> Preparation of tert-butyl 4-((6-fluoro-4-(4-fluorophenyl)-1,2,3,4-tetrahydroquinoxaline-1-carboxamido)methyl)piperidine-1-carboxylate

The title compound was prepared in the same manner as Preparation Example a-5, using 1-Boc-4-(aminomethyl)piperidine in place of (*R*)-(+)-1-Boc-3-aminopyrrolidine to give *tert-butyl* 4-((6-fluoro-4-(4-fluorophenyl)-1,2,3,4-tetrahydroquinoxaline-1-carboxamido)methyl)piperidine-1-carboxylate.

White solid (yield: 95%); ¹H NMR (400 MHz, CD₃OD) δ 7.37-7.25 (m, 2H), 7.24-7.13 (m, 3H), 6.45 (ddd, *J* = 8.9, 8.0, 2.8 Hz, 1H), 6.20 (dd, *J* = 11.4, 2.8 Hz, 1H), 4.17-3.99 (m, 2H), 3.94-3.81 (m, 2H), 3.71-3.60 (m, 2H), 3.12 (d, *J* = 6.6 Hz, 2H), 2.75 (brs, 2H), 1.83-1.63 (m, 3H), 1.46 (s, 9H), 1.21-1.01 (m, 2H).

### <Example 33> Synthesis of 6-fluoro-4-(4-fluorophenyl)-N-(piperidin-4-ylmethyl)-3,4-dihydroquinoxaline-1(2H)-carboxamide

Following the procedure as described for Example 1, reaction of **Preparation Example a-14** (1.0 equiv.) in DCM (0.5 M) with TFA (10.0 equiv.) gave 6-fluoro-4-(4-fluorophenyl)-*N*-(piperidin-4-ylmethyl)-3,4-dihydroquinoxaline-1(*2H*)-carboxamide.

White solid (yield: 80%); ¹H NMR (400 MHz, CD₃OD) δ 7.37-7.27 (m, 2H), 7.25-7.15 (m, 3H), 6.45 (ddd, *J* = 8.8, 8.0, 2.8 Hz, 1H), 6.20 (dd, *J =* 11.4, 2.8 Hz, 1H), 3.86 (dd, *J =* 5.8, 4.5 Hz, 2H), 3.64 (dd, *J =* 5.8, 4.5 Hz, 2H), 3.17-3.09 (m, 3H), 3.09-3.01 (m, 1H), 2.67-2.55 (m, 2H), 1.78-1.62 (m, 3H), 1.27-1.12 (m, 2H); LC/MS ESI (+): 387.1 (M+1).

### <Example 34> Synthesis of 6-fluoro-4-(4-fluorophenyl)-N-((1-methylpiperidin-4-yl)methyl)-3,4-dihydroquinoxaline-1(2H)-carboxamide

Following the procedure as described for Example 2, reaction of **Example 33** (1.0 equiv.) in DCM (0.3 M) with sodium triacetoxyborohydride (1.5 equiv.) and formaldehyde (2.0 equiv.) gave 6-fluoro-4-(4-fluorophenyl)-*NI*-((1-methylpiperidin-4-yl)methyl)-3,4-dihydroquinoxaline-1(2*H*)-carboxamide.

White solid (yield: 68%); ¹H NMR (400 MHz, CD₃OD) δ 7.37-7.27 (m, 2H), 7.25-7.15 (m, 3H), 6.50-6.41 (m, 1H), 6.24-6.16 (m, 1H), 3.86 (dd, *J* = 5.8, 4.5 Hz, 2H), 3.64 (dd, *J* = 5.8, 4.5 Hz, 2H), 3.14 (d, *J* = 6.7 Hz, 2H), 3.09 (d, *J* = 11.7 Hz, 2H), 2.46 (s, 3H), 2.39-2.28 (m, 2H), 1.87-1.76 (m, 2H), 1.74-1.58 (m, 1H), 1.46-1.26 (m, 2H); LC/MS ESI (+): 401.2 (M+1).

### <Example 35> Synthesis of 6-fluoro-4-(4-fluorophenyl)-N-((1-isobutylpiperidin-4-yl)methyl)-3,4-dihydroquinoxaline-1(2H)-carboxamide

The title compound was synthesized in the same manner as Example 34, using isobutyraldehyde in place of formaldehyde to give 6-fluoro-4-(4-fluorophenyl)-*N*-((1-isobutylpiperidin-4-yl)methyl)-3,4-dihydroquinoxaline-1(*2H*)-carboxamide.

White solid (yield: 46%); ¹H NMR (400 MHz, CDCl₃) δ 7.26-7.07 (m, 5H), 6.47-6.38 (m, 1H), 6.29-6.21 (m, 1H), 5.45-5.32 (m, 1H), 3.94-3.87 (m, 2H), 3.64-3.57 (m, 2H), 3.38-2.93 (m, 3H), 2.78-2.19 (m, 3H), 2.17-1.88 (m, 2H), 1.87-1.37 (m, 6H), 1.15-0.89 (m, 6H); LC/MS ESI (+): 443.2 (M+1).

### <Example 36> Synthesis of 6-fluoro-4-(4-fluorophenyl)-N-((1-isopropylpiperidin-4-yl)methyl)-3,4-dihydroquinoxaline-1(2H)-carboxamide

The title compound was synthesized in the same manner as Example 34, using acetone in place of formaldehyde to give 6-fluoro-4-(4-fluorophenyl)-*N*-((1-isopropylpiperidin-4-yl)methyl)-3,4-dihydroquinoxaline-1(*2H*)-carboxamide.

White solid (yield: 67%); ¹H NMR (400 MHz, CD₃OD) δ 7.37-7.15 (m, 5H), 6.50-6.41 (m, 1H), 6.25-6.17 (m, 1H), 3.86 (dd, *J* = 5.8, 4.5 Hz, 2H), 3.64 (dd, *J* = 5.8, 4.6 Hz, 2H), 3.22 (d, *J* = 12.0 Hz, 2H), 3.16 (d, *J* = 6.7 Hz, 3H), 2.65 (t, *J* = 12.1 Hz, 2H), 1.97-1.83 (m, 2H), 1.82-1.65 (m, 1H), 1.51-1.35 (m, 2H), 1.23 (d, *J* = 6.6 Hz, 6H); LC/MS ESI (+): 429.2 (M+1).

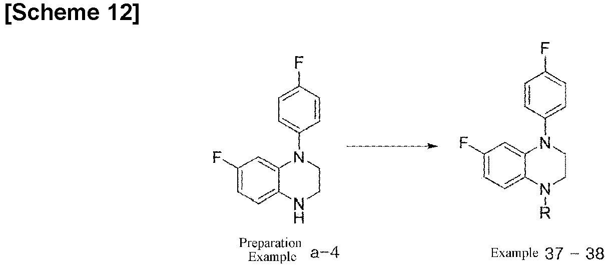

### <Example 37> Synthesis of N-((1H-imidazol-4-)methyl)-6-fluoro-4-(4-fluorophenyl)-3,4-dihydroquinoxaline-1(2H)-carboxamide

The title compound was synthesized in the same manner as Preparation Example a-5, using (1*H*-imidazol-4-yl)methanamine hydrochloride in place of (*R*)-(+)-1-Boc-3-aminopyrrolidine to give *N*-((1*H*-imidazol-4-)methyl)-6-fluoro-4-(4-fluorophenyl)-3,4-dihydroquinoxaline-1(*2H*)-carboxamide.

Light yellow solid (yield: 11%); ¹H NMR (400 MHz, CD₃OD) δ 7.61 (s, 1H), 7.33-7.28 (m, 2H), 7.23-7.15 (m, 3H), 6.96 (s, 1H), 6.44-6.39 (m, 1H), 6.17 (dd, *J* = 11.3, 2.7 Hz, 1H), 4.34 (s, 2H), 3.87 (t, *J* = 5.1 Hz, 2H), 3.63 (t, *J* = 5.1 Hz, 2H); LC/MS ESI (+): 370.0 (M+1).

### <Example 38> Synthesis of 6-fluoro-4-(4-fluorophenyl)-N-(pyrazin-2-ylmethyl)-3,4-dihydroquinoxaline-1(2H)-carboxamide

The title compound was synthesized in the same manner as Preparation Example a-5, using 4-(aminomethyl)pyrimidine in place of (*R*)-(+)-1-Boc-3-aminopyrrolidine to give 6-fluoro-4-(4-fluorophenyl)-*N*-(pyrazin-2-ylmethyl)-3,4-dihydroquinoxaline-1(2*H*)-carboxamide.

Ivory solid (yield: 61%); ¹H NMR (400 MHz, CDCl₃) δ 9.14 (s, 1H), 8.68 (d, *J* = 5.2 Hz, 1H), 7.33 (d, *J* = 5.5 Hz, 1H), 7.30-7.27 (m, 1H), 7.25-7.21 (m, 2H), 7.17-7.11 (m, 2H), 6.49-6.44 (m, 1H), 6.33-6.26 (m, 2H) 4.59 (d, *J* = 5.5 Hz, 1H), 3.96 (t, *J* = 5.1 Hz, 1H), 3.64 (t, *J* = 5.1 Hz, 1H); LC/MS ESI (+): 381.9 (M+1).

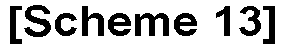

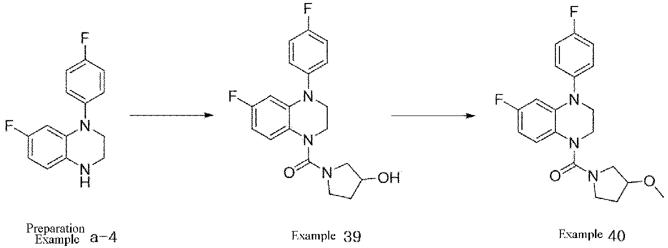

### <Example 39> Synthesis of (6-fluoro-4-(4-fluorophenyl)-3,4-dihydroquinoxalin-1(2H)-yl)(3-hydropyrrolidin-1-yl)methanone

The title compound was synthesized in the same manner as Preparation Example a-5, using 3-pyrrolidinol in place of (*R*)-(+)-1-Boc-3-aminopyrrolidine to give (6-fluoro-4-(4-fluorophenyl)-3,4-dihydroquinoxalin-1(2*H*)-yl)(3-hydropyrrolidin-1-yl)methanone.

Ivory solid (yield: 89%); ¹H NMR (400 MHz, CDCl₃) δ 7.24-7.19 (m, 2H), 7.14-7.08 (m, 2H), 6.90 (dd, *J* = 8.6, 5.9 Hz, 1H), 6.41-6.36 (m, 1H), 6.27 (dd, *J* = 11.3, 2.7 Hz, 1H), 5.48 (d, *J* = 7.4 Hz, 1H), 4.47 (brs, 1H), 4.07-4.02 (m, 1H), 3.76-3.70 (m, 2H), 3.69-3.53 (m, 3H), 3.45-3.39 (m, 1H), 3.34-3.31 (m, 1H), 2.03-1.89 (m, 2H), 1.76 (d, *J* = 3.5 Hz, 1H); LC/MS ESI (+): 360.0 (M+1).

### <Example 40> Synthesis of (6-fluoro-4-(4-fluorophenyl)-3,4-dihydroquinoxalin-1(2H)-yl)(3-methoxypyrrolidin-1-yl)methanone

To a solution of Example 39 (1.0 equiv.) in THF (0.3 M) were added sequentially NaH (2.0 equiv.) and iodomethane (1.2 equiv.). The resulting mixture was stirred at room temperature for 2 h. The reaction mixture was quenched with water and extracted with DCM. The organic layer was washed with brine, dried with MgSO₄, filtered, and then concentrated under reduced pressure. The residue was purified by silica column chromatography (with DCM/MeOH) to give (6-fluoro-4-(4-fluorophenyl)-3,4-dihydroquinoxalin-1(2*H*)-yl)(3-methoxypyrrolidin-1-yl)methanone.

Yellow oil (yield: 96%); ¹H NMR (400 MHz, CDCl₃) δ 7.24-7.19 (m, 2H), 7.13-7.07 (m, 2H), 6.89 (dd, *J* = 9.0, 5.9 Hz, 1H), 6.41-6.36 (m, 1H), 6.26 (dd, *J* = 11.0, 2.7 Hz, 1H), 4.04-3.99 (m, 1H), 3.95-3.90 (m, 1H), 3.75-3.70 (m, 2H), 3.68-3.61 (m, 1H), 3.53-3.46 (m, 2H), 3.44-3.36 (m, 2H), 3.32 (s, 3H), 2.05-1.97 (m, 1H), 1.94-1.85 (m, 1H); LC/MS ESI (+): 374.0 (M+1).

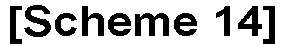

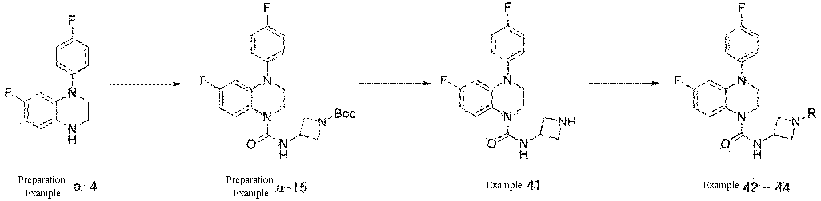

### <Preparation Example a-15> Preparation of tert-butyl 3-(6-fluoro-4-(4-fluorophenyl)-1,2,3,4-tetrahydroquinoxaline-1-carboxamido)azetidine-1-carboxylate

The title compound was synthesized in the same manner as Preparation Example a-5, using 1-Boc-3-aminoazetidine in place of (*R*)-(+)-1-Boc-3-aminopyrrolidine to give *tert*-butyl 3-(6-fluoro-4-(4-fluorophenyl)-1,2,3,4-tetrahydroquinoxaline-1-carboxamido)azetidine-1-carboxylate.

White solid (yield: 94%); ¹H NMR (400 MHz, CDCl₃) δ 7.25-7.20 (m, 2H), 7.17-7.09 (m, 3H), 6.47-6.41 (m, 1H), 6.26 (dd, *J* = 11.3, 2.7 Hz, 1H), 5.56 (d, *J =* 6.7 Hz, 1H), 4.63-4.54 (m, 1H), 4.26-4.24 (m, 2H), 3.92 (t, *J* = 5.1 Hz, 2H), 3.73-3.71 (m, 2H), 3.62 (t, *J* = 5.1 Hz, 2H), 1.44 (s, 9H).

### <Example 41> Synthesis of N-(azetidin-3-yl)-6-fluoro-4-(4-fluorophenyl)-3,4-dihydroquinoxaline-1(2H)-carboxamide

Following the procedure as described for Example 1, reaction of **Preparation Example a-15** (1.0 equiv.) in DCM (0.5 M) with TFA (10.0 equiv.) gave *N*-(azetidin-3-yl)-6-fluoro-4-(4-fluorophenyl)-3,4-dihydroquinoxaline-1(2*H*)-carboxamide.

White solid (yield: 43%); ¹H NMR (400 MHz, CDCl₃) δ 7.25-7.20 (m, 2H), 7.17-7.11 (m, 3H), 6.46-6.41 (m, 1H), 6.26 (dd, *J* = 11.3, 2.7 Hz, 1H), 5.91 (d, *J* = 7.4 Hz, 1H), 4.79-4.70 (m, 1H), 4.10-4.06 (m, 2H), 3.92 (t, *J* = 5.3 Hz, 2H), 3.74-3.65 (m, 2H), 3.62 (t, *J = 5.1* Hz, 2H); LC/MS ESI (+): 345.0 (M+1).

### <Example 42> Synthesis of 6-fluoro-4-(4-fluorophenyl)-N-(1-isopropylazetidin-3-yl)-3,4-dihydroquinoxaline-1(2H)-carboxamide

Following the procedure as described for Example 2, reaction of **Example 41** (1.0 equiv.) in DCM (0.3 M) with sodium triacetoxyborohydride (1.5 equiv.) and acetone (2.0 equiv.) gave 6-fluoro-4-(4-fluorophenyl)-*N*-((1-methylpiperidin-4-yl)methyl)-3,4-dihydroquinoxaline-1(2*H*)-carboxamide.

White solid (yield: 39%); ¹H NMR (400 MHz, CDCl₃) δ 7.25-7.21 (m, 2H), 7.16-7.10 (m, 3H), 6.45-6.40 (m, 5H), 6.25 (dd, *J* = 11.3, 2.7 Hz, 1H), 5.94-5.67 (m, 1H), 4.54-4.45 (m, 1H), 3.91 (t, *J* = 5.1 Hz, 2H), 3.75-3.68 (m, 2H), 3.62 (t, *J* = 5.1 Hz, 2H), 3.26-3.03 (m, 4H) 2.54-2.37 (m, 2H), 0.99 (d, *J =* 6.3 Hz, 6H); LC/MS ESI (+): 387.2 (M+1).

### <Example 43> Synthesis of 6-fluoro-4-(4-fluorophenyl)-N-(1-isobutylazetidin-3-yl)-3,4-dihydroquinoxaline-1(2H)-carboxamide

The title compound was synthesized in the same manner as Example 42, using isobutyraldehyd in place of acetone to give 6-fluoro-4-(4-fluorophenyl)-N-(1-isobutylazetidin-3-yl)-3,4-dihydroquinoxaline-1(2*H*)-carboxamide.

White solid (yield: 56%); ¹H NMR (400 MHz, CDCl₃) δ 7.25-7.21 (m, 2H), 7.16-7.11 (m, 3H), 6.45-6.41 (m, 1H), 6.26 (dd, *J* = 11.3, 2.7 Hz, 1H), 5.59-5.47 (m, 1H), 4.54-4.46 (m, 1H), 3.91 (t, *J =* 5.1 Hz, 2H), 3.69-3.63 (m, 2H), 3.62 (t, *J* = 5.1 Hz, 2H), 2.95-2.80 (m, 2H), 2.25 (d, *J =* 7.0 Hz, 2H), 1.63-1.53 (m, 1H), 0.87 (d, *J* = 6.7 Hz, 6H); LC/MS ESI (+): 401.2 (M+1).

### <Example 44> Synthesis of N-(1-acetylazetidin-3-yl)-6-fluoro-4-(4-fluorophenyl)-3,4-dihydroquinoxaline-1(2H)-carboxamide

To a solution of **Example 41** (1.0 equiv.), TEA (2.5 equiv.) and THF (0.3 M) was added dropwise acetyl chloride (1.8 equiv.). The resulting mixture was stirred at room temperature for 4.5 h. The reaction mixture was quenched with water, and the pH was adjusted to 8 with Conc. NaHCO₃ (aq.). The mixture was extracted with DCM, the organic layer dried with MgSO₄, filtered, and then concentrated under reduced pressure. The residue was purified by silica column chromatography (with DCM/MeOH) to give N-(1-acetylazetidin-3-yl)-6-fluoro-4-(4-fluorophenyl)-3,4-dihydroquinoxaline-1(*2H*)-carboxamide.

White solid (yield: 37%); ¹H NMR (400 MHz, CDCl₃) δ 7.25-7.20 (m, 2H), 7.17-7.11 (m, 3H), 6.46-6.42 (m, 1H), 6.27 (dd, *J* = 11.3, 2.7 Hz, 1H), 5.59 (d, *J = 6.7* Hz, 1H), 4.67-4.59 (m, 1H), 4.47-4.43 (m, 1H), 4.35-4.30 (m, 1H),3.97-3.88 (m, 3H), 3.81-3.77 (m, 1H), 3.63 (t, *J =* 5.1 Hz, 2H), 1.88 (s, 3H); LC/MS ESI (+): 387.1 (M+1).

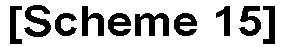

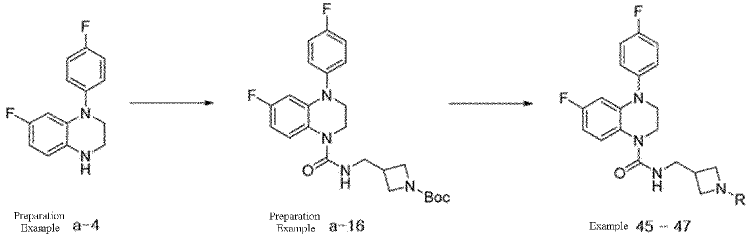

### <Preparation Example a-16> Preparation of tert-butyl 3-((6-fluoro-4-(4-fluorophenyl)-1,2,3,4-tetrahydroquinoxaline-1-carboxamido)methyl)azetidine-1-carboxylate

The title compound was prepared in the same manner as Preparation Example a-5, using 1-Boc-3-(aminomethyl)azetidine in place of (*R*)-(+)-1-Boc-3-aminopyrrolidine to give *tert-butyl* 3-((6-fluoro-4-(4-fluorophenyl)-1,2,3,4-tetrahydroquinoxaline-1-carboxamido)methyl)azetidine-1-carboxylate.

White solid (yield: 81%); ¹H NMR (400 MHz, CD₃OD) δ 7.36-7.28 (m, 2H), 7.26-7.11 (m, 3H), 6.51-6.40 (m, 1H), 6.19 (dd, *J* = 11.4, 2.8 Hz, 1H), 3.98 (dd, *J* = 8.5, 8.5 Hz, 2H), 3.87 (brs, 2H), 3.77-3.67 (m, 2H), 3.66-3.61 (m, 2H), 3.41 (d, *J =* 6.3 Hz, 2H), 2.87-2.71 (m, 1H), 1.44 (s, 9H).

### <Example 45> Synthesis of 6-fluoro-4-(4-fluorophenyl)-N-((1-methylazetidin-3-yl)methyl)-3,4-dihydroquinoxaline-1(2H)-carboxamide

To a solution of **Preparation Example a-16** (1.0 equiv.) in DCM (0.3 M) was added TFA (10.0 equiv.). The reaction mixture was stirred at room temperature for 24 h. The reaction mixture was diluted with DCM, and the pH was adjusted to 9 with 10M NaOH (aq.). The mixture was extracted with DCM, the organic layer dried with MgSO₄, filtered, and then concentrated under reduced pressure. A solution of the obtained concentrate, sodium triacetoxyborohydride (1.5 equiv.) and formaldehyde (2.0 equiv.) in DCM (0.3 M) was stirred at room temperature for 1 h. The reaction mixture was quenched with water and extracted with DCM. The organic layer was washed with brine, dried with MgSO₄, filtered, and then concentrated under reduced pressure. The residue was purified by silica column chromatography (with n-hexane/EtOAc) to give 6-fluoro-4-(4-fluorophenyl)-*N*-((1-methylazetidin-3-yl)methyl)-3,4-dihydroquinoxaline-1(*2H*)-carboxamide.

Ivory solid (yield: 2%); ¹H NMR (400 MHz, CD₃OD) δ 7.36-7.27 (m, 2H), 7.26-7.16 (m, 3H), 6.45 (ddd, *J* = 8.8, 8.0, 2.8 Hz, 1H), 6.20 (dd, *J* = 11.4, 2.8 Hz, 1H), 4.20-4.08 (m, 2H), 4.03-3.92 (m, 2H), 3.87 (dd, *J* = 5.9, 4.5 Hz, 2H), 3.65 (dd, *J* = 5.8, 4.6 Hz, 2H), 3.42 (d, *J* = 6.5 Hz, 2H), 3.18-3.01 (m, 1H), 2.88 (s, 3H); LC/MS ESI (+): 373.1 (M+1).

### <Example 46> Synthesis of 6-fluoro-4-(4-fluorophenyl)-N-((1-isopropylazetidin-3-yl)methyl)-3,4-dihydroquinoxaline-1(2H)-carboxamide

The title compound was synthesized in the same manner as Example 45, using acetone in place of formaldehyde to give 6-fluoro-4-(4-fluorophenyl)-*N*-((1-isopropylazetidin-3-yl)methyl)-3,4-dihydroquinoxaline-1(2*H*)-carboxamide.

White solid (yield: 25%); ¹H NMR (400 MHz, CD₃OD) δ 7.36-7.25 (m, 2H), 7.24-7.14 (m, 3H), 6.45 (ddd, *J* = 8.8, 8.0, 2.8 Hz, 1H), 6.20 (dd, *J =* 11.4, 2.8 Hz, 1H), 3.86 (dd, *J* = 5.8, 4.5 Hz, 2H), 3.76-3.60 (m, 4H), 3.37 (d, *J* = 6.5 Hz, 2H), 3.33 (brs, 1H), 2.84-2.71 (m, 2H), 1.39-1.22 (m, 1H), 1.04 (d, *J* = 6.3 Hz, 6H); LC/MS ESI (+): 401.2 (M+1).

### <Example 47> Synthesis of 6-fluoro-4-(4-fluorophenyl)-N-((1-isobutylazetidin-3-yl)methyl)-3,4-dihydroquinoxaline-1(2H)-carboxamide

The title compound was synthesized in the same manner as Example 45, using isobutyraldehyde in place of formaldehyde to give 6-fluoro-4-(4-fluorophenyl)-N-((1-isobutylazetidin-3-yl)methyl)-3,4-dihydroquinoxaline-1(2*H*)-carboxamide.

White solid (yield: 27%); ¹H NMR (400 MHz, CD₃OD) δ 7.36-7.26 (m, 2H), 7.25-7.14 (m, 3H), 6.45 (ddd, *J* = 8.9, 8.0, 2.8 Hz, 1H), 6.20 (dd, *J =* 11.4, 2.8 Hz, 1H), 3.86 (dd, *J* = 5.8, 4.5 Hz, 2H), 3.76 (dd, *J* = 8.2, 1.6 Hz, 2H), 3.64 (dd, *J* = 5.8, 4.6 Hz, 2H), 3.47-3.35 (m, 4H), 2.96-2.81 (m, 1H), 2.62 (d, *J =* 7.1 Hz, 2H), 1.84-1.69 (m, 1H), 0.93 (d, *J =* 6.7 Hz, 6H); LC/MS ESI (+): 415.2 (M+1).

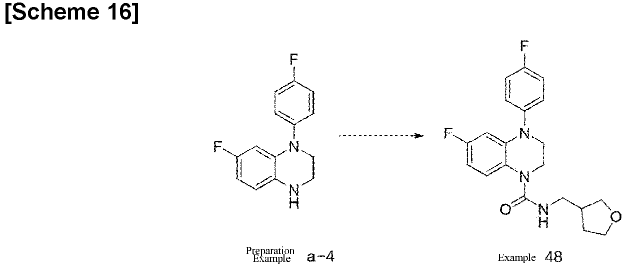

### <Example 48> Synthesis of 6-fluoro-4-(4-fluorophenyl)-N-((tetrahydrofuran-3-yl)methyl)-3,4-dihydroquinoxaline-1(2H)-carboxamide

The title compound was synthesized in the same manner as Preparation Example a-5, using (tetrahydrofuran-3-yl)methanamine in place of (*R*)-(+)-1-Boc-3-aminopyrrolidine to give 6-fluoro-4-(4-fluorophenyl)-*N*-((tetrahydrofuran-3-yl)methyl)-3,4-dihydroquinoxaline-1(2H)-carboxamide.

White solid (yield: 57%); ¹H NMR (400 MHz, CDCl₃) δ 7.26-7.17 (m, 2H), 7.16-7.05 (m, 3H), 6.41 (ddd, *J* = 8.7, 7.7, 2.8 Hz, 1H), 6.26 (dd, *J* = 11.4, 2.8 Hz, 1H), 5.46-5.33 (m, 1H), 3.96-3.90 (m, 2H), 3.89-3.69 (m, 3H), 3.64-3.58 (m, 2H), 3.57-3.50 (m, 1H), 3.39-3.21 (m, 2H), 2.59-2.44 (m, 1H), 2.10-1.97 (m, 1H), 1.70-1.58 (m, 1H); LC/MS ESI (+): 374.2 (M+1).

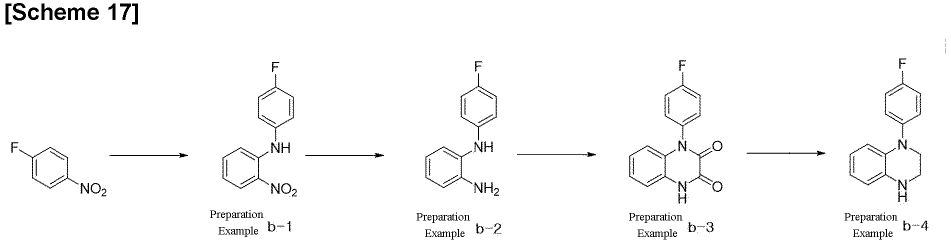

### <Preparation Process b> Preparation of 1-(4-fluorophenyl)-1,2,3,4-tetrahydroquinoxaline

1-(4-Fluorophenyl)-1,2,3,4-tetrahydroquinoxaline according to the present invention was prepared through Preparation Examples b-1 to b-4 below.

### <Preparation Example b-1> Preparation of N-(4-fluorophenyl)-2-nitroaniline

The title compound was prepared in the same manner as Preparation Example a-1, using 1-fluoro-2-nitrobenzene in place of 2,4-difluoro-1-nitrobenzene to give N-(4-fluorophenyl)-2-nitroaniline.

Yellow solid (yield: 30%); ¹H NMR (400 MHz, CDCl₃) δ 9.40 (s, 1H), 8.21 (dd, *J* = 8.6, 1.6 Hz, 1H), 7.38-7.34 (m, 1H), 7.27-7.22 (m, 2H), 7.15-7.09 (m, 2H), 7.05 (dd, *J* = 8.6, 1.2 Hz, 1H), 6.77 (ddd, *J* = 8.4, 6.8, 1.2 Hz, 1H).

### <Preparation Example b-2> Preparation of N¹-(4-fluorophenyl)benzene-1,2-diamine

The title compound was s prepared in the same manner as Preparation Example a-2, using **Preparation Example b-1** in place of Preparation Example a-1 to give *N¹*-(4-fluorophenyl)benzene-1,2-diamine.

Orange solid (yield: 61%); ¹H NMR (400 MHz, CDCl₃) δ 7.06 (dd, *J* = 7.8, 1.2 Hz, 1H), 7.01 (dd, *J* = 7.6, 1.2 Hz, 1H), 6.95-6.89 (m, 2H), 6.81 (dd, *J* = 7.8, 1.2 Hz, 1H), 6.79-6.73 (m. 1H), 6.73-6.67 (m, 2H), 5.09 (s, 1H), 3.74 (s, 2H); LC/MS ESI (+): 202.6 (M+1).

### <Preparation Example b-3> Preparation of 1-(4-fluorophenyl)quinoxalin-2,3(1H,4H)-dione

The title compound was prepared in the same manner as Preparation Example a-3, using **Preparation Example b-2** in place of Preparation Example a-2 to give 1-(4-fluorophenyl)quinoxalin-2,3(*1H,4H*)-dione.

White solid (yield: 81%); ¹H NMR (400 MHz, DMSO-*d₆*) δ 12.11 (s, 1H), 7.45 (d, *J* = 6.7 Hz, 4H), 7.24-7.19 (m, 1H), 7.16-7.10 (m, 1H), 7.00-6.96 (m, 1H), 6.32 (dd, *J* = 8.2, 0.8 Hz, 1H); LC/MS ESI (+): 256.6 (M+1).

### <Preparation Example b-4> Preparation of 1-(4-fluorophenyl)-1,2,3,4-tetrahydroquinoxaline

The title compound was prepared in the same manner as Preparation Example a-4, using **Preparation Example b-3** in place of Preparation Example a-3 to give 1-(4-fluorophenyl)-1,2,3,4-tetrahydroquinoxaline.

Brown solid (yield: 38%); ¹H NMR (400 MHz, CDCl₃) δ 7.18-7.13 (m, 2H), 7.05-6.99 (m, 2H), 6.70-6.53 (m, 4H), 3.66-3.64 (m, 2H), 3.48-3.45 (m, 2H); LC/MS ESI (+): 228.8 (M+1).

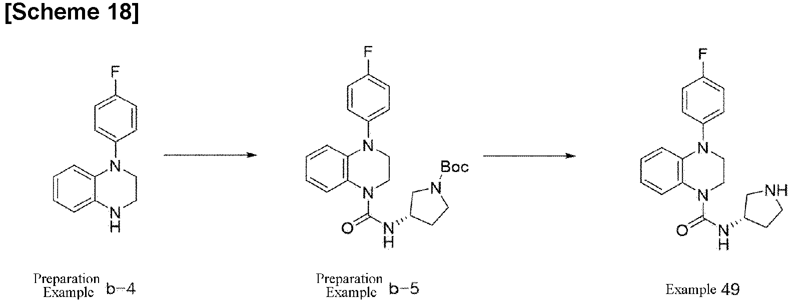

### <Preparation Example b-5> Preparation of tert-butyl (S)-3-(4-(4-fluorophenyl)-1,2,3,4-tetrahydroquinoxaline-1-carboxamido)pyrrolidine-1-carboxylate

To a solution of **Preparation Example b-4** (200 mg, 0.88 mmol), TEA (0.5 mL, 3.50 mmol) and DCM (8.0 mL) cooled to 0 °C under a nitrogen atmosphere was added dropwise triphosgene (156 mg, 0.53 mmol). The reaction mixture was stirred for 1 h then TEA (0.5 mL, 3.5 mmol) and (S)-(-)-1-Boc-3-aminopyrrolidine (0.2 mL, 1.31 mmol) were added slowly. The mixture warmed to room temperature and stirred for 3 h. The resulting mixture was partitioned between DCM and water. The organic layer was washed with brine, dried with MgSO₄, filtered and then concentrated under reduced pressure. The residue was purified by silica column chromatography (with *n*-hexane/EtOAC) to give *tert-butyl* (*S*)-3-(4-(4-fluorophenyl)-1,2,3,4-tetrahydroquinoxaline-1-carboxamido)pyrrolidine-1-carboxylate.

Yellow oil (yield: 98%); ¹H NMR (400 MHz, CDCl₃) δ 7.25-7.19 (m, 2H), 7.15-7.07 (m, 3H), 6.94-6.86 (m, 1H), 6.74-6.68 (m, 1H), 6.65-6.59 (m, 1H), 5.38 (d, *J* = 6.9 Hz, 1H), 4.45-4.40 (m, 1H), 3.99-3.90 (m, 2H), 3.71-3.61 (m, 3H), 3.44-3.39 (m, 2H), 3.21-3.15 (m, 1H), 2.19-2.15 (m, 1H), 1.83-1.79 (m, 1H), 1.46 (s, 9H).

### <Example 49> Synthesis of (S)-4-(4-fluorophenyl)-N-(pyrrolidin-3-yl)-3,4-dihydroquinoxaline-1(2H)-carboxamide

Following the procedure as described for Example 1, reaction of Preparation **Example b-5** (1.0 equiv.) in DCM (0.5 M) with TFA (10.0 equiv.) gave (*S*)-4-(4-fluorophenyl)-*N*-(pyrrolidin-3-yl)-3,4-dihydroquinoxaline-1(*2H*)-carboxamide.

White solid (yield: 31%); ¹H NMR (400 MHz, CDCl₃) δ 8.12 (s, 1H), 7.27-7.18 (m, 3H), 7.12-7.03 (m, 2H), 6.91-6.83 (m, 1H), 6.76-6.68 (m, 1H), 6.62 (d, *J* = 8.1 Hz, 1H), 6.30 (d, *J = 7.2* Hz, 1H), 4.62-4.57 (m, 1H), 3.93 (t, *J* = 5.0 Hz, 2H), 3.64 (t, *J =* 5.2 Hz, 2H), 3.36-3.22 (m, 2H), 3.21-3.09 (m, 2H), 2.38-2.24 (m, 1H), 2.00-1.95 (m, 1H); LC/MS ESI (+): 341.0 (M+1).

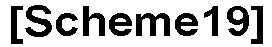

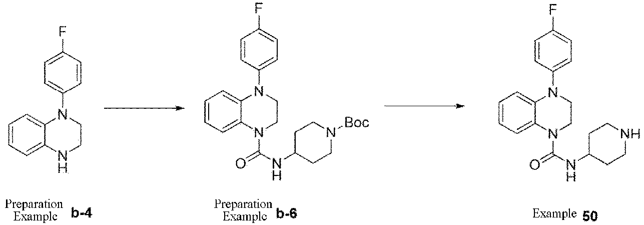

### <Preparation Example b-6> Preparation of tert-butyl 4-(4-(4-fluorophenyl)-1,2,3,4-tetrahydroquinoxaline-1-carboxamido)piperidine-1-carboxylate

The title compound was prepared in the same manner as Preparation Example b-5, using 1-Boc-4-aminopiperidine in place of (*S*)-(-)-1-Boc-3-aminopyrrolidine.to give *tert*-butyl 4-(4-(4-fluorophenyl)-1,2,3,4-tetrahydroquinoxaline-1-carboxamido)piperidine-1-carboxylate.

White solid (yield: 85%); ¹H NMR (400 MHz, CDCl₃) δ 7.24-7.19 (m, 2H), 7.16-7.07 (m, 3H), 6.93-6.86 (m, 1H), 6.73-6.69 (m, 1H), 6.62 (d, *J* = 8.2 Hz, 1H), 5.23 (d, *J* = 7.4 Hz, 1H), 4.09-3.82 (m, 5H), 3.63 (t, *J* = 5.1 Hz, 1H), 2.97-2.85 (m, 2H), 2.00-1.90 (m, 2H), 1.45 (s, 9H), 1.37-1.24 (m, 2H).

### <Example 50> Synthesis of 4-(4-fluorophenyl)-N-(piperidin-4-yl)-3,4-dihydroquinoxaline-1(2H)-carboxamide

Following the procedure as described for Example 1, reaction of **Preparation Example b-6** (1.0 equiv.) in DCM (0.5 M) with TFA (10.0 equiv.) gave 4-(4-fluorophenyl)-*N*-(piperidin-4-yl)-3,4-dihydroquinoxaline-1(*2H*)-carboxamide.

White solid (yield: 49%); ¹H NMR (400 MHz, CDCl₃) δ 7.24-7.15 (m, 3H), 7.12-7.06 (m, 2H), 6.92-6.86 (m, 1H), 6.74-6.69 (m, 1H), 6.63 (d, *J = 8.2* Hz, 1H), 5.25 (d, *J* = 7.8 Hz, 1H), 3.94 (t, *J =* 5.3 Hz, 1H), 3.87-3.79 (m, 1H), 3.63 (t, *J* = 5.1 Hz, 1H), 3.08-2.99 (m, 2H), 2.75-2.65 (m, 2H), 2.02-1.93 (m, 2H), 1.36-1.23 (m, 2H); LC/MS ESI (+): 355.1 (M+1).

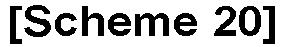

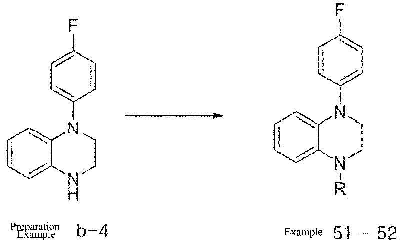

### <Example 51> Synthesis of (S)-4-(4-fluorophenyl)-N-(tetrahydrofuran-3-yl)-3,4-dihydroquinoxaline-1(2H)-carboxamide

The title compound was synthesized in the same manner as Preparation Example b-5, using (*S*)-3-aminotetrahydrofuran in place of (*S*)-(-)-1-Boc-3-aminopyrrolidine.to give (*S*)-4-(4-fluorophenyl)-*N*-(tetrahydrofuran-3-yl)-3,4-dihydroquinoxaline-1(*2H*)-carboxamide.

Brown solid (yield: 67%); ¹H NMR (400 MHz, CDCl₃) δ 7.25-7.20 (m, 2H), 7.16 (dd, *J* = 7.9, 1.5 Hz, 1H), 7.13-7.07 (m, 2H), 6.90 (ddd, *J* = 8.4, 7.2, 1.5 Hz, 1H), 6.75-6.70 (m, 1H), 6.62 (dd, *J* = 8.3, 1.3 Hz, 1H), 5.48 (d, *J =* 6.9 Hz, 1H), 4.52-4.46 (m, 1H), 4.01-3.85 (m, 4H), 3.81-3.76 (m, 1H), 3.70-3.66 (m, 1H), 3.64 (t, *J* = 5.2 Hz, 1H), 2.32-2.24 (m, 1H), 1.82-1.77 (m, 1H); LC/MS ESI (+): 342.2 (M+1).

### <Example 52> Synthesis of 4-(4-fluorophenyl)-N-((tetrahydro-2H-pyran-4-yl)methyl)-3,4-dihydroquinoxaline-1(2H)-carboxamide

The title compound was synthesized in the same manner as Preparation Example b-5, using 4-aminomethyltetrahydropyran in place of (*S*)-(-)-1-Boc-3-aminopyrrolidine.to give 4-(4-fluorophenyl)-*N*-((tetrahydro-*2H*-pyran-4-yl)methyl)-3,4-dihydroquinoxaline-1(*2H*)-carboxamide

Light yellow solid (yield: 74%); ¹H NMR (400 MHz, CDCl₃) δ 7.25-7.21 (m, 3H), 7.15-7.09 (m, 2H), 6.95-6.91 (m, 1H), 6.79-6.74 (m, 1H), 6.66 (dd, *J* = 8.3, 1.3 Hz, 1H), 5.48 (t, *J* = 5.8 Hz, 1H), 4.03-3.94 (m, 4H), 3.67-3.64 (m, 2H), 3.44-3.37 (m, 2H), 3.21 (t, *J =* 6.4 Hz, 1H), 1.87-1.76 (m, 1H), 1.65-1.62 (m, 2H), 1.40-1.29 (m, 2H); LC/MS ESI (+): 370.2 (M+1).

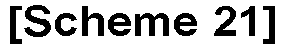

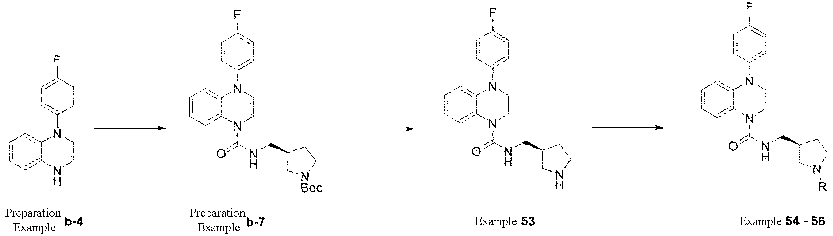

### <Preparation Example b-7> Preparation of tert-butyl (R)-3-((4-(4-fluorophenyl)-1,2,3,4-tetrahydroquinoxaline-1-carboxamido)methyl)pyrrolidine-1-carboxylate

The title compound was synthesized in the same manner as Preparation Example b-5, using (*R*)-1-Boc-3-(aminomethyl)pyrrolidine in place of (*S*)-(-)-1-Boc-3-aminopyrrolidine.to give *tert-butyl* (*R*)-3-((4-(4-fluorophenyl)-1,2,3,4-tetrahydroquinoxaline-1-carboxamido)methyl)pyrrolidine-1-carboxylate.

White solid (yield: 91%); ¹H NMR (400 MHz, CDCl₃) δ 7.25-7.12 (m, 3H), 7.11-7.03 (m, 2H), 6.95-6.85 (m, 1H), 6.77-6.69 (m, 1H), 6.65-6.58 (m, 1H), 5.46 (t, *J =* 5.9 Hz, 1H), 4.01-3.86 (m, 2H), 3.62 (t, *J* = 5.2 Hz, 2H), 3.56-3.34 (m, 3H), 3.33-3.25 (m, 2H), 3.08-2.94 (m, 1H), 2.51-2.37 (m, 1H), 2.02-1.91 (m, 1H), 1.67-1.54 (m, 1H), 1.45 (s, 9H).

### <Example 53> Synthesis of (S)-4-(4-fluorophenyl)-N-(pyrrolidin-3-ylmethyl)-3,4-dihydroquinoxaline-1(2H)-carboxamide

Following the procedure as described for Example 1, reaction of **Preparation Example b-7** (1.0 equiv.) in DCM (0.5 M) with TFA (10.0 equiv.) gave (S)-4-(4-fluorophenyl)-*N*-(pyrrolidin-3-ylmethyl)-3,4-dihydroquinoxaline-1(*2H*)-carboxamide.

White solid (yield: 56.4%); ¹H NMR (400 MHz, CDCl₃) δ 7.24-7.18 (m, 2H), 7.17 (dd, *J* = 7.9, 1.6 Hz, 1H), 7.13-7.05 (m, 2H), 6.94-6.86 (m, 1H), 6.77-6.69 (m, 1H), 6.62 (dd, *J =* 8.3, 1.4 Hz, 1H), 5.65 (t, *J =* 5.7 Hz, 1H), 3.93 (t, *J* = 5.2 Hz, 2H), 3.63 (t, *J* = 5.1 Hz, 2H), 3.53-3.34 (m, 1H), 3.33-3.22 (m, 2H), 3.21-3.12 (m, 2H), 3.11-3.01 (m, 1H), 2.89-2.79 (m, 1H), 2.53-2.41 (m, 1H), 2.05-1.95 (m, 1H), 1.65-1.52 (m, 1H); LC/MS ESI (+): 355.2 (M+1).

### <Example 54> Synthesis of (R)-4-(4-fluorophenyl)-N-((1-methylpyrrolidin-3-yl)methyl)-3,4-dihydroquinoxaline-1(2H)-carboxamide

Following the procedure as described for Example 2, reaction of Example 53 (1.0 equiv.) in DCM (0.3 M) with sodium triacetoxyborohydride (1.5 equiv.) and formaldehyde (2.0 equiv.) gave (R)-4-(4-fluorophenyl)-N-((1-methylpyrrolidin-3-yl)methyl)-3,4-dihydroquinoxaline-1(*2H*)-carboxamide.

Light yellow oil (yield: 76.9%); ¹H NMR (400 MHz, CDCl₃) δ 7.24-7.03 (m, 5H), 6.93-6.83 (m, 1H), 6.76-6.68 (m, 1H), 6.62 (dd, *J* = 8.3, 1.4 Hz, 1H), 4.05-3.94 (m, 1H), 3.93-3.82 (m, 1H), 3.63 (t, *J* = 5.2 Hz, 2H), 3.39-3.19 (m, 2H), 2.75-2.64 (m, 1H), 2.63-2.54 (m, 1H), 2.53-2.39 (m, 3H), 2.31 (s, 3H), 2.10-1.96 (m, 1H), 1.64-1.51 (m, 1H); LC/MS ESI (+): 369.2 (M+1).

### <Example 55> Synthesis of (R)-4-(4-fluorophenyl)-N-((1-isopropylpyrrolidin-3-yl)methyl)-3,4-dihydroquinoxaline-1(2H)-carboxamide

The title compound was synthesized in the same manner as Example 54, using acetone in place of formaldehyde to give (*R*)-4-(4-fluorophenyl)-*N*-((1-isopropylpyrrolidin-3-yl)methyl)-3,4-dihydroquinoxaline-1(*2H*)-carboxamide.

Light yellow oil (yield: 56.9%); ¹H NMR (400 MHz, CDCl₃) δ 7.25-7.14 (m, 3H), 7.12-7.01 (m, 2H), 6.92-6.82 (m, 1H), 6.76-6.69 (m, 1H), 6.63 (dd, *J* = 8.3, 1.4 Hz, 1H), 5.95 (t, *J* = 5.1 Hz, 1H), 4.09-4.00 (m, 1H), 3.83 (ddd, *J* = 13.1, 6.2, 4.6 Hz, 1H), 3.65-3.61 (m, 2H), 3.36-3.21 (m, 2H), 2.69-2.58 (m, 2H), 2.51 (q, *J* = 6.6 Hz, 1H), 2.44-2.32 (m, 2H), 2.30-2.23 (m, 1H), 2.03-1.90 (m, 1H), 1.60-1.43 (m, 1H), 0.99 (dd, *J* = 9.8, 6.3 Hz, 6H); LC/MS ESI (+): 397.2 (M+1).

### <Example 56> Synthesis of (R)-4-(4-fluorophenyl)-N-((1-isobutylpyrrolidin-3-yl)methyl)-3,4-dihydroquinoxaline-1(2H)-carboxamide

The title compound was synthesized in the same manner as Example 54, using isobutyraldehyde in place of formaldehyde to give (*R*)-4-(4-fluorophenyl)-*N*-((1-isobutylpyrrolidin-3-yl)methyl)-3,4-dihydroquinoxaline-1(*2H*)-carboxamide.

Light yellow oil (yield: 91.7%); ¹H NMR (400 MHz, CDCl₃) δ 7.27-7.04 (m, 5H), 6.95-6.83 (m, 1H), 6.78-6.67 (m, 1H), 6.63 (dd, *J* = 8.3, 1.4 Hz, 1H), 6.10 (s, 1H), 4.24-4.12 (m, 1H), 3.83-3.69 (m, 1H), 3.68-3.59 (m, 2H), 3.42-3.22 (m, 2H), 2.83-2.62 (m, 1H), 2.61-2.30 (m, 5H), 2.23-2.10 (m, 1H), 2.09-1.93 (m, 1H), 1.76-1.50 (m, 2H), 0.94-0.67 (m, 6H); LC/MS ESI (+): 411.2 (M+1).

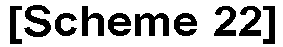

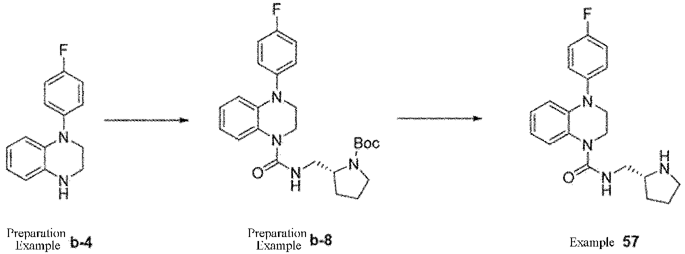

### <Preparation Example b-8> Preparation of tert-butyl (R)-2-((4-(4-fluorophenyl)-1,2,3,4-tetrahydroquinoxaline-1-carboxamido)methyl)pyrrolidine-1-carboxylate

The title compound was prepared in the same manner as Preparation Example b-5, using (*R*)-1-Boc-2-(aminomethyl)pyrrolidine in place of (*S*)-(-)-1-Boc-3-aminopyrrolidine.to give *tert-butyl* (*R*)-2-((4-(4-fluorophenyl)-1,2,3,4-tetrahydroquinoxaline-1-carboxamido)methyl)pyrrolidine-1-carboxylate.

Light yellow solid (yield: 93%); ¹H NMR (400 MHz, CDCl₃) δ 7.35-7.26 (m, 1H), 7.23-7.18 (m, 2H), 7.13-7.00 (m, 2H), 6.94-6.83 (m, 1H), 6.78-6.68 (m, 1H), 6.62 (d, *J =* 8.1 Hz, 1H), 6.40 (brs, 1H), 4.04-3.83 (m, 3H), 3.65-3.62 (m, 2H), 3.55-3.41 (m, 1H), 3.40-3.20 (m, 3H), 2.00-1.75 (m, 4H), 1.40 (s, 9H).

### <Example 57> Synthesis of (R)-4-(4-fluorophenyl)-N-(pyrrolidin-2-ylmethyl)-3,4-dihydroquinoxaline-1(2H)-carboxamide

Following the procedure as described for Example 1, reaction of **Preparation Example b-8** (1.0 equiv.) in DCM (0.5 M) with TFA (10.0 equiv.) gave (*R*)-4-(4-fluorophenyl)-*N*-(pyrrolidin-2-ylmethyl)-3,4-dihydroquinoxaline-1(2*H*)-carboxamide.

Yellow oil (yield: 65%); ¹H NMR (400 MHz, CDCl₃) δ 7.24-7.19 (m, 3H), 7.12-7.06 (m, 2H), 6.91-6.87 (m, 1H), 6.75-6.70 (m, 1H), 6.62 (dd, *J* = 8.3, 1.3 Hz, 1H), 5.86-5.78 (m, 1H), 3.98-3.92 (m, 2H), 3.67-3.61 (m, 2H), 3.42-3.32 (m, 2H), 3.19-3.12 (m, 1H), 2.97-2.86 (m, 2H), 2.17 (brs, 1H), 1.94-1.85 (m, 1H), 1.82-1.68 (m, 2H), 1.46-1.40 (m, 1H); LC/MS ESI (+): 355.2 (M+1).

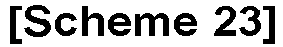

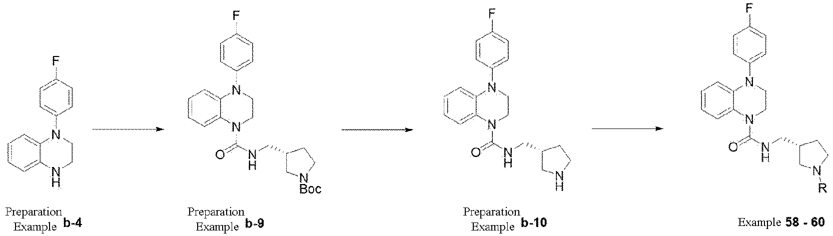

### <Preparation Example b-9> Preparation of tert-butyl (R)-3-((4-(4-fluorophenyl)-1,2,3,4-tetrahydroquinoxaline-1-carboxamido)methyl)pyrrolidine-1-carboxylate

The title compound was prepared in the same manner as Preparation Example b-5, using (*R*)-1-Boc-3-(aminomethyl)pyrrolidine in place of (*S*)-(-)-1-Boc-3-aminopyrrolidine.to give *tert*-butyl (*R*)-3-((4-(4-fluorophenyl)-1,2,3,4-tetrahydroquinoxaline-1-carboxamido)methyl)pyrrolidine-1-carboxylate.

White solid (yield: 70%); ¹H NMR (400 MHz, CDCl₃) δ 7.24-7.20 (m, 2H), 7.17-7.07 (m, 3H), 6.95-6.87 (m, 1H), 6.76-6.71 (m, 1H), 6.66-6.60 (m, 1H), 5.50-5.43 (m, 1H), 4.01-3.88 (m, 2H), 3.63 (t, *J* = 5.1 Hz, 2H), 3.55-3.16 (m, 5H), 3.06-2.97 (m, 1H), 2.50-2.38 (m, 1H), 2.03-1.93 (m, 1H), 1.67-1.58 (m, 1H), 1.46 (s, 9H).

### <Preparation Example b-10> Preparation of (R)-4-(4-fluorophenyl)-N-(pyrrolidin-3-ylmethyl)-3,4-dihydroquinoxaline-1(2H)-carboxamide

Following the procedure as described for Example 1, reaction of **Preparation Example b-9** (1.0 equiv.) in DCM (0.5 M) with TFA (10.0 equiv.) gave (*R*)-4-(4-fluorophenyl)-*N*-(pyrrolidin-3-ylmethyl)-3,4-dihydroquinoxaline-1(2H)-carboxamide.

White solid (yield: 70%); ¹H NMR (400 MHz, CDCl₃) δ 7.22-7.17 (m, 3H), 7.12-7.06 (m, 2H), 6.91-6.87 (m, 1H), 6.75-6.70 (m, 1H), 6.62 (dd, *J* = 8.3, 1.3 Hz, 1H), 5.69 (t, *J* = 5.4 Hz, 1H), 3.92 (t, *J* = 5.1 Hz, 2H), 3.62 (t, *J* = 5.1 Hz, 2H), 3.31-3.24 (m, 2H), 3.15-3.09 (m, 2H), 3.05-2.98 (m, 1H), 2.81-2.77 (m, 1H), 2.45-2.36 (m, 1H), 2.01-1.91 (m, 1H), 1.57-1.48 (m, 1H); LC/MS ESI (+): 355.2 (M+1).

### <Example 58> Synthesis of (S)-4-(4-fluorophenyl)-N-((1-methylpyrrolidin-3-yl)methyl)-3,4-dihydroquinoxaline-1(2H)-carboxamide

Following the procedure as described for Example 2, reaction of **Preparation Example b-10** (1.0 equiv.) in DCM (0.3 M) with sodium triacetoxyborohydride (1.5 equiv.) and formaldehyde (2.0 equiv.) gave (*S*)-4-(4-fluorophenyl)-*N*-((1-methylpyrrolidin-3-yl)methyl)-3,4-dihydroquinoxaline-1(*2H*)-carboxamide.

Yellow oil (yield: 67%); ¹H NMR (400 MHz, CDCl₃) δ 7.23-7.17 (m, 3H), 7.13-7.07 (m, 2H), 6.91-6.87 (m, 1H), 6.74-6.70 (m, 1H), 6.63 (dd, *J* = 8.3, 1.3 Hz, 1H), 6.10-6.00 (m, 1H), 4.03-3.97 (m, 1H), 3.91-3.85 (m, 1H), 3.63 (t, *J* = 5.1 Hz, 2H), 3.35-3.23 (m, 2H), 2.65-2.59 (m, 1H), 2.55-2.50 (m, 1H), 2.48-2.34 (m, 3H), 2.25 (s, 3H), 2.05-1.96 (m, 1H), 1.58-1.51 (m, 1H); LC/MS ESI (+): 369.3 (M+1).

### <Example 59> Synthesis of (S)-4-(4-fluorophenyl)-N-((1-isopropylpyrrolidin-3-yl)methyl)-3,4-dihydroquinoxaline-1 (2H)-carboxamide

The title compound was synthesized in the same manner as Example 58, using acetone in place of formaldehyde to give (*S*)-4-(4-fluorophenyl)-*N*-((1-isopropylpyrrolidin-3-yl)methyl)-3,4-dihydroquinoxaline-1(2*H*)-carboxamide.

Colorless oil (yield: 71%); ¹H NMR (400 MHz, CDCl₃) δ 7.23-7.17 (m, 3H), 7.12-7.05 (m, 2H), 6.90-6.85 (m, 1H), 6.74-6.70 (m, 1H), 6.63 (dd, *J* = 8.3, 1.3 Hz, 1H), 5.99-5.92 (m, 1H), 4.08-4.02 (m, 1H), 3.86-3.80 (m, 1H), 3.65-3.62 (m, 2H), 3.36-3.24 (m, 2H), 2.67-2.61 (m, 2H), 2.54-2.48 (m, 1H), 2.45-2.34 (m, 2H), 2.30-2.24 (m, 1H), 2.02-1.94 (m, 1H), 1.58-1.49 (m, 1H), 0.99 (dd, *J* = 9.8, 6.4 Hz, 6H); LC/MS ESI (+): 397.3 **(M+1).**

### <Example 60> Synthesis of (S)-4-(4-fluorophenyl)-N-((1-isobutylpyrrolidin-3-yl)methyl)-3,4-dihydroquinoxaline-1(2H)-carboxamide

The title compound was synthesized in the same manner as Example 58, using isobutyraldehyde in place of formaldehyde to give (*S*)-4-(4-fluorophenyl)-*N*-((1-isobutylpyrrolidin-3-yl)methyl)-3,4-dihydroquinoxaline-1(2*H*)-carboxamide.

Colorless oil (yield: 69%); ¹H NMR (400 MHz, CDCl₃) δ 7.23-7.17 (m, 3H), 7.12-7.05 (m, 2H), 6.90-6.85 (m, 1H), 6.74-6.69 (m, 1H), 6.61 (dd, *J* = 8.3, 1.3 Hz, 1H), 6.10 (brs, 1H), 4.21-4.14 (m, 1H), 3.76-3.68 (m, 1H), 3.65-3.61 (m, 2H), 3.36-3.24 (m, 2H), 2.69-2.64 (m, 1H), 2.50-2.29 (m, 4H), 2.17-1.93 (m, 3H), 1.70-1.51 (m, 3H), 0.81 (d, *J* = 6.6 Hz, 3H), 0.73 (d, *J* = 6.6 Hz, 3H); LC/MS ESI (+): 411.3 (M+1).

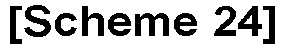

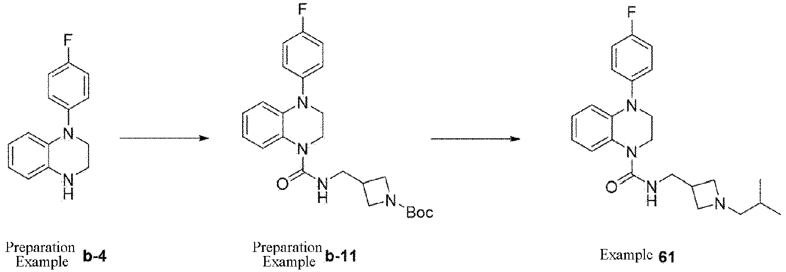

### <Preparation Example b-11> Preparation of tert-butyl 3-((4-(4-fluorophenyl)-1,2,3,4-tetrahydroquinoxaline-1-carboxamido)methyl)azetidine-1-carboxylate

The title compound was prepared in the same manner as Preparation Example b-5, using 1-Boc-3-(aminomethyl)azetidine in place of (*S*)-(-)-1-Boc-3-aminopyrrolidine.to give tert-butyl 3-((4-(4-fluorophenyl)-1,2,3,4-tetrahydroquinoxaline-1-carboxamido)methyl)azetidine-1-carboxylate.

Light yellow solid (yield: 92%); ¹HNMR (400 MHz, CDCl₃) δ 7.26-7.20 (m, 2H), 7.17-7.09 (m, 3H), 6.95-6.90 (m, 1H), 6.77-6.72 (m, 1H), 6.64 (dd, *J* = 8.3, 1.3 Hz, 1H), 5.58-5.54 (m, 1H), 4.04-4.00 (m, 2H), 3.98-3.95 (m, 2H), 3.69-3.62 (m, 4H), 3.54-3.45 (m, 2H), 2.84-2.74 (m, 1H), 1.46 (s, 9H).

### <Example 61> Synthesis of 4-(4-fluorophenyl)-N-((1-isobutylazetidin-3-yl)methyl)-3,4-dihydroquinoxaline-1(2H)-carboxamide

To a solution of **Preparation Example -11** (1.0 equiv.) in DCM (0.3 M) was added TFA (10.0 equiv.). The reaction mixture was stirred at room temperature for 24 h. The reaction mixture was diluted with DCM, and the pH was adjusted to 9 with 10M NaOH (aq.). The mixture was extracted with DCM, the organic layer dried with MgSO₄, filtered, and then concentrated under reduced pressure. A solution of the obtained concentrate, sodium triacetoxyborohydride (1.5 equiv.) and isobutyraldehyde (2.0 equiv.) in DCM (0.3 M) was stirred at room temperature for 1 h. The reaction mixture was quenched with water and extracted with DCM. The organic layer was washed with brine, dried with MgSO₄, filtered, and then concentrated under reduced pressure. The residue was purified by silica column chromatography (with DCM/MeOH) to give 4-(4-fluorophenyl)-*N*-((1-isobutylazetidin-3-yl)methyl)-3,4-dihydroquinoxaline-1(2*H*)-carboxamide.

Colorless oil (yield: 19%); ¹H NMR (400 MHz, CDCl₃) δ 7.24-7.18 (m, 3H), 7.12-7.05 (m, 2H), 6.90-6.86 (m, 1H), 6.73-6.69 (m, 1H), 6.62 (dd, *J* = 8.3, 1.3 Hz, 1H), 5.85 (t, *J* = 5.2 Hz, 1H), 3.69-3.65 (m, 2H), 3.64-3.62 (m, 2H), 3.44 (t, *J* = 5.7 Hz, 2H), 3.31-3.27 (m, 2H), 2.91-2.88 (m, 2H), 2.69-2.59 (m, 1H), 2.17 (d, *J =* 7.1 Hz, 2H), 1.58-1.48 (m, 1H), 0.81 (d, *J* = 6.6 Hz, 6H); LC/MS ESI (+): 396.5 (M+1).

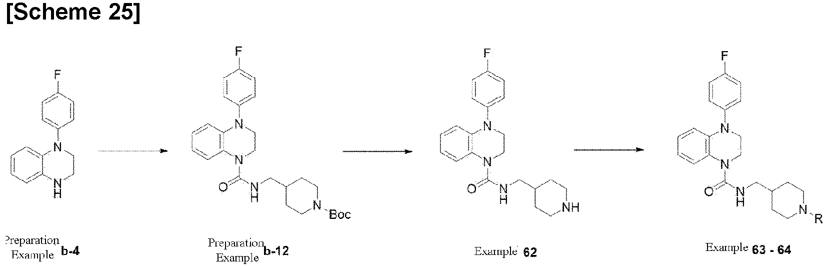

### <Preparation Example b-12> Preparation of tert-butyl 4-((4-(4-fluorophenyl)-1,2,3,4-tetrahydroquinoxaline-1-carboxamido)methyl)piperidine-1-carboxylate

The title compound was synthesized in the same manner as Preparation Example b-5, using 1-Boc-4-(aminomethyl)piperidine in place of (*S*)-(-)-1-Boc-3-aminopyrrolidine.to give *tert-butyl* 4-((4-(4-fluorophenyl)-1,2,3,4-tetrahydroquinoxaline-1-carboxamido)methyl)piperidine-1-carboxylate.

White solid (yield: 67%); ¹H NMR (400 MHz, CDCl₃) δ 7.22-7.16 (m, 3H), 7.12-7.07 (m, 2H), 6.95-6.87 (m, 1H), 6.77-6.70 (m, 1H), 6.65-6.61 (m, 1H), 5.47-5.42 (m, 1H), 4.18-4.06 (m, 2H), 3.94 (t, *J* = 4.4 Hz, 2H), 3.63 (t, *J =* 5.1 Hz, 2H), 3.17 (t, *J =* 5.4 Hz, 2H), 2.76-2.62 (m, 2H), 1.72-1.65 (m, 3H), 1.45 (s, 9H), 1.18-1.08 (m, 2H).

### <Example 62> Synthesis of 4-(4-fluorophenyl)-N-(piperidin-4-ylmethyl)-3,4-dihydroquinoxaline-1(2H)-carboxamide

Following the procedure as described for Example 1, reaction of **Preparation Example b-12** (1.0 equiv.) in DCM (0.5 M) with TFA (10.0 equiv.) gave 4-(4-fluorophenyl)-*N*-(piperidin-4-ylmethyl)-3,4-dihydroquinoxaline-1(2*H*)-carboxamide.

White solid (yield: 96%); ¹H NMR (400 MHz, CDCl₃) δ 7.23-7.17 (m, 3H), 7.12-7.07 (m, 2H), 6.93-6.88 (m, 1H), 6.76-6.72 (m, 1H), 6.66 (dd, *J* = 8.3, 1.3 Hz, 1H), 5.45 (t, *J* = 5.9 Hz, 2H), 3.96-3.93 (m, 2H), 3.64-3.62 (m, 2H), 3.18-3.09 (m, 4H), 2.65-2.58 (m, 2H), 2.15-1.98 (m, 2H), 1.74-1.62 (m, 3H), 1.24-1.11 (m, 2H); LC/MS ESI (+): 369.2 (M+1).

### <Example 63> Synthesis of 4-(4-fluorophenyl)-N-((1-isopropylpiperidin-4-yl)methyl)-3,4-dihydroquinoxaline-1(2H)-carboxamide

Following the procedure as described for Example 2, reaction of **Example 62** (1.0 equiv.) in DCM (0.3 M) with sodium triacetoxyborohydride (1.5 equiv.) and acetone (2.0 equiv.) gave 4-(4-fluorophenyl)-*N*-((1-isopropylpiperidin-4-yl)methyl)-3,4-dihydroquinoxaline-1(2*H*)-carboxamide.

Light yellow solid (yield: 81%); ¹H NMR (400 MHz, CDCl₃) δ 7.23-7.17 (m, 3H), 7.12-7.07 (m, 2H), 6.92-6.88 (m, 1H), 6.75-6.71 (m, 1H), 6.63 (dd, *J* = 8.3, 1.3 Hz, 1H), 5.44 (t, *J* = 5.7 Hz, 1H), 3.96-3.93 (m, 2H), 3.64-3.62 (m, 2H), 3.17 (t, *J* = 6.3 Hz, 2H), 2.92-2.85 (m, 2H), 2.75-2.65 (m, 1H), 2.16-2.06 (m, 2H), 2.05-1.67 (m, 2H), 1.58-1.47 (m, 1H), 1.32-1.20 (m, 2H), 1.03 (d, *J =* 6.6 Hz, 6H); LC/MS ESI (+): 411.3 (M+1).

### <Example 64> Synthesis of 4-(4-fluorophenyl)-N-((1-isobutylpiperidin-4-yl)methyl)-3,4-dihydroquinoxaline-1(2H)-carboxamide

The title compound was synthesized in the same manner as Example 63, using isobutyraldehyde in place of acetone to give 4-(4-fluorophenyl)-*N*-((1-isobutylpiperidin-4-yl)methyl)-3,4-dihydroquinoxaline-1(2*H*)-carboxamide.

Light yellow solid (yield: 69%); ¹H NMR (400 MHz, CDCl₃) δ 7.23-7.17 (m, 3H), 7.12-7.07 (m, 2H), 6.92-6.88 (m, 1H), 6.75-6.71 (m, 1H), 6.63 (dd, *J* = 8.3, 1.3 Hz, 1H), 5.43 (t, *J* = 5.8 Hz, 1H), 3.96-3.93 (m, 2H), 3.64-3.62 (m, 2H), 3.17 (t, *J* = 6.3 Hz, 2H), 2.89-2.83 (m, 2H), 2.05 (d, *J =* 7.4 Hz, 2H), 1.88-1.81 (m, 2H), 1.80-1.74 (m, 1H), 1.64-1.61 (m, 2H), 1.57-1.45 (m, 1H), 1.32-1.22 (m, 2H), 0.88 (d, *J* = 6.5 Hz, 6H); LC/MS ESI (+): 425.3 (M+1).

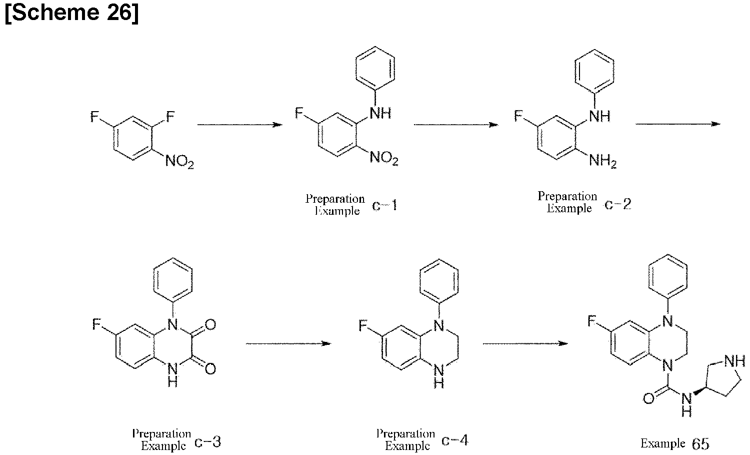

### <Preparation Process c> Preparation of 7-fluoro-1-phenyl-1,2,3,4-tetrahydroquinoxaline

7-Fluoro-1-phenyl-1,2,3,4-tetrahydroquinoxaline according to the present invention was synthesized through Preparation Examples c-1 to c-4 below.

### <Preparation Example c-1> Preparation of 5-fluoro-2-nitro-N-phenylaniline

A mixture of 2,4-difluoro-1-nitrobenzene (1.0 equiv.) and aniline (1.0 equiv.) was stirred at 130 °C for 24 h. After cooling the reaction mixture was quenched with water and extracted with DCM. The organic layer was washed with brine, dried with MgSO₄, filtered, and then concentrated under reduced pressure. The residue was purified by silica column chromatography (with n-hexane/EtOAc) to give 5-fluoro-2-nitro-N-phenylaniline.

Yellow solid (yield: 63%); ¹H NMR (400 MHz, CDCl₃) δ 9.65 (s, 1H), 8.26 (dd, *J* = 9.4, 5.9 Hz, 1H), 7.448-7.43 (m, 2H), 7.31-7.26 (m, 3H), 6.80 (dd, *J* = 11.3, 2.3 Hz, 1H), 6.48 (ddd, *J* = 9.5, 6.9, 2.7 Hz, 1H).

### <Preparation Example c-2> Preparation of 5-fluoro-N¹-phenylbenzene-1,2-diamine

Tin (II) chloride dihydrate (3.0 equiv.) was added to a solution of **Preparation Example c-1** (1.0 equiv.) in EtOAc (0.5 M). The reaction mixture was stirred at 90 °C for 5 h. After cooling the reaction mixture was diluted with water, adjusted to pH 9 with 10M NaOH (aq.) and extracted with EtOAc. The combined organic layer dried with MgSO₄, filtered, and then concentrated under reduced pressure. The residue was purified by silica column chromatography (with *n*-hexane/EtOAc) to give 5-fluoro-N¹-phenylbenzene-1,2-diamine.

Brown oil (yield: 86%); ¹H NMR (400 MHz, CDCl₃) δ 7.28-7.24 (m, 2H), 6.93-6.86 (m, 3H), 6.75-6.71 (m, 1H), 6.69-6.64 (m,1H), 5.34 (s, 1H), 3.89 (s, 2H); LC/MS ESI (+): 202.7 (M+1).

### <Preparation Example c-3> Preparation of 7-fluoro-1-phenylquinoxalin-2,3(1H,4H)-dione

A mixture of **Preparation Example c-2** (1.0 equiv.) in diethyl oxalate (6.0 equiv.) was heated to160 °C for 24 h and then cooled to room temperature. The precipitate formed in the mixture was collected by filtration, washed with EtOAc and then dried vacuo to give 7-fluoro-1-phenylquinoxalin-2,3(1H,4H)-dione.

White solid (yield: 86%); ¹H NMR (400 MHz, DMSO-*d₆*) δ 12.11 (s, 1H), 7.64-7.54 (m, 3H), 7.40-7.38 (m, 2H), 7.22 (dd, *J* = 8.8, 5.3 Hz, 1H), 7.04-6.68 (m, 1H), 5.99 (dd, *J =* 10.2, 2.7 Hz, 1H); LC/MS ESI (+): 256.6 (M+1).

### <Preparation Example c-4> Preparation of 7-fluoro-1-phenyl-1,2,3,4-tetrahydroquinoxaline

To a solution of **Preparation Example c-3** (1.0 equiv.) in THF (0.5 M) was added dropwise 1M borane-THF solution (3.0 equiv.) and stirred at room temperature. The reaction mixture was heated at 65°C for 16 h. After cooling the reaction mixture was quenched with water, and the pH was adjusted to 9 with Conc. NaHCO₃ (aq.). The mixture was extracted with EtOAc, and the organic layer was dried with MgSO₄, filtered, and then concentrated under reduced pressure. The residue was purified by silica column chromatography (with n-hexane/EtOAc) to give 7-fluoro-1-phenyl-1,2,3,4-tetrahydroquinoxaline.

Brown solid (yield: 38%); ¹H NMR (400 MHz, CDCl₃) δ 7.38-7.34 (m, 2H), 7.25-7.20 (m, 2H), 7.13-7.09 (m, 1H), 6.52-6.46 (m, 2H), 6.39-6.33 (m, 1H), 3.71-3.68 (m, 2H), 3.46-3.44 (m, 2H); LC/MS ESI (+): 228.9 (M+1).

### <Example 65> Synthesis of (R)-6-fluoro-4-phenyl-N-(pyrrolidin-3-yl)-3,4-dihydroquinoxaline-1(2H)-carboxamide

To a solution of **Preparation Example c-4** (120 mg, 0.52 mmol), TEA (220.0 µL, 1.57 mmol) and DCM (5.0 mL) cooled to 0 °C under a nitrogen atmosphere was added dropwise triphosgene (78 mg, 0.26 mmol). The reaction mixture was stirred for 1 h then TEA (220.0 µL, 1.57 mmol) and (R)-(+)-1-Boc-3-aminopyrrolidine (0.16 mL, 0.97 mmol) were added slowly. The mixture warmed to room temperature and stirred for 3 h. The resulting mixture was partitioned between DCM and water. The organic layer was washed with brine, dried with MgSO₄, filtered and then concentrated under reduced pressure. A solution of the obtained concentrate in DCM (2.0 mL) was added TFA (490.0 µL, 5.20 mmol). The reaction mixture was stirred at room temperature for 24 h. The reaction mixture was diluted with DCM, and the pH was adjusted to 9 with 10M NaOH (aq.). The mixture was extracted with DCM, the organic layer dried with MgSO₄, filtered, and then concentrated under reduced pressure. The residue was purified by silica column chromatography (with *n*-hexane/EtOAc) to give (*R*)-6-fluoro-4-phenyl-N-(pyrrolidin-3-yl)-3,4-dihydroquinoxaline-1(*2H*)-carboxamide.

White solid (yield: 60.6%); ¹H NMR (400 MHz, CDCl₃) δ 7.49-7.32 (m, 2H), 7.25-7.19 (m, 3H), 7.13-7.09 (m, 1H), 6.43-6.33 (m, 2H), 5.39 (s, 1H), 4.35-4.31 (m, 1H), 3.92-3.88 (m, 2H), 3.66-3.62 (m, 2H), 3.25-3.20 (m, 1H), 3.06 (s, 1H), 3.02-2.90 (m, 1H), 2.89-2.70 (m, 1H), 2.21-2.12 (m, 1H), 1.91-1.87 (m, 1H), 1.69-1.50 (m, 1H); LC/MS ESI (+): 341.0 (M+1).

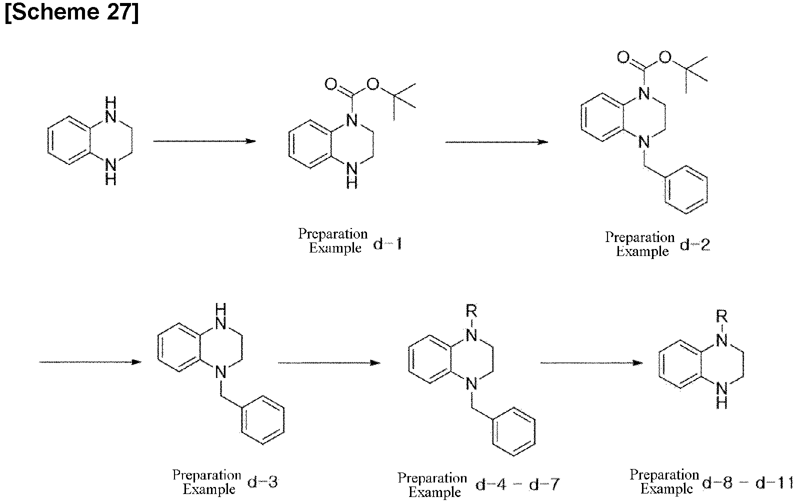

### <Preparation Process d> Preparation of chloro-substituted phenyl or pyridine-1,2,3,4-tetrahydroquinoxaline

Chloro-substituted phenyl or pyridine-1,2,3,4-tetrahydroquinoxaline according to the present invention was prepared through Preparation Examples d-1 to d-11 below.

### <Preparation Example d-1> Preparation of tert-butyl 3,4-dihydroquinoxaline-1(2H)-carboxylate

To a solution of 1,2,3,4-tetrahydroquinoxaline (10.8 g, 80.48 mmol), di-*tert*-butyldicarbonate (18.5 mL, 80.48 mmol) and THF (200 mL) was added 0.02 M NaOH (50 mL). The reaction mixture was stirred at room temperature for 24 h. The resulting mixture was concentrated under reduced pressure and then extracted with DCM. The organic layer was washed with brine, dried with MgSO₄, filtered and then concentrated under reduced pressure. The residue was purified by silica column chromatography (with *n-*hexane/EtOAc) to give *tert*-butyl 3,4-dihydroquinoxaline-1(2*H*)-carboxylate.

Yellow solid (yield: 94%); ¹H NMR (400 MHz, DMSO-*d₆*) δ 7.29 (d, *J* = 8.1 Hz, 1H), 6.79 (ddd, *J* = 8.5, 7.2, 1.5 Hz, 1H), 6.54 (dd, *J* = 8.0, 1.5 Hz, 1H), 6.45 (ddd, *J* = 8.5, 7.2, 1.5 Hz, 1H), 6.01 (s, 1H), 3.60-3.53 (m, 2H), 3.27-3.20 (m, 2H), 1.44 (s, 9H).

### <Preparation Example d-2> Preparation of tert-butyl 4-benzyl-3,4-dihydroquinoxaline-1(2H)-carboxylate

To a solution of Preparation Example d-1 (2.0 g, 8.54 mmol) in DMF (15.0 mL) were added sequentially DIPEA (4.4 g, 34.15 mmol) and benzyl bromide (1.7 g, 10.24 mmol). The resulting mixture was stirred at 130 °C for 2 h. The reaction mixture was quenched with water and extracted with DCM. The organic layer was washed with brine, dried with MgSO₄, filtered, and then concentrated under reduced pressure. The residue was purified by silica column chromatography (with *n*-hexane/EtOAc) to give *tert-butyl* 4-benzyl-3,4-dihydroquinoxaline-1(*2H*)-carboxylate.

Brown oil (yield: 98.9%); ¹H NMR (400 MHz, CD₃CN) δ 7.40-7.29 (m, 3H), 7.28-7.19 (m, 3H), 6.84 (ddd, *J* = 8.6, 7.3, 1.6 Hz, 1H), 6.62-6.51 (m, 2H), 4.53 (s, 2H), 3.80-3.73 (m, 2H), 3.48-3.41 (m, 2H), 1.47 (s, 9H).

### <Preparation Example d-3> Preparation of 1-benzyl-1,2,3,4-tetrahydroquinoxaline

Following the procedure as described for Example 1, reaction of **Preparation Example d-2** (1.0 equiv.) in DCM (0.5 M) with TFA (10.0 equiv.) gave 1-benzyl-1,2,3,4-tetrahydroquinoxaline.

Brown solid (yield: 80%); ¹H NMR (400 MHz, CD₃CN) δ 7.37-7.19 (m, 5H), 6.47-6.39 (m, 4H), 4.41 (s, 2H), 4.29 (brs, 1H), 3.37 (s, 4H); LC/MS ESI (+): 225.2 (M+1).

### <Preparation Example d-4> Preparation of 1-benzyl-4-(2-chlorophenyl)-1,2,3,4-tetrahydroquinoxaline

A solution of **Preparation Example d-3** (1.0 equiv.), sodium *tert*-butoxide (2.0 equiv.), 1-chloro-2-iodobenzene (3.0 equiv.), Pd₂dba (0.05 equiv.) and Xphos (0.15 equiv.) in toluene (0.6 M) was heated at 110 °C for 15 and then cooled to room temperature. The slurry was filtered through a Celite pad and partitioned between DCM and water. The organic layer was washed with brine, dried with MgSO₄, filtered, and then concentrated under reduced pressure. The residue was purified by silica column chromatography (with *n*-hexane/EtOAc) to give 1-benzyl-4-(2-chlorophenyl)-1,2,3,4-tetrahydroquinoxaline.

Colorless oil (yield: 99%); ¹H NMR (400 MHz, DMSO-*d₆*) δ7.57 (dd, *J* = 8.0, 1.4 Hz, 1H), 7.47-7.31 (m, 6H), 7.30-7.21 (m, 2H), 6.61-6.49 (m, 2H), 6.37 (dd, *J* = 7.4, 1.9 Hz, 1H), 5.99 (dd, *J*= 8.0, 0.9 Hz, 1H), 3.72-3.59 (m, 2H), 3.57-3.42 (m, 2H); LC/MS ESI (+): 335.1 (M+1).

### <Preparation Example d-5> Preparation of 1-benzyl-4-(pyridin-2-yl)-1,2,3,4-tetrahydroquinoxaline

The title compound was prepared in the same manner as Preparation Example d-4, using 2-bromopyridine in place of 1-chloro-2-iodobenzene to give 1-benzyl-4-(pyridin-2-yl)-1,2,3,4-tetrahydroquinoxaline.

Yellow solid (yield: 73%); ¹H NMR (400 MHz, DMSO-*d₆*) δ 8.22 (dd, *J* = 5.1, 1.2 Hz, 1H), 7.53 (ddd, *J* = 8.7, 6.9, 1.9 Hz, 1H), 7.35-7.19 (m, 5H), 7.15 (dd, *J* = 7.8, 1.6 Hz, 1H), 7.03 (d, *J* = 8.6 Hz, 1H), 6.86 (ddd, *J* = 8.6, 7.3, 1.5 Hz, 1H), 6.77 (dd, *J* = 7.0, 5.5 Hz, 1H), 6.70 (dd, *J* = 8.2, 1.2 Hz, 1H), 6.60-6.53 (m, 1H), 4.65-4.50 (m, 2H), 4.10-4.02 (m, 2H), 3.40 (t, *J =* 5.1 Hz, 2H); LC/MS ESI (+): 302.2 (M+1).

### <Preparation Example d-6> Preparation of 1-benzyl-4-(pyridin-3-yl)-1,2,3,4-tetrahydroquinoxaline

The title compound was prepared in the same manner as Preparation Example d-4, using 3-iodopyridine in place of 1-chloro-2-iodobenzene to give 1-benzyl-4-(pyridin-3-yl)-1,2,3,4-tetrahydroquinoxaline.

Yellow oil (yield: 100%); ¹H NMR (400 MHz, CD₃CN) δ 8.46 (d, *J* = 2.7 Hz, 1H), 8.19 (dd, *J* = 4.7, 1.2 Hz, 1H), 7.50 (ddd, *J* = 8.4, 2.9, 1.6 Hz, 1H), 7.40-7.31 (m, 4H), 7.30-7.24 (m, 2H), 6.82 (dd, *J* = 8.2, 1.6 Hz, 1H), 6.78-6.72 (m, 1H), 6.70-6.65 (m, 1H), 6.55-6.49 (m, 1H), 4.57 (s, 2H), 3.80-3.74 (m, 2H), 3.52-3.46 (m, 2H); LC/MS ESI (+): 302.2 (M+1).

### <Preparation Example d-7> Preparation of 1-benzyl-4-(pyridin-4-yl)-1,2,3,4-tetrahydroquinoxaline

The title compound was prepared in the same manner as Preparation Example d-4, using 4-iodopyridine in place of 1-chloro-2-iodobenzene to give 1-benzyl-4-(pyridin-4-yl)-1,2,3,4-tetrahydroquinoxaline.

Red oil (yield: 100%); ¹H NMR (400 MHz, DMSO-*d₆*) δ 8.26-8.19 (m, 2H), 7.31 (dd, *J* = 8.1, 6.8 Hz, 2H), 7.26-7.18 (m, 3H), 7.09 (dd, *J* = 7.9, 1.5 Hz, 1H), 7.05-6.99 (m, 2H), 6.89 (ddd, *J* = 8.6, 7.2, 1.5 Hz, 1H), 6.73 (dd, *J* = 8.3, 1.3 Hz, 1H), 6.62-6.53 (m, 1H), 4.56 (s, 2H), 3.84 (q, *J =* 5.9 Hz, 2H), 3.40 (t, *J =* 5.1 Hz, 2H); LC/MS ESI (+): 302.2 (M+1).

### <Preparation Example d-8> Preparation of 1-(2-chlorophenyl)-1,2,3,4-tetrahydroquinoxaline

A suspension of **Preparation Example d-4** (1.0 equiv.), Pd(OH)₂ (0.6 equiv.) were stirred in MeOH/THF (v/v = 2: 1, 0.3 M) under a hydrogen atmosphere for 3 h. The resulting mixture was filtered through a Celite pad and concentrated under reduced pressure. The residue was purified by silica column chromatography (with *n-*hexane/EtOAc) to give 1-(2-chlorophenyl)-1,2,3,4-tetrahydroquinoxaline.

Gray solid (yield: 62%); ¹H NMR (400 MHz, DMSO-*d₆*) δ 7.55 (dd, *J* = 8.0, 1.4 Hz, 1H), 7.43-7.35 (m, 1H), 7.34-7.30 (m, 1H), 7.28-7.21 (m, 1H), 6.54-6.49 (m, 2H), 6.31 (ddd, *J* = 8.2, 5.1, 3.5 Hz, 1H), 5.96 (d, *J* = 7.8 Hz, 1H), 3.54-3.46 (m, 2H), 3.35 (dd, *J* = 4.9, 2.2 Hz, 2H); LC/MS ESI (+): 245.1 (M+1).

### <Preparation Example d-9> Preparation of 1-(pyridin-2-yl)-1,2,3,4-tetrahydroquinoxaline

The title compound was prepared in the same manner as Preparation Example d-8, using **Preparation Example d-5** in place of Preparation Example d-4 to give 1-(pyridin-2-yl)-1,2,3,4-tetrahydroquinoxaline.

Yellow solid (yield: 78.2%); ¹H NMR (400 MHz, CDCl₃) δ 8.26 (d, *J* = 4.1 Hz, 1H), 7.42 (ddd, *J* = 8.5, 7.1, 2.0 Hz, 1H), 7.22 (dd, *J* = 8.4, 1.5 Hz, 1H), 7.16 (d, *J =* 8.5 Hz, 1H), 6.93-6.83 (m, 1H), 6.70 (ddd, *J* = 7.1, 4.9, 1.0 Hz, 1H), 6.67-6.55 (m, 2H), 4.08 (t, *J = 4.8* Hz, 2H), 3.99 (brs, 1H), 3.41 (t, *J =* 4.8 Hz, 2H); LC/MS ESI (+): 212.2 (M+1).

### <Preparation Example d-10> Preparation of 1-(pyridin-3-yl)-1,2,3,4-tetrahydroquinoxaline

The title compound was prepared in the same manner as Preparation Example d-8, using **Preparation Example d-6** in place of Preparation Example d-4 to give 1-(pyridin-3-yl)-1,2,3,4-tetrahydroquinoxaline.

Brown oil (yield: 83.1%); ¹H NMR (400 MHz, CD₃CN) δ 8.45 (d, *J =* 2.7 Hz, 1H), 8.17 (dd, *J* = 4.7, 1.6 Hz, 1H), 7.50 (ddd, *J* = 8.3, 2.8, 1.8 Hz, 1H), 7.26 (dd, *J* = 8.2, 4.7 Hz, 1H), 6.81-6.71 (m, 2H), 6.66-6.61 (m, 1H), 6.54-6.47 (m, 1H), 4.63 (brs, 1H), 3.68-3.63 (m, 2H), 3.39-3.34 (m, 2H); LC/MS ESI (+): 212.2 (M+1).

### <Preparation Example d-11> Preparation of 1-(pyridin-4-yl)-1,2,3,4-tetrahydroquinoxaline

The title compound was prepared in the same manner as Preparation Example d-8, using **Preparation Example d-7** in place of Preparation Example d-4 to give 1-(pyridin-4-yl)-1,2,3,4-tetrahydroquinoxaline.

Yellow solid (yield: 76.7%); ¹H NMR (400 MHz, DMSO-*d₆*) δ 8.25-8.18 (m, 2H), 7.06-7.00 (m, 3H), 6.85 (ddd, *J* = 8.4, 7.2, 1.4 Hz, 1H), 6.65 (dd, *J* = 8.1, 1.5 Hz, 1H), 6.50 (ddd, *J* = 8.0, 7.2, 1.5 Hz, 1H), 6.09 (s, 1H), 3.70-3.63 (m, 2H), 3.29-3.21 (m, 2H); LC/MS ESI (+): 212.1 (M+1).

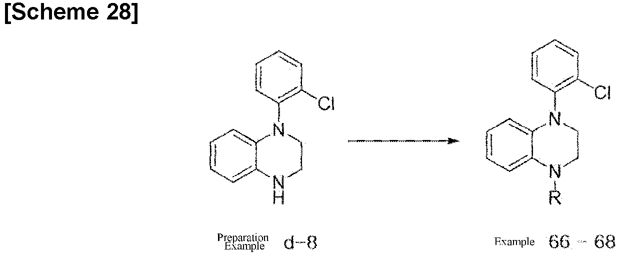

### <Example 66> Synthesis of (4-(2-chlorophenyl)-3,4-dihydroquinoxalin-1(2H)-yl)(piperidin-1-yl)methanone

To a solution of **Preparation Example d-8** (1.0 equiv.), TEA (3.0 equiv.) and DCM (0.3 M) cooled to 0 °C under a nitrogen atmosphere was added dropwise triphosgene (0.6 equiv.). The reaction mixture was stirred for 1 h then TEA (2.0 equiv.) and piperidine (1.2 equiv.) were added slowly. The mixture warmed to room temperature and stirred for 3 h. The resulting mixture was partitioned between DCM and water. The organic layer was washed with brine, dried with MgSO₄, filtered and then concentrated under reduced pressure. The residue was purified by silica column chromatography (with *n-*hexane/EtOAc) to give (4-(2-chlorophenyl)-3,4-dihydroquinoxalin-1(2H)-yl)(piperidin-1-yl)methanone.

Light yellow oil (yield: 34.8%); ¹H NMR (400 MHz, CDCl₃) δ 7.55-7.48 (m, 1H), 7.36-7.31 (m, 2H), 7.26-7.21 (m, 1H), 7.02 (dd, *J* = 8.0, 1.6 Hz, 1H), 6.77-6.70 (m, 1H), 6.69-6.62 (m, 1H), 6.21 (dd, *J* = 8.0, 1.5 Hz, 1H), 3.84 (t, *J* = 5.0 Hz, 2H), 3.69 (t, *J* = 4.1 Hz, 2H), 3.38-3.31 (m, 4H), 1.61-1.48 (m, 6H); LC/MS ESI (+): 342.2 (M+1).

### <Example 67> Synthesis of (4-(2-chlorophenyl)-3,4-dihydroquinoxalin-1(2H)-yl)(pyrrolidin-1-yl)methanone

The title compound was synthesized in the same manner as Example 66, using pyrrolidine in place of piperidine to give (4-(2-chlorophenyl)-3,4-dihydroquinoxalin-1 (2H)-yl)(pyrrolidin-1-yl)methanone.

Light yellow oil (yield: 54.5%); ¹H NMR (400 MHz, CDCl₃) δ 7.54-7.49 (m, 1H), 7.36-7.32 (m, 2H), 7.28-7.26 (m, 1H), 6.95 (dd, *J* = 7.9, 1.6 Hz, 1H), 6.77-6.70 (m, 1H), 6.69-6.63 (m, 1H), 6.19 (dd, *J* = 8.0, 1.5 Hz, 1H), 3.90 (t, *J =* 5.1 Hz, 2H), 3.75-3.69 (m, 2H), 3.40-3.33 (m, 4H), 1.89-1.78 (m, 4H); LC/MS ESI (+): 356.2 (M+1).

### <Example 68> Synthesis of (4-(2-chlorophenyl)-3,4-dihydroquinoxalin-1(2H)-yl)(4-methylpiperazin-1-yl)methanone

The title compound was synthesized in the same manner as Example 66, using *N*-methylpiperazine in place of piperidine to give (4-(2-chlorophenyl)-3,4-dihydroquinoxalin-1(*2H*)-yl)(4-methylpiperazin-1-yl)methanone.

Light yellow oil (yield: 51.6%); ¹H NMR (400 MHz, CDCl₃) δ 7.53-7.48 (m, 1H), 7.40-7.26 (m, 3H), 7.05 (dd, *J* = 8.0, 1.6 Hz, 1H), 6.80-6.71 (m, 1H), 6.70-6.62 (m, 1H), 6.20 (dd, *J* = 8.1, 1.5 Hz, 1H), 3.87 (t, *J* = 5.0 Hz, 2H), 3.70 (t, *J =* 4.3 Hz, 2H), 3.46-3.39 (m, 4H), 2.38 (t, *J* = 5.1 Hz, 5H), 2.29 (s, 3H); LC/MS ESI (+): 371.2 (M+1).

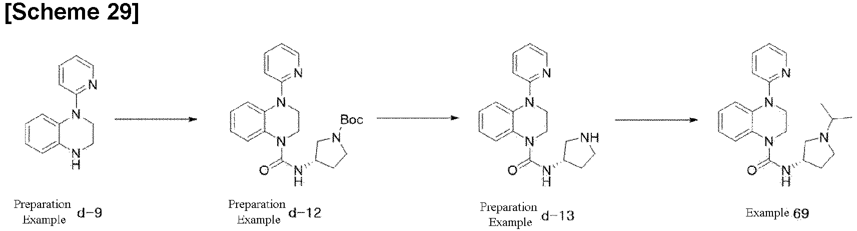

### <Preparation Example d-12> Preparation of tert-butyl (S)-3-(4-(pyridin-2-yl)-1,2,3,4-tetrahydroquinoxaline-1-carboxamido)pyrrolidine-1-carboxylate

To a solution of **Preparation Example d-9** (1.0 equiv.), TEA (3.0 equiv.) and DCM (0.3 M) cooled to 0 °C under a nitrogen atmosphere was added dropwise triphosgene (0.6 equiv.). The reaction mixture was stirred at 0 °C for 1 h then TEA (2.0 equiv.) and (S)-(-)-1-Boc-3-aminopyrrolidine (1.2 equiv.) were added slowly. The mixture warmed to room temperature and stirred for 3 h. The resulting mixture was partitioned between DCM and water. The organic layer was washed with brine, dried with MgSO₄, filtered and then concentrated under reduced pressure. The residue was purified by silica column chromatography (with *n*-hexane/EtOAc) to give *tert-butyl* (*S*)-3-(4-(pyridin-2-yl)-1,2,3,4-tetrahydroquinoxaline-1-carboxamido)pyrrolidine-1-carboxylate.

White solid (yield: 97%); ¹H NMR (400 MHz, CD₃OD) δ 8.25-8.24 (m, 1H), 7.67-7.62 (m, 1H), 7.45 (dd, *J* = 8.3, 1.3 Hz, 1H), 7.34-7.32 (m, 1H), 7.24-7.19 (m, 1H), 7.10-7.01 (m, 2H), 6.92 (ddd, *J* = 7.3, 5.0, 0.9 Hz, 1H), 4.34-4.28 (m, 1H), 4.02-3.97 (m, 2H), 3.92-3.84 (m, 2H), 3.65-3.59 (m, 1H), 3.49-3.36 (m, 2H), 3.24-3.20 (m, 1H), 2.20-2.11 (m, 1H), 1.95-1.87 (m, 1H), 1.47 (s, 9H).

### <Preparation Example d-13> Preparation of (S)-4-(pyridin-2-yl)-N-(pyrrolidin-3-yl)-3,4-dihydroquinoxaline-1(2H)-carboxamide

Following the procedure as described for Example 1, reaction of **Preparation Example d-12** (1.0 equiv.) in DCM (0.5 M) with TFA (10.0 equiv.) gave (*S*)-4-(pyridin-2-yl)-*N*-(pyrrolidin-3-yl)-3,4-dihydroquinoxaline-1(*2H*)-carboxamide.

White solid (yield: 48%); ¹H NMR (400 MHz, CDCl₃) δ 8.32-8.30 (m, 1H), 7.56-7.51 (m, 1H), 7.47 (dd, *J* = 8.1, 1.4 Hz, 1H), 7.33 (dd, *J* = 7.9, 1.5 Hz, 1H), 7.16-7.15 (m, 1H), 7.08-7.03 (m, 1H), 7.01-6.94 (m, 1H), 6.84 (ddd, *J* = 7.2, 4.9, 0.8 Hz, 1H), 5.34 (d, J= 6.9 Hz, 1H), 4.36-4.28 (m, 1H), 4.03-3.94 (m, 4H), 3.19-3.14 (m, 1H), 3.05-2.99 (m, 1H), 2.95-2.88 (m, 1H), 2.80-2.76 (m, 1H), 2.46 (brs, 1H), 2.19-2.10 (m, 1H), 1.63-1.55 (m, 1H); LC/MS ESI (+): 324.2 (M+1).

### <Example 69> Synthesis of (S)-N-(1-isopropylpyrrolidin-3-yl)-4-(pyridin-2-yl)-3,4-dihydroquinoxaline-1(2H)-carboxamide

Following the procedure as described for Example 2, reaction of **Preparation Example d-13** (1.0 equiv.) in DCM (0.3 M) with sodium triacetoxyborohydride (1.5 equiv.) and acetone (2.0 equiv.) gave (*S*)-*N*-(1-isopropylpyrrolidin-3-yl)-4-(pyridin-2-yl)-3,4-dihydroquinoxaline-1(2*H*)-carboxamide.

Light yellow oil (yield: 48%); ¹H NMR (400 MHz, CD₃OD) δ 8.25-8.21 (m, 1H), 7.64-7.58 (m, 1H), 7.48-7.46 (m, 1H), 7.34-7.31 (m, 1H), 7.22-7.18 (m, 1H), 7.10-7.01 (m, 2H), 6.90 (ddd, *J* = 7.2, 5.1, 0.9 Hz, 1H), 4.37-4.30 (m, 1H), 3.99-3.95 (m, 2H), 3.93-3.80 (m, 2H), 2.90-2.81 (m, 2H), 2.62-2.59 (m, 1H), 2.54-2.48 (m, 1H), 2.45-2.39 (m, 1H), 2.32-2.23 (m, 1H), 1.71-1.63 (m, 1H), 1.10 (t, *J* = 7.1, 6.6 Hz, 6H); LC/MS ESI (+): 366.3 (M+1).

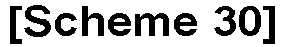

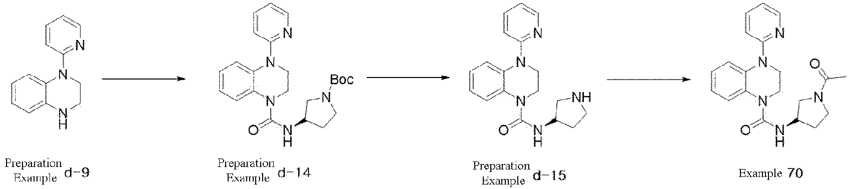

### <Preparation Example d-14> Preparation of tert-butyl (R)-3-(4-(pyridin-2-yl)-1,2,3,4-tetrahydroquinoxaline-1-carboxamido)pyrrolidine-1-carboxylate

The title compound was prepared in the same manner as Preparation Example d-12, using (*R*)-(+)-1-Boc-3-aminopyrrolidine in place of (*S*)-(-)-1-Boc-3-aminopyrrolidine to give *tert*-butyl *(R*)-3-((4-pyridin-2-yl)-1,2,3,4-tetrahydroquinoxaline-1-carboxamido)methyl)pyrrolidine-1-carboxylate.

Light yellow oil (yield: 89%); ¹H NMR (400 MHz, CDCl₃) δ 8.33-8.27 (m, 1H), 7.58-7.49 (m, 1H), 7.46 (dd, *J* = 8.2, 1.4 Hz, 1H), 7.28-7.22 (m, 1H), 7.18-7.11 (m, 1H), 7.10-7.02 (m, 1H), 6.99-6.93 (m, 1H), 6.87-6.80 (m, 1H), 5.20 (d, *J* = 6.7 Hz, 1H), 4.38 (s, 1H), 4.02-3.97 (m, 2H), 3.66-3.58 (m, 1H), 3.42-3.35 (m, 3H), 3.13 (s, 1H), 1.45-1.41 (m, 12H); LC/MS ESI (+): 424.1 (M+1).

### <Preparation Example d-15> Preparation of (R)-4-(pyridin-2-yl)-N-(pyrrolidin-3-yl)-3,4-dihydroquinoxaline-1(2H)-carboxamide

Following the procedure as described for Example 1, reaction of **Preparation Example d-14** (1.0 equiv.) in DCM (0.5 M) with TFA (10.0 equiv.) gave (*R*)-4-(pyridin-2-yl)-*N*-(pyrrolidin-3-yl)-3,4-dihydroquinoxaline-1(*2H*)-carboxamide.

Ivory solid (yield: 99%); ¹H NMR (400 MHz, DMSO-*d₆*) δ 13.04 (s, 1H), 10.33 (s, 1H), 8.35-8.16 (m, 1H), 7.99-7.94 (m, 1H), 7.67-7.60 (m, 1H), 7.56-7.48 (m, 1H), 7.47-7.36 (m, 4H), 7.26-7.14 (m, 1H), 5.10-4.71 (m, 1H), 2.32 (d, *J* = 12.3 Hz, 1H), 1.32-1.19 (m, 1H), 1.03 (d, *J* = 6.7 Hz, 6H), 0.90-0.81 (m, 1H); LC/MS ESI (+): 324.1 (M+1).

### <Example 70> Synthesis of (R)-N-(1-acetylpyrrolidin-3-yl)-4-(pyridin-2-yl)-3,4-dihydroquinoxaline-1(2H)-carboxamide

To a solution of **Preparation Example d-15** (1.0 equiv.), TEA (1.0 equiv.) and THF (0.3 M) were added dropwise acetyl chloride (1.1 equiv.). The resulting mixture was stirred at room temperature for 1.5 h. The reaction mixture was quenched with water, and the pH was adjusted to 8 by Conc. NaHCO₃ (aq.). The mixture was extracted with DCM, the organic layer dried with MgSO₄, filtered, and then concentrated under reduced pressure. The residue was purified by silica column chromatography (with DCM/MeOH) to give (*R*)-*N*-(1-acetylpyrrolidin-3-yl)-4-(pyridin-2-yl)-3,4-dihydroquinoxaline-1(*2H*)-carboxamide.

Ivory solid (yield: 45%); ¹H NMR (400 MHz, CDCl₃) δ 8.33-8.27 (m, 1H), 7.58-7.43 (m, 2H), 7.28-7.20 (m, 1H), 7.18-7.02 (m, 2H), 7.01-6.90 (m, 1H), 6.88-6.80 (m, 1H), 5.23 (d, *J* = 6.2 Hz, 1H), 4.47-4.37 (m, 1H), 4.04-3.93 (m, 3H), 3.82-3.68 (m, 1H), 3.56-3.44 (m, 2H), 3.33-3.22 (m, 1H), 2.20-2.10 (m, 1H), 2.02 (d, *J* = 9.0 Hz, 3H), 1.83-1.70 (m, 1H), 1.28-1.20 (m, 1H); LC/MS ESI (+): 366.1 (M+1).

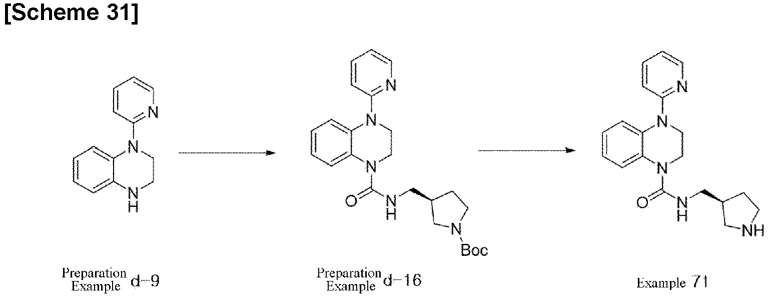

### <Preparation Example d-16> Preparation of tert-butyl (R)-3-((4-pyridin-2-yl)-1,2,3,4-tetrahydroquinoxaline-1-carboxamido)methyl)pyrrolidine-1-carboxylate

The title compound was prepared in the same manner as Preparation Example d-12, using (*R*)-1-Boc-3-(aminomethyl)pyrrolidine in place of (*S*)-(-)-1-Boc-3-aminopyrrolidine to give *tert*-butyl (*R*)-3-((4-pyridin-2-yl)-1,2,3,4-tetrahydroquinoxaline-1-carboxamido)methyl)pyrrolidine-1-carboxylate.

Yellow oil (yield: 100%); ¹H NMR (400 MHz, CD₃OD) δ 8.24 (ddd, *J* = 5.0, 2.0, 0.9 Hz, 1H), 7.62 (ddd, *J* = 8.5, 7.2, 2.0 Hz, 1H), 7.47-7.40 (m, 1H), 7.37-7.30 (m, 1H), 7.23-7.16 (m, 1H), 7.13-7.00 (m, 2H), 6.90 (ddd, *J* = 7.2, 5.0, 0.9 Hz, 1H), 4.02-3.95 (m, 2H), 3.93-3.81 (m, 2H), 3.47-3.35 (m, 2H), 3.30-3.13 (m, 2H), 3.08-2.98 (m, 1H), 2.50-2.35 (m, 1H), 2.02-1.86 (m, 1H), 1.71-1.56 (m, 1H), 1.44 (s, 9H); LC/MS ESI (+): 438.0 (M+1).

### <Example 71> Synthesis of (S)-4-(pyridin-2-yl)-N-(pyrrolidin-3-ylmethyl)-3,4-dihydroquinoxaline-1(2H)-carboxamide

Following the procedure as described for Example 1, reaction of **Preparation Example d-16** (1.0 equiv.) in DCM (0.5 M) with TFA (10.0 equiv.) gave (*S*)-4-(pyridin-2-yl)-*N*-(pyrrolidin-3-ylmethyl)-3,4-dihydroquinoxaline-1(*2H*)-carboxamide.

Yellow oil (yield: 100%); ¹H NMR (400 MHz CD₃OD) δ 8.27-8.21 (m, 1H), 7.64 (ddd, *J* = 8.5, 7.2, 2.0 Hz, 1H), 7.48-7.41 (m, 1H), 7.39-7.31 (m, 1H), 7.23-7.16 (m, 1H), 7.14-7.01 (m, 3H), 6.92 (ddd, *J* = 7.2, 5.0, 0.9 Hz, 1H), 4.07-3.95 (m, 2H), 3.92-3.84 (m, 2H), 3.38-3.33 (m, 2H), 3.29-3.18 (m, 3H), 3.02-2.93 (m, 1H), 2.65-2.49 (m, 1H), 2.16-2.01 (m, 1H), 1.81-1.67 (m, 1H); LC/MS ESI (+): 338.0 (M+1).

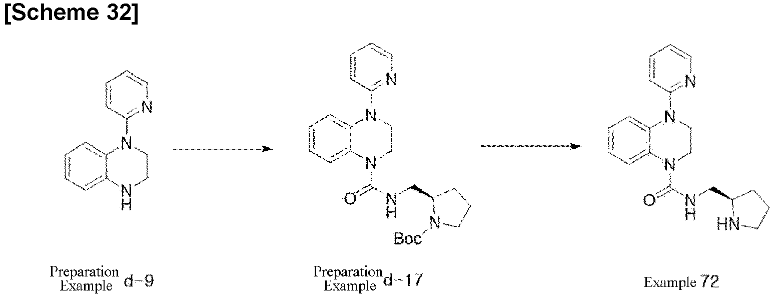

### <Preparation Example d-17> Preparation of tert-butyl (R)-2-((4-pyridin-2-yl)-1,2,3,4-tetrahydroquinoxaline-1-carboxamido)methyl)pyrrolidine-1-carboxylate

The title compound was prepared in the same manner as Preparation Example d-12, using (*R*)-1-Boc-2-(aminomethyl)pyrrolidine in place of (*S*)-(-)-1-Boc-3-aminopyrrolidine to give *tert-butyl* (*R*)-3-((4-pyridin-2-yl)-1,2,3,4-tetrahydroquinoxaline-1-carboxamido)methyl)pyrrolidine-1-carboxylate.

Yellow oil (yield: 100%); ¹H NMR (400 MHz, CDCl₃) δ 8.32-8.26 (m, 1H), 7.61-7.56 (m, 1H), 7.50-7.42 (m, 1H), 7.40-7.36 (m, 1H), 7.18-7.11 (m, 1H), 7.05-6.99 (m, 2H), 6.81-6.75 (m, 1H), 6.59-6.54 (m, 1H), 4.10-3.83 (m, 4H), 3.46-3.19 (m, 3H), 2.03-1.66 (m, 4H), 1.40 (s, 9H); LC/MS ESI (+): 438.0 (M+1).

### <Example 72> Synthesis of (R)-4-(pyridin-2-yl)-N-(pyrrolidin-3-ylmethyl)-3,4-dihydroquinoxaline-1(2H)-carboxamide

Following the procedure as described for Example 1, reaction of **Preparation Example d-17** (1.0 equiv.) in DCM (0.5 M) with TFA (10.0 equiv.) gave (*R*)-4-(pyridin-2-yl)-*N*-(pyrrolidin-3-ylmethyl)-3,4-dihydroquinoxaline-1(*2H*)-carboxamide.

Yellow oil (yield: 100%); ¹H NMR (400 MHz, CD₃OD) δ 8.24 (ddd, *J* = 5.0, 2.0, 0.9 Hz, 1H), 7.64 (ddd, *J* = 8.5, 7.2, 2.0 Hz, 1H), 7.50 (dd, *J* = 8.0, 1.6 Hz, 1H), 7.36 (dd, *J =* 8.0, 1.5 Hz, 1H), 7.20 (dd, *J* = 8.5, 0.9 Hz, 1H), 7.15-7.00 (m, 3H), 6.92 (ddd, *J* = 7.2, 5.0, 0.9 Hz, 1H), 4.04-3.98 (m, 2H), 3.95-3.89 (m, 2H), 3.73-3.61 (m, 1H), 3.53-3.41 (m, 2H), 3.28-3.15 (m, 2H), 2.16-1.90 (m, 3H), 1.81-1.69 (m, 1H); LC/MS ESI (+): 338.0 (M+1).

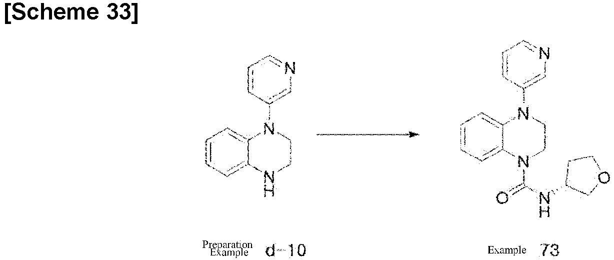

### <Example 73> Synthesis of (R)-4-(pyridin-3-yl)-N-(tetrahydrofuran-3-yl)-3,4-dihydroquinoxaline-1(2H)-carboxamide

To a solution of **Preparation Example d-10** (1.0 equiv.), TEA (3.0 equiv.) and DCM (0.3 M) cooled to 0 °C under a nitrogen atmosphere was added dropwise triphosgene (0.6 equiv.). The reaction mixture was stirred for 1 h then TEA (2.0 equiv.) and (*R*)-3-aminotetrahydrofuran (1.2 equiv.) were added slowly. The mixture warmed to room temperature and stirred for 3 h. The resulting mixture was partitioned between DCMand water. The organic layer was washed with brine, dried with MgSO₄, filtered and then concentrated under reduced pressure. The residue was purified by silica column chromatography (with *n*-hexane/EtOAc) to give (*R*)-4-(pyridin-3-yl)-*N*-(tetrahydrofuran-3-yl)-3,4-dihydroquinoxaline-1(*2H*)-carboxamide.

Yellow solid (yield: 97%); ¹H NMR (400 MHz, CDCl₃) δ 8.58 (d, *J* = 2.4 Hz, 1H), 8.41 (dd, *J* = 4.8, 1.4 Hz, 1H), 7.61-7.58 (m, 1H), 7.34-7.31 (m, 1H), 7.24-7.20 (m, 1H), 6.98-6.93 (m, 1H), 6.84-6.82 (m, 2H), 5.45 (d, *J =* 6.9 Hz, 1H), 4.53-4.46 (m, 1H), 4.03-3.84 (m, 4H), 3.82-3.67 (m, 4H), 2.33-2.24 (m, 1H), 1.84-1.76 (m, 1H); LC/MS ESI (+): 325.0 (M+1).

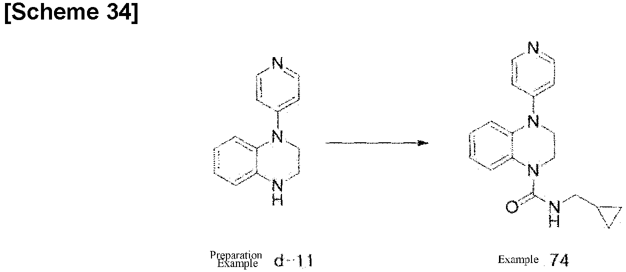

### <Example 74> Synthesis of N-(cyclopropylmethyl)-4-(pyridin-4-yl)-3,4-dihydroquinoxaline-1(2H)-carboxamide

To a solution of **Preparation Example d-11** (1.0 equiv.), TEA (3.0 equiv.) and DCM (0.3 M) cooled to 0 °C under a nitrogen atmosphere was added dropwise triphosgene (0.6 equiv.). The reaction mixture was stirred for 1 h then TEA (2.0 equiv.) and (R)-3-aminotetrahydrofuran (1.2 equiv.) were added slowly. The mixture warmed to room temperature and stirred for 3 h. The resulting mixture was partitioned between DCM and water. The organic layer was washed with brine, dried with MgSO₄, filtered and then concentrated under reduced pressure. The residue was purified by silica column chromatography (with n-hexane/EtOAc) to give N-(cyclopropylmethyl)-4-(pyridin-4-yl)-3,4-dihydroquinoxaline-1(*2H*)-carboxamide.

Light yellow solid (yield: 37%); ¹H NMR (400 MHz, CDCl₃) δ 8.40-8.34 (m, 2H), 7.43-7.40 (m, 1H), 7.39-7.36 (m, 1H), 7.14-7.10 (m, 1H), 7.07-7.03 (m, 3H), 5.28-5.24 (m, 1H), 4.00-3.97 (m, 2H), 3.84-3.80 (m, 2H), 3.14 (dd, *J* = 7.1, 5.4 Hz, 1H), 1.01-0.91 (m, 1H), 0.51-0.46 (m, 2H), 0.20-0.16 (m, 2H); LC/MS ESI (+): 309.1 (M+1).

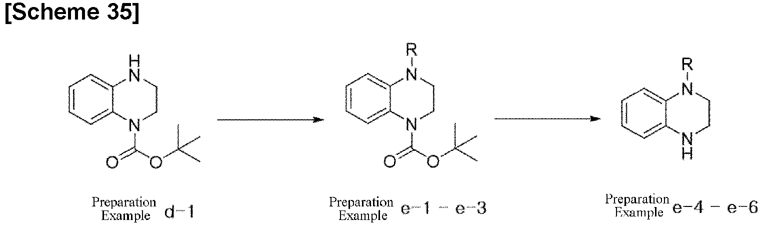

### <Preparation Process e> Preparation of halogen-substituted pyridine, cyanophenyl or pyrazin-1,2,3,4-tetrahydroquinoxaline

Halogen-substituted pyridine, cyanophenyl or pyrazin-1,2,3,4-tetrahydroquinoxaline according to the present invention was prepared through Preparation Examples e-1 to e-6 below.

### <Preparation Example e-1> Preparation of tert-butyl 4-(5-fluoropyridin-2-yl)-3,4-dihydroquinoxaline-1(2H)-carboxylate

A solution of **Preparation Example d-1** (1.0 equiv.), sodium *tert*-butoxide (2.0 equiv.), 5-fluoro-2-bromopyridine (3.0 equiv.), Pd₂dba (0.05 equiv.) and Xphos (0.15 equiv.) in toluene (0.6 M) was heated at 110 °C for 15 and then cooled to room temperature. The slurry was filtered through a Celite pad and partitioned between DCM and water. The organic layer was washed with brine, dried with MgSO₄, filtered, and then concentrated under reduced pressure. The residue was purified by silica column chromatography (with *n*-hexane/EtOAc) to give *tert*-butyl 4-(5-fluoropyridin-2-yl)-3,4-dihydroquinoxaline-1(2*H*)-carboxylate.

Red oil (yield: 93%); ¹H NMR (400 MHz, CDCl₃) δ 8.14 (d, *J =* 3.0 Hz, 1H), 7.79 (s, 1H), 7.25-7.20 (m, 2H), 7.19-7.12 (m, 1H), 7.03-6.92 (m, 2H), 4.06-3.99 (m, 2H), 3.91-3.83 (m, 2H), 1.53 (s, 9H); LC/MS ESI (+): 330.1 (M+1).

### <Preparation Example e-2> Preparation of tert-butyl 4-(4-cyanophenyl)-3,4-dihydroquinoxaline-1(2H)-carboxylate

The title compound was prepared in the same manner as Preparation Example e-1, using 4-iodobenzonitrile in place of 5-fluoro-2-bromopyridine to give *tert-butyl* 4-(4-cyanophenyl)-3,4-dihydroquinoxaline-1(*2H*)-carboxylate.

Yellow solid (yield: 89%); ¹H NMR (400 MHz, CD₃OD) δ 7.68-7.61 (m, 1H), 7.61-7.54 (m, 2H), 7.32-7.24 (m, 2H), 7.17-7.08 (m, 1H), 7.03-6.91 (m, 2H), 3.90-3.77 (m, 4H), 1.50 (s, 9H).

### <Preparation Example e-3> Preparation of tert-butyl 4-(pyrazin-2-yl)-3,4-dihydroquinoxaline-1(2H)-carboxylate

The title compound was prepared in the same manner as Preparation Example e-1, using 2-chloropyrazine in place of 5-fluoro-2-bromopyridine to give *tert*-butyl 4-(pyrazin-2-yl)-3,4-dihydroquinoxaline-1(*2H*)-carboxylate.

Orange solid (yield: 85%); ¹H NMR (400 MHz, CDCl₃) δ 8.59 (d, *J =* 1.6 Hz, 1H), 8.16 (dd, *J* = 2.7, 1.5 Hz, 1H), 7.98 (d, *J* = 2.7 Hz, 1H), 7.84 (d, *J* = 8.0 Hz, 1H), 7.40-7.34 (m, 1H), 7.12-7.00 (m, 2H), 4.12-4.04 (m, 2H), 3.93-3.86 (m, 2H), 1.53 (s, 9H).

### <Preparation Example e-4> Preparation of 1-(5-fluoropyridin-2-yl)-1,2,3,4-tetrahydroquinoxaline

Following the procedure as described for Example 1, reaction of **Preparation Example e-1** (1.0 equiv.) in DCM (0.5 M) with TFA (10.0 equiv.) gave 1-(5-fluoropyridin-2-yl)-1,2,3,4-tetrahydroquinoxaline.

Red oil (yield: 91%); ¹H NMR (400 MHz, CDCl₃) δ 8.12 (d, *J* = 3.0 Hz, 1H), 7.23-7.09 (m, 3H), 6.92-6.83 (m, 1H), 6.69-6.59 (m, 2H), 4.07-3.89 (m, 3H), 3.43-3.37 (m, 2H); LC/MS ESI (+): 230.1 (M+1).

### <Preparation Example e-5> Preparation of 4-(3,4-dihydroquinoxalin-1(2H)-yl)benzonitrile

Following the procedure as described for Example 1, reaction of **Preparation Example e-2** (1.0 equiv.) in DCM (0.5 M) with TFA (10.0 equiv.) gave 4-(3,4-dihydroquinoxalin-1(2*H*)-yl)benzonitrile.

Yellow solid (yield: 89%); ¹H NMR (400 MHz, CD₃OD) δ 7.56-7.47 (m, 2H), 7.24-7.14 (m, 2H), 7.01-6.94 (m, 1H), 6.85-6.76 (m, 1H), 6.69-6.62 (m, 1H), 6.57-6.48 (m, 1H), 3.74-3.67 (m, 2H), 3.35-3.28 (m, 2H); LC/MS ESI (+): 236.1 (M+1).

### <Preparation Example e-6> Preparation of 1-(pyrazin-2-yl)-1,2,3,4-tetrahydroquinoxaline

Following the procedure as described for Example 1, reaction of Preparation **Example e-3** (1.0 equiv.) in DCM (0.5 M) with TFA (10.0 equiv.) gave 1-(pyrazin-2-yl)-1,2,3,4-tetrahydroquinoxaline.

Yellow oil (yield: 88%); ¹H NMR (400 MHz, CDCl₃) δ 8.60 (d, *J =* 1.6 Hz, 1H), 8.13 (dd, *J* = 2.7, 1.6 Hz, 1H), 7.92 (d, *J* = 2.7 Hz, 1H), 7.26-7.21 (m, 1H), 6.99-6.89 (m, 1H), 6.72-6.63 (m, 2H), 4.11-4.01 (m, 3H), 3.48-3.40 (m, 2H); LC/MS ESI (+): 230.1 (M+1).

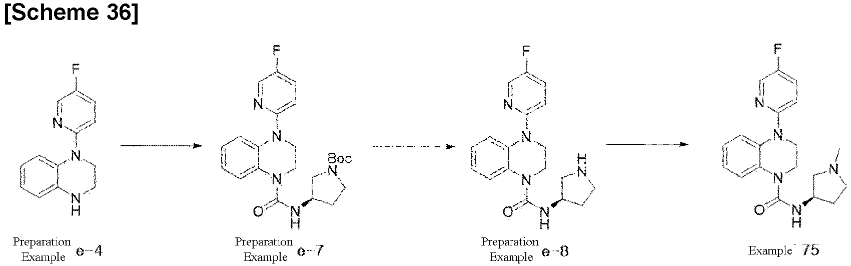

### <Preparation Example e-7> Preparation of tert-butyl (R)-3-(4-(5-fluoropyridin-2-yl)-1,2,3,4-tetrahydroquinoxaline-1-carboxamido)pyrrolidine-1-carboxylate

To a solution of **Preparation Example e-4** (1.0 equiv.), TEA (3.0 equiv.) and DCM (0.3 M) cooled to 0 °C under a nitrogen atmosphere was added dropwise triphosgene (0.6 equiv.). The reaction mixture was stirred for 1 h then TEA (2.0 equiv.) and (R)-(+)-1-Boc-3-aminopyrrolidine (1.2 equiv.) were added slowly. The mixture warmed to room temperature and stirred for 3 h. The resulting mixture was partitioned between DCM and water. The organic layer was washed with brine, dried with MgSO₄, filtered and then concentrated under reduced pressure. The residue was purified by silica column chromatography (with *n*-hexane/EtOAc) to give *tert-butyl (R)*-3-(4-(5-fluoropyridin-2-yl)-1,2,3,4-tetrahydroquinoxaline-1-carboxamido)pyrrolidine-1-carboxylate.

Light yellow solid (yield: 95.5%); ¹H NMR (400 MHz, CDCl₃) δ 8.18 (d, *J =* 3.0 Hz, 1H), 7.39-7.28 (m, 2H), 7.26-7.13 (m, 2H), 7.10-7.02 (m, 1H), 6.99-6.90 (m, 1H), 5.27-5.19 (m, 1H), 4.49-4.31 (m, 1H), 4.01-3.87 (m, 4H), 3.68-3.59 (m, 1H), 3.47-3.33 (m, 2H), 3.22-3.08 (m, 1H), 2.22-2.09 (m, 1H), 1.89-1.70 (m, 1H), 1.45 (s, 9H); LC/MS ESI (+): 442.2 (M+1).

### <Preparation Example e-8> Preparation of (R)-4-(5-fluoropyridin-2-yl)-N-(pyrrolidin-3-yl)-3,4-dihydroquinoxaline-1(2H)-carboxamide

Following the procedure as described for Example 1, reaction of **Preparation Example e-7** (1.0 equiv.) in DCM (0.5 M) with TFA (10.0 equiv.) gave (*R*)-4-(5-fluoropyridin-2-yl)-*N*-(pyrrolidin-3-yl)-3,4-dihydroquinoxaline-1(*2H*)-carboxamide.

Light yellow solid (yield: 68.9%); ¹H NMR (400 MHz, CDCl₃) δ 8.17 (d, *J* = 3.0 Hz, 1H), 7.38-7.28 (m, 3H), 7.16 (dd, *J* = 9.1, 3.6 Hz, 1H), 7.09-7.00 (m, 1H), 6.98-6.91 (m, 1H), 5.31 (d, *J* = 6.9 Hz, 1H), 4.37-4.25 (m, 1H), 4.02-3.88 (m, 4H), 3.16 (dd, *J* = 11.3, 6.5 Hz, 1H), 3.06-2.96 (m, 1H), 2.95-2.85 (m, 1H), 2.77 (dd, *J* = 11.3, 4.0 Hz, 1H), 2.20-2.09 (m, 1H), 2.01 (s, 1H), 1.63-1.53 (m, 1H); LC/MS ESI (+): 342.2 (M+1).

### <Example 75> Synthesis of (R)-4-(5-fluoropyridin-2-yl)-N-(1-methylpyrrolidin-3-yl)-3,4-dihydroquinoxaline-1(2H)-carboxamide

Following the procedure as described for Example 2, reaction of **Preparation Example e-8** (1.0 equiv.) in DCM (0.3 M) with sodium triacetoxyborohydride (1.5 equiv.) and formaldehyde (1.1 equiv.) gave (*R*)-4-(5-fluoropyridin-2-yl)-*N*-(1-methylpyrrolidin-3-yl)-3,4-dihydroquinoxaline-1(2*H*)-carboxamide.

Light yellow oil (yield: 85.6%); ¹H NMR (400 MHz, CDCl₃) δ 8.17 (d, *J* = 3.0 Hz, 1H), 7.36-7.31 (m, 2H), 7.31-7.27 (m, 1H), 7.20-7.14 (m, 1H), 7.06-7.00 (m, 1H), 6.98-6.92 (m, 1H), 5.44 (d, *J* = 7.7 Hz, 1H), 4.46-4.31 (m, 1H), 4.03-3.82 (m, 4H), 2.83-2.74 (m, 1H), 2.61-2.48 (m, 2H), 2.37-2.26 (m, 4H), 2.25-2.14 (m, 1H), 1.64-1.51 (m, 1H); LC/MS ESI (+): 356.2 (M+1).

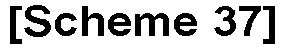

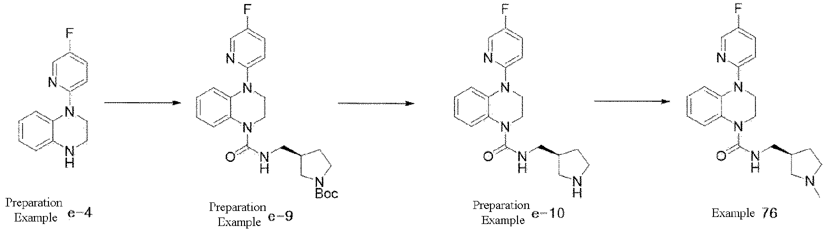

### <Preparation Example e-9> Preparation of tert-butyl (R)-3-((4-(5-fluoropyridin-2-yl)-1,2,3,4-tetrahydroquinoxaline-1-carboxamido)methyl)pyrrolidine-1-carboxylate

The title compound was prepared in the same manner as Preparation Example e-7, using (*R*)-1-Boc-3-(aminomethyl)pyrrolidine in place of (*R*)-(+)-1-Boc-3-aminopyrrolidine to give *tert-butyl* (*R*)-3-((4-(5-fluoropyridin-2-yl)-1,2,3,4-tetrahydroquinoxaline-1-carboxamido)methyl)pyrrolidine-1-carboxylate.

White solid (yield: 87.5%); ¹H NMR (400 MHz, CDCl₃) δ 8.18 (d, *J* = 3.0 Hz, 1H), 7.40-7.27 (m, 3H), 7.20-7.03 (m, 2H), 6.99-6.93 (m, 1H), 5.33-5.28 (m, 1H), 4.00-3.90 (m, 4H), 3.55-3.32 (m, 2H), 3.31-3.21 (m, 2H), 3.20-3.09 (m, 1H), 3.06-2.92 (m, 1H), 2.44-2.37 (m, 1H), 2.01-1.89 (m, 1H), 1.64 (s, 1H), 1.45 (s, 9H); LC/MS ESI (+): 456.2 (M+1).

### <Preparation Example e-10> Preparation of (S)-4-(5-fluoropyridin-2-yl)-N-(1-pyrrolidin-3-yl)methyl)-3,4-dihydroquinoxaline-1(2H)-carboxamide

Following the procedure as described for Example 1, reaction of **Preparation Example e-9** (1.0 equiv.) in DCM (0.5 M) with TFA (10.0 equiv.) gave (S)-4-(5-fluoropyridin-2-yl)-*N*-(1-pyrrolidin-3-yl)methyl)-3,4-dihydroquinoxaline-1(*2H*)-carboxamide.

Light yellow solid (yield: 83.1%); ¹H NMR (400 MHz, CDCl₃) δ 8.17 (d, *J* = 3.0 Hz, 1H), 7.38-7.27 (m, 3H), 7.18-7.12 (m, 1H), 7.08-7.02 (m, 1H), 6.98-6.93 (m, 1H), 5.49 (t, *J =* 5.5 Hz, 1H), 3.97-3.92 (m, 4H), 3.31-3.20 (m, 2H), 3.04-2.97 (m, 2H), 2.93-2.84 (m, 1H), 2.70-2.61 (m, 1H), 2.37-2.29 (m, 2H), 1.96-1.83 (m, 1H), 1.52-1.37 (m, 1H); LC/MS ESI (+): 356.2 (M+1).

### <Example 76> Synthesis of (R)-4-(5-fluoropyridin-2-yl)-N-(1-methylpyrrolidin-3-yl)methyl)-3,4-dihydroquinoxaline-1(2H)-carboxamide

Following the procedure as described for Example 2, reaction of **Preparation Example e-10** (1.0 equiv.) in DCM (0.3 M) with sodium triacetoxyborohydride (1.5 equiv.) and formaldehyde (1.1 equiv.) gave (R)-4-(5-fluoropyridin-2-yl)-N-(1-methylpyrrolidin-3-yl)methyl)-3,4-dihydroquinoxaline-1(2h)-carboxamide.

Light yellow oil (yield: 83.7%); ¹H NMR (400 MHz, CDCl₃) δ 8.18 (d, *J* = 3.0 Hz, 1H), 7.37-7.31 (m, 2H), 7.30-7.27 (m, 1H), 7.18-7.10 (m, 1H), 7.07-7.00 (m, 1H), 6.99-6.91 (m, 1H), 6.06-6.02 (m, 1H), 4.07-3.77 (m, 4H), 3.33-3.18 (m, 2H), 2.67-2.57 (m, 1H), 2.48-2.34 (m, 3H), 2.34-2.24 (m, 1H), 2.04-1.91 (m, 1H), 1.57-1.47 (m, 1H); LC/MS ESI (+): 370.3 (M+1).

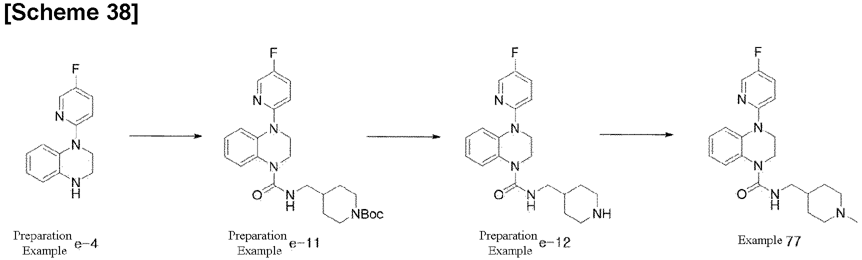

### <Preparation Example e-11> Preparation of tert-butyl 4-((4-(5-fluoropyridin-2-yl)-1,2,3,4-tetrahydroquinoxaline-1-carboxamido)methyl)piperidine-1-carboxylate

The title compound was prepared in the same manner as Preparation Example e-7, using 1-Boc-4-(aminomethyl)piperidine in place of (*R*)-(+)-1-Boc-3-aminopyrrolidine to give *tert-butyl* 4-((4-(5-fluoropyridin-2-yl)-1,2,3,4-tetrahydroquinoxaline-1-carboxamido)methyl)piperidine-1-carboxylate.

White solid (yield: 88.4%); ¹H NMR (400 MHz, CDCl₃) δ 8.18 (d, *J* = 3.0 Hz, 1H), 7.40-7.27 (m, 3H), 7.18-7.11 (m, 1H), 7.07 (ddd, *J* = 8.2, 7.3, 1.6 Hz, 1H), 7.00-6.94 (m, H), 5.32-5.25 (m, 1H), 4.16-4.03 (m, 2H), 3.97-3.92 (m, 4H), 3.20-3.06 (m, 2H), 2.75-2.55 (m, 2H), 1.68-1.62 (m, 3H), 1.44 (s, 9H), 1.18-1.02 (m, 2H); LC/MS ESI (+): 470.2 (M+1).

### <Preparation Example e-12> Preparation of 4-(5-fluoropyridin-2-yl)-N-(1-piperidin-4-ylmethyl)-3,4-dihydroquinoxaline-1(2H)-carboxamide

Following the procedure as described for Example 1, reaction of **Preparation Example e-11** (1.0 equiv.) in DCM (0.5 M) with TFA (10.0 equiv.) gave 4-(5-fluoropyridin-2-yl)-*N*-(1-piperidin-4-ylmethyl)-3,4-dihydroquinoxaline-1(*2H*)-carboxamide.

Light yellow solid (yield: 78.8%); ¹H NMR (400 MHz, CDCl₃) δ 8.17 (d, *J =* 3.0 Hz, 1H), 7.39-7.27 (m, 3H), 7.19-7.11 (m, 1H), 7.10-7.03 (m, 1H), 7.00-6.93 (m, 1H), 5.32-5.24 (m, 1H), 3.97-3.92 (m, 4H), 3.17-3.04 (m, 4H), 2.64-2.50 (m, 2H), 2.15-2.10 (m, 1H), 1.73-1.54 (m, 3H), 1.23-1.07 (m, 2H); LC/MS ESI (+): 370.2 (M+1).

### <Example 77> Synthesis of 4-(5-fluoropyridin-2-yl)-N-(1-methylpiperidin-4-yl)methyl)-3,4-dihydroquinoxaline-1(2H)-carboxamide

Following the procedure as described for Example 2, reaction of **Preparation Example e-12** (1.0 equiv.) in DCM (0.3 M) with sodium triacetoxyborohydride (1.5 equiv.) and formaldehyde (1.1 equiv.) gave 4-(5-fluoropyridin-2-yl)-*N*-(1-methylpiperidin-4-yl)methyl)-3,4-dihydroquinoxaline-1(2*H*)-carboxamide.

White solid (yield: 88.5%); ¹H NMR (400 MHz, CDCl₃) δ 8.19-8.17 (m, 1H), 7.36 (dd, *J* = 8.2, 1.4 Hz, 1H), 7.34-7.27 (m, 2H), 7.18-7.12 (m, 1H), 7.09-7.03 (m, 1H), 7.00-6.93 (m, 1H), 5.31-5.21 (m, 1H), 3.97-3.92 (m, 4H), 3.19-3.11 (m, 2H), 2.87-2.78 (m, 2H), 2.25 (s, 3H), 1.94-1.83 (m, 2H), 1.70-1.66 (m, 2H), 1.55-1.41 (m, 1H), 1.33-1.19 (m, 2H); LC/MS ESI (+): 384.3 (M+1).

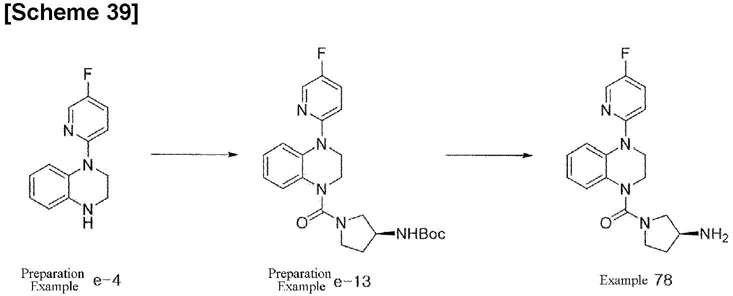

### <Preparation Example e-13> Preparation of tert-butyl (S)-(1-(4-(5-fluoropyridin-2-yl)-1,2,3,4-tetrahydroquinoxaline-1-carbonyl)pyrrolidin-3-yl)carbamate

The title compound was prepared in the same manner as Preparation Example e-7, using (*S*)-(-)-3-(Boc-amino)pyrrolidine in place of (*R*)-(+)-1-Boc-3-aminopyrrolidine to give *tert-butyl* (*S*)-(1-(4-(5-fluoropyridin-2-yl)-1,2,3,4-tetrahydroquinoxaline-1-carbonyl)pyrrolidin-3-yl)carbamate.

White solid (yield: 98.4%); ¹H NMR (400 MHz, CDCl₃) δ 8.15 (d, *J* = 3.0 Hz, 1H), 7.36-7.29 (m, 1H), 7.25-7.21 (m, 1H), 7.18-7.11 (m, 1H), 7.06-6.98 (m, 1H), 6.97-6.90 (m, 2H), 4.20-4.06 (m, 1H), 4.05-3.96 (m, 2H), 3.91-3.77 (m, 2H), 3.58-3.29 (m, 4H), 3.14-3.05 (m, 1H), 2.13-2.06 (m, 1H), 1.82-1.73 (m, 1H), 1.39 (s, 9H); LC/MS ESI (+): 456.3 (M+1).

### <Example 78> Synthesis of (S)-(3-aminopyrrolidin-1-yl)(4-(5-fluoropyridin-2-yl)-3,4-dihydroquinoxaline-1(2H)-methanone

Following the procedure as described for Example 1, reaction of **Preparation Example e-13** (1.0 equiv.) in DCM (0.5 M) with TFA (10.0 equiv.) gave (S)-(3-aminopyrrolidin-1-yl)(4-(5-fluoropyridin-2-yl)-3,4-dihydroquinoxaline-1(*2H*)-methanone.

White solid (yield: 71.5%); ¹H NMR (400 MHz, CDCl₃) δ 8.15 (d, *J* = 3.0 Hz, 1H), 7.36-7.28 (m, 1H), 7.26-7.20 (m, 1H), 7.14 (dd, *J* = 9.2, 3.6 Hz, 1H), 7.07-7.01 (m, 1H), 6.97-6.90 (m, 2H), 4.01 (t, *J* = 6.3 Hz, 2H), 3.92-3.75 (m, 2H), 3.58-3.31 (m, 4H), 3.00 (dd, *J* = 10.3, 4.5 Hz, 1H), 2.07-1.97 (m, 1H), 1.68-1.55 (m, 1H), 1.33-1.27 (m, 2H); LC/MS ESI (+): 342.2 (M+1).

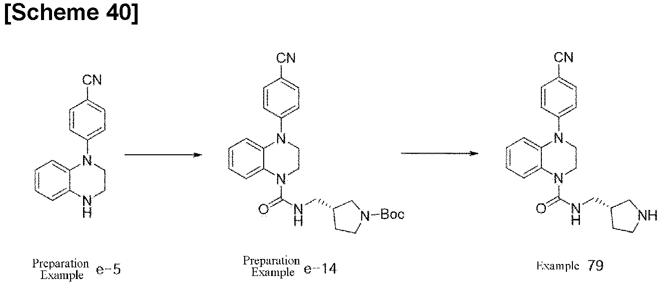

### <Preparation Example e-14> Preparation of tert-butyl (R)-3-((4-(4-cyanophenyl)-1,2,3,4-tetrahydroquinoxaline-1-carboxamido)methyl)pyrrolidine-1-carboxylate

To a solution of **Preparation Example e-5** (1.0 equiv.), TEA (3.0 equiv.) and DCM (0.3 M) cooled to 0 °C under a nitrogen atmosphere was added dropwise triphosgene (0.6 equiv.). The reaction mixture was stirred for 1 h then TEA (2.0 equiv.) and (R)-1-Boc-3-(aminomethyl)pyrrolidine (1.2 equiv.) were added slowly. The mixture warmed to room temperature and stirred for 3 h. The resulting mixture was partitioned between DCM and water. The organic layer was washed with brine, dried with MgSO₄, filtered and then concentrated under reduced pressure. The residue was purified by silica column chromatography (with DCM/MeOH) to give *tert-butyl* (R)-3-((4-(4-cyanophenyl)-1,2,3,4-tetrahydroquinoxaline-1-carboxamido)methyl)pyrrolidine-1-carboxylate.

Ivory solid (yield: 91%); ¹H NMR (400 MHz, CD₃OD) δ 7.63-7.54 (m, 2H), 7.39 (d, *J* = 8.0 Hz, 1H), 7.35-7.27 (m, 2H), 7.22 (dd, *J* = 7.9, 1.7 Hz, 1H), 7.12-6.98 (m, 2H), 3.93-3.78 (m, 4H), 3.44-3.32 (m, 2H), 3.29-3.10 (m, 3H), 3.05-2.95 (m, 1H), 2.46-2.34 (m, 1H), 1.99-1.86 (m, 1H), 1.69-1.54 (m, 1H), 1.42 (d, *J =* 5.2 Hz, 9H).

### <Example 79> Synthesis of (S)-4-(4-cyanophenyl)-N-(pyrrolidin-3-ylmethyl)-3,4-dihydroquinoxaline-1(2H)-carboxamide

Following the procedure as described for Example 1, reaction of **Preparation Example e-14** (1.0 equiv.) in DCM (0.5 M) with TFA (10.0 equiv.) gave (S)-4-(4-cyanophenyl)-*N*-(pyrrolidin-3-ylmethyl)-3,4-dihydroquinoxaline-1(*2H*)-carboxamide.

Ivory solid (yield: 17%); ¹H NMR (400 MHz, CD₃OD) δ 7.67-7.56 (m, 2H), 7.44-7.36 (m, 1H), 7.35-7.28 (m, 2H), 7.26-7.20 (m, 1H), 7.12-6.99 (m, 2H), 3.94-3.79 (m, 4H), 3.24-3.08 (m, 2H), 2.99-2.78 (m, 3H), 2.58 (dd, *J* = 11.2, 6.5 Hz, 1H), 2.43-2.27 (m, 1H), 1.94-1.81 (m, 1H), 1.52-1.39 (m, 1H); LC/MS ESI (+): 362.1 (M+1).

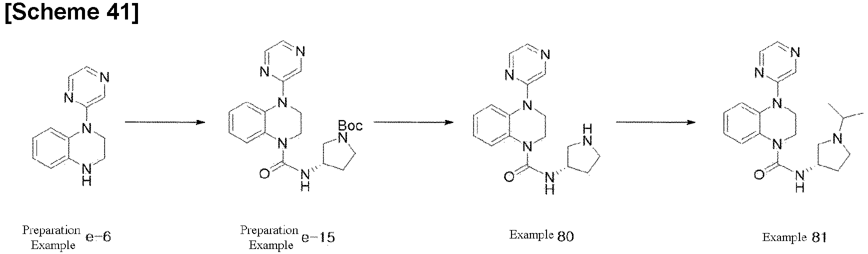

### <Preparation Example e-15> Preparation of tert-butyl (S)-3-(4-(pyrazin-2-yl)-1,2,3,4-tetrahydroquinoxaline-1-carboxamido)pyrrolidine-1-carboxylate

To a solution of Preparation **Example e-6** (1.0 equiv.), TEA (3.0 equiv.) and DCM (0.3 M) cooled to 0 °C under a nitrogen atmosphere was added dropwise triphosgene (0.6 equiv.). The reaction mixture was stirred for 1 h then TEA (2.0 equiv.) and (S)-(-)-1-Boc-3-aminopyrrolidine (1.2 equiv.) were added slowly. The mixture warmed to room temperature and stirred for 3 h. The resulting mixture was partitioned between DCM and water. The organic layer was washed with brine, dried with MgSO₄, filtered and then concentrated under reduced pressure. The residue was purified by silica column chromatography (with *n*-hexane/EtOAc) to give *tert-butyl* (*S*)-3-(4-(pyrazin-2-yl)-1,2,3,4-tetrahydroquinoxaline-1-carboxamido)pyrrolidine-1-carboxylate.

Light yellow solid (yield: 87.6%); ¹H NMR (400 MHz, CDCl₃) δ 8.61 (d, *J* = 1.5 Hz, 1H), 8.21 (dd, *J* = 2.7, 1.5 Hz, 1H) 8.05 (d, *J* = 2.7 Hz, 1H), 7.54 (dd, *J* = 8.1, 1.5 Hz, 1H), 7.32 (dd, *J* = 7.9, 1.6 Hz, 1H), 7.19-7.02 (m, 2H), 5.18 (d, *J =* 6.7 Hz, 1H), 4.40 (s, 1H), 4.07-3.91 (m, 4H), 3.65 (t, *J* = 9.4 Hz, 1H), 3.47-3.32 (m, 2H), 3.24-3.11 (m, 1H), 2.23-2.07 (m, 1H), 1.80 (s, 1H), 1.45 (s, 9H); LC/MS ESI (+): 425.2 (M+1).

### <Example 80> Synthesis of (S)-4-(pyrazin-2-yl)-N-(pyrrolidin-3-yl)-3,4-dihydroquinoxaline-1(2H)-carboxamide

Following the procedure as described for Example 1, reaction of **Preparation Example e-15** (1.0 equiv.) in DCM (0.5 M) with TFA (10.0 equiv.) gave (*S*)-4-(pyrazin-2-yl)-*N*-(pyrrolidin-3-yl)-3,4-dihydroquinoxaline-1(*2H*)-carboxamide.

Ivory solid (yield: 76.9%); ¹H NMR (400 MHz, CDCl₃) δ 8.60 (d, *J =* 1.5 Hz, 1H), 8.20 (dd, *J* = 2.7, 1.5 Hz, 1H), 8.03 (d, *J*= 2.7 Hz, 1H), 7.51 (dd, *J* = 7.9, 1.6 Hz, 1H), 7.37 (dd, *J* = 7.8, 1.7 Hz, 1H), 7.16-7.01 (m, 2H), 5.29 (d, *J =* 6.9 Hz, 1H), 4.38-4.26 (m, 1H), 4.05-4.00 (m, 2H), 4.00-3.91 (m, 2H), 3.20-3.12 (m, 1H), 3.07-2.97 (m, 1H), 2.96-2.85 (m, 1H), 2.82-2.74 (m, 1H), 2.24-2.08 (m, 1H), 2.01-1.98 (m, 1H), 1.65-1.52 (m, 1H); LC/MS ESI (+): 325.1 (M+1).

### <Example 81> Synthesis of (S)-N-(1-isopropylpyrrolidin-3-yl)-4-(pyrazin-2-yl)-3,4-dihydroquinoxaline-1(2H)-carboxamide

Following the procedure as described for Example 2, reaction of **Example 80** (1.0 equiv.) in DCM (0.3 M) with sodium triacetoxyborohydride (1.5 equiv.) and acetone (2.0 equiv.) gave (*S*)-*N*-(1-isopropylpyrrolidin-3-yl)-4-(pyrazin-2-yl)-3,4-dihydroquinoxaline-1(*2H*)-carboxamide.

Yellow solid (yield: 64.6%); ¹H NMR (400 MHz, CH₃CN) δ 8.62 (d, *J =* 1.5 Hz, 1H), 8.20 (dd, *J* = 2.7, 1.5 Hz, 1H), 8.03 (d, *J* = 2.7 Hz, 1H), 7.50 (dd, *J* = 7.9, 1.6 Hz, 1H), 7.39 (dd, *J* = 7.7, 1.8 Hz, 1H), 7.15-7.01 (m, 2H), 5.43 (d, *J =* 7.6 Hz, 1H), 4.44-4.33 (m, 1H), 4.05-3.90 (m, 4H), 2.90-2.79 (m, 1H), 2.73-2.60 (m, 2H), 2.39-2.21 (m, 3H), 1.65-1.51 (m, 1H), 1.09-1.01 (m, 6H); LC/MS ESI (+): 367.2 (M+1).

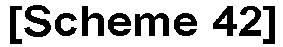

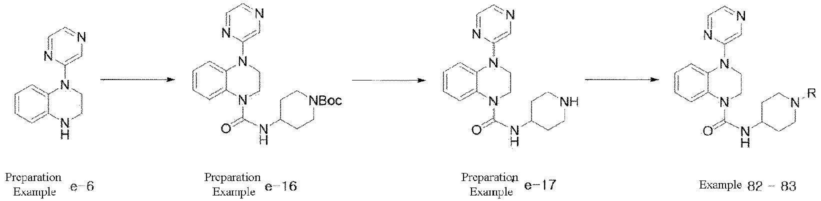

### <Preparation Example e-16> Preparation of tert-butyl (4-(pyrazin-2-yl)-1,2,3,4-tetrahydroquinoxaline-1-carboxamido)piperidine-1-carboxylate

The title compound was prepared in the same manner as Preparation Example e-15, using 1-Boc-4-aminopiperidine in place of (*S*)-(-)-1-Boc-3-aminopyrrolidine to give *tert-butyl* (4-(pyrazin-2-yl)-1,2,3,4-tetrahydroquinoxaline-1-carboxamido)piperidine-1-carboxylate.

Light yellow solid (yield: 85.3%); ¹H NMR (400 MHz, CDCl₃) δ 8.59 (d, *J* = 1.5 Hz, 1H), 8.21 (dd, *J* = 2.7, 1.5 Hz, 1H), 8.04 (d, *J* = 2.7 Hz, 1H), 7.53 (dd, *J* = 8.1, 1.5 Hz, 1H), 7.34 (dd, *J* = 7.8, 1.6 Hz, 1H), 7.17-7.02 (m, 2H), 5.03 (d, *J* = 7.6 Hz, 1H), 4.17-3.93 (m, 6H), 3.92-3.78 (m, 1H), 2.87 (t, *J* = 12.9 Hz, 1H), 1.99-1.88 (m, 2H), 1.44 (s, 9H), 1.35-1.21 (m, 2H); LC/MS ESI (+): 425.2.

### <Preparation Example e-17> Preparation of N-(1-piperidin-4-yl)-4-(pyrazin-2-yl)-3,4-dihydroquinoxaline-1(2H)-carboxamide

Following the procedure as described for Example 1, reaction of **Preparation Example e-16** (1.0 equiv.) in DCM (0.5 M) with TFA (10.0 equiv.) gave *N*-(1-piperidin-4-yl)-4-(pyrazin-2-yl)-3,4-dihydroquinoxaline-1(*2H*)-carboxamide.

Light yellow solid (yield: 84.0 %); ¹H NMR (400 MHz, CDCl₃) δ 8.60 (dd, *J* = 1.5, 0.4 Hz, 1H), 8.20 (dd, *J* = 2.7, 1.5 Hz, 1H), 8.04 (dd, *J* = 2.7, 0.4 Hz, 1H), 7.56-7.48 (m, 1H), 7.40-7.32 (m, 1H), 7.17-7.02 (m, 2H), 5.06 (d, *J* = 7.7 Hz, 1H), 4.06-3.92 (m, 4H), 3.87-3.73 (m, 1H), 3.09-2.99 (m, 2H), 2.75-2.63 (m, 2H), 2.02-1.92 (m, 2H), 1.67-1.64 (m, 1H), 1.36-1.22 (m, 2H); LC/MS ESI (+): 339.2 (M+1).

### <Example 82> Synthesis of N-(1-isobutylpiperidin-4-yl)-4-(pyrazin-2-yl)-3,4-dihydroquinoxaline-1(2H)-carboxamide

Following the procedure as described for Example 2, reaction of **Preparation Example e-17** (1.0 equiv.) in DCM (0.3 M) with sodium triacetoxyborohydride (1.5 equiv.) and isobutyraldehyde (2.0 equiv.) gave *N*-(1-isobutylpiperidin-4-yl)-4-(pyrazin-2-yl)-3,4-dihydroquinoxaline-1(2*H*)-carboxamide.

Light yellow solid (yield: 81.8%) ¹H NMR (400 MHz, CDCl₃) δ 8.60 (d, *J =* 1.5 Hz, 1H), 8.20 (dd, *J* = 2.6, 1.5 Hz, 1H), 8.03 (d, *J* = 2.6 Hz, 1H), 7.52 (dd, *J* = 8.0, 1.5 Hz, 1H), 7.36 (dd, *J* = 7.9, 1.6 Hz, 1H), 7.16-7.02 (m, 2H), 5.05 (d, *J* = 7.7 Hz, 1H), 4.06-4.00 (m, 2H), 4.00-3.93 (m, 2H), 3.78-3.64 (m, 1H), 2.72 (d, *J =* 10.9 Hz, 2H), 2.09-1.97 (m, 4H), 1.96-1.86 (m, 2H), 1.80-1.66 (m, 1H), 1.50-1.35 (m, 2H), 0.86 (d, *J* = 6.5 Hz, 6H); LC/MS ESI (+): 395.3 (M+1).

### <Example 83> Synthesis of N-(1-isopropylpiperidin-4-yl)-4-(pyrazin-2-yl)-3,4-dihydroquinoxaline-1(2H)-carboxamide

The title compound was synthesized in the same manner as Example 82, using acetone in place of isobutyraldehyde to give *N*-(1-isopropylpiperidin-4-yl)-4-(pyrazin-2-yl)-3,4-dihydroquinoxaline-1(2*H*)-carboxamide.

Light yellow solid (yield: 81.6%); ¹H NMR (400 MHz, CDCl₃) δ 8.59 (d, *J* = 1.6 Hz, 1H), 8.20 (dd, *J* = 2.6, 1.5 Hz, 1H), 8.03 (d, *J* = 2.6 Hz, 1H), 7.52 (dd, *J* = 8.1, 1.4 Hz, 1H), 7.35 (dd, *J* = 7.9, 1.6 Hz, 1H), 7.15-7.00 (m, 2H), 5.05 (d, *J* = 7.7 Hz, 1H), 4.06-3.92 (m, 4H), 3.77-3.63 (m, 1H), 2.78 (d, *J* = 11.9 Hz, 2H), 2.74-2.63 (m, 1H), 2.25 (t, *J* = 2.6 Hz, 2H), 2.04-1.94 (m, 2H), 1.48-1.34 (m, 2H), 1.02 (d, *J* = 6.6 Hz, 6H); LC/MS ESI (+): 381.3 (M+1).

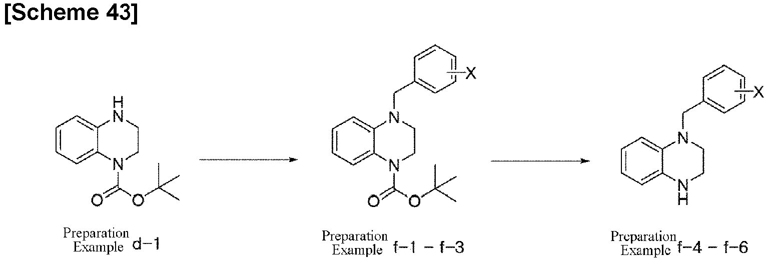

### <Preparation Process f> Preparation of benzyl- or halogen-substituted benzyl-1,2,3,4-tetrahydroquinoxaline

Benzyl- or halogen-substituted benzyl-1,2,3,4-tetrahydroquinoxaline according to the present invention was prepared through Preparation Examples f-1 to f-6 below.

### <Preparation Example f-1> Preparation of tert-butyl 4-(2-fluorobenzyl)-3,4-dihydroquinoxaline-1(2H)-carboxylate

The title compound was prepared in the same manner as Preparation Example d-2, using 2-fluorobenzyl in place of benzyl bromide to give *tert-butyl* 4-(2-fluorobenzyl)-3,4-dihydroquinoxaline-1(2*H*)-carboxylate.

Yellow oil (yield: 90%); ¹H NMR (400 MHz, CDCl₃) δ 7.57-7.41 (m, 1H), 7.32-7.17 (m, 2H), 7.16-7.05 (m, 2H), 6.98-6.90 (m, 1H), 6.72-6.58 (m, 2H), 4.59 (s, 2H), 3.91-3.84 (m, 2H), 3.49 (dd, *J* = 5.7, 4.5 Hz, 2H), 1.55 (s, 9H).

### <Preparation Example f-2> Preparation of tert-butyl 4-(4-fluorobenzyl)-3,4-dihydroquinoxaline-1(2H)-carboxylate

The title compound was prepared in the same manner as Preparation Example d-2, using 4-fluorobenzyl in place of benzyl bromide to give *tert-butyl* 4-(4-fluorobenzyl)-3,4-dihydroquinoxaline-1(2*H*)-carboxylate.

Yellow oil (yield: 95%); ¹H NMR (400 MHz, CDCl₃) δ 7.53-7.45 (m, 1H), 7.28-7.18 (m, 2H), 7.09-6.99 (m, 2H), 7.03-6.90 (m, 1H), 6.73-6.59 (m, 2H), 4.51 (d, *J* = 1.3 Hz, 2H), 3.89-3.82 (m, 2H), 3.51-3.40 (m, 2H), 1.55 (s, 9H).

### <Preparation Example f-3> Preparation of tert-butyl 4-(4-chlorobenzyl)-3,4-dihydroquinoxaline-1(2H)-carboxylate

The title compound was prepared in the same manner as Preparation Example d-2, using 4-chlorobenzyl in place of benzyl bromide to give *tert*-butyl 4-(3-chlorobenzyl)-3,4-dihydroquinoxaline-1(2H)-carboxylate.

Light yellow oil (yield: 100%); ¹H NMR (400 MHz, CDCl₃) δ 7.48 (d, *J* = 8.1 Hz, 1H), 7.31-7.27 (m, 2H), 7.21-7.15 (m, 2H), 6.91 (ddd, *J* = 8.1, 7.3, 1.5 Hz, 1H), 6.66 (ddd, *J* = 8.4, 7.2, 1.3 Hz, 1H), 6.58 (dd, *J* = 8.3, 1.3 Hz, 1H), 4.48 (s, 2H), 3.87-3.80 (m, 2H), 3.45-3.38 (m, 2H), 1.53 (s, 9H).

### <Preparation Example f-4> Preparation of 1-(2-fluorobenzyl)-1,2,3,4-tetrahydroquinoxaline

Following the procedure as described for Example 1, reaction of **Preparation Example f-1** (1.0 equiv.) in DCM (0.5 M) with TFA (10.0 equiv.) gave 1-(2-fluorobenzyl)-1,2,3,4-tetrahydroquinoxaline.

Gray solid (yield: 96%); ¹H NMR (400 MHz, CD₃OD) δ 7.37-7.22 (m, 2H), 7.15-7.06 (m, 2H), 6.61-6.44 (m, 4H), 4.50 (s, 2H), 3.47-3.34 (m, 4H); LC/MS ESI (+): 243.1 (M).

### <Preparation Example f-5> Preparation of 1-(4-fluorobenzyl)-1,2,3,4-tetrahydroquinoxaline

Following the procedure as described for Example 1, reaction of **Preparation Example f-2** (1.0 equiv.) in DCM (0.5 M) with TFA (10.0 equiv.) gave 1-(2-fluorobenzyl)-1,2,3,4-tetrahydroquinoxaline.

Light brown solid (yield: 93%); ¹H NMR (400 MHz, CD₃OD) δ 7.37-7.28 (m, 2H), 7.10-6.99 (m, 2H), 6.60-6.45 (m, 4H), 4.42 (s, 2H), 3.42-3.33 (m, 4H); LC/MS ESI (+): 243.1 (M).

### <Preparation Example f-6> Preparation of 1-(4-chlorobenzyl)-1,2,3,4-tetrahydroquinoxaline

Following the procedure as described for Example 1, reaction of **Preparation Example f-3** (1.0 equiv.) in DCM (0.5 M) with TFA (10.0 equiv.) gave 1-(4-chlorobenzyl)-1,2,3,4-tetrahydroquinoxaline.

Light brown oil (yield: 94%); ¹H NMR (400 MHz, DMSO-*d₆*) δ 7.42-7.32 (m, 2H), 7.31-7.26 (m, 2H), 6.47-6.32 (m, 4H), 5.48 (s, 1H), 4.38 (s, 2H), 3.30 (d, *J =* 1.5 Hz, 4H); LC/MS ESI (+): 258.0 (M).

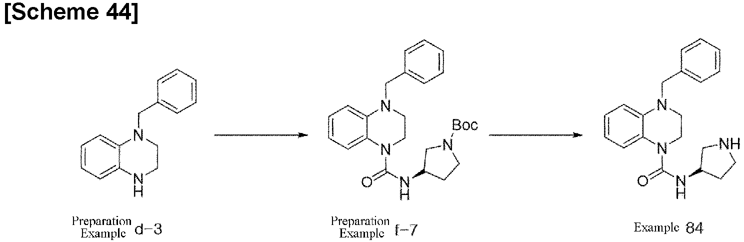

### <Preparation Example f-7> Preparation of tert-butyl (R)-3-(4-benzyl-1,2,3,4-tetrahydroquinoxaline-1-carboxamido)pyrrolidine-1-carboxylate

To a solution of **Preparation Example d-3** (1.0 equiv.), TEA (3.0 equiv.) and DCM (0.3 M) cooled to 0 °C under a nitrogen atmosphere was added dropwise triphosgene (0.6 equiv.). The reaction mixture was stirred for 1 h then TEA (2.0 equiv.) and (R)-(+)-1-Boc-3-aminopyrrolidine (1.2 equiv.) were added slowly. The mixture warmed to room temperature and stirred for 3 h. The resulting mixture was partitioned between DCM and water. The organic layer was washed with brine, dried with MgSO₄, filtered and then concentrated under reduced pressure. The residue was purified by silica column chromatography (with n-hexane/EtOAC) to give *tert-butyl* (R)-3-(4-benzyl-1,2,3,4-tetrahydroquinoxaline-1-carboxamido)pyrrolidine-1-carboxylate.

Brown solid (yield: 100%); ¹H NMR (400 MHz, CDCl₃) δ 7.36-7.27 (m, 2H), 7.25-7.17 (m, 2H), 7.08 (d, *J* = 7.8 Hz, 1H), 7.00-6.94 (m, 1H), 6.71-6.59 (m, 2H), 5.34 (d, *J* = 6.9 Hz, 1H), 5.08-5.03 (m, 2H), 4.56 (s, 2H), 4.49-4.35 (m, 1H), 3.95-3.75 (m, 2H), 3.73-3.52 (m, 1H), 3.46-3.31 (m, 2H), 3.20-3.04 (m, 1H), 2.24-2.02 (m, 1H), 1.94-1.64 (m, 1H), 1.51-1.48 (m, 1H), 1.45 (s, 9H).

### <Example 84> Synthesis of (R)-4-benzyl-N-(pyrrolidin-3-yl)-3,4-dihydroquinoxaline-1(2H)-carboxamide

Following the procedure as described for Example 1, reaction of Preparation **Example f-7** (1.0 equiv.) in DCM (0.5 M) with TFA (10.0 equiv.) gave (R)-4-benzyl-N-(pyrrolidin-3-yl)-3,4-dihydroquinoxaline-1(*2H*)-carboxamide.

Yellow solid (yield: 49%); ¹H NMR (400 MHz, CDCl₃) δ 7.36-7.27 (m, 3H), 7.25-7.18 (m, 3H), 7.15-7.09 (m, 1H), 6.97 (ddd, *J* = 8.6, 7.3, 1.6 Hz, 1H), 6.70-6.61 (m, 2H), 5.41 (d, *J = 6.8* Hz, 1H), 4.55 (s, 2H), 4.37-4.25 (m, 1H), 3.94-3.78 (m, 2H), 3.47-3.40 (m, 2H), 3.23-3.14 (m, 1H), 3.07-2.88 (m, 2H), 2.80-2.72 (m, 1H), 2.25-2.08 (m, 1H), 1.64-1.51 (m, 1H); LC/MS ESI (+): 337.1 (M+1).

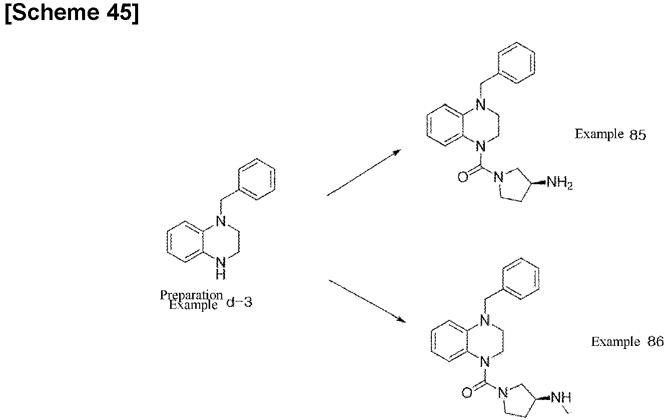

### <Example 85> Synthesis of (S)-(3-aminopyrrolidin-1-yl)(4-benzyl-3,4-dihydroquinoxalin-1(2H)-yl)methanone

To a solution of **Preparation Example d-3** (1.0 equiv.), TEA (3.0 equiv.) and DCM (0.4 M) cooled to 0 °C under a nitrogen atmosphere was added dropwise triphosgene (0.6 equiv.). The reaction mixture was stirred for 1 h then TEA (2.0 equiv.) and (S)-(-)-3-(Boc-amino)pyrrolidine (1.2 equiv.) were added slowly. The mixture warmed to room temperature and stirred for 3 h. The resulting mixture was partitioned between DCM and water. The organic layer was washed with brine, dried with MgSO₄, filtered and then concentrated under reduced pressure. A solution of the obtained concentrate in DCM (0.5 M) was added TFA (10.0 equiv.) The reaction mixture was stirred at room temperature for 24 h. The reaction mixture was diluted with DCM, and the pH was adjusted to 9 with 10M NaOH (aq.). The mixture was extracted with DCM, the organic layer dried with MgSO₄, filtered, and then concentrated under reduced pressure. The residue was purified by silica column chromatography (with *n*-hexane/EtOAc) to give (*S*)-(3-aminopyrrolidin-1-yl)(4-benzyl-3,4-dihydroquinoxalin-1(*2H*)-yl)methanone.

Yellow oil (yield: 23%); ¹H NMR (400 MHz, CD₃OD) δ 7.36-7.15 (m, 5H), 6.90-6.74 (m, 2H), 6.68-6.52 (m, 2H), 4.54 (s, 2H), 3.80-3.59 (m, 2H), 3.57-3.31 (m, 6H), 3.06-2.95 (m, 1H), 2.10-1.95 (m, 1H), 1.73-1.59 (m, 1H); LC/MS ESI (+): 337.0 (M+1).

### <Example 86> Synthesis of (S)-(4-benzyl-3,4-dihydroquinoxalin-1(2H)-yl)(3-(methylamino)pyrrolidin-1-yl)methanone

To a solution of **Preparation Example d-3** (1.0 equiv.), TEA (3.0 equiv.) and DCM (0.4 M) cooled to 0 °C under a nitrogen atmosphere was added dropwise triphosgene (0.6 equiv.). The reaction mixture was stirred for 1 h then TEA (2.0 equiv.) and (S)-(-)-3-(Boc-amino)pyrrolidine (1.2 equiv.) were added slowly. The mixture warmed to room temperature and stirred for 3 h. The resulting mixture was partitioned between DCM and water. The organic layer was washed with brine, dried with MgSO₄, filtered and then concentrated under reduced pressure. To a solution of the obtained concentrate in THF (0.3 M) were added sequentially NaH (1.5 equiv.) and iodomethane (1.0 equiv.). The resulting mixture was stirred at room temperature for 3 h. The reaction mixture was quenched with water and extracted with DCM. The organic layer was washed with brine, dried with MgSO₄, filtered, and then concentrated under reduced pressure. A solution of the obtained concentrate in DCM (0.5 M) was added TFA (10.0 equiv.) The reaction mixture was stirred at room temperature for 24 h. The reaction mixture was diluted with DCM, and the pH was adjusted to 9 with 10M NaOH (aq.). The mixture was extracted with DCM, the organic layer dried with MgSO₄, filtered, and then concentrated under reduced pressure. The residue was purified by silica column chromatography (with *n-*hexane/EtOAc) to give (*S*)-(4-benzyl-3,4-dihydroquinoxalin-1(*2H*)-yl)(3-(methylamino)pyrrolidin-1-yl)methanone.

Yellow oil (yield: 47%); ¹H NMR (400 MHz, CD₃OD) δ 7.35-7.16 (m, 5H), 6.86-6.76 (m, 2H), 6.67-6.52 (m, 2H), 4.55 (s, 2H), 3.80-3.70 (m, 1H), 3.68-3.59 (m, 1H), 3.58-3.40 (m, 4H), 3.39-3.33 (m, 1H), 3.21-3.06 (m, 2H), 2.31 (s, 3H), 2.12-2.01 (m, 1H), 1.76-1.63 (m, 1H); LC/MS ESI (+): 351.3 (M+1).

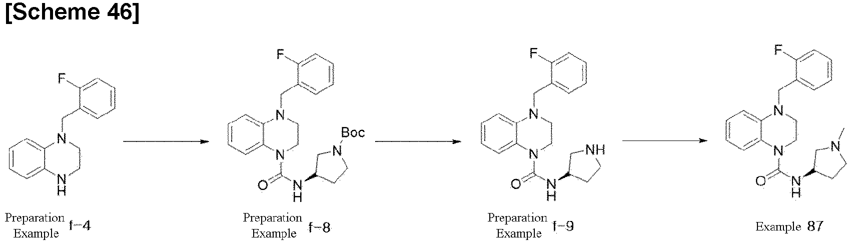

### <Preparation Example f-8> Preparation of tert-butyl (R)-3-(4-(2-fluorobenzyl)-1,2,3,4-tetrahydroquinoxaline-1-carboxamido)pyrrolidine-1-carboxylate

To a solution of **Preparation Example f-4** (1.0 equiv.), TEA (3.0 equiv.) and DCM (0.3 M) cooled to 0 °C under a nitrogen atmosphere was added dropwise triphosgene (0.6 equiv.). The reaction mixture was stirred for 1 h then TEA (2.0 equiv.) and (R)-(+)-1-Boc-3-aminopyrrolidine (1.2 equiv.) were added slowly. The mixture warmed to room temperature and stirred for 3 h. The resulting mixture was partitioned between DCM and water. The organic layer was washed with brine, dried with MgSO₄, filtered and then concentrated under reduced pressure. The residue was purified by silica column chromatography (with n-hexane/EtOAc) to give *tert*-butyl (*R*)-3-(4-(2-fluorobenzyl)-1,2,3,4-tetrahydroquinoxaline-1-carboxamido)pyrrolidine-1-carboxylate.

White solid (yield: 98%); ¹H NMR (400 MHz, CDCl₃) δ 7.32-7.22 (m, 1H), 7.20-7.05 (m, 5H), 7.05-6.96 (m, 1H), 6.72-6.65 (m, 2H), 5.36 (d, *J* = 6.9 Hz, 1H), 4.62 (s, 2H), 4.46-4.39 (m, 1H), 3.95-3.86 (m, 3H), 3.67 (d, *J* = 7.2 Hz, 1H), 3.51-3.37 (m, 3H), 3.23-3.10 (m, 1H), 1.85-1.72 (m, 1H), 1.48 (s, 9H).

### <Preparation Example f-9> Preparation of (R)-4-(2-fluorobenzyl)-N-(pyrrolidin-3-yl)-3,4-dihydroquinoxaline-1(2H)-carboxamide

Following the procedure as described for Example 1, reaction of **Preparation Example f-8** (1.0 equiv.) in DCM (0.5 M) with TFA (10.0 equiv.) gave (*R*)-4-(2-fluorobenzyl)-*N*-(pyrrolidin-3-yl)-3,4-dihydroquinoxaline-1(*2H*)-carboxamide.

White solid (yield: 98%); ¹H NMR (400 MHz, CDCl₃) δ 7.32-7.21 (m, 1H), 7.15-7.05 (m, 3H), 7.05-6.94 (m, 1H), 6.73-6.59 (m, 2H), 5.55 (d, *J* = 6.7 Hz, 1H), 4.61 (s, 2H), 4.44-4.32 (m, 1H), 3.98-3.81 (m, 2H), 3.46 (dt, *J* = 9.2, 5.2 Hz, 2H), 3.34-3.25 (m, 1H), 3.24-3.13 (m, 1H), 3.11-2.92 (m, 2H), 2.65-2.54 (m, 1H), 2.26-2.17 (m, 1H), 1.79-1.55 (m, 1H); LC/MS ESI (+): 355.2 (M+1).

### <Example 87> Synthesis of (R)-4-(2-fluorobenzyl)-N-(1-methylpyrrolidin-3-yl)-3,4-dihydroquinoxaline-1(2H)-carboxamide

Following the procedure as described for Example 2, reaction of **Preparation Example f-9** (1.0 equiv.) in DCM (0.3 M) with sodium triacetoxyborohydride (1.5 equiv.) and formaldehyde (2.0 equiv.) gave (*R*)-4-(2-fluorobenzyl)-*N*-(1-methylpyrrolidin-3-yl)-3,4-dihydroquinoxaline-1(2*H*)-carboxamide.

Light yellow oil (yield: 81%); ¹H NMR (400 MHz, CDCl₃) δ 7.32-7.21 (m, 1H), 7.19-7.03 (m, 4H), 7.02-6.94 (m, 1H), 6.72-6.62 (m, 2H), 5.54 (d, *J* = 7.5 Hz, 1H), 4.61 (s, 2H), 4.48-4.36 (m, 1H), 4.01-3.91 (m, 1H), 3.90-3.78 (m, 1H), 3.46 (t, *J* = 5.2 Hz, 2H), 2.82-2.73 (m, 1H), 2.65-2.53 (m, 2H), 2.41-2.20 (m, 5H), 1.64-1.51 (m, 1H); LC/MS ESI (+): 369.3 (M+1).

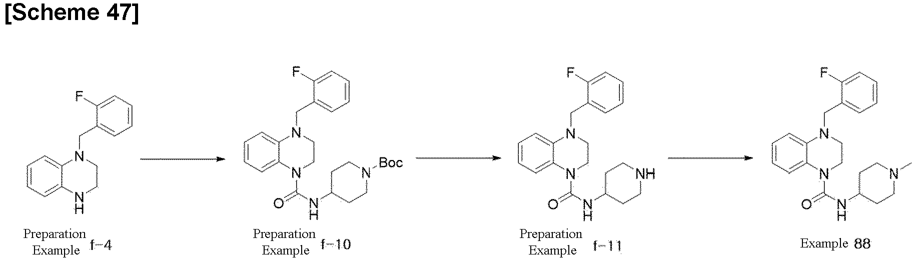

### <Preparation Example f-10> Preparation of tert-butyl 4-(4-(2-fluorobenzyl)-1,2,3,4-tetrahydroquinoxaline-1-carboxamido)piperidine-1-carboxylate

The title compound was prepared in the same manner as Preparation Example f-8, using 1-Boc-4-aminopiperidine in place of (R)-(+)-1-Boc-3-aminopyrrolidine to give *tert-butyl* 4-(4-(2-fluorobenzyl)-1,2,3,4-tetrahydroquinoxaline-1-carboxamido)piperidine-1-carboxylate.

White solid (yield: 98%); ¹H NMR (400 MHz, CDCl₃) δ 7.32-7.21 (m, 1H), 7.17-6.96 (m, 5H), 6.72-6.63 (m, 2H), 5.21 (d, *J =* 7.7 Hz, 1H), 4.62 (s, 2H), 4.00 (s, 2H), 3.95-3.82 (m, 3H), 3.47 (t, *J* = 5.2 Hz, 2H), 2.97-2.86 (m, 2H), 2.00-1.91 (m, 2H), 1.47 (s, 9H), 1.38-1.23 (m, 2H).

### <Preparation Example f-11> Preparation of 4-(2-fluorobenzyl)-N-(piperidin-4-yl)-3,4-dihydroquinoxaline-1(2H)-carboxamide

Following the procedure as described for Example 1, reaction of **Preparation Example f-10** (1.0 equiv.) in DCM (0.5 M) with TFA (10.0 equiv.) gave 4-(2-fluorobenzyl)-*N*-(piperidin-4-yl)-3,4-dihydroquinoxaline-1(2*H*)-carboxamide.

White solid (yield: 97%); ¹H NMR (400 MHz, CDCl₃) δ 7.36-7.21 (m, 1H), 7.19-7.04 (m, 4H), 7.04-6.96 (m, 1H), 6.72-6.64 (m, 2H), 5.24 (d, *J* = 7.8 Hz, 1H), 4.62 (s, 2H), 3.90 (dd, *J* = 6.5, 3.9 Hz, 2H), 3.88-3.76 (m, 1H), 3.47 (t, *J* = 5.2 Hz, 2H), 3.11-3.01 (m, 2H), 2.78-2.67 (m, 2H), 2.04-1.94 (m, 2H), 1.37-1.23 (m, 3H); LC/MS ESI (+): 369.1 (M+1).

### <Example 88> Synthesis of 4-(2-fluorobenzyl)-N-(1-methylpiperidin-4-yl)-3,4-dihydroquinoxaline-1(2H)-carboxamide

Following the procedure as described for Example 2, reaction of **Preparation Example f-11** (1.0 equiv.) in DCM (0.3 M) with sodium triacetoxyborohydride (1.5 equiv.) and formaldehyde (2.0 equiv.) gave 4-(2-fluorobenzyl)-*N*-(1-methylpiperidin-4-yl)-3,4-dihydroquinoxaline-1(*2H*)-carboxamide.

White solid (yield: 90%); ¹H NMR (400 MHz, CDCl₃) δ 7.32-7.21 (m, 1H), 7.19-6.96 (m, 5H), 6.71-6.63 (m, 2H), 5.22 (d, *J =* 7.8 Hz, 1H), 4.62 (s, 2H), 3.90 (t, *J* = 5.2 Hz, 2H), 3.81-3.65 (m, 1H), 3.47 (t, *J* = 5.2 Hz, 2H), 2.77-2.70 (m, 2H), 2.28 (s, 3H), 2.18-2.08 (m, 2H), 2.03-1.93 (m, 2H), 1.53-1.37 (m, 2H); LC/MS ESI (+): 383.3 (M+1).

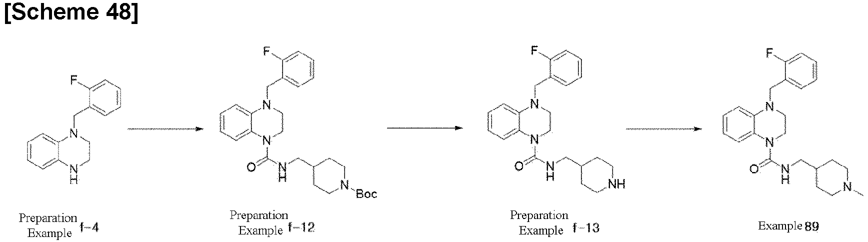

### <Preparation Example f-12> Preparation of tert-butyl 4-((4-(2-fluorobenzyl)-1,2,3,4-tetrahydroquinoxaline-1-carboxamido)methyl)piperidine-1-carboxylate

The title compound was prepared in the same manner as Preparation Example f-8, using 1-Boc-4-(aminomethyl)piperidine in place of (*R*)-(+)-1-Boc-3-aminopyrrolidine to give *tert-butyl* 4-((4-(2-fluorobenzyl)-1,2,3,4-tetrahydroquinoxaline-1-carboxamido)methyl)piperidine-1-carboxylate.

Yellow oil (yield: 99%); ¹H NMR (400 MHz, CDCl₃) δ 7.32-7.21 (m, 1H), 7.19-6.98 (m, 5H), 6.74-6.65 (m, 2H), 5.43 (t, *J* = 6.0 Hz, 1H), 4.62 (s, 2H), 3.90 (t, *J* = 5.1 Hz, 2H), 3.47 (t, *J* = 5.2 Hz, 2H), 3.17 (s, 2H), 2.70 (s, 2H), 1.83-1.55 (m, 4H), 1.47 (s, 9H), 1.21-1.06 (m, 2H), 0.95-0.79 (m, 1H).

### <Preparation Example f-13> Preparation of 4-(2-fluorobenzyl)-N-(piperidin-4-ylmethyl)-3,4-dihydroquinoxaline-1(2H)-carboxamide

Following the procedure as described for Example 1, reaction of **Preparation Example f-12** (1.0 equiv.) in DCM (0.5 M) with TFA (10.0 equiv.) gave 4-(2-fluorobenzyl)-*N*-(piperidin-4-yl)-3,4-dihydroquinoxaline-1(2*H*)-carboxamide.

Light yellow solid (yield: 81%); ¹H NMR (400 MHz, CDCl₃) δ 7.19-7.12 (m, 2H), 7.10-7.06 (m, 2H), 7.03-6.98 (m, 1H), 6.74-6.64 (m, 2H), 5.50-5.38 (m, 1H), 7.32-7.21 (m, 1H), 4.62 (s, 2H), 3.90 (td, *J* = 5.2, 2.8 Hz, 2H), 3.29 (d, *J* = 4.1 Hz, 1H), 3.21-3.13 (m, 2H), 3.07-2.96 (m, 1H), 2.79-2.67 (m, 2H), 2.03-1.93 (m, 1H), 1.84-.76 (m, 2H), 1.79-1.62 (m, 1H), 1.60-1.34 (m, 2H), 1.32-1.18 (m, 1H), 0.94-0.77 (m, 1H); LC/MS ESI (+): 383.1 (M+1).

### <Example 89> Synthesis of 4-(2-fluorobenzyl)-N-((1-methylpiperidin-4-yl)methyl)-3,4-dihydroquinoxaline-1(2H)-carboxamide

Following the procedure as described for Example 2, reaction of **Preparation Example f-13** (1.0 equiv.) in DCM (0.3 M) with sodium triacetoxyborohydride (1.5 equiv.) and formaldehyde (2.0 equiv.) gave 4-(2-fluorobenzyl)-*N*-((1-methylpiperidin-4-yl)methyl)-3,4-dihydroquinoxaline-1(*2H*)-carboxamide.

Light yellow oil (yield: 85%); ¹H NMR (400 MHz, CDCl₃) δ 7.32-7.21 (m, 1H), 7.19-7.06 (m, 4H), 7.05-6.97 (m, 1H), 6.74-6.64 (m, 2H), 5.43 (t, *J* = 6.0 Hz, 1H), 4.62 (s, 2H), 3.90 (dd, *J* = 6.6, 3.8 Hz, 2H), 3.47 (t, *J* = 5.2 Hz, 2H), 3.18 (t, *J* = 6.3 Hz, 2H), 2.91-2.82 (m, 2H), 2.28 (s, 3H), 1.98-1.87 (m, 2H), 1.71 (s, 1H), 1.60-1.44 (m, 1H), 1.38-1.22 (m, 3H); LC/MS ESI (+): 397.3 (M+1).

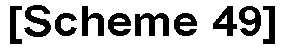

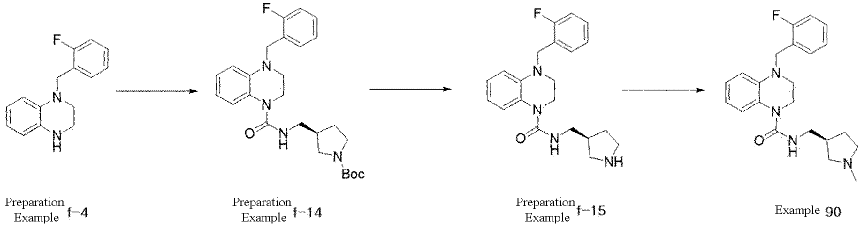

### <Preparation Example f-14> Preparation of tert-butyl (R)-3-((4-(2-fluorobenzyl)-1,2,3,4-tetrahydroquinoxaline-1-carboxamido)methyl)pyrrolidine-1-carboxylate

The title compound was prepared in the same manner as Preparation Example f-8, using (*S*)-1-Boc-3-(aminomethyl)pyrrolidine in place of (*R*)-(+)-1-Boc-3-aminopyrrolidine to give *tert*-butyl (*R*)-3-((4-(2-fluorobenzyl)-1,2,3,4-tetrahydroquinoxaline-1-carboxamido)methyl)pyrrolidine-1-carboxylate.

Light yellow solid (yield: 98%); ¹H NMR (400 MHz, CDCl₃) δ 7.32-7.21 (m, 1H), 7.18-7.11 (m, 2H), 7.11-7.05 (m, 2H), 7.05-6.97 (m, 1H), 6.74-6.65 (m, 2H), 5.44 (t, *J* = 5.6 Hz, 1H), 4.62 (s, 2H), 3.97-3.87 (m, 2H), 3.58-3.40 (m, 3H), 3.40-3.14 (m, 2H), 3.09-2.95 (m, 1H), 2.44 (s, 1H), 2.02-1.94 (m, 1H), 1.73-1.51 (m, 1H), 1.50-1.46 (m, 10H), 0.95-0.74 (m, 1H).

### <Preparation Example f-15> Preparation of (S)-4-(2-fluorobenzyl)-N-(pyrrolidin-3-ylmethyl)-3,4-dihydroquinoxaline-1(2H)-carboxamide

Following the procedure as described for Example 1, reaction of **Preparation Example f-14** (1.0 equiv.) in DCM (0.5 M) with TFA (10.0 equiv.) gave (*S*)-4-(2-fluorobenzyl)-*N*-(pyrrolidin-3-ylmethyl)-3,4-dihydroquinoxaline-1(*2H*)-carboxamide.

Light yellow solid (yield: 89%); ¹H NMR (400 MHz, CDCl₃) δ 7.32-7.21 (m, 1H), 7.18-7.13 (m, 2H), 7.11-7.04 (m, 2H), 7.04-6.94 (m, 1H), 6.73-6.61 (m, 2H), 5.62 (t, *J =* 5.8 Hz, 1H), 4.60 (d, *J* = 8.1 Hz, 2H), 3.89 (t, *J* = 5.2 Hz, 2H), 3.46 (t, *J* = 5.1 Hz, 2H), 3.31-3.23 (m, 2H), 3.27-3.10 (m, 2H), 3.14-3.00 (m, 1H), 2.90-2.81 (m, 1H), 2.53-2.44 (m, 1H), 2.44-2.27 (m, 1H), 2.10-1.86 (m, 1H), 1.66-1.44 (m, 1H); LC/MS ESI (+): 369.1 (M+1).

### <Example 90> Synthesis of (R)-4-(2-fluorobenzyl)-N-((1-methylpyrrolidin-3-yl)methyl)-3,4-dihydroquinoxaline-1(2H)-carboxamide

Following the procedure as described for Example 2, reaction of **Preparation Example f-15** (1.0 equiv.) in DCM (0.3 M) with sodium triacetoxyborohydride (1.5 equiv.) and formaldehyde (2.0 equiv.) gave (*R*)-4-(2-fluorobenzyl)-*N*-((1-methylpyrrolidin-3-yl)methyl)-3,4-dihydroquinoxaline-1(*2H*)-carboxamide.

Light yellow oil (yield: 85%); ¹H NMR (400 MHz, CDCl₃) δ 7.32-7.21 (m, 1H), 7.20-7.04 (m, 3H), 7.03-6.95 (m, 2H), 6.75-6.63 (m, 2H), 6.05-5.98 (m, 1H), 4.62 (s, 2H), 4.02-3.92 (m, 1H), 3.91-3.78 (m, 1H), 3.47 (dd, *J*= 5.9, 4.6 Hz, 2H), 3.36-3.21 (m, 2H), 2.65-2.31 (m, 5H), 2.24 (s, 3H), 2.06-1.93 (m, 1H), 1.60-1.45 (m, 1H); LC/MS ESI (+): 383.3 (M+1).

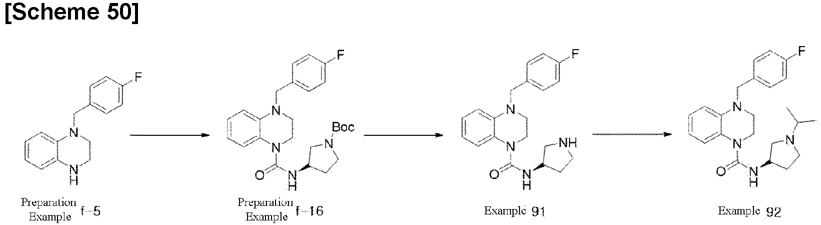

### <Preparation Example f-16> Preparation of tert-butyl (R)-3-(4-(4-fluorobenzyl)-1,2,3,4-tetrahydroquinoxaline-1-carboxamido)pyrrolidine-1-carboxylate

To a solution of **Preparation Example f-5** (1.0 equiv.), TEA (3.0 equiv.) and DCM (0.3 M) cooled to 0 °C under a nitrogen atmosphere was added dropwise triphosgene (0.6 equiv.). The reaction mixture was stirred for 1 h then TEA (2.0 equiv.) and (*R*)-(+)-1-Boc-3-aminopyrrolidine (1.2 equiv.) were added slowly. The mixture warmed to room temperature and stirred for 3 h. The resulting mixture was partitioned between DCM and water. The organic layer was washed with brine, dried with MgSO₄, filtered and then concentrated under reduced pressure. The residue was purified by silica column chromatography (with *n*-hexane/EtOAc) to give *tert*-butyl (*R*)-3-(4-(4-fluorobenzyl)-1,2,3,4-tetrahydroquinoxaline-1-carboxamido)pyrrolidine-1-carboxylate.

White solid (yield: 94%); ¹H NMR (400 MHz, CDCl₃) δ 7.23-7.07 (m, 3H), 7.06-6.94 (m, 3H), 6.72-6.65 (m, 2H), 5.38-5.30 (m, 2H), 4.54 (s, 2H), 4.44-4.40 (m, 1H), 4.03-3.77 (m, 3H), 3.66 (d, *J* = 6.9 Hz, 1H), 3.44 (t, *J* = 5.2 Hz, 3H), 3.16 (s, 1H), 1.50-1.45 (m, 10H).

### <Example 91> Synthesis of (R)-4-(4-fluorobenzyl)-N-(pyrrolidin-3-yl)-3,4-dihydroquinoxaline-1(2H)-carboxamide

Following the procedure as described for Example 1, reaction of **Preparation Example f-16** (1.0 equiv.) in DCM (0.5 M) with TFA (10.0 equiv.) gave (*R*)-4-(4-fluorobenzyl)-*N*-(pyrrolidin-3-yl)-3,4-dihydroquinoxaline-1(2*H*)-carboxamide.

White solid (yield: 94%); ¹H NMR (400 MHz, CDCl₃) δ 7.27-7.13 (m, 3H), 7.11-6.93 (m, 3H), 6.74-6.70 (m, 1H), 6.67-6.60 (m, 1H), 5.76 (d, *J* = 6.4 Hz, 1H), 4.54-4.47 (m, 2H), 3.96-3.76 (m, 2H), 3.48-3.36 (m, 3H), 3.30-3.19 (m, 2H), 2.64 (d, *J* = 5.0 Hz, 1H), 2.44-2.18 (m, 1H), 1.69-1.55 (m, 1H), 0.94-0.79 (m, 2H); LC/MS ESI (+): 355.1 (M+1).

### <Example 92> Synthesis of (R)-4-(4-fluorobenzyl)-N-(1-isopropylpyrrolidin-3-yl)-3,4-dihydroquinoxaline-1(2H)-carboxamide

Following the procedure as described for Example 2, reaction of **Example 91** (1.0 equiv.) in DCM (0.3 M) with sodium triacetoxyborohydride (1.5 equiv.) and acetone (2.0 equiv.) gave (*R*)-4-(4-fluorobenzyl)-*N*-(1-isopropylpyrrolidin-3-yl)-3,4-dihydroquinoxaline-1(*2H*)-carboxamide.

Light yellow oil (yield: 78%); ¹H NMR (400 MHz, CDCl₃) δ 7.23-7.13 (m, 3H), 7.10-6.94 (m, 3H), 6.72-6.63 (m, 2H), 5.55 (d, *J* = 7.7 Hz, 1H), 4.53 (s, 2H), 4.00-3.89 (m, 1H), 3.89-3.78 (m, 1H), 3.42 (t, *J* = 5.2 Hz, 2H), 2.85 (td, *J* = 8.3, 3.4 Hz, 1H), 2.79-2.71 (m, 1H), 2.66-2.54 (m, 1H), 2.42-2.19 (m, 4H), 1.63-1.51 (m, 1H), 1.09 (dd, *J* = 6.3, 5.2 Hz, 6H); LC/MS ESI (+): 397.3 (M+1).

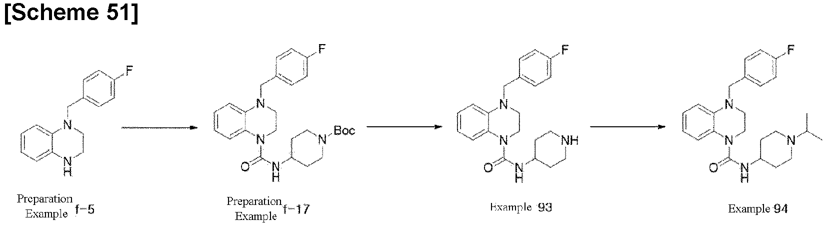

### <Preparation Example f-17> Preparation of tert-butyl 4-(4-(4-fluorobenzyl)-1,2,3,4-tetrahydroquinoxaline-1-carboxamido)piperidine-1-carboxylate

To a solution of **Preparation Example f-5** (1.0 equiv.), TEA (3.0 equiv.) and DCM (0.3 M) cooled to 0 °C under a nitrogen atmosphere was added dropwise triphosgene (0.6 equiv.). The reaction mixture was stirred for 1 h then TEA (2.0 equiv.) and 1-Boc-4-aminopiperidine (1.2 equiv.) were added slowly. The mixture warmed to room temperature and stirred for 3 h. The resulting mixture was partitioned between DCM and water. The organic layer was washed with brine, dried with MgSO₄, filtered and then concentrated under reduced pressure. The residue was purified by silica column chromatography (with n-hexane/EtOAc) to give *tert-butyl* 4-(4-(4-fluorobenzyl)-1,2,3,4-tetrahydroquinoxaline-1-carboxamido)piperidine-1-carboxylate.

White solid (yield: 80%); ¹H NMR (400 MHz, CDCl₃) δ 7.23-7.10 (m, 3H), 7.08-6.95 (m, 3H), 6.72-6.63 (m, 2H), 4.54 (s, 2H), 4.08-3.78 (m, 6H), 3.43 (t, *J* = 5.2 Hz, 2H), 2.96-2.86 (m, 2H), 2.00-1.90 (m, 2H), 1.47 (s, 9H), 1.37-1.23 (m, 2H).

### <Example 93> Synthesis of 4-(4-fluorobenzyl)-N-(piperidin-4-yl)-3,4-dihydroquinoxaline-1(2H)-carboxamide

Following the procedure as described for Example 1, reaction of **Preparation Example f-17** (1.0 equiv.) in DCM (0.5 M) with TFA (10.0 equiv.) gave 4-(4-fluorobenzyl)-*N*-(piperidin-4-yl)-3,4-dihydroquinoxaline-1(*2H*)-carboxamide.

White solid (yield: 90%); ¹H NMR (400 MHz, CDCl₃) δ 7.23-7.08 (m, 3H), 7.06-6.95 (m, 3H), 6.73-6.62 (m, 2H), 5.29-5.19 (m, 1H), 4.54 (s, 2H), 3.97-3.83 (m, 3H), 3.37-3.27 (m, 1H), 2.94-2.82 (m, 3H), 2.14 (s, 1H), 1.98-1.91 (m, 1H), 1.75-1.55 (m, 2H), 1.48-1.25 (m, 1H), 0.94-0.79 (m, 2H); LC/MS ESI (+): 369.3 (M+1).

### <Example 94> Synthesis of 4-(4-fluorobenzyl)-N-(1-isopropylpiperidin-4-yl)-3,4-dihydroquinoxaline-1(2H)-carboxamide

Following the procedure as described for Example 2, reaction of **Example 93** (1.0 equiv.) in DCM (0.3 M) with sodium triacetoxyborohydride (1.5 equiv.) and acetone (2.0 equiv.) gave 4-(4-fluorobenzyl)-*N*-(1-isopropylpiperidin-4-yl)-3,4-dihydroquinoxaline-1(*2H*)-carboxamide.

Light yellow oil (yield: 58%); ¹H NMR (400 MHz, CDCl₃) δ 7.23-7.10 (m, 3H), 7.08-6.95 (m, 3H), 6.71-6.60 (m, 2H), 5.23 (d, *J* = 7.9 Hz, 1H), 4.53 (s, 2H), 3.88 (dd, *J* = 6.2, 4.2 Hz, 2H), 3.81-3.67 (m, 1H), 3.43 (t, *J* = 5.2 Hz, 2H), 2.83-2.66 (m, 3H), 2.34-2.23 (m, 2H), 2.05-1.95 (m, 2H), 1.49-1.35 (m, 2H), 1.05 (d, *J* = 6.5 Hz, 6H); LC/MS ESI (+): 411.3 (M+1).

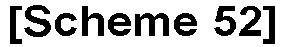

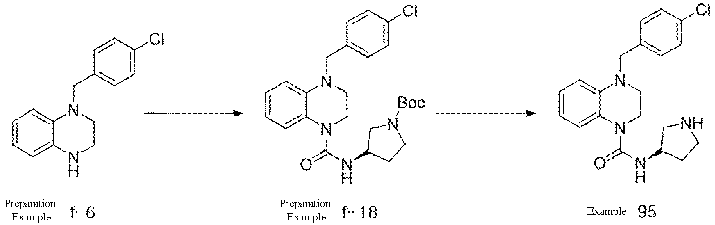

### <Preparation Example f-18> Preparation of tert-butyl (R)-3-(4-(4-chlorobenzyl)-1,2,3,4-tetrahydroquinoxaline-1-carboxamido)pyrrolidine-1-carboxylate

To a solution of **Preparation Example f-6** (1.0 equiv.), TEA (3.0 equiv.) and DCM (0.3 M) cooled to 0 °C under a nitrogen atmosphere was added dropwise triphosgene (0.6 equiv.). The reaction mixture was stirred for 1 h then TEA (2.0 equiv.) and (R)-(+)-1-Boc-3-aminopyrrolidine (1.2 equiv.) were added slowly. The mixture warmed to room temperature and stirred for 3 h. The resulting mixture was partitioned between DCM and water. The organic layer was washed with brine, dried with MgSO₄, filtered and then concentrated under reduced pressure. The residue was purified by silica column chromatography (with *n*-hexane/EtOAc) to give *tert*-butyl (*R*)-3-(4-(4-chlorobenzyl)-1,2,3,4-tetrahydroquinoxaline-1-carboxamido)pyrrolidine-1-carboxylate.

Brown oil (yield: 82%); ¹H NMR (400 MHz, CDCl₃) δ 7.32-7.27 (m, 2H), 7.14 (d, *J =* 8.3 Hz, 2H), 7.09 (d, *J =* 7.8 Hz, 1H), 7.02-6.94 (m, 1H), 6.69-6.60 (m, 2H), 5.32 (d, *J* = 6.9 Hz, 1H), 4.51 (s, 2H), 4.46-4.35 (m, 1H), 3.95-3.76 (m, 2H), 3.67 (d, *J* = 15.3 Hz, 1H), 3.49-3.28 (m, 4H), 3.24-3.10 (m, 1H), 2.22-2.11 (m, 1H), 1.91-1.68 (m, 1H), 1.45 (s, 9H).

### <Example 95> Synthesis of (R)-4-(4-chlorobenzyl)-N-(pyrrolidin-3-yl)-3,4-dihydroquinoxaline-1(2H)-carboxamide

Following the procedure as described for Example 1, reaction of **Preparation Example f-18** (1.0 equiv.) in DCM (0.5 M) with TFA (10.0 equiv.) gave (*R*)-4-(4-chlorobenzyl)-*N*-(pyrrolidin-3-yl)-3,4-dihydroquinoxaline-1(2*H*)-carboxamide.

White solid (yield: 27%); ¹H NMR (400 MHz, CDCl₃) δ 7.31-7.27 (m, 2H), 7.17-7.12 (m, 3H), 7.00-6.94 (m, 1H), 6.70-6.57 (m, 2H), 5.50 (d, *J* = 6.7 Hz, 1H), 4.50 (s, 2H), 4.41-4.28 (m, 1H), 3.93-3.78 (m, 2H), 3.44-3.38 (m, 2H), 3.28-3.21 (m, 1H), 3.19-3.09 (m, 1H), 3.05-2.97 (m, 1H), 2.93-2.87 (m, 1H), 2.26-2.14 (m, 2H), 1.74-1.61 (m, 1H); LC/MS ESI (+): 371.3 (M+1).

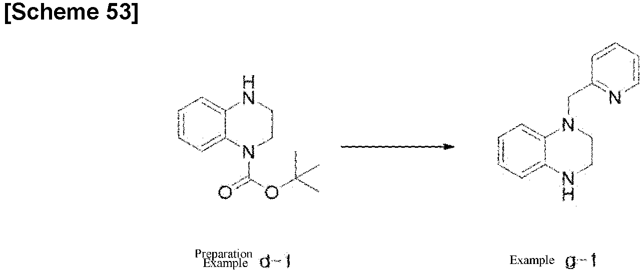

### <Preparation Process g> Preparation of 1-(pyridin-2-ylmethyl)-1,2,3,4-tetrahydroquinoxaline

1-(Pyridin-2-ylmethyl)-1,2,3,4-tetrahydroquinoxaline according to the present invention was prepared through Preparation Example g-1 below.

### <Preparation Example g-1> Preparation of 1-(pyridin-2-ylmethyl)-1,2,3,4-tetrahydroquinoxaline

To a solution of **Preparation Example d-1** (3.5 g, 14.94 mmol) in THF (25.0 mL) was added 2-(bromomethyl)pyridine hydrobromide (5.3 g, 20.91 mmol), DIPEA (11.6 g, 89.63 mmol) and NaOH (1.2 g, 29.88 mmol). The reaction mixture was stirred at room temperature for 1 h. The reaction mixture was quenched with water and extracted with DCM. The organic layer was washed with brine, dried with MgSO₄, filtered, and then concentrated under reduced pressure. A solution of the obtained concentrate in DCM (20.0 mL) was added TFA (10.3 mL, 134.76 mmol). The reaction mixture was stirred at room temperature for 24 h. The reaction mixture was diluted with DCM, and the pH was adjusted to 9 with 10M NaOH (aq.). The mixture was extracted with DCM, the organic layer dried with MgSO₄, filtered, and then concentrated under reduced pressure. The residue was purified by silica column chromatography (with n-hexane/EtOAc) to give 1-(pyridin-2-ylmethyl)-1,2,3,4-tetrahydroquinoxaline.

Light brown solid (yield: 86.7%); ¹H NMR (400 MHz, CD₃CN) δ 8.54-8.47 (m, 1H), 7.68-7.59 (m, 1H), 7.29-7.22 (m, 1H), 7.21-7.12 (m, 1H), 6.47-6.35 (m, 3H), 6.34-6.27 (m, 1H), 4.46 (s, 2H), 4.29 (brs, 1H), 3.50-3.43 (m, 5H); LC/MS ESI (+): 226.1 (M+1).

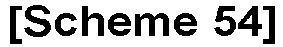

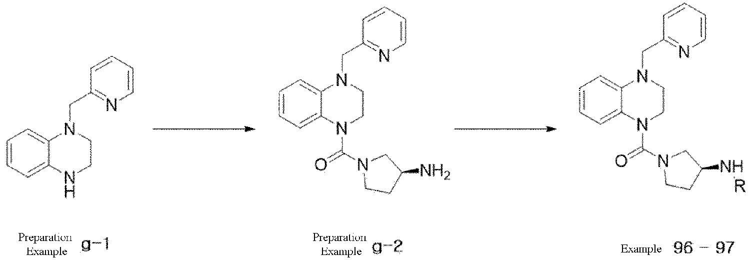

### <Preparation Example g-2> Preparation of (S)-(3-aminopyrrolidin-1-yl)(4-(pyridin-2-ylmethyl)-3,4-dihydroquinoxalin-1(2H)-yl)methanone

To a solution of **Preparation Example g-1** (1.0 equiv.), TEA (3.0 equiv.) and DCM (0.4 M) cooled to 0 °C under a nitrogen atmosphere was added dropwise triphosgene (0.6 equiv.). The reaction mixture was stirred for 1 h then TEA (2.0 equiv.) and (S)-(-)-3-(Boc-amino)pyrrolidine (1.2 equiv.) were added slowly. The mixture warmed to room temperature and stirred for 3 h. The resulting mixture was partitioned between DCM and water. The organic layer was washed with brine, dried with MgSO₄, filtered and then concentrated under reduced pressure. A solution of the obtained concentrate in DCM (0.3 M) was added TFA (10.0 equiv.). The reaction mixture was stirred at room temperature for 24 h. The reaction mixture was diluted with DCM, and the pH was adjusted to 9 with 10M NaOH (aq.). The mixture was extracted with DCM, the organic layer dried with MgSO₄, filtered, and then concentrated under reduced pressure. The residue was purified by silica column chromatography (with n-hexane/EtOAc) to give (*S*)-(3-aminopyrrolidin-1-yl)(4-(pyridin-2-ylmethyl)-3,4-dihydroquinoxalin-1(*2H*)-yl)methanone.

Yellow oil (yield: 70%); ¹H NMR (400 MHz, CD₃OD) δ 8.53-8.45 (m, 1H), 7.79-7.70 (m, 1H), 7.33-7.24 (m, 2H), 6.87-6.80 (m, 1H), 6.79-6.72 (m, 1H), 6.62-6.53 (m, 1H), 6.52-6.45 (m, 1H), 4.65-4.59 (m, 2H), 3.82-3.71 (m, 1H), 3.70-3.64 (m, 1H), 3.63-3.55 (m, 2H), 3.54-3.32 (m, 4H), 3.10-2.94 (m, 1H), 2.09-1.96 (m, 1H), 1.73-1.60 (m, 1H); LC/MS ESI (+): 338.2 (M+1).

### <Example 96> Synthesis of (S)-(3-(isobutylamino)pyrrolidin-1-yl)(4-(pyridin-2-ylmethyl)-3,4-dihydroquinoxalin-1(2H)-yl)methanone

Following the procedure as described for Example 2, reaction of **Preparation Example e-12** (1.0 equiv.) in DCM (0.3 M) with sodium triacetoxyborohydride (1.5 equiv.) and isobutyraldehyde (1.1 equiv.) gave (*S*)-(3-(isobutylamino)pyrrolidin-1-yl)(4-(pyridin-2-ylmethyl)-3,4-dihydroquinoxalin-1(2*H*)-yl)methanone.

Light yellow oil (yield: 71%); ¹H NMR (400 MHz, CD₃OD) δ 8.55-8.42 (m, 1H), 7.78-7.69 (m, 1H), 7.32-7.23 (m, 2H), 6.86-6.79 (m, 1H), 6.78-6.71 (m, 1H), 6.61-6.52 (m, 1H), 6.52-6.45 (m, 1H), 4.61 (d, *J* = 4.3 Hz, 2H), 3.88-3.77 (m, 1H), 3.69-3.52 (m, 3H), 3.51-3.40 (m, 2H), 3.39-3.29 (m, 1H), 3.26-3.17 (m, 1H), 3.13-3.04 (m, 1H), 2.42-2.25 (m, 2H), 2.12-1.98 (m, 1H), 1.76-1.61 (m, 2H), 0.96-0.81 (dd, *J* = 6.6, 1.0 Hz, 6H); LC/MS ESI (+): 394.3 (M+1).

### <Example 97> Synthesis of (S)-(3-(dimethylamino)pyrrolidin-1-yl)(4-(pyridin-2-ylmethyl)-3,4-dihydroquinoxalin-1(2H)-yl)methanone

The title compound was synthesized in the same manner as Example 96, using formaldehyde in place of isobutyraldehyde to give (*S*)-(3-(dimethylamino)pyrrolidin-1-yl)(4-(pyridin-2-ylmethyl)-3,4-dihydroquinoxalin-1(2*H*)-yl)methanone.

Light yellow oil (yield: 53%); ¹H NMR (400 MHz, CD₃OD) δ 8.53-8.46 (m, 1H), 7.78-7.69 (m, 1H), 7.33-7.24 (m, 2H), 6.86-6.74 (m, 2H), 6.62-6.53 (m, 1H), 6.52-6.47 (m, 1H), 4.75-4.53 (m, 2H), 4.02-3.93 (m, 1H), 3.73-3.62 (m, 1H), 3.61-3.52 (m, 1H), 3.54-3.31 (m, 4H), 3.19-3.10 (m, 1H), 2.80-2.67 (m, 1H), 2.21 (s, 6H), 2.19-2.02 (m, 1H), 1.78-1.63 (m, 1H); LC/MS ESI (+): 366.3 (M+1).

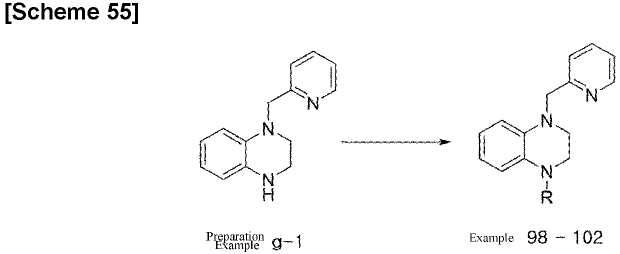

### <Example 98> Synthesis of (R)-4-(pyridin-2-ylmethyl)-N-(tetrahydrofuran-3-yl)-3,4-dihydroquinoxaline-1(2H)-carboxamide

To a solution of **Preparation Example g-1** (1.0 equiv.), TEA (3.0 equiv.) and DCM (0.3 M) cooled to 0 °C under a nitrogen atmosphere was added dropwise triphosgene (0.6 equiv.). The reaction mixture was stirred at 0 °C for 1 h then TEA (2.0 equiv.) and (S)-3-aminotetrahydrofuran (1.2 equiv.) were added slowly. The mixture warmed to room temperature and stirred for 3 h. The resulting mixture was partitioned between DCM and water. The organic layer was washed with brine, dried with MgSO₄, filtered and then concentrated under reduced pressure. The residue was purified by silica column chromatography (with *n*-hexane/EtOAc) to give (*R*)-4-(pyridin-2-ylmethyl)-*N-*(tetrahydrofuran-3-yl)-3,4-dihydroquinoxaline-1(*2H*)-carboxamide.

Ivory solid (yield: 52%); ¹H NMR (400 MHz, CD₃OD) δ 8.55-8.49 (m, 1H), 7.77 (dd, *J* = 7.7, 1.8 Hz, 1H), 7.36-7.27 (m, 2H), 7.19 (dd, *J* = 7.9, 1.5 Hz, 1H), 6.93 (dd, *J =* 7.4, 1.6 Hz, 1H), 6.71-6.58 (m, 2H), 4.67 (s, 2H), 4.41-4.31 (m, 1H), 3.94-3.82 (m, 4H), 3.81-3.73 (m, 1H), 3.67-3.60 (m, 1H), 3.54 (t, *J* = 4.7 Hz, 2H), 2.30-2.16 (m, 1H), 1.94-1.79 (m, 1H); LC/MS ESI (+): 339.2 (M+1).

### <Example 99> Synthesis of N-(oxetan-3-yl)-4-(pyridin-2-ylmethyl)-3,4-dihydroquinoxaline-1(2H)-carboxamide

The title compound was synthesized in the same manner as Example 98, using 3-axetanamine in place of (*S*)-3-aminotetrahydrofuran to give *N*-(oxetan-3-yl)-4-(pyridin-2-ylmethyl)-3,4-dihydroquinoxaline-1(*2H*)-carboxamide.

White solid (yield: 77%); ¹H NMR (400 MHz, CD₃CN) δ 8.55-8.48 (m, 1H), 7.64 (ddd, *J* = 7.7, 7.7, 1.8 Hz, 1H), 7.24-7.15 (m, 3H), 6.90 (ddd, *J* = 8.3, 7.4, 1.6 Hz, 1H), 6.67-6.58 (m, 2H), 6.02 (d, *J* = 6.6 Hz, 1H), 4.92-4.77 (m, 1H), 4.75-4.66 (m, 2H), 4.60 (s, 2H), 4.49-4.39 (m, 2H), 3.78-3.71 (m, 2H), 3.52-3.45 (m, 2H); LC/MS ESI (+): 325.1 (M+1).

### <Example 100> Synthesis of 4-(pyridin-2-ylmethyl)-N-((tetrahydrofuran-3-yl)methyl)-3,4-dihydroquinoxaline-1(2H)-carboxamide

The title compound was synthesized in the same manner as Example 98, using (tetrahydrofuran-3-yl) methanamine in place of (*S*)-3-aminotetrahydrofuran to give 4-(pyridin-2-ylmethyl)-*N*-((tetrahydrofuran-3-yl)methyl)-3,4-dihydroquinoxaline-1(*2H*)-carboxamide.

White solid (yield: 51%); ¹H NMR (400 MHz, CDCl₃) δ 8.59 (dd, *J* = 4.9, 0.9 Hz, 1H), 7.61 (ddd, *J* = 7.7, 7.7, 1.8 Hz, 1H), 7.23-7.09 (m, 3H), 6.98 (ddd, *J* = 8.3, 7.4, 1.5 Hz, 1H), 6.71-6.60 (m, 2H), 5.47 (t, *J* = 5.8 Hz, 1H), 4.65 (s, 2H), 3.97-3.88 (m, 2H), 3.87-3.68 (m, 3H), 3.60-3.48 (m, 3H), 3.37-3.19 (m, 2H), 2.60-2.44 (m, 1H), 2.09-1.96 (m, 1H), 1.75-1.59 (m, 1H); LC/MS ESI (+): 353.2 (M+1).

### <Example 101> Synthesis of 4-(pyridin-2-ylmethyl)-N-((tetrahydro-2H-pyran-4-yl)methyl)-3,4-dihydroquinoxaline-1(2H)-carboxamide

The title compound was synthesized in the same manner as Example 98, using 4-aminomethyl tetrahydropyran in place of (*S*)-3-aminotetrahydrofuran to give 4-(pyridin-2-ylmethyl)-*N*-((tetrahydro-*2H*-pyran-4-yl)methyl)-3,4-dihydroquinoxaline-1(2*H*)-carboxamide.

Ivory solid (yield: 84%); ¹H NMR (400 MHz, CD₃CN) δ 8.49 (dd, *J* = 4.9, 1.0 Hz, 1H), 7.62 (ddd, *J* = 7.7, 7.7, 1.8 Hz, 1H), 7.23-7.08 (m, 3H), 6.88 (ddd, *J* = 8.6, 7.3, 1.6 Hz, 1H), 6.64-6.55 (m, 2H), 5.67-5.49 (m, 1H), 4.58 (s, 2H), 3.88-3.79 (m, 2H), 3.74 (dd, *J* = 5.8, 4.5 Hz, 2H), 3.46 (dd, *J* = 5.7, 4.5 Hz, 2H), 3.27 (ddd, *J* = 11.8, 11.8, 2.2 Hz, 2H), 3.02 (dd, *J* = 6.8, 6.0 Hz, 2H), 1.74-1.58 (m, 1H), 1.56-1.45 (m, 2H), 1.22-1.07 (m, 2H); LC/MS ESI (+): 367.2 (M+1).

### <Example 102> Synthesis of N-(cyclopropylmethyl)-4-(pyridin-2-ylmethyl)-3,4-dihydroquinoxaline-1(2H)-carboxamide

The title compound was synthesized in the same manner as Example 98, using cyclopropylmethanamine in place of (*S*)-3-aminotetrahydrofuran to give *N-*(cyclopropylmethyl)-4-(pyridin-2-ylmethyl)-3,4-dihydroquinoxaline-1(*2H*)-carboxamide.

Light yellow solid (yield: 68%); ¹H NMR (400 MHz, CD₃OD) δ 8.52 (dd, *J* = 4.9, 0.9 Hz, 1H), 7.76 (ddd, *J* = 7.7, 7.7, 1.8 Hz, 1H), 7.34-7.23 (m, 2H), 7.19 (dd, *J* = 7.9, 1.5 Hz, 1H), 6.94 (ddd, *J* = 8.3, 7.4, 1.6 Hz, 1H), 6.67 (ddd, *J* = 7.6, 7.5, 1.3 Hz, 1H), 6.61 (dd, *J* = 8.3, 1.3 Hz, 1H), 4.66 (s, 2H), 3.84 (dd, *J* = 5.7, 4.6 Hz, 2H), 3.54 (dd, *J =* 5.8, 4.6 Hz, 2H), 3.09 (d, *J* = 6.9 Hz, 2H), 1.10-0.95 (m, 1H), 0.53-0.41 (m, 2H), 0.25-0.16 (m, 2H); LC/MS ESI (+): 323.2 (M+1).

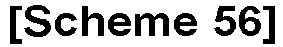

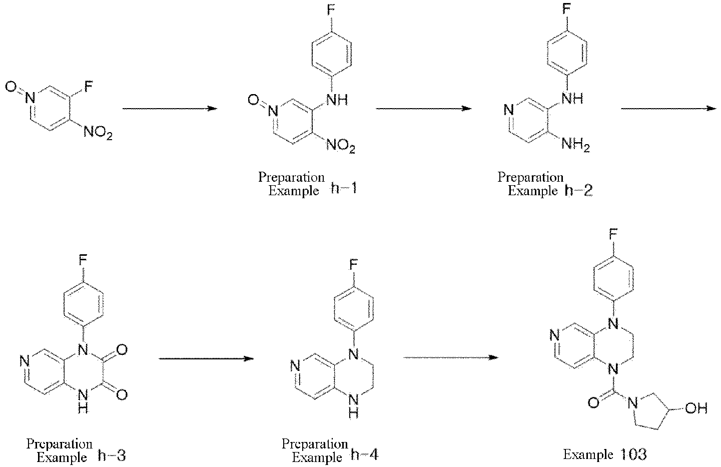

### <Preparation Process h> Preparation of 4-(4-fluorophenyl)-1,2,3,4-tetrahydropyrido[3,4-b]pyrazine

4-(4-Fluorophenyl)-1,2,3,4-tetrahydropyrido[3,4-*b*]pyrazine according to the present invention was prepared through Preparation Examples h-1 to h-4 below.

### <Preparation Example h-1> Preparation of 3-((4-fluorophenyl)amino)-4-nitropyridine-1-oxide

3-Fluoro-4-nitropyridine 1-oxide (5.0 g, 31.6 mmol) and 4-fluoroaniline (10.5 g, 94.9 mmol) were dissolved in pyridine (28 mL). The resulting mixture was heated at 70 °C for 1h. The reaction mixture was cooled to room temperature and added EtOH (30.0 mL). The precipitate formed in the mixture was collected by filtration, washed with EtOAc and then dried vacuo to give 3-((4-fluorophenyl)amino)-4-nitropyridine-1-oxide.

Orange solid (yield: 90.3%); ¹H NMR (400 MHz, DMSO-*d*₆) δ 9.56 (s, 1H), 8.13-8.07 (m, 1H), 7.65-7.60 (m, 2H), 7.46-7.40 (m, 2H), 7.36-7.27 (m, 2H); LC/MS ESI (+): 250.0 (M+1).

### <Preparation Example h-2> Preparation of N³-(4-fluorophenyl)pyridine-3,4-diamine

The **Preparation Example h-1** (7.0 g, 28.1 mmol) was dissolved in AcOH/H₂O (4:1 = v/v, 50.0 mL) and then Fe powder (9.4 g, 168.0 mmol) was added. The reaction mixture was heated at 100 °C for 3 h. After cooling the reaction mixture was filtered through a Celite pad and the pH was adjusted to 9 with 10M NaOH (aq.). The mixture was extracted with EtOAc, the organic layer dried with MgSO₄, filtered, and then concentrated under reduced pressure. The residue was purified by silica column chromatography (with *n*-hexane/EtOAc) to give *N*³-(4-fluorophenyl)pyridine-3,4-diamine.

Purple oil (yield: 68.4%); ¹H NMR (400 MHz, DMSO-*d₆*) δ 7.94 (s, 1H), 7.85 (d, *J* = 5.4 Hz, 1H), 7.16 (s, 1H), 7.02-6.91 (m, 2H), 6.66-6.56 (m, 3H), 5.65 (s, 2H); LC/MS ESI (+): 204.0 (M+1).

### <Preparation Example h-3> Preparation of 4-(4-fluorophenyl)pyrido[3,4-b]pyrazin-2,3(1H,4H)-dione

A mixture of **Preparation Example h-2** (3.9 g, 19.2 mmol) in diethyl oxalate (7.8 mL, 57.6 mmol) was heated to160 °C for 24 h and then cooled to room temperature. The precipitate formed in the mixture was collected by filtration, washed with EtOH and then dried vacuo to give 4-(4-fluorophenyl)pyrido[3,4-*b*]pyrazin-2,3(*1H*,*4H*)-dione.

White solid (yield: 69.7%); ¹H NMR (400 MHz, DMSO-*d₆*) δ 12.37 (s, 1H), 8.23 (d, *J* = 5.3 Hz, 1H), 7.53-7.46 (m, 5H), 7.16 (d, *J* = 5.3 Hz, 1H); LC/MS ESI (+): 258.0 (M+1).

### <Preparation Example h-4> Preparation of 4-(4-fluorophenyl)-1,2,3,4-tetrahydropyrido[3,4-b]pyrazine

To a solution of **Preparation Example h-3** (1.0 g, 3.89 mmol) in THF (30 mL) was added dropwise 1M borane-THF solution (23.3 mL, 23.3 mmol) and stirred at room temperature. The reaction mixture was heated at 65°C for 16 h. After cooling the reaction mixture was quenched with water, and the pH was adjusted to 9 with Conc. NaHCO₃ (aq.). The mixture was extracted with EtOAc, and the organic layer was dried with MgSO₄, filtered, and then concentrated under reduced pressure. The residue was purified by silica column chromatography (with DCM/MeOH) to give 4-(4-fluorophenyl)-1,2,3,4-tetrahydropyrido[3,4-b]pyrazine.

White solid (yield: 43.1%); ¹H NMR (400 MHz, CDCl₃) δ 7.80 (d, *J* = 5.3 Hz, 2H), 7.18-7.09 (m, 2H), 7.08-6.99 (m, 2H), 6.42 (d, *J* = 5.3 Hz, 1H), 4.38 (s, 1H), 3.65-3.58 (m, 2H), 3.55-3.46 (m, 2H); LC/MS ESI (+): 230.0 (M+1).

### <Example 103> Synthesis of (4-(4-fluorophenyl)-3,4-dihydropyrido[3,4-b]pyrazine-1(2H)-yl)(3-hydroxypyrrolidin-1-yl)methanone

To a solution of **Preparation Example h-4** (300 mg, 0.13 mmol), TEA (36.5 µL, 0.26 mmol) and DCM (1.0 mL) cooled to 0 °C under a nitrogen atmosphere was added dropwise triphosgene (23.3 mg, 1.62 mmol). The reaction mixture was stirred for 1 h then TEA (36.5 µL, 0.26 mmol) and pyrrolidin-3-ol (12.8 µL, 0.16 mmol) were added slowly. The mixture warmed to room temperature and stirred for 1 h. The resulting mixture was partitioned between DCM and water. The organic layer was washed with brine, dried with MgSO₄, filtered and then concentrated under reduced pressure. The residue was purified by silica column chromatography (with n-hexane/EtOAc) to give (4-(4-fluorophenyl)-3,4-dihydropyrido[3,4-*b*]pyrazine-1(*2H*)-yl)(3-hydroxypyrrolidin-1-yl)methanone.

Yellow solid (yield: 33%); ¹H NMR (400 MHz, CDCl₃) δ 7.89-7.84 (m, 2H), 7.24-7.16 (m, 2H), 7.15-7.05 (m, 2H), 6.82 (d, *J* = 5.4 Hz, 1H), 4.55-4.49 (m, 1H), 3.95-3.86 (m, 1H), 3.77-3.57 (m, 5H), 3.55-3.38 (m, 2H), 2.10-1.91 (m, 3H); LC/MS ESI (+): 343.0 (M+1).

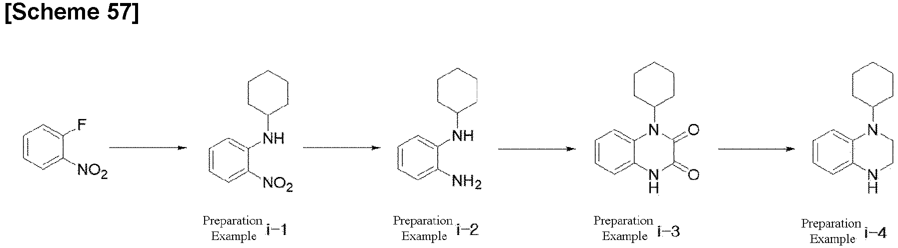

### <Preparation Process i> Preparation of 1-cyclohexyl-1,2,3,4-tetrahydroquinoxaline

1-Cyclohexyl-1,2,3,4-tetrahydroquinoxaline according to the present invention was synthesized through Preparation Examples i-1 to i-4 below.

### <Preparation Example i-1> Preparation of N-cyclohexyl-2-nitroaniline

A mixture of 1-fluoro-2-nitrobenzene (1 g, 7.09 mmol) and cyclohexylamine (1.0 mL, 8.50 mmol) was stirred at 110 °C for 6 h. After cooling the reaction mixture was quenched with water and extracted with DCM. The organic layer was washed with brine, dried with MgSO₄, filtered, and then concentrated under reduced pressure. The residue was purified by silica column chromatography (with *n*-hexane/EtOAc) to give *N-*cyclohexyl-2-nitroaniline.

Orange solid (yield: 99%); ¹H NMR (400 MHz, CDCl₃) δ 8.20-8.08 (m, 2H), 7.42-7.36 (m, 1H), 6.86 (d, *J* = 8.6 Hz, 1H), 6.59 (ddd, *J* = 8.4, 6.8, 1.2 Hz, 1H), 3.55-3.48 (m, 1H), 2.10-2.01 (m, 2H), 1.85-1.76 (m, 2H), 1.70-1.62 (m, 1H), 1.48-1.25 (m, 6H); LC/MS ESI (+): 221.1 (M+1).

### <Preparation Example i-2> Preparation of N¹-cyclohexylbenzene-1,2-diamine

A suspension of **Preparation Example i-1** (1.6 g, 7.04 mmol), 10% Pd/C (74 mg, 0.70 mmol) were stirred in MeOH/EtOAc (v/v = 3:1, 20 mL) under a hydrogen atmosphere for 6 h. The resulting mixture was filtered through a Celite pad and concentrated under reduced pressure. The residue was purified by silica column chromatography (with *n*-hexane/EtOAc) to give *N¹*-cyclohexylbenzene-1,2-diamine.

Brown oil (yield: 86%); ¹H NMR (400 MHz, CDCl₃) δ 6.83-6.77 (m, 1H), 6.74-6.70 (m, 1H), 6.69-6.62 (m, 2H), 3.27-3.19 (m, 4H), 2.10-2.06 (m, 2H), 1.82-1.73 (m, 2H), 1.69-1.62 (m, 1H), 1.45-1.32 (m, 2H), 1.29-1.16 (m, 3H); LC/MS ESI (+): 191.2 (M+1).

### <Preparation Example i-3> Preparation of 1-cyclohexylquinoxalin-2,3(1H,4H)-dione

A mixture of **Preparation Example i-2** (1.0 g, 5.26 mmol) in diethyl oxalate (4.3 mL, 31.53 mmol) was heated to130 °C for 2 h and then cooled to room temperature. The precipitate formed in the mixture was collected by filtration, washed with EtOAc and then dried vacuo to give 1-cyclohexylquinoxalin-2,3(1H,4H)-dione.

Gray solid (yield: 80%); ¹H NMR (400 MHz, DMSO-*d₆*) δ 11.96 (s, 1H), 7.65-7.56 (m, 1H), 7.19-7.13 (m, 3H), 4.58-4.39 (m, 1H), 2.47-2.37 (m, 2H), 1.85-1.79 (m, 2H), 1.74-1.62 (m, 3H), 1.51-1.38 (m, 2H), 1.31-1.16 (m, 1H); LC/MS ESI (+): 245.1 (M+1).

### <Preparation Example i-4> Preparation of 1-cyclohexyl-1,2,3,4-tetrahydroquinoxaline

To a solution of **Preparation Example i-3** (980 mg, 4.01 mmol) in THF (7.0 mL) was added dropwise 1M borane-THF solution (1 M) (24.0 mL, 24.07 mmol) and stirred at room temperature. The reaction mixture was heated at 65°C for 16 h. After cooling the reaction mixture was quenched with water, and the pH was adjusted to 9 with Conc. NaHCO₃ (aq.). The mixture was extracted with EtOAc, and the organic layer was dried with MgSO₄, filtered, and then concentrated under reduced pressure. The residue was purified by silica column chromatography (with n-hexane/EtOAc) to give 1-cyclohexyl-1,2,3,4-tetrahydroquinoxaline.

Orange oil (yield: 91%); ¹H NMR (400 MHz, CDCl₃) δ 6.70-6.60 (m, 2H), 6.55-6.47 (m, 2H), 3.61-3.49 (m, 1H), 3.44-3.35 (m, 2H), 3.34-3.24 (m, 2H), 1.92-1.78 (m, 4H), 1.76-1.66 (m, 1H), 1.50-1.31 (m, 4H), 1.22-1.07 (m, 1H); LC/MS ESI (+): 217.2 (M+1).

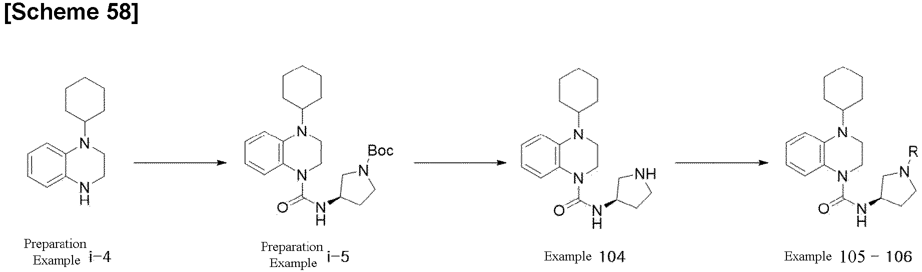

### <Preparation Example i-5> Preparation of tert-butyl (R)-3-(4-cyclohexyl-1,2,3,4-tetrahydroquinoxaline-1-carboxamido)pyrrolidine-1-carboxylate

To a solution of **Preparation Example i-4** (1.0 equiv.), TEA (3.0 equiv.) and DCM (0.3 M) cooled to 0 °C under a nitrogen atmosphere was added dropwise triphosgene (0.6 equiv.). The reaction mixture was stirred at 0 °C for 1 h then TEA (2.0 equiv.) and (R)-(+)-1-Boc-3-aminopyrrolidine (1.2 equiv.) were added slowly. The mixture warmed to room temperature and stirred for 3 h. The resulting mixture was partitioned between DCM and water. The organic layer was washed with brine, dried with MgSO₄, filtered and then concentrated under reduced pressure. The residue was purified by silica column chromatography (with *n*-hexane/EtOAc) to give *tert*-butyl (*R*)-3-(4-cyclohexyl-1,2,3,4-tetrahydroquinoxaline-1-carboxamido)pyrrolidine-1-carboxylate.

White solid (yield: 85%); ¹H NMR (400 MHz, CDCl₃) δ 7.10-7.03 (m, 2H), 6.82-6.77 (m, 1H), 6.64-6.56 (m, 1H), 5.31-5.27 (m, 1H), 4.46-4.34 (m, 1H), 3.85-3.60 (m, 4H), 3.46-3.30 (m, 4H), 3.19-3.10 (m, 1H), 2.20-2.10 (m, 1H), 1.91-1.70 (m, 6H), 1.52-1.37 (m, 13H), 1.23-1.12 (m, 1H).

### <Example 104> Synthesis of (R)-4-cyclohexyl-N-(pyrrolidin-3-yl)-3,4-dihydroquinoxaline-1(2H)-carboxamide

Following the procedure as described for Example 1, reaction of **Preparation Example i-5** (1.0 equiv.) in DCM (0.5 M) with TFA (10.0 equiv.) gave (*R*)-4-cyclohexyl-*N*-(pyrrolidin-3-yl)-3,4-dihydroquinoxaline-1(*2H*)-carboxamide.

White solid (yield: 67%); ¹H NMR (400 MHz, CDCl₃) δ 7.11-7.03 (m, 2H), 6.80-6.78 (m, 1H), 6.64-6.58 (m, 1H), 5.39 (d, *J* = 6.8 Hz, 1H), 4.34-4.25 (m, 1H), 3.78-3.71 (m, 2H), 3.67-3.59 (m, 1H), 3.31 (t, *J* = 5.4 Hz, 2H), 3.23-3.18 (m, 1H), 3.08-3.02 (m, 1H), 2.98-2.92 (m, 1H), 2.82-2.78 (m, 1H), 2.21-2.12 (m, 1H), 1.91-1.82 (m, 4H), 1.75-1.71 (m, 1H), 1.65-1.55 (m, 1H), 1.52-1.35 (m, 4H), 1.23-1.12 (m, 1H); LC/MS ESI (+): 329.3 (M+1).

### <Example 105> Synthesis of (R)-4-cyclohexyl-N-(1-methylpyrrolidin-3-yl)-3,4-dihydroquinoxaline-1(2H)-carboxamide

Following the procedure as described for Example 2, reaction of **Example 104** (1.0 equiv.) in DCM (0.3 M) with sodium triacetoxyborohydride (1.3 equiv.) and formaldehyde (1.1 equiv.) gave (*R*)-4-cyclohexyl-*N*-(1-methylpyrrolidin-3-yl)-3,4-dihydroquinoxaline-1(*2H*)-carboxamide.

Ivory solid (yield: 43%); ¹H NMR (400 MHz, CDCl₃) δ 7.11 (dd, *J* = 7.8, 1.6 Hz, 1H), 7.07-7.02 (m, 1H), 6.79-6.76 (m, 1H), 6.64-6.59 (m, 1H), 5.48 (d, *J* = 7.4 Hz, 1H), 4.43-4.35 (m, 1H), 3.83-3.77 (m, 1H), 3.71-3.60 (m, 2H), 3.33-3.28 (m, 2H), 2.77-2.72 (m, 1H), 2.63-2.59 (m, 1H), 2.55-2.51 (m, 1H), 2.34-2.22 (m, 5H), 1.90-1.82 (m, 4H), 1.75-1.72 (m, 1H), 1.58-1.52 (m, 1H), 1.49-1.37 (m, 4H), 1.21-1.14 (m, 1H); LC/MS ESI (+): 343.3 (M+1).

### <Example 106> Synthesis of (R)-4-cyclohexyl-N-(1-isobutylpyrrolidin-3-yl)-3,4-dihydroquinoxaline-1(2H)-carboxamide

The title compound was synthesized in the same manner as Example 105, using isobutyraldehyde in place of formaldehyde to give (*R*)-4-cyclohexyl-*N*-(1-isobutylpyrrolidin-3-yl)-3,4-dihydroquinoxaline-1(*2H*)-carboxamide.

White solid (yield: 46%); ¹H NMR (400 MHz, CDCl₃) δ 7.09 (dd, *J* = 7.8, 1.5 Hz, 1H), 7.05-7.00 (m, 1H), 6.77-6.75 (m, 1H), 6.61-6.57 (m, 1H), 5.59 (d, *J* = 8.0 Hz, 1H), 4.37-4.30 (m, 1H), 3.78-3.66 (m, 2H), 3.64-3.58 (m, 1H), 3.28 (t, *J* = 5.3 Hz, 1H), 2.77-2.72 (m, 1H), 2.53-2.46 (m, 2H), 2.25-2.11 (m, 4H), 1.88-1.79 (m, 4H), 1.74-1.64 (m, 2H), 1.54-1.32 (m, 5H), 1.21-1.10 (m, 1H), 0.87 (dd, *J* = 6.6, 3.9 Hz, 6H); LC/MS ESI (+): 385.4 (M+1).

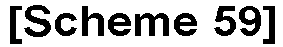

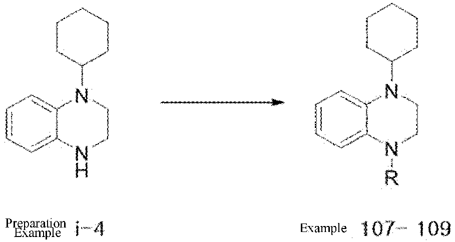

### <Example 107> Synthesis of (4-cyclohexyl-3,4-dihydroquinoxalin-1(2H)-yl)(4-methylpiperazin-1-yl)methanone

The title compound was synthesized in the same manner as Preparation Example i-5, using 1-methylpiperazine in place of (*R*)-(+)-1-Boc-3-aminopyrrolidine to give (4-cyclohexyl-3,4-dihydroquinoxalin-1(*2H*)-yl)(4-methylpiperazin-1-yl)methanone.

Brown oil (yield: 81%); ¹H NMR (400 MHz, CDCl₃) δ 6.96 (dd, *J* = 7.9, 1.5 Hz, 1H), 6.93-6.89 (m, 1H), 6.72-6.70 (m, 1H), 6.59-6.63 (m, 1H), 3.65-3.57 (m, 3H), 3.38-3.33 (m, 6H), 2.26-2.33 (m, 4H), 2.28 (s, 3H), 1.89-1.81 (m, 4H), 1.73-1.70 (m, 1H), 1.50-1.33 (m, 4H), 1.17-1.13 (m, 1H); LC/MS ESI (+): 343.3 (M+1).

### <Example 108> Synthesis of (4-cyclohexyl-3,4-dihydroquinoxalin-1(2H)-yl)(pyrrolidin-1-yl)methanone

The title compound was synthesized in the same manner as Preparation Example i-5, using pyrrolidine in place of (*R*)-(+)-1-Boc-3-aminopyrrolidine to give (4-cyclohexyl-3,4-dihydroquinoxalin-1(*2H*)-yl)(pyrrolidin-1-yl)methanone.

Ivory solid (yield: 54%); ¹H NMR (400 MHz, CDCl₃) δ 6.91-6.84 (m, 2H), 6.70-6.68 (m, 1H), 6.57-6.53 (m, 1H), 3.67 (t, *J* = 5.4 Hz, 2H), 3.63-3.57 (m, 1H), 3.37 (t, *J =* 5.3 Hz, 2H), 3.28-3.25 (m, 4H), 1.88-1.76 (m, 8H), 1.73-1.69 (m, 1H), 1.51-1.33 (m, 4H), 1.21-1.10 (m, 1H); LC/MS ESI (+): 314.3 (M+1).

### <Example 109> Synthesis of (4-cyclohexyl-3,4-dihydroquinoxalin-1(2H)-yl)(piperidin-1-yl)methanone

The title compound was synthesized in the same manner as Preparation Example i-5, using piperidine in place of (*R*)-(+)-1-Boc-3-aminopyrrolidine to give (4-cyclohexyl-3,4-dihydroquinoxalin-1(*2H*)-yl)(piperidin-1-yl)methanone.

Red oil (yield: 80%); ¹H NMR (400 MHz, CDCl₃) δ 6.94-6.87 (m, 2H), 6.71-6.69 (m, 1H), 6.58-6.53 (m, 1H), 3.62-3.58 (m, 3H), 3.36 (t, *J* = 5.3 Hz, 2H), 3.27-3.24 (m, 4H), 1.88-1.82 (m, 4H), 1.73-1.69 (m, 1H), 1.55-1.36 (m, 10H), 1.21-1.11 (m, 1H); LC/MS ESI (+): 328.0 (M+1).

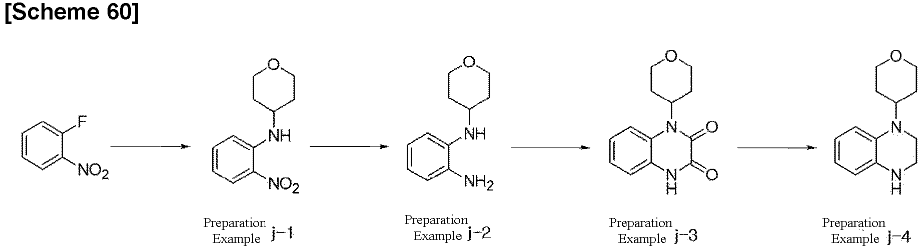

### <Preparation Process j> Preparation of 1-(tetrahydro-2H-pyran-4-yl)-1,2,3,4-tetrahydroquinoxaline

1-(Tetrahydro-*2H*-pyran-4-yl)-1,2,3,4-tetrahydroquinoxaline according to the present invention was synthesized through Preparation Examples j-1 to j-4 below.

### <Preparation Example j-1> Preparation of N-(2-nitrophenyl)tetrahydro-2H-pyran-4-amine

A mixture of 1-fluoro-2-nitrobenzene (1.0 equiv.) and tetrahydro-*2H*-pyran-4-amine (1.2 equiv.) was stirred at 110 °C for 6 h. After cooling the reaction mixture was quenched with water and extracted with DCM. The organic layer was washed with brine, dried with MgSO₄, filtered, and then concentrated under reduced pressure. The residue was purified by silica column chromatography (with n-hexane/EtOAc) to give *N*-(2-nitrophenyl)tetrahydro-*2H*-pyran-4-amine.

Orange solid (yield: 91%); ¹H NMR (400 MHz, CDCl₃) δ 8.25-8.18 (m, 1H), 8.12 (s, 1H), 7.49-7.40 (m, 1H), 6.93-6.86 (m, 1H), 6.72-6.63 (m, 1H), 4.10-4.00 (m, 2H), 3.83-3.70 (m, 1H), 3.65-3.54 (m, 2H), 2.16-2.04 (m, 2H), 1.78-1.63 (m, 2H); LC/MS ESI (+): 223.1 (M+1).

### <Preparation Example j-2> Preparation of N¹-tetrahydro-2H-pyran-4-yl)benzene-1,2-diamine

A suspension of **Preparation Example j-1** (1.0 equiv.), 10% Pd/C (0.2 equiv.) were stirred in MeOH/EtOAc (v/v = 3:1, 20 mL) under a hydrogen atmosphere for 6 h. The resulting mixture was filtered through a Celite pad and concentrated under reduced pressure. The residue was purified by silica column chromatography (with *n-*hexane/EtOAc) to give *N¹*-tetrahydro-*2H*-pyran-4-yl)benzene-1,2-diamine.

Brown solid (yield: 91%); ¹H NMR (400 MHz, CDCl₃) δ 6.86-6.80 (m, 1H), 6.79-6.68 (m, 3H), 4.08-3.99 (m, 2H), 3.61-3.43 (m, 3H), 3.37 (s, 2H), 3.27 (s, 1H), 2.12-2.01 (m, 2H), 1.62-1.48 (m, 2H); LC/MS ESI (+): 193.2 (M+1).

### <Preparation Example j-3> Preparation of 1-(tetrahydro-2H-pyran-4-yl)hydroquinoxalin-2,3-dione

A mixture of **Preparation Example j-2** (1.0 equiv.) in diethyl oxalate (6.0 equiv.) was heated to130 °C for 2 h and then cooled to room temperature. The precipitate formed in the mixture was collected by filtration, washed with EtOAc and then dried vacuo to give 1-(tetrahydro-*2H*-pyran-4-yl)hydroquinoxalin-2,3-dione.

Brown solid (yield: 70%); ¹H NMR (400 MHz, DMSO-*d₆*) δ 11.99 (s, 1H), 7.78-7.57 (m, 1H), 7.25-7.13 (m, 3H), 4.31-4.21 (m, 3H), 2.79-2.64 (m, 2H), 1.66-1.57 (m, 2H), 1.10-1.02 (m, 2H); LC/MS ESI (+): 247.1 (M+1).

### <Preparation Example j-4> Preparation of 1-(tetrahydro-2H-pyran-4-yl)-1,2,3,4-tetrahydroquinoxaline

To a solution of **Preparation Example j-3** (1.0 equiv.) in THF (0.5 M) was added dropwise 1M borane-THF solution (1.0 M) (6.0 equiv.) and stirred at room temperature. The reaction mixture was heated at 65°C for 16 h. After cooling the reaction mixture was quenched with water, and the pH was adjusted to 9 with Conc. NaHCO₃ (aq). The mixture was extracted with EtOAc, and the organic layer was dried with MgSO₄, filtered, and then concentrated under reduced pressure. The residue was purified by silica column chromatography (with *n*-hexane/EtOAc) to give 1-(tetrahydro-*2H*-pyran-4-yl)-1,2,3,4-tetrahydroquinoxaline.

White solid (yield: 79%); ¹H NMR (400 MHz, CD₃OD) δ 6.77-6.70 (m, 1H), 6.67-6.46 (m, 3H), 4.09-4.01 (m, 2H), 3.93-3.81 (m, 1H), 3.62-3.51 (m, 2H), 3.34-3.29 (m, 3H), 3.28-3.25 (m, 2H), 1.94-1.77 (m, 2H), 1.76-1.66 (m, 2H); LC/MS ESI (+): 219.2 (M+1).

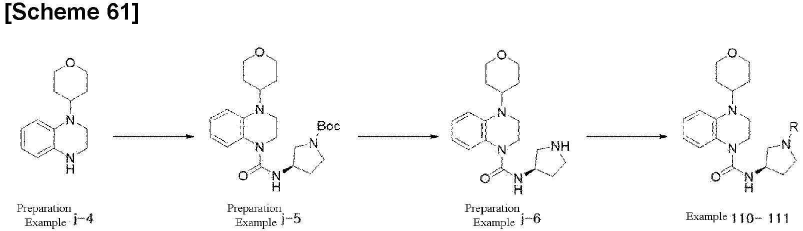

### <Preparation Example j-5> Preparation of tert-butyl (R)-3-(4-(tetrahydro-2H-pyran-4-yl)-1,2,3,4-tetrahydroquinoxaline-1-carboxamido)pyrrolidine-1-carboxylate

To a solution of **Preparation Example j-4** (1.0 equiv.), TEA (3.0 equiv.) and DCM (0.3 M) cooled to 0 °C under a nitrogen atmosphere was added dropwise triphosgene (0.6 equiv.). The reaction mixture was stirred at 0 °C for 1 h then TEA (2.0 equiv.) and (R)-(+)-1-Boc-3-aminopyrrolidine (1.2 equiv.) were added slowly. The mixture warmed to room temperature and stirred for 3 h. The resulting mixture was partitioned between DCM and water. The organic layer was washed with brine, dried with MgSO₄, filtered and then concentrated under reduced pressure. The residue was purified by silica column chromatography (with *n*-hexane/EtOAc) to give *tert*-butyl (*R*)-3-(4-(tetrahydro-*2H*-pyran-4-yl)-1,2,3,4-tetrahydroquinoxaline-1-carboxamido)pyrrolidine-1-carboxylate.

White solid (yield: 94.3%); ¹H NMR (400 MHz, CDCl₃) δ 7.10-7.02 (m, 2H), 6.85-6.77 (m, 1H), 6.68-6.58 (m, 1H), 5.27 (d, *J* = 6.8 Hz, 1H), 4.46-4.32 (m, 1H), 4.12-4.04 (m, 2H), 3.94-3.83 (m, 1H), 3.82-3.74 (m, 1H), 3.73-3.58 (m, 1H), 3.57-3.46 (m, 2H), 3.45-3.23 (m, 5H), 3.16-3.09 (m, 1H), 2.17-2.10 (m, 1H), 1.93-1.79 (m, 2H), 1.74-1.66 (m, 2H), 1.44 (s, 9H); LC/MS ESI (+): 431.2 (M+1).

### <Preparation Example j-6> Preparation of (R)-N-(pyrrolidin-3-yl)-4-(tetrahydro-2H-pyran-4-yl)-3,4-dihydroquinoxaline-1(2H)-carboxamide

Following the procedure as described for Example 1, reaction of **Preparation Example j-5** (1.0 equiv.) in DCM (0.5 M) with TFA (10.0 equiv.) gave (*R*)-*N*-(pyrrolidin-3-yl)-4-(tetrahydro-*2H*-pyran-4-yl)-3,4-dihydroquinoxaline-1(*2H*)-carboxamide.

White solid (yield: 84.3%); ¹H NMR (400 MHz, CDCl₃) δ 7.13 (dd, *J* = 7.8, 1.6 Hz, 1H), 7.08-7.03 (m, 1H), 6.86-6.78 (m, 1H), 6.67-6.61 (m, 1H), 5.44 (d, *J*= 6.7 Hz, 1H), 4.40-4.28 (m, 1H), 4.11-4.05 (m, 2H), 3.92-3.79 (m, 1H), 3.78-3.63 (m, 2H), 3.55-3.47 (m, 2H), 3.34-3.18 (m, 4H), 3.17-3.05 (m, 1H), 3.04-2.96 (m, 1H), 2.92-2.86 (m, 1H), 2.24-2.11 (m, 1H), 1.95-1.77 (m, 2H), 1.76-1.57 (m, 3H); LC/MS ESI (+): 331.2 (M+1).

### <Example 110> Synthesis of (R)-N-(1-methylpyrrolidin-3-yl)-4-(tetrahydro-2H-pyran-4-yl)-3,4-dihydroquinoxaline-1(2H)-carboxamide

Following the procedure as described for Example 2, reaction of **Preparation Example j-6** (1.0 equiv.) in DCM (0.3 M) with sodium triacetoxyborohydride (1.3 equiv.) and formaldehyde (1.1 equiv.) gave (*R*)-*N*-(1-methylpyrrolidin-3-yl)-4-(tetrahydro-2H-pyran-4-yl)-3,4-dihydroquinoxaline-1(*2H*)-carboxamide.

Light yellow oil (yield: 82.5%); ¹H NMR (400 MHz, CDCl₃) δ 7.11 (dd, *J* = 7.8, 1.6 Hz, 1H), 7.08-6.98 (m, 1H), 6.79 (dd, *J* = 8.3, 1.1 Hz, 1H), 6.67-6.59 (m, 1H), 5.44 (d, *J* = 7.5 Hz, 1H), 4.42-4.30 (m, 1H), 4.13-4.04 (m, 2H), 3.94-3.77 (m, 2H), 3.74-3.62 (m, 1H), 3.56-3.45 (m, 2H), 3.35-3.22 (m, 2H), 2.79-2.69 (m, 1H), 2.60-2.49 (m, 2H), 2.35-2.26 (m, 4H), 2.25-2.16 (m, 1H), 1.93-1.77 (m, 2H), 1.75-1.72 (m, 2H), 1.59-1.46 (m, 1H); LC/MS ESI (+): 345.2 (M+1).

### <Example 111> Synthesis of (R)-N-(1-isobutylpyrrolidin-3-yl)-4-(tetrahydro-2H-pyran-4-yl)-3,4-dihydroquinoxaline-1(2H)-carboxamide

The title compound was synthesized in the same manner as Example 110, using isobutyraldehyde in place of formaldehyde to give (*R*)-*N*-(1-isobutylpyrrolidin-3-yl)-4-(tetrahydro-2*H*-pyran-4-yl)-3,4-dihydroquinoxaline-1(2*H*)-carboxamide.

Light yellow oil (yield: 86.3%); ¹H NMR (400 MHz, CDCl₃) δ 7.11 (dd, *J* = 7.8, 1.6 Hz, 1H), 7.07-6.98 (m, 1H), 6.83-6.76 (m, 1H), 6.67-6.58 (m, 1H), 5.59 (d, *J* = 7.9 Hz, 1H), 4.39-4.28 (m, 1H), 4.13-4.04 (m, 2H), 3.95-3.83 (m, 1H), 3.83-3.67 (m, 2H), 3.51 (t, *J* = 2.1 Hz, 2H), 3.29 (t, *J* = 5.3 Hz, 2H), 2.82-2.71 (m, 1H), 2.56-2.49 (m, 1H), 2.49-2.41 (m, 1H), 2.27-2.08 (m, 4H), 1.93-1.78 (m, 2H), 1.76-1.67 (m, 3H), 1.59-1.48 (m, 1H), 0.92-0.80 (m, 6H); LC/MS ESI (+): 387.3 (M+1).

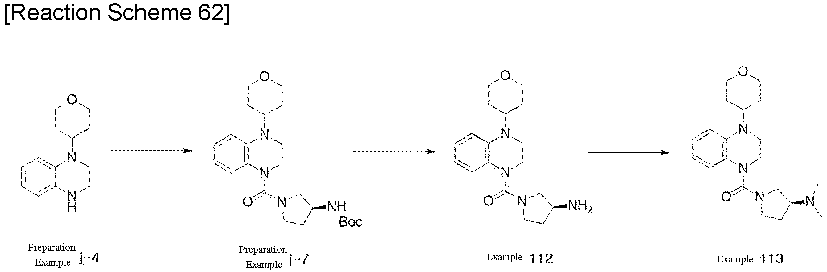

### <Preparation Example j-7> Preparation of tert-butyl (S)-(1-(4-(tetrahydro-2H-pyran-4-yl)-1,2,3,4-tetrahydroquinoxaline-1-carboxamido)pyrrolidin-3-yl)carbamate

The title compound was prepared in the same manner as Preparation Example j-5, using (*S*)-(-)-3-(Boc-amino)pyrrolidine in place of (*R*)-(+)-1-Boc-3-aminopyrrolidine to give *tert*-butyl (*S*)-(1-(4-(tetrahydro-*2H*-pyran-4-yl)-1,2,3,4-tetrahydroquinoxaline-1-carboxamido)pyrrolidin-3-yl)carbamate.

Ivory solid (yield: 90.2%); ¹H NMR (400 MHz, CDCl₃) δ 6.96-6.88 (m, 1H), 6.86 (dd, *J* = 7.9, 1.5 Hz, 1H), 6.77-6.70 (m, 1H), 6.64-6.55 (m, 1H), 4.60-4.47 (m, 1H), 4.15-4.06 (m, 3H), 3.91-3.81 (m, 1H), 3.68 (t, *J* = 4.5 Hz, 2H), 3.57-3.29 (m, 6H), 3.09-3.01 (m, 1H), 2.13-2.03 (m, 1H), 1.95-1.79 (m, 2H), 1.77-1.71 (m, 3H), 1.42 (s, 9H); LC/MS ESI (+): 431.2 (M+1).

### <Example 112> Synthesis of (S)-(3-aminopyrrolidin-1-yl)(4-(tetrahydro-2H-pyran-4-yl)-3,4-dihydroquinoxalin-1(2H)-yl)methanone

Following the procedure as described for Example 1, reaction of **Preparation Example j-7** (1.0 equiv.) in DCM (0.5 M) with TFA (10.0 equiv.) gave (*S*)-(3-aminopyrrolidin-1-yl)(4-(tetrahydro-2H-pyran-4-yl)-3,4-dihydroquinoxalin-1(*2H*)-yl)methanone.

Light yellow oil (yield: 69.7%); ¹H NMR (400 MHz, CDCl₃) δ 6.96-6.85 (m, 2H), 6.76-6.71 (m, 1H), 6.65-6.55 (m, 1H), 4.13-4.04 (m, 2H), 3.86 (t, *J =* 3.9 Hz, 1H), 3.78-3.69 (m, 1H), 3.68-3.56 (m, 1H), 3.56-3.41 (m, 5H), 3.41-3.26 (m, 3H), 3.03-2.89 (m, 1H), 2.07-1.95 (m, 1H), 1.93-1.78 (m, 2H), 1.78-1.69 (m, 2H), 1.67-1.54 (m, 1H), 1.44-1.37 (m, 2H); LC/MS ESI (+): 331.2 (M+1).

### <Example 113> Synthesis of (S)-(3-(dimethylamino)pyrrolidin-1-yl)(4-(tetrahydro-2H-pyran-4-yl)-3,4-dihydroquinoxalin-1(2H)-yl)methanone

Following the procedure as described for Example 2, reaction of **Example 112** (1.0 equiv.) in DCM (0.1 M) with sodium triacetoxyborohydride (3.0 equiv.) and formaldehyde (3.0 equiv.) gave (*S*)-(3-(dimethylamino)pyrrolidin-1-yl)(4-(tetrahydro-*2H*-pyran-4-yl)-3,4-dihydroquinoxalin-1(*2H*)-yl)methanone.

Light yellow oil (yield: 16.5%); ¹H NMR (400 MHz, CDCl₃) δ 6.94-6.83 (m, 2H), 6.73 (dd, *J* = 8.1, 1.2 Hz, 1H), 6.63-6.55 (m, 1H), 4.09 (d, *J* = 1.5 Hz, 2H), 4.03-3.94 (m, 1H), 3.92-3.83 (m, 1H), 3.58-3.43 (m, 3H), 3.42-3.25 (m, 5H), 3.15 (dd, *J* = 10.7, 8.9 Hz, 1H), 2.68-2.56 (m, 1H), 2.20 (s, 6H), 2.05-1.95 (m, 1H), 1.94-1.78 (m, 2H), 1.77-1.67 (m, 3H); LC/MS ESI (+): 359.3 (M+1).

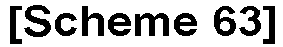

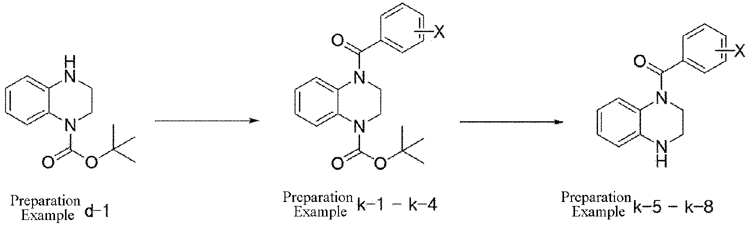

### <Preparation Process k> Preparation of (3,4-dihydroquinoxalin-1(2H)-yl)(phenyl)methanone or halogen-substituted (3,4-dihydroquinoxalin-1(2H)-yl)(phenyl)methanone

(3,4-Dihydroquinoxalin-1(*2H*)-yl)(phenyl)methanone or halogen-substituted (3,4-dihydroquinoxalin-1(*2H*)-yl)(phenyl)methanone according to the present invention was synthesized through Preparation Examples k-1 to k-8 below.

### <Preparation Example k-1> Preparation of tert-butyl 4-benzoyl-3,4-dihydroquinoxaline-1(2H)-carboxylate

To a solution of **Preparation Example d-1** (473 mg, 2.02 mmol) in THF (20 mL) was added benzoyl chloride (117 µL, 1.01 mmol). The resulting mixture was stirred at room temperature for 3 h. The reaction mixture was quenched with water, and the pH was adjusted to 8 with Conc. NaHCO₃ (aq.). The mixture was extracted with DCM, the organic layer dried with MgSO₄, filtered, and then concentrated under reduced pressure. The residue was purified by silica column chromatography (with n-hexane/EtOAc) to give *tert*-butyl 4-benzoyl-3,4-dihydroquinoxaline-1(*2H*)-carboxylate.

Yellow oil (yield: 100%); ¹H NMR (400 MHz, CDCl₃) δ 7.92-7.83 (m, 1H), 7.45-7.34 (m, 3H), 7.31-7.26 (m, 2H), 7.05 (d, *J* = 1.6 Hz, 1H), 6.73 (s, 1H), 6.65-6.54 (m, 1H), 4.09-4.02 (m, 2H), 3.98-3.91 (m, 2H), 1.57 (s, 9H).

### <Preparation Example k-2> Preparation of tert-butyl 4-(2-fluorobenzoyl)-3,4-dihydroquinoxaline-1(2H)-carboxylate

The title compound was prepared in the same manner as Preparation Example k-1, using 2-fluorobenzoyl chloride in place of benzoyl chloride to give *tert*-butyl 4-(2-fluorobenzoyl)-3,4-dihydroquinoxaline-1(*2H*)-carboxylate.

Colorless oil (yield: 84%); ¹H NMR (400 MHz, CDCl₃) δ 7.96-7.82 (m, 1H), 7.51-7.45 (m, 1H), 7.42-7.32 (m, 1H), 7.22-7.15 (m, 1H), 7.13-7.07 (m, 1H), 7.02-6.89 (m, 1H), 6.85-6.40 (m, 2H), 4.12-3.95 (m, 4H), 1.57 (s, 9H).

### <Preparation Example k-3> Preparation of tert-butyl 4-(3-fluorobenzoyl)-3,4-dihydroquinoxaline-1(2H)-carboxylate

The title compound was prepared in the same manner as Preparation Example k-1, using 3-fluorobenzoyl chloride in place of benzoyl chloride to give *tert*-butyl 4-(3-fluorobenzoyl)-3,4-dihydroquinoxaline-1(*2H*)-carboxylate.

Colorless oil (yield: 72%); ¹H NMR (400 MHz, CDCl₃) δ 7.93-7.83 (m, 1H), 7.25-7.21 (m, 1H), 7.20-7.16 (m, 1H), 7.15-7.12 (m, 1H), 7.11-7.06 (m, 2H), 6.80-6.78 (m, 1H), 6.65-6.55 (m, 1H), 4.08-4.03 (m, 2H), 3.97-3.93 (m, 2H), 1.57 (s, 9H).

### <Preparation Example k-4> Preparation of tert-butyl 4-(4-fluorobenzoyl)-3,4-dihydroquinoxaline-1(2H)-carboxylate

The title compound was prepared in the same manner as Preparation Example k-1, using 4-fluorobenzoyl chloride in place of benzoyl chloride to give *tert*-butyl 4-(4-fluorobenzoyl)-3,4-dihydroquinoxaline-1(*2H*)-carboxylate.

Colorless oil (yield: 93%); ¹H NMR (400 MHz, CDCl₃) δ 7.95-7.85 (m, 1H), 7.46-7.41 (m, 2H), 7.10-7.05 (m, 1H), 7.01-6.94 (m, 2H), 6.77-6.73 (m, 1H), 6.57-6.50 (m, 1H), 4.08-4.05 (m, 2H), 3.97-3.93 (m, 2H), 1.57 (s, 9H).

### <Preparation Example k-5> Preparation of (3,4-dihydroquinoxalin-1(2H)-yl)(phenyl)methanone

Following the procedure as described for Example 1, reaction of **Preparation Example k-1** (1.0 equiv.) in DCM (0.5 M) with TFA (10.0 equiv.) gave (3,4-dihydroquinoxalin-1(*2H*)-yl)(phenyl)methanone.

Light yellow oil (yield: 92%); ¹H NMR (400 MHz, CDCl₃) δ 7.49-7.35 (m, 3H), 7.34-7.28 (m, 2H), 6.91-6.83 (m, 1H), 6.59 (dd, *J* = 8.0, 0.9 Hz, 2H), 6.40-6.34 (m, 1H), 4.07 (brs, 1H), 3.98 (t, *J* = 5.1 Hz, 2H), 3.56 (t, *J* = 5.1 Hz, 2H); LC/MS ESI (+): 239.1 (M+1).

### <Preparation Example k-6> Preparation of (3,4-dihydroquinoxalin-1(2H)-yl)(2-fluorophenyl)methanone

Following the procedure as described for Example 1, reaction of **Preparation Example k-2** (1.0 equiv.) in DCM (0.5 M) with TFA (10.0 equiv.) gave (3,4-dihydroquinoxalin-1(*2H*)-yl)(2-fluorophenyl)methanone.

White solid (yield: 87%); ¹H NMR (400 MHz, CDCl₃) δ 7.52-7.46 (m, 1H), 7.42-7.28 (m, 1H), 7.24-7.12 (m, 1H), 7.05-6.75 (m, 2H), 6.58 (d, *J =* 7.9 Hz, 1H), 6.42-6.17 (m, 1H), 4.50-3.87 (m, 2H), 3.80-3.20 (m, 3H); LC/MS ESI (+): 257.1 (M+1).

### <Preparation Example k-7> Preparation of (3,4-dihydroquinoxalin-1(2H)-yl)(3-fluorophenyl)methanone

Following the procedure as described for Example 1, reaction of **Preparation Example k-3** (1.0 equiv.) in DCM (0.5 M) with TFA (10.0 equiv.) gave (3,4-dihydroquinoxalin-1(*2H*)-yl)(3-fluorophenyl)methanone.

White solid (yield: 87%); ¹H NMR (400 MHz, CDCl₃) δ 7.30-7.27 (m, 1H), 7.21-7.16 (m, 2H), 7.09-7.05 (m, 1H), 6.92-6.87 (m, 1H), 6.68-6.45 (m, 2H), 6.44-6.30 (m, 1H), 4.12 (brs, 1H), 3.97 (t, *J* = 4.9 Hz, 2H), 3.62-3.50 (m, 2H); LC/MS ESI (+): 257.1 (M+1).

### <Preparation Example k-8> Preparation of (3,4-dihydroquinoxalin-1(2H)-yl)(4-fluorophenyl)methanone

Following the procedure as described for Example 1, reaction of **Preparation Example k-4** (1.0 equiv.) in DCM (0.5 M) with TFA (10.0 equiv.) gave (3,4-dihydroquinoxalin-1(*2H*)-yl)(4-fluorophenyl)methanone.

White solid (yield: 93%); ¹H NMR (400 MHz, CDCl₃) δ 7.48-7.44 (m, 1H), 7.01-6.96 (m, 2H), 6.90-6.85 (m, 1H), 6.65-6.45 (m, 2H), 6.38-6.34 (m, 1H), 4.14 (brs, 1H), 3.98-3.96 (m, 2H), 3.56 (t, *J* = 4.9 Hz, 2H); LC/MS ESI (+): 257.1 (M+1).

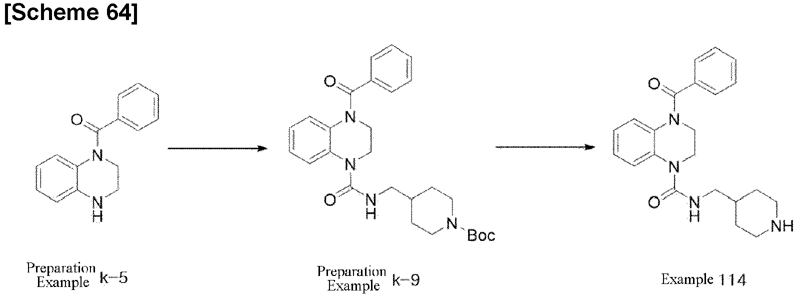

### <Preparation Example k-9> Preparation of tert-butyl 4-((4-benzoyl-1,2,3,4-tetrahydroquinoxaline-1-carboxamido)methyl)piperidine-1-carboxylate

To a solution of **Preparation Example k-5** (1.0 equiv.), TEA (3.0 equiv.) and DCM (0.3 M) cooled to 0 °C under a nitrogen atmosphere was added dropwise triphosgene (0.6 equiv.). The reaction mixture was stirred at 0 °C for 1 h then TEA (2.0 equiv.) and 1-Boc-4-(aminomethyl)piperidine (1.2 equiv.) were added slowly. The mixture warmed to room temperature and stirred for 3 h. The resulting mixture was partitioned between DCM and water. The organic layer was washed with brine, dried with MgSO₄, filtered and then concentrated under reduced pressure. The residue was purified by silica column chromatography (with n-hexane/EtOAc) to give 4-((4-benzoyl-1,2,3,4-tetrahydroquinoxaline-1-carboxamido)methyl)piperidine-1-carboxylate.

White solid (yield: 90%); ¹H NMR (400 MHz, CDCl₃) δ 7.43-7.40 (m, 4H), 7.35-7.31 (m, 2H), 7.15-7.10 (m, 1H), 6.94-6.85 (m, 2H), 5.33-5.31 (m, 1H), 4.22-4.04 (m, 4H), 4.03-3.96 (m, 2H), 3.30-3.17 (m, 2H), 2.80-2.65 (m, 2H), 1.80-1.70 (m, 3H), 1.47 (s, 9H), 1.22-1.14 (m, 2H).

### <Example 114> Synthesis of 4-benzoyl-N-(piperidin-4-ylmethyl)-3,4-dihydroquinoxaline-1(2H)-carboxamide

Following the procedure as described for Example 1, reaction of **Preparation Example K-9** (1.0 equiv.) in DCM (0.5 M) with TFA (10.0 equiv.) gave 4-benzoyl-*N-*(piperidin-4-ylmethyl)-3,4-dihydroquinoxaline-1(*2H*)-carboxamide.

White solid (yield: 67%); ¹H NMR (400 MHz, CD₃OD) δ 7.55-7.53 (m, 1H), 7.43-7.39 (m, 3H), 7.34-7.30 (m, 2H), 7.14-7.10 (m, 1H), 6.84-6.79 (m, 1H), 6.68 (d, *J* = 7.9 Hz, 1H), 4.08-4.05 (m, 2H), 3.93-3.89 (m, 2H), 3.17-3.10 (m, 4H), 2.69-2.62 (m, 2H), 1.81-1.74 (m, 3H), 1.32-1.21 (m, 2H);LC/MS ESI (+): 379.3 (M+1).

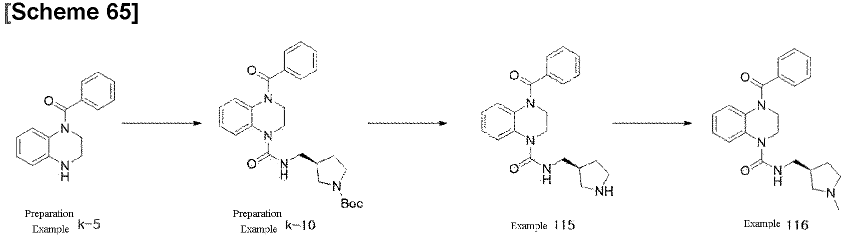

### <Preparation Example k-10> Preparation of tert-butyl (R)-3-((4-benzoyl-1,2,3,4-tetrahydroquinoxaline-1-carboxamido)methyl)pyrrolidine-1-carboxylate

The title compound was prepared in the same manner as Preparation Example k-9, using (*R*)-1-Boc-3-(aminomethyl)pyrrolidine in place of 1-Boc-4-(aminomethyl)piperidine to give *tert*-butyl (*R*)-3-((4-benzoyl-1,2,3,4-tetrahydroquinoxaline-1-carboxamido)methyl)pyrrolidine-1-carboxylate.

White solid (yield: 94%); ¹H NMR (400 MHz, CDCl₃) δ 7.42-7.38 (m, 4H), 7.34-7.31 (m, 2H), 7.15-7.07 (m, 1H), 6.95-6.80 (m, 2H), 5.39-5.27 (m, 1H), 4.07-4.03 (m, 2H), 4.00-3.94 (m, 2H), 3.59-3.18 (m, 5H), 3.12-2.99 (m, 1H), 2.56-2.42 (m, 1H), 2.05-1.97 (m, 1H), 1.72-1.60 (m, 1H), 1.46 (s, 9H).

### <Example 115> Synthesis of (S)-4-benzoyl-N-(pyrrolidin-3-ylmethyl)-3,4-dihydroquinoxaline-1(2H)-carboxamide

Following the procedure as described for Example 1, reaction of **Preparation Example K-10** (1.0 equiv.) in DCM (0.5 M) with TFA (10.0 equiv.) gave (*S*)-4-benzoyl-*N-*(pyrrolidin-3-ylmethyl)-3,4-dihydroquinoxaline-1(*2H*)-carboxamide.

White solid (yield: 92%); ¹H NMR (400 MHz, CD₃OD) δ 7.56 (dd, *J* = 8.3, 1.1 Hz, 1H), 7.48-7.42 (m, 3H), 7.37-7.33 (m, 2H), 7.18-7.13 (m, 1H), 6.87-6.83 (m, 1H), 6.75-6.65 (m, 1H), 4.11-4.07 (m, 2H), 3.96-3.91 (m, 2H), 3.36-3.30 (m, 1H), 3.27-3.19 (m, 2H), 3.14-3.07 (m, 1H), 3.91-2.85 (m, 1H), 2.62-2.53 (m, 1H), 2.13-2.06 (m, 1H), 1.75-1.66 (m, 1H); LC/MS ESI (+): 365.2 (M+1).

### <Example 116> Synthesis of (R)-4-benzoyl-N-((1-methylpyrrolidin-3-yl)methyl)-3,4-dihydroquinoxaline-1(2H)-carboxamide

Following the procedure as described for Example 2, reaction of **Example 115** (1.0 equiv.) in DCM (0.3 M) with sodium triacetoxyborohydride (1.5 equiv.) and formaldehyde (2.0 equiv.) gave (*R*)-4-benzoyl-*N*-((1-methylpyrrolidin-3-yl)methyl)-3,4-dihydroquinoxaline-1(*2H*)-carboxamide.

White solid (yield: 82%); ¹H NMR (400 MHz, CD₃OD) δ 7.52 (dd, *J* = 8.2, 1.2 Hz, 1H), 7.45-7.39 (m, 3H), 7.34-7.30 (m, 2H), 7.15-7.10 (m, 1H), 6.84-6.80 (m, 1H), 6.68 (d, *J* = 7.9 Hz, 1H), 4.08-4.04 (m, 2H), 3.92-3.89 (m, 2H), 3.26 (d, *J* = 7.1 Hz, 2H), 2.79-2.74 (m, 1H), 2.65-2.61 (m, 2H), 2.58-2.52 (m, 1H), 2.43-2.39 (m, 1H), 2.36 (s, 3H), 2.07-2.00 (m, 1H), 1.65-1.58 (m, 1H); LC/MS ESI (+): 379.1 (M+1).

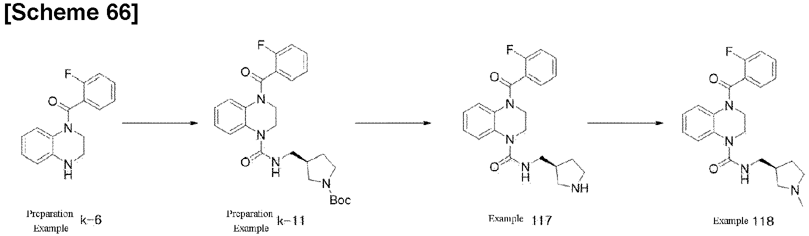

### <Preparation Example k-11> Preparation of tert-butyl (R)-3-((4-(2-fluorophenyl)-1,2,3,4-tetrahydroquinoxaline-1-carboxamido)methyl)pyrrolidine-1-carboxylate

To a solution of **Preparation Example k-6** (1.0 equiv.), TEA (3.0 equiv.) and DCM (0.3 M) cooled to 0 °C under a nitrogen atmosphere was added dropwise triphosgene (0.6 equiv.). The reaction mixture was stirred at 0 °C for 1 h then TEA (2.0 equiv.) and (*R*)-1-Boc-3-(aminomethyl)pyrrolidine (1.2 equiv.) were added slowly. The mixture warmed to room temperature and stirred for 3 h. The resulting mixture was partitioned between DCM and water. The organic layer was washed with brine, dried with MgSO₄, filtered and then concentrated under reduced pressure. The residue was purified by silica column chromatography (with *n*-hexane/EtOAc) to give *tert*-butyl (*R*)-3-((4-(2-fluorophenyl)-1,2,3,4-tetrahydroquinoxaline-1-carboxamido)methyl)pyrrolidine-1-carboxylate.

White solid (yield: 97%); ¹H NMR (400 MHz, CDCl₃) δ 7.65-7.55 (m, 1H), 7.42-7.31 (m, 2H), 7.25-7.22 (m, 1H), 7.15-7.11 (m, 1H), 6.95-6.75 (m, 3H), 5.25-5.15 (m, 1H), 4.10-3.90 (m, 4H), 3.55-3.40 (m, 3H), 3.35-3.25 (m, 2H), 3.10-2.95 (m, 1H), 2.50-2.40 (m, 1H), 2.04-1.95 (m, 1H), 1.70-1.55 (m, 1H), 1.45 (s, 9H).

### <Example 117> Synthesis of (S)-4-(2-fluorobenzoyl)-N-(pyrrolidin-3-ylmethyl)-3,4-dihydroquinoxaline-1(2H)-carboxamide

Following the procedure as described for Example 1, reaction of **Preparation Example K-10** (1.0 equiv.) in DCM (0.5 M) with TFA (10.0 equiv.) gave (*S*)-4-(2-fluorobenzoyl)-*N*-(pyrrolidin-3-ylmethyl)-3,4-dihydroquinoxaline-1(*2H*)-carboxamide.

White solid (yield: 71%); ¹H NMR (400 MHz, CD₃OD) δ 7.54-7.43 (m, 3H), 7.26-7.23 (m, 1H), 7.17-7.13 (m, 1H), 7.09-6.91 (m, 1H), 6.90-6.50 (m, 2H), 4.19-4.00 (m, 2H), 3.98-3.85 (m, 2H), 3.28-3.24 (m, 2H), 3.15-3.09 (m, 2H), 3.04-2.97 (m, 1H), 2.80-2.76 (m, 1H), 2.53-2.46 (m, 1H), 2.05-1.96 (m, 1H), 1.66-1.57 (m, 1H); LC/MS ESI (+): 383.3 (M+1).

### <Example 118> Synthesis of (R)-4-(2-fluorobenzoyl)-N-((1-methylpyrrolidin-3-yl)methyl)-3,4-dihydroquinoxaline-1(2H)-carboxamide

Following the procedure as described for Example 2, reaction of **Example 117** (1.0 equiv.) in DCM (0.3 M) with sodium triacetoxyborohydride (1.5 equiv.) and formaldehyde (2.0 equiv.) gave (*R*)-4-(2-fluorobenzoyl)-*N*-((1-methylpyrrolidin-3-yl)methyl)-3,4-dihydroquinoxaline-1(*2H*)-carboxamide.

White solid (yield: 76%); ¹H NMR (400 MHz, CD₃OD) δ 7.52-7.42 (m, 3H), 7.26-7.22 (m, 1H), 7.16-7.12 (m, 1H), 7.09-6.91 (m, 1H), 6.90-6.50 (m, 2H), 4.15-3.96 (m, 2H), 3.95-3.82 (m, 2H), 3.25 (d, *J* = 7.0 Hz, 2H), 2.78-2.73 (m, 1H), 2.66-2.61 (m, 2H), 2.59-2.52 (m, 1H), 2.43-2.39 (m, 1H), 2.36 (s, 3H), 2.07-1.98 (m, 1H), 1.65-1.56 (m, 1H); LC/MS ESI (+): 397.3 (M+1).

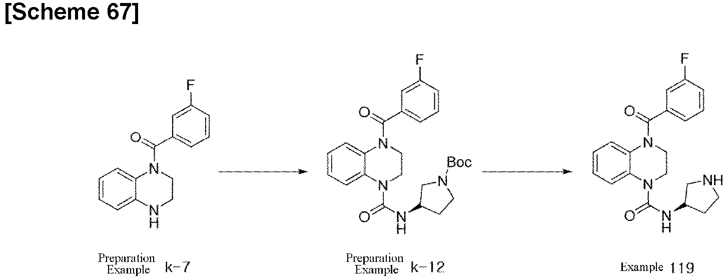

### <Preparation Example k-12> Preparation of tert-butyl (R)-3-(4-(3-fluorobenzoyl)-1,2,3,4-tetrahydroquinoxaline-1-carboxamido)pyrrolidine-1-carboxylate

To a solution of **Preparation Example k-7** (1.0 equiv.), TEA (3.0 equiv.) and DCM (0.3 M) cooled to 0 °C under a nitrogen atmosphere was added dropwise triphosgene (0.6 equiv.). The reaction mixture was stirred at 0 °C for 1 h then TEA (2.0 equiv.) and (*R*)-(+)-1-Boc-3-aminopyrrolidine (1.2 equiv.) were added slowly. The mixture warmed to room temperature and stirred for 3 h. The resulting mixture was partitioned between DCM and water. The organic layer was washed with brine, dried with MgSO₄, filtered and then concentrated under reduced pressure. The residue was purified by silica column chromatography (with *n*-hexane/EtOAc) to give *tert*-butyl (*R*)-3-(4-(3-fluorobenzoyl)-1,2,3,4-tetrahydroquinoxaline-1-carboxamido)pyrrolidine-1-carboxylate.

White solid (yield: 96%); ¹H NMR (400 MHz, CD₃OD) δ 7.58 (d, *J* = 8.0 Hz, 1H), 7.40-7.34 (m, 1H), 7.25-7.20 (m, 3H), 7.18-7.13 (m, 1H), 6.87-6.83 (m, 1H), 6.73 (d, *J =* 7.5 Hz, 1H), 4.42-4.37 (m, 1H), 4.09-4.05 (m, 2H), 3.95-3.92 (m, 2H), 3.72-3.66 (m, 1H), 3.54-3.46 (m, 1H), 3.45-3.37 (m, 1H), 3.30-3.27 (m, 1H), 2.27-2.18 (m, 1H), 2.02-1.98 (m, 1H), 1.48 (s, 9H).

### <Example 119> Synthesis of (R)-4-(3-fluorobenzoyl)-N-(pyrrolidin-3-yl)-3,4-dihydroquinoxaline-1(2H)-carboxamide

Following the procedure as described for Example 1, reaction of **Preparation Example K-12** (1.0 equiv.) in DCM (0.5 M) with TFA (10.0 equiv.) gave (*R*)-4-(3-fluorobenzoyl)-*N*-(pyrrolidin-3-yl)-3,4-dihydroquinoxaline-1(*2H*)-carboxamide.

White solid (yield: 67%); ¹H NMR (400 MHz, CD₃OD) δ 7.63-7.61 (m, 1H), 7.40-7.35 (m, 1H), 7.26-7.14 (m, 4H), 6.88-6.84 (m, 1H), 6.74 (d, *J* = 7.6 Hz, 1H), 4.41-4.35 (m, 1H), 4.15-4.03 (m, 2H), 3.96-3.91 (m, 2H), 3.30-3.21 (m, 2H), 3.09-2.97 (m, 2H), 2.31-2.23 (m, 1H), 1.97-1.88 (m, 1H); LC/MS ESI (+): 369.3 (M+1).

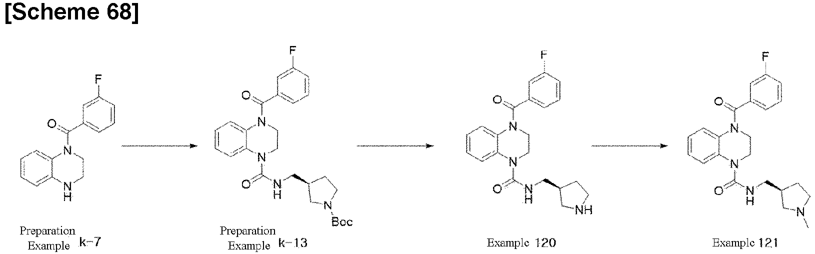

### <Preparation Example k-13> Preparation of tert-butyl (R)-3-((4-(3-fluorophenyl)-1,2,3,4-tetrahydroquinoxaline-1-carboxamido)methyl)pyrrolidine-1-carboxylate

The title compound was prepared in the same manner as **Preparation Example k-12,** using (R)-1-Boc-3-(aminomethyl)pyrrolidine in place of (*R*)-(+)-1-Boc-3-aminopyrrolidine to give *tert*-butyl (*R*)-3-((4-(3-fluorophenyl)-1,2,3,4-tetrahydroquinoxaline-1-carboxamido)methyl)pyrrolidine-1-carboxylate.

White solid (yield: 97%); ¹H NMR (400 MHz, CDCl₃) δ 7.40 (d, *J* = 7.9 Hz, 1H), 7.33-7.28 (m, 1H), 7.18-7.07 (m, 4H), 6.96-6.80 (m, 2H), 5.35-5.27 (m, 1H), 4.07-4.03 (m, 2H), 4.01-3.94 (m, 2H), 3.57-3.39 (m, 3H), 3.38-3.18 (m, 2H), 3.12-2.99 (m, 1H), 2.53-2.43 (m, 1H), 2.04-1.95 (m, 1H), 1.72-1.61 (m, 1H), 1.45 (s, 9H).

### <Example 120> Synthesis of (S)-4-(3-fluorobenzoyl)-N-(pyrrolidin-3-yl)methyl)-3,4-dihydroquinoxaline-1(2H)-carboxamide

Following the procedure as described for Example 1, reaction of **Preparation Example k-13** (1.0 equiv.) in DCM (0.5 M) with TFA (10.0 equiv.) gave (S)-4-(3-fluorobenzoyl)-*N*-(pyrrolidin-3-yl)methyl)-3,4-dihydroquinoxaline-1(*2H*)-carboxamide.

White solid (yield: 82%); ¹H NMR (400 MHz, CD₃OD) δ 7.57-7.52 (m, 1H), 7.37-7.31 (m, 1H), 7.21-7.13 (m, 4H), 6.88-6.83 (m, 1H), 6.72 (d, *J* = 7.6 Hz, 1H), 4.08-4.02 (m, 2H), 3.93-3.87 (m, 2H), 3.33-3.22 (m, 1H), 3.19-3.13 (m, 2H), 3.06-3.00 (m, 1H), 2.83-2.77 (m, 1H), 2.56-2.46 (m, 1H), 2.08-1.96 (m, 1H), 1.68-1.57 (m, 1H);LC/MS ESI (+): 383.3 (M+1).

### <Example 121> Synthesis of (R)-4-(3-fluorobenzoyl)-N-((1-methylpyrrolidin-3-yl)methyl)-3,4-dihydroquinoxaline-1(2H)-carboxamide

Following the procedure as described for Example 2, reaction of **Example 120** (1.0 equiv.) in DCM (0.3 M) with sodium triacetoxyborohydride (1.5 equiv.) and formaldehyde (2.0 equiv.) gave (*R*)-4-(3-fluorobenzoyl)-*N*-((1-methylpyrrolidin-3-yl)methyl)-3,4-dihydroquinoxaline-1(*2H*)-carboxamide.

White solid (yield: 76%); ¹H NMR (400 MHz, CD₃OD) δ 7.54-7.51 (m, 1H), 7.37-7.31 (m, 1H), 7.20-7.13 (m, 4H), 6.87-6.83 (m, 1H), 6.71 (d, *J* = 7.9 Hz, 1H), 4.08-4.04 (m, 2H), 3.92-3.89 (m, 2H), 3.26 (d, *J* = 7.0 Hz, 2H), 2.78-2.74 (m, 1H), 2.65-2.61 (m, 2H), 2.59-2.52 (m, 1H), 2.43-2.39 (m, 1H), 2.36 (s, 3H), 2.08-1.99 (m, 1H), 1.65-1.57 (m, 1H); LC/MS ESI (+): 397.2 (M+1).

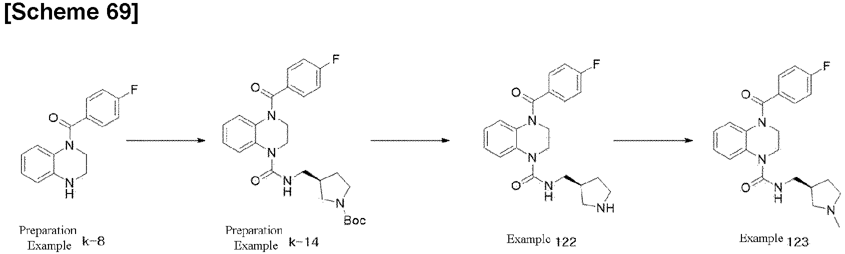

### <Preparation Example k-14> Preparation of tert-butyl (R)-3-((4-(4-fluorophenyl)-1,2,3,4-tetrahydroquinoxaline-1-carboxamido)methyl)pyrrolidine-1-carboxylate

To a solution of **Preparation Example k-8** (1.0 equiv.), TEA (3.0 equiv.) and DCM (0.3 M) cooled to 0 °C under a nitrogen atmosphere was added dropwise triphosgene (0.6 equiv.). The reaction mixture was stirred at 0 °C for 1 h then TEA (2.0 equiv.) and (*R*)-1-Boc-3-(aminomethyl)pyrrolidine (1.2 equiv.) were added slowly. The mixture warmed to room temperature and stirred for 3 h. The resulting mixture was partitioned between DCM and water. The organic layer was washed with brine, dried with MgSO₄, filtered and then concentrated under reduced pressure. The residue was purified by silica column chromatography (with *n*-hexane/EtOAc) to give *tert*-butyl (*R*)-3-((4-(4-fluorophenyl)-1,2,3,4-tetrahydroquinoxaline-1-carboxamido)methyl)pyrrolidine-1-carboxylate.

White solid (yield: 53%); ¹H NMR (400 MHz, CDCl₃) δ 7.45-7.40 (m, 3H), 7.17-7.11 (m, 1H), 7.06-6.99 (m, 2H), 6.95-6.87 (m, 1H), 6.85-6.73 (m, 1H), 5.40-5.25 (m, 1H), 4.10-4.05 (m, 2H), 4.02-3.95 (m, 2H), 3.60-3.40 (m, 3H), 3.39-3.30 (m, 2H), 3.15-2.99 (m, 1H), 2.57-2.43 (m, 1H), 2.05-1.98 (m, 1H), 1.71-1.62 (m, 1H), 1.48 (s, 9H).

### <Example 122> Synthesis of (S)-4-(4-fluorobenzoyl)-N-(pyrrolidin-3-ylmethyl)-3,4-dihydroquinoxaline-1(2H)-carboxamide

Following the procedure as described for Example 1, reaction of **Preparation Example K-14** (1.0 equiv.) in DCM (0.5 M) with TFA (10.0 equiv.) gave (*S*)-4-(4-fluorobenzoyl)-*N*-(pyrrolidin-3-ylmethyl)-3,4-dihydroquinoxaline-1(2*H*)-carboxamide.

White solid (yield: 96%); ¹H NMR (400 MHz, CD₃OD) δ 7.56-7.53 (m, 1H), 7.48-7.43 (m, 2H), 7.16-7.12 (m, 1H), 7.08-7.02 (m, 2H), 6.88-6.83 (m, 1H), 6.67 (d, *J* = 7.9 Hz, 1H), 4.08-4.04 (m, 2H), 3.93-3.89 (m, 2H), 3.29-3.23 (m, 2H), 3.15-3.07 (m, 2H), 3.02-2.96 (m, 1H), 2.79-2.74 (m, 1H), 2.55-2.44 (m, 1H), 2.05-1.97 (m, 1H), 1.66-1.57 (m, 1H); LC/MS ESI (+): 383.3 (M+1).

### <Example 123> Synthesis of (R)-4-(4-fluorobenzoyl)-N-((1-methylpyrrolidin-3-yl)methyl)-3,4-dihydroquinoxaline-1(2H)-carboxamide

Following the procedure as described for Example 2, reaction of **Example 122** (1.0 equiv.) in DCM (0.3 M) with sodium triacetoxyborohydride (1.5 equiv.) and formaldehyde (2.0 equiv.) gave (*R*)-4-(4-fluorobenzoyl)-*N*-((1-methylpyrrolidin-3-yl)methyl)-3,4-dihydroquinoxaline-1(*2H*)-carboxamide.

White solid (yield: 82%); ¹H NMR (400 MHz, CD₃OD) δ 7.53 (dd, *J* = 8.1, 1.1 Hz, 1H), 7.48-7.43 (m, 2H), 7.16-7.12 (m, 1H), 7.07-7.02 (m, 2H), 6.87-6.83 (m, 1H), 6.66 (d, *J* = 7.9 Hz, 1H), 4.08-4.04 (m, 2H), 3.92-3.89 (m, 2H), 3.26 (d, *J* = 7.1 Hz, 2H), 2.77-2.73 (m, 1H), 2.63-2.60 (m, 2H), 2.58-2.52 (m, 1H), 2.41-2.37 (m, 1H), 2.36 (s, 3H), 2.08-1.99 (m, 1H), 1.65-1.56 (m, 1H);LC/MS ESI (+): 397.2 (M+1).

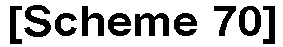

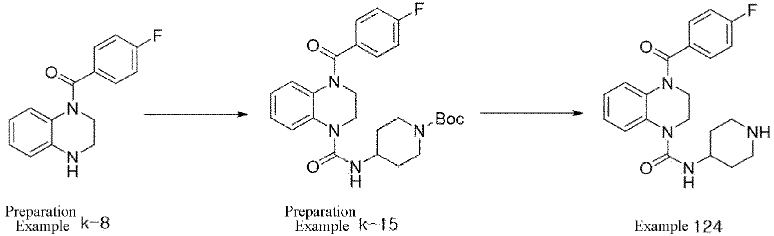

### <Preparation Example k-15> Preparation of tert-butyl 4-(4-(4-fluorobenzoyl)-1,2,3,4-tetrahydroquinoxaline-1-carboxamido)piperidine-1-carboxylate

The title compound was prepared in the same manner as Preparation Example k-14, using 1-Boc-4-aminopiperidine in place of (*R*)-1-Boc-3-(aminomethyl)pyrrolidine to give *tert*-butyl 4-(4-(4-fluorobenzoyl)-1,2,3,4-tetrahydroquinoxaline-1-carboxamido)piperidine-1-carboxylate.

White solid (yield: 96%); ¹H NMR (400 MHz, CDCl₃) δ 7.44-7.38 (m, 3H), 7.14-7.08 (m, 1H), 7.03-6.98 (m, 2H), 6.91-6.86 (m, 1H), 6.81-6.76 (m, 1H), 5.03 (d, *J* = 7.6 Hz, 1H), 4.07-3.93 (m, 7H), 2.93-2.87 (m, 2H), 2.02-1.98 (m, 2H), 1.46 (s, 9H), 1.39-1.29 (m, 2H).

### <Example 124> Synthesis of 4-(4-fluorobenzoyl-N-(piperidin-4-yl)-3,4-dihydroquinoxaline-1(2H)-carboxamide

Following the procedure as described for Example 1, reaction of **Preparation Example K-15** (1.0 equiv.) in DCM (0.5 M) with TFA (10.0 equiv.) gave 4-(4-fluorobenzoyl-*N*-(piperidin-4-yl)-3,4-dihydroquinoxaline-1(*2H*)-carboxamide.

White solid (yield: 87%); ¹H NMR (400 MHz, CD₃OD) δ 7.55 (dd, *J* = 8.3, 1.1 Hz, 1H), 7.50-7.45 (m, 2H), 7.14-7.10 (m, 1H), 7.09-7.03 (m, 2H), 6.85-6.81 (m, 1H), 6.66 (d, *J*= 7.9 Hz, 1H), 4.07-4.04 (m, 2H), 3.92-3.89 (m, 2H), 3.83-3.74 (m, 1H), 3.14-3.08 (m, 2H), 2.74-2.68 (m, 2H), 2.01-1.93 (m, 2H), 1.57-1.47 (m, 2H); LC/MS ESI (+): 383.3 (M+1).

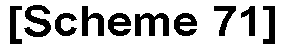

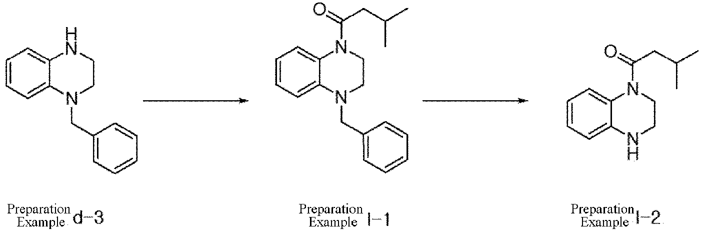

### <Preparation Process I> Preparation of 1-(3,4-dihydroquinoxalin-1(2H)-yl)-3-methylbutan-1-one

1-(3,4-Dihydroquinoxalin-1(*2H*)-yl)-3-methylbutan-1-one according to the present invention was synthesized through Preparation Examples I-1 to I-2 below.

### <Preparation Example I-1> Preparation of 1-(4-benzyl-3,4-dihydroquinoxalin-1(2H)-yl)-3-methylbutan-1-one

To a solution of **Preparation Example d-3** (1.0 equiv.), TEA (2.0 equiv.) and THF (0.3 M) cooled to 0 °C under a nitrogen atmosphere was added dropwise isovaleryl chloride (1.5 equiv.). The resulting mixture was stirred at 0 °C for 1.5 h. The reaction mixture was quenched with water and extracted with DCM. The organic layer was washed with brine, dried with MgSO₄, filtered, and then concentrated under reduced pressure. The residue was purified by silica column chromatography (with *n-*hexane/EtOAc) to give 1-(4-benzyl-3,4-dihydroquinoxalin-1(*2H*)-yl)-3-methylbutan-1-one.

Colorless oil (yield: 100%); ¹H NMR (400 MHz, CD₃CN) δ 7.38-7.30 (m, 2H), 7.29-7.02 (m, 4H), 7.01-6.92 (m, 1H), 6.68 (d, *J* = 8.2 Hz, 1H), 6.62 (dd, *J* = 7.6, 7.6 Hz, 1H), 4.59 (s, 2H), 3.89 (t, *J* = 5.3 Hz, 2H), 3.54-3.42 (m, 2H), 2.42 (d, *J* = 7.0 Hz, 2H), 2.12-2.00 (m, 1H), 0.89 (s, 6H); LC/MS ESI (+): 309.3 (M+1).

### <Preparation Example I-2> Preparation of 1-(3,4-dihydroquinoxalin-1(2H)-yl)-3-methylbutan-1-one

A suspension of **Preparation Example I-1** (1.0 equiv.), 10% Pd/C (0.5 equiv.) were stirred in MeOH/THF (v/v = 2: 1, 0.4 M) under a hydrogen atmosphere for 1.5 h. The resulting mixture was filtered through a Celite pad and concentrated under reduced pressure. The residue was purified by silica column chromatography (with *n-*hexane/EtOAc) to give 1-(3,4-dihydroquinoxalin-1(*2H*)-yl)-3-methylbutan-1-one.

Yellow solid (yield: 92.5%); ¹H NMR (400 MHz, CD₃CN) δ 7.21-6.88 (m, 2H), 6.68-6.53 (m, 2H), 4.78 (brs, 1H), 3.77-3.66 (m, 2H), 3.42-3.31 (m, 2H), 2.42 (d, *J* = 7.0 Hz, 2H), 2.10-2.02 (m, 1H), 0.89 (s, 6H); LC/MS ESI (+): 219.1 (M+1).

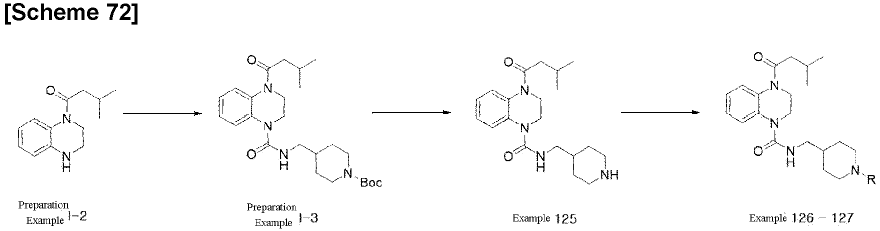

### <Preparation Example I-3> Preparation of tert-butyl 4-((4-(3-methylbutanoyl)-1,2,3,4-tetrahydroquinoxaline-1-carboxamido)methyl)piperidine-1-carboxylate

To a solution of **Preparation Example I-2** (1.0 equiv.), TEA (3.0 equiv.) and DCM (0.3 M) cooled to 0 °C under a nitrogen atmosphere was added dropwise triphosgene (0.6 equiv.). The reaction mixture was stirred at 0 °C for 1 h then TEA (2.0 equiv.) and 1-Boc-4-(aminomethyl)piperidine (1.2 equiv.) were added slowly. The mixture warmed to room temperature and stirred for 3 h. The resulting mixture was partitioned between DCM and water. The organic layer was washed with brine, dried with MgSO₄, filtered and then concentrated under reduced pressure. The residue was purified by silica column chromatography (with *n*-hexane/EtOAc) to give *tert*-butyl 4-((4-(3-methylbutanoyl)-1,2,3,4-tetrahydroquinoxaline-1-carboxamido)methyl)piperidine-1-carboxylate.

White solid (yield: 93%); ¹H NMR (400 MHz, CD₃OD) δ 7.62-7.46 (m, 1H), 7.46-7.18 (m, 2H), 7.17-7.05 (m, 1H), 4.11-3.98 (m, 2H), 3.92 (dd, *J* = 6.4, 6.4 Hz, 2H), 3.76 (dd, *J* = 6.4, 6.4 Hz, 2H), 3.07 (d, *J* = 6.6 Hz, 2H), 2.69 (brs, 2H), 2.42 (d, *J* = 7.2 Hz, 2H), 2.12-1.95 (m, 1H), 1.76-1.59 (m, 3H), 1.42 (s, 9H), 1.13-0.97 (m, 2H), 0.93-0.69 (d, *J* = 6.2 Hz, 6H).

### <Example 125> Synthesis of 4-(3-methylbutanoyl)-N-(piperidin-4-ylmethyl)-3,4-dihydroquinoxaline-1(2H)-carboxamide

Following the procedure as described for Example 1, reaction of **Preparation Example I-3** (1.0 equiv.) in DCM (0.5 M) with TFA (10.0 equiv.) gave 4-(3-methylbutanoyl)-*N*-(piperidin-4-ylmethyl)-3,4-dihydroquinoxaline-1(*2H*)-carboxamide.

Ivory solid (yield: 95%); ¹H NMR (400 MHz, CD₃OD) δ 7.55 (d, *J* = 8.1 Hz, 1H), 7.47-7.19 (m, 2H), 7.18-7.09 (m, 1H), 3.92 (dd, *J* = 6.4, 6.4 Hz, 2H), 3.77 (dd, *J* = 6.4, 6.4 Hz, 2H), 3.07 (d, *J* = 6.4 Hz, 2H), 3.06-2.93 (m, 2H), 2.59-2.48 (m, 2H), 2.43 (d, *J* = 7.2 Hz, 2H), 2.13-1.96 (m, 1H), 1.73-1.58 (m, 3H), 1.21-1.05 (m, 2H), 0.83 (d, *J* = 6.2 Hz, 6H); LC/MS ESI (+): 359.0 (M+1).

### <Example 126> Synthesis of 4-(3-methylbutanoyl)-N-((1-methylpiperidin-4-yl)methyl)-3,4-dihydroquinoxaline-1(2H)-carboxamide

Following the procedure as described for Example 2, reaction of **Example 125** (1.0 equiv.) in DCM (0.3 M) with sodium triacetoxyborohydride (1.5 equiv.) and formaldehyde (2.0 equiv.) gave 4-(3-methylbutanoyl)-N-((1-methylpiperidin-4-yl)methyl)-3,4-dihydroquinoxaline-1(*2H*)-carboxamide.

White solid (yield: 29%); ¹H NMR (400 MHz, CD₃OD) δ 7.55 (d, *J =* 8.2 Hz, 1H), 7.49-7.19 (m, 2H), 7.18-7.09 (m, 1H), 3.93 (dd, *J* = 6.5, 6.5 Hz, 2H), 3.77 (dd, *J* = 6.4, 6.4 Hz, 2H), 3.08 (d, *J* = 6.8 Hz, 2H), 2.86 (d, *J* = 11.4 Hz, 2H), 2.44 (d, *J* = 7.3 Hz, 2H), 2.25 (s, 3H), 2.13-1.90 (m, 3H), 1.78-1.64 (m, 2H), 1.60-1.46 (m, 1H), 1.33-1.18 (m, 2H), 0.83 (d, *J* = 6.4 Hz, 6H); LC/MS ESI (+): 373.3 (M+1).

### <Example 127> Synthesis of N-((1-isobutylpiperidin-4-yl)methyl)-4-(3-methylbutanoyl)-3,4-dihydroquinoxaline-1(2H)-carboxamide

The title compound was synthesized in the same manner as Example 126, using isobutyraldehyde in place of formaldehyde to give *N*-((1-isobutylpiperidin-4-yl)methyl)-4-(3-methylbutanoyl)-3,4-dihydroquinoxaline-1(*2H*)-carboxamide.

White solid (yield: 86%); ¹H NMR (400 MHz, CD₃OD) δ 7.56 (d, *J =* 8.2 Hz, 1H), 7.49-7.18 (m, 2H), 7.18-7.09 (m, 1H), 3.93 (dd, *J* = 6.4, 6.4 Hz, 2H), 3.77 (dd, *J* = 6.4, 6.4 Hz, 2H), 3.08 (d, *J* = 6.8 Hz, 2H), 2.90 (d, *J* = 11.4 Hz, 2H), 2.44 (d, *J* = 7.2 Hz, 2H), 2.10 (d, *J* = 7.2 Hz, 2H), 2.07-1.95 (m, 1H), 1.94-1.75 (m, 3H), 1.66 (d, *J* = 13.1 Hz, 2H), 1.59-1.45 (m, 1H), 1.35-1.20 (m, 3H), 0.90 (d, *J*= 6.6 Hz, 6H), 0.83 (d, *J* = 6.9 Hz, 6H); LC/MS ESI (+): 415.1 (M+1).

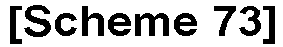

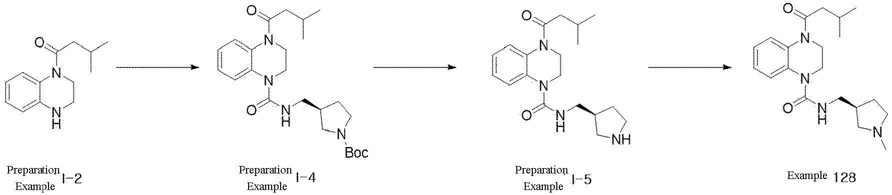

### <Preparation Example I-4> Preparation of tert-butyl (R)-4-(3-methylbutanoyl)-N-((1-methylpyrrolidin-3-yl)methyl)-3,4-dihydroquinoxaline-1(2H)-carboxamide

The title compound was prepared in the same manner as Preparation Example I-3, using (*R*)-1-Boc-3-(aminomethyl)pyrrolidine in place of 1-Boc-4-(aminomethyl)piperidine to give tert-butyl (*R*)-4-(3-methylbutanoyl)-*N*-((1-methylpyrrolidin-3-yl)methyl)-3,4-dihydroquinoxaline-1(*2H*)-carboxamide.

White solid (yield: 90%); ¹H NMR (400 MHz, CD₃OD) δ 7.54 (d, *J =* 8.1 Hz, 1H), 7.46-7.19 (m, 2H), 7.17-7.09 (m, 1H), 3.92 (dd, *J* = 6.5, 1.8 Hz, 2H), 3.76 (dd, *J* = 6.5, 6.5 Hz, 2H), 3.45-3.33 (m, 2H), 3.28-3.11 (m, 3H), 3.06-2.97 (m, 1H), 2.43 (d, *J* = 7.2 Hz, 3H), 2.13-2.00 (m, 1H), 1.98-1.86 (m, 1H), 1.73-1.54 (m, 1H), 1.42 (s, 9H), 0.82 (brs, 6H).

### <Preparation Example I-5> Preparation of (S)-4-(3-methylbutanoyl)-N-(pyrrolidin-3-ylmethyl)-3,4-dihydroquinoxaline-1(2H)-carboxamide

Following the procedure as described for Example 1, reaction of **Preparation Example I-4** (1.0 equiv.) in DCM (0.5 M) with TFA (10.0 equiv.) gave (*S*)-4-(3-methylbutanoyl)-*N*-(pyrrolidin-3-ylmethyl)-3,4-dihydroquinoxaline-1(*2H*)-carboxamide.

Ivory solid (yield: 81%); ¹H NMR (400 MHz, CD₃OD) δ 7.55 (d, *J* = 8.2 Hz, 1H), 7.49-7.20 (m, 2H), 7.19-7.10 (m, 1H), 3.93 (dd, *J* = 6.7, 6.7 Hz, 2H), 3.78 (dd, *J* = 6.4, 6.4 Hz, 2H), 3.23-3.13 (m, 2H), 3.08-2.98 (m, 2H), 2.96-2.84 (m, 1H), 2.73-2.61 (m, 1H), 2.50-2.34 (m, 3H), 2.12-1.99 (m, 1H), 1.98-1.85 (m, 1H), 1.58-1.45 (m, 1H), 0.84 (d, *J* = 6.4 Hz, 6H); LC/MS ESI (+): 344.9 (M+1).

### <Example 128> Synthesis of (R)-4-(3-methylbutanoyl)-N-((1-methylpyrrolidin-3-yl)methyl)-3,4-dihydroquinoxaline-1(2H)-carboxamide

Following the procedure as described for Example 2, reaction of **Preparation Example I-5** (1.0 equiv.) in DCM (0.3 M) with sodium triacetoxyborohydride (1.5 equiv.) and formaldehyde (2.0 equiv.) gave (*R*)-4-(3-methylbutanoyl)-*N*-((1-methylpyrrolidin-3-yl)methyl)-3,4-dihydroquinoxaline-1(*2H*)-carboxamide.

Ivory solid (yield: 48%); ¹H NMR (400 MHz, CD₃OD) δ 7.54 (d, *J =* 8.2 Hz, 1H), 7.46-7.20 (m, 2H), 7.19-7.10 (m, 1H), 4.00-3.85 (m, 2H), 3.81-3.73 (m, 2H), 3.19 (d, *J* = 7.1 Hz, 2H), 2.69 (dd, *J* = 9.7, 7.8 Hz, 1H), 2.64-2.54 (m, 2H), 2.53-2.40 (m, 3H), 2.33 (s, 4H), 2.13-1.89 (m, 2H), 1.61-1.48 (m, 1H), 0.84 (d, *J* = 6.5 Hz, 6H); LC/MS ESI (+): 359.2 (M+1).

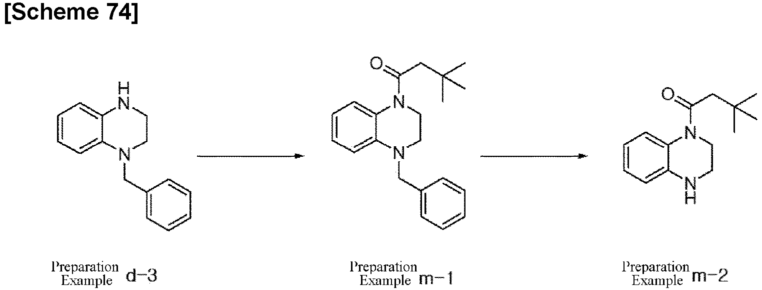

### <Preparation Process m> Preparation of 1-(3,4-dihydroquinoxalin-1(2H)-yl)-3,3-dimethylbutan-1-one

1-(3,4-Dihydroquinoxalin-1(*2H*)-yl)-3,3-dimethylbutan-1-one according to the present invention was synthesized through Preparation Examples m-1 to m-2 below.

### <Preparation Example m-1> Preparation of 1-(4-benzyl-3,4-dihydroquinoxalin-1(2H)-yl)-3,3-dimethylbutan-1-one

The title compound was prepared in the same manner as Preparation Example I-1, using 3,3-dimethylbutyryl chloride in place of isovaleryl chloride to give 1-(4-benzyl-3,4-dihydroquinoxalin-1(*2H*)-yl)-3,3-dimethylbutan-1-one.

Yellow oil (yield: 100%); ¹H NMR (400 MHz, CD₃CN) δ 7.39-7.30 (m, 2H), 7.29-7.04 (m, 4H), 7.02-6.92 (m, 1H), 6.68 (d, *J* = 8.22 Hz, 1H), 6.65-6.58 (m, 1H), 4.58 (s, 2H), 3.89 (t, *J* = 5.48 Hz, 2H), 3.49 (t, *J* = 5.28 Hz, 2H), 2.50 (s, 2H), 0.96 (s, 9H); LC/MS ESI (+): 323.3 (M+1).

### <Preparation Example m-2> Preparation of 1-(3,4-dihydroquinoxalin-1(2H)-yl)-3,3-dimethylbutan-1-one

The title compound was prepared in the same manner as Preparation Example I-2, using **Preparation Example m-1** in place of Preparation Example I-1 to give 1-(3,4-dihydroquinoxalin-1(*2H*)-yl)-3,3-dimethylbutan-1-one.

Yellow solid (yield: 80.1%); ¹H NMR (400 MHz, CD₃CN) δ 7.26-6.89 (m, 2H), 6.66-6.52 (m, 2H), 4.78 (brs, 1H), 3.49-3.44 (m, 2H), 3.43-3.38 (m, 2H), 2.51 (s, 2H), 0.96 (s, 9H); LC/MS ESI (+): 233.0 (M+1).

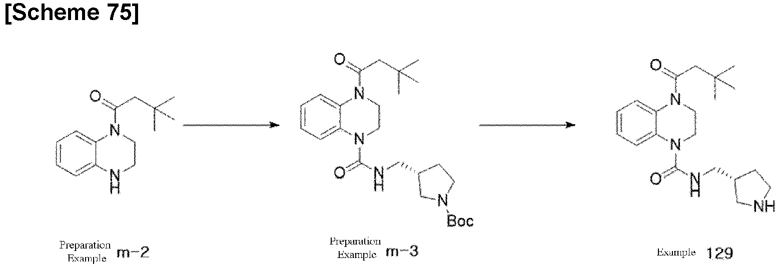

### <Preparation Example m-3> Preparation of tert-butyl (S)-3-((4-(3,3-dimethylbutanoyl)-1,2,3,4-tetrahydroquinoxaline-1-carboxamido)methyl)pyrrolidine-1-carboxylate

To a solution of **Preparation Example m-2** (1.0 equiv.), TEA (3.0 equiv.) and DCM (0.3 M) cooled to 0 °C under a nitrogen atmosphere was added dropwise triphosgene (0.6 equiv.). The reaction mixture was stirred at 0 °C for 1 h then TEA (2.0 equiv.) and (R)-1-Boc-3-(aminomethyl)pyrrolidine (1.2 equiv.) were added slowly. The mixture warmed to room temperature and stirred for 3 h. The resulting mixture was partitioned between DCM and water. The organic layer was washed with brine, dried with MgSO₄, filtered and then concentrated under reduced pressure. The residue was purified by silica column chromatography (with n-hexane/EtOAc) to give *tert*-butyl (*S*)-3-((4-(3,3-dimethylbutanoyl)-1,2,3,4-tetrahydroquinoxaline-1-carboxamido)methyl)pyrrolidine-1-carboxylate.

White solid (yield: 88%); ¹H NMR (400 MHz, CD₃OD) δ 7.55 (d, *J =* 8.1 Hz, 1H), 7.49-7.19 (m, 2H), 7.17-7.09 (m, 1H), 3.98-3.82 (m, 2H), 3.81-3.70 (m, 2H), 3.48-3.34 (m, 2H), 3.28-3.11 (m, 3H), 3.01 (dd, *J* = 10.9, 7.1 Hz, 1H), 2.55-2.46 (m, 2H), 2.45-2.29 (m, 1H), 2.00-1.84 (m, 1H), 1.71-1.54 (m, 1H), 1.42 (s, 9H), 0.89 (s, 9H).

### <Example 129> Synthesis of (R)-4-(3,3-dimethylbutanoyl)-N-(pyrrolidin-3-ylmethyl)-3,4-dihydroquinoxaline-1(2H)-carboxamide

Following the procedure as described for Example 1, reaction of **Preparation Example m-3** (1.0 equiv.) in DCM (0.5 M) with TFA (10.0 equiv.) gave (*R*)-4-(3,3-dimethylbutanoyl)-*N*-(pyrrolidin-3-ylmethyl)-3,4-dihydroquinoxaline-1(*2H*)-carboxamide.

Ivory solid (yield: 67%); ¹H NMR (400 MHz, CD₃OD) δ 7.56 (d, *J* = 8.1 Hz, 1H), 7.50-7.20 (m, 2H), 7.19-7.08 (m, 1H), 4.00-3.87 (m, 2H), 3.82-3.74 (m, 2H), 3.25-3.15 (m, 2H), 3.11-3.00 (m, 2H), 3.00-2.90 (m, 1H), 2.70 (dd, *J* = 11.4, 6.8 Hz, 1H), 2.51 (s, 2H), 2.50-2.34 (m, 1H), 2.04-1.88 (m, 1H), 1.61-1.48 (m, 1H), 0.91 (s, 9H); LC/MS ESI (+): 359.0 (M+1).

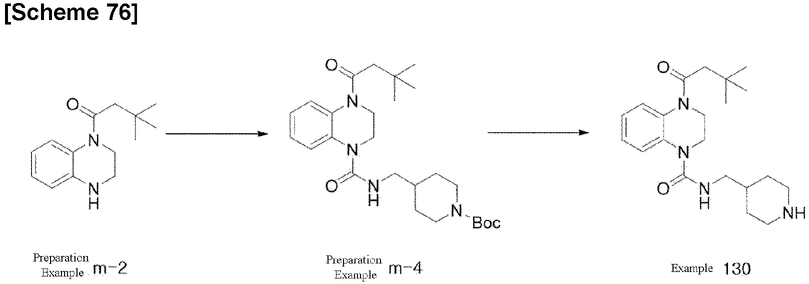

### <Preparation Example m-4> Preparation of tert-butyl 4-((4-(3,3-dimethylbutanoyl)-1,2,3,4-tetrahydroquinoxaline-1-carboxamido)methyl)piperidine-1-carboxylate

The title compound was prepared in the same manner as Preparation Example m-3, using 1-Boc-4-(aminomethyl)piperidine in place of (*R*)-1-Boc-3-(aminomethyl)pyrrolidine to give tert-butyl 4-((4-(3,3-dimethylbutanoyl)-1,2,3,4-tetrahydroquinoxaline-1-carboxamido)methyl)piperidine-1-carboxylate.

White solid (yield: 93%); ¹H NMR (400 MHz, CD₃OD) δ 7.55 (d, *J* = 8.3 Hz, 1H), 7.50-7.17 (m, 2H), 7.16-7.07 (m, 1H), 4.12-3.98 (m, 2H), 3.96-3.85 (m, 2H), 3.84-3.69 (m, 2H), 3.08 (d, *J* = 6.5 Hz, 2H), 2.69 (brs, 2H), 2.50 (s, 2H), 1.77-1.58 (m, 3H), 1.42 (s, 9H), 1.12-0.98 (m, 2H), 0.89 (s, 9H).

### <Example 130> Synthesis of 4-(3,3-dimethylbutanoyl)-N-(piperidin-4-ylmethyl)-3,4-dihydroquinoxaline-1(2H)-carboxamide

Following the procedure as described for Example 1, reaction of **Preparation Example m-4** (1.0 equiv.) in DCM (0.5 M) with TFA (10.0 equiv.) gave 4-(3,3-dimethylbutanoyl)-*N*-(piperidin-4-ylmethyl)-3,4-dihydroquinoxaline-1(*2H*)-carboxamide.

White solid (yield: 46%); ¹H NMR (400 MHz, CD₃OD) δ 7.57 (d, *J* = 8.1 Hz, 1H), 7.51-7.20 (m, 2H), 7.19-7.07 (m, 1H), 3.99-3.87 (m, 2H), 3.81-3.74 (m, 2H), 3.24-3.13 (m, 2H), 3.13-3.03 (m, 2H), 2.75-2.63 (m, 2H), 2.51 (s, 2H), 1.84-1.63 (m, 2H), 1.30-1.16 (m, 2H), 0.90 (s, 9H); LC/MS ESI (+): 373.1 (M+1).

The compounds of the present invention prepared through Examples are summarized in Table 1 below. The term "compound" used throughout the specification may refer to the term "Example." For example, Example 1 may refer to Compound 1.

**[Table 1]**

| No. | Structure | IUPAC Name |
|---|---|---|
| 1 | 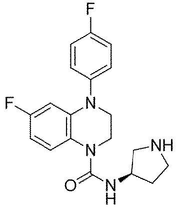 | (*R*)-6-fluoro-4-(4-fluorophenyl)-*N*-(pyrrolidin-3-yl)-3,4-dihydroquinoxaline-1(2H)-carboxamide |
| 2 | 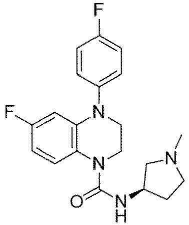 | (*R*)-6-fluoro-4-(4-fluorophenyl)-*N*-(1-methylpyrrolidin-3-yl)-3,4-dihydroquinoxaline-1(2H)-carboxamide |
| 3 | 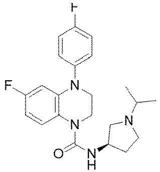 | *R*)-6-fluoro-4-(4-fluorophenyl)-*N*-(1-isopropylpyrrolidin-3-yl)-3,4-dihydroquinoxaline-1(2H)-carboxamide |
| 4 | 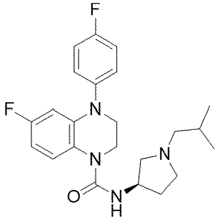 | (*R*)-6-fluoro-4-(4-fluorophenyl)-*N*-(1-isobutylpyrrolidin-3-yl)-3,4-dihydroquinoxaline-1(2H)-carboxamide |
| 5 | 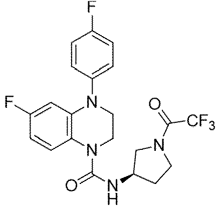 | (*R*)-6-fluoro-4-(4-fluorophenyl)-*N*-(1-(2,2,2-trifluoroacetyl)pyrrolidin-3-yl)-3,4-dihydroquinoxaline-1(2H)-carboxamide |
| 6 | 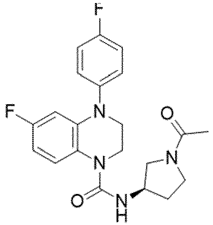 | (*R*)-*N*-(1-acetylpyrrolidin-3-yl)-6-fluoro-4-(4-fluorophenyl)-3,4-dihydroquinoxaline-1(2H)-carboxamide |
| 7 | 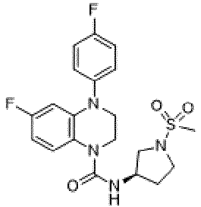 | (*R*)-6-fluoro-4-(4-fluorophenyl)-*N*-(1-(methylsulfonyl)pyrrolidin-3-yl)-3,4-dihydroquinoxaline-1(2H)-carboxamide |
| 8 | 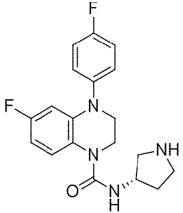 | (*S*)-6-fluoro-4-(4-fluorophenyl)-*N*-(pyrrolidin-3-yl)-3,4-dihydroquinoxaline-1(2H)-carboxamide |
| 9 | 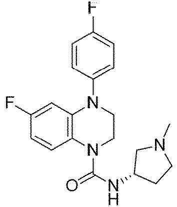 | (*S*)-6-fluoro-4-(4-fluorophenyl)-*N*-(1-methylpyrrolidin-3-yl)-3,4-dihydroquinoxaline-1(2H)-carboxamide |
| 10 | 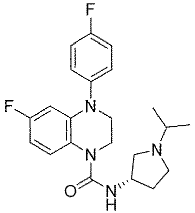 | (*S*)-6-fluoro-4-(4-fluorophenyl)-*N*-(1-isopropylpyrrolidin-3-yl)-3,4-dihydroquinoxaline-1(2H)-carboxamide |
| 11 | 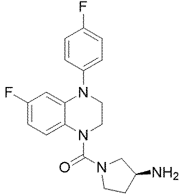 | (*S*)-(3-aminopyrrolidin-1-yl)(6-fluoro-4-(4-fluorophenyl)-3,4-dihydroquinoxalin-1(2H)-yl)methanone |
| 12 | 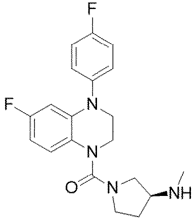 | (*S*)-(6-fluoro-4-(4-fluorophenyl)-3,4-dihydroquinoxalin-1(2H)-yl)(3-(methylamino)pyrrolidin-1-yl)methanone |
| 13 | 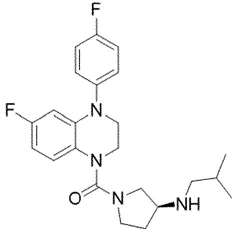 | (*S*)-(6-fluoro-4-(4-fluorophenyl)-3,4-dihydroquinoxalin-1(2H)-yl)(3-(isobutylamino)pyrrolidin-1-yl)methanone |
| 14 | 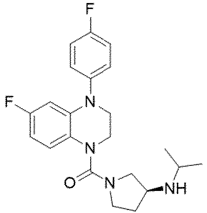 | (*S*)-(6-fluoro-4-(4-fluorophenyl)-3,4-dihydroquinoxalin-1(2H)-yl)(3-(isopropylamino)pyrrolidin-1-yl)methanone |
| 15 | 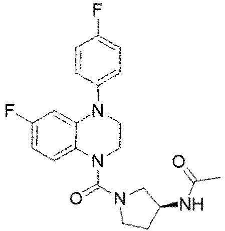 | (*S*)-*N*-(1-(6-fluoro-4-(4-fluorophenyl)-1,2,3,4-tetrahydroquinoxaline-1-carbonyl)pyrrolidin-3-yl)acetamide |
| 16 | 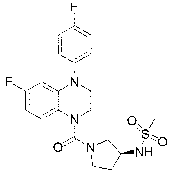 | (*S*)-*N*-(1-(6-fluoro-4-(4-fluorophenyl)-1,2,3,4-tetrahydroquinoxaline-1-carbonyl)pyrrolidin-3-yl)methanesulfonamide |
| 17 | 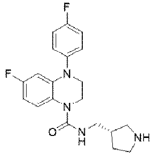 | (*S*)-6-fluoro-4-(4-fluorophenyl)-*N*-(pyrrolidin-3-ylmethyl)-3,4-dihydroquinoxaline-1(2H)-carboxamide |
| 18 | 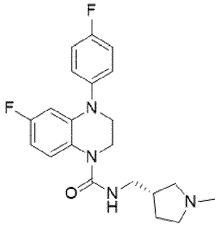 | (*R*)-6-fluoro-4-(4-fluorophenyl)-*N*-((1-methylpyrrolidin-3-yl)methyl)-3,4-dihydroquinoxaline-1(2H)-carboxamide |
| 19 | 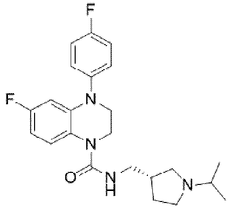 | (*R*)-6-fluoro-4-(4-fluorophenyl)-*N*-((1-isopropylpyrrolidin-3-yl)methyl)-3,4-dihydroquinoxaline-1(2H)-carboxamide |
| 20 | 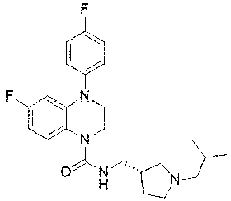 | (*R*)-6-fluoro-4-(4-fluorophenyl)-*N*-((1-isobutylpyrrolidin-3-yl)methyl)-3,4-dihydroquinoxaline-1(2H)-carboxamide |
| 21 | 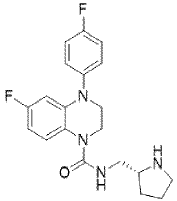 | (*R*)-6-fluoro-4-(4-fluorophenyl)-*N*-(pyrrolidin-2-ylmethyl)-3,4-dihydroquinoxaline-1(2H)-carboxamide |
| 22 | 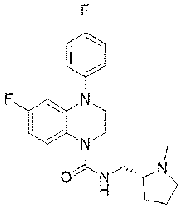 | (*R*)-6-fluoro-4-(4-fluorophenyl)-*N*-((1-methylpyrrolidin-2-yl)methyl)-3,4-dihydroquinoxaline-1(2H)-carboxamide |
| 23 | 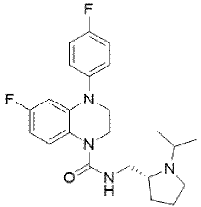 | (*R*)-6-fluoro-4-(4-fluorophenyl)-*N*-((1-isopropylpyrrolidin-2-yl)methyl)-3,4-dihydroquinoxaline-1(2H)-carboxamide |
| 24 | 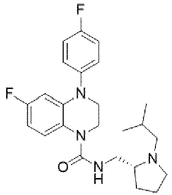 | (*R*)-6-fluoro-4-(4-fluorophenyl)-*N*-((1-isobutylpyrrolidin-2-yl)methyl)-3,4-dihydroquinoxaline-1(2H)-carboxamide |
| 25 | 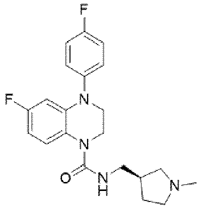 | (*S*)-6-fluoro-4-(4-fluorophenyl)-*N*-((1-methylpyrrolidin-3-yl)methyl)-3,4-dihydroquinoxaline-1(2H)-carboxamide |
| 26 | 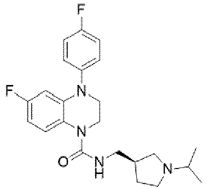 | (*S*)-6-fluoro-4-(4-fluorophenyl)-*N*-((1-isopropylpyrrolidin-3-yl)methyl)-3,4-dihydroquinoxaline-1(2H)-carboxamide |
| 27 | 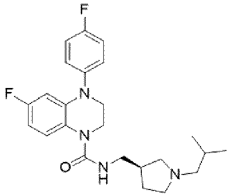 | (*S*)-6-fluoro-4-(4-fluorophenyl)-*N*-((1-isobutylpyrrolidin-3-yl)methyl)-3,4-dihydroquinoxaline-1(2H)-carboxamide |
| 28 | 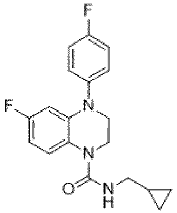 | *N*-(cyclopropylmethyl)-6-fluoro-4-(4-fluorophenyl)-3,4-dihydroquinoxaline-1(2H)-carboxamide |
| 29 | 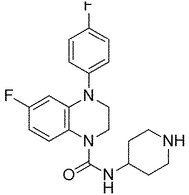 | 6-fluoro-4-(4-fluorophenyl)-*N*-(piperidin-4-yl)-3,4-dihydroquinoxaline-1(2H)-carboxamide |
| 30 | 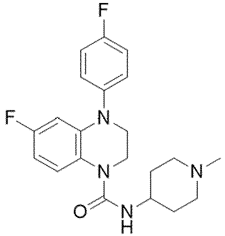 | 6-fluoro-4-(4-fluorophenyl)-*N*-(1-methylpiperidin-4-yl)-3,4-dihydroquinoxaline-1(2H)-carboxamide |
| 31 | 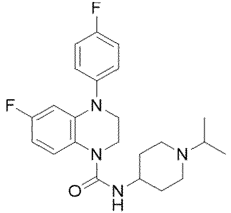 | 6-fluoro-4-(4-fluorophenyl)-*N*-(1-isopropylpiperidin-4-yl)-3,4-dihydroquinoxaline-1(2H)-carboxamide |
| 32 | 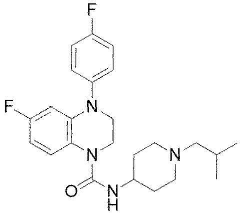 | 6-fluoro-4-(4-fluorophenyl)-*N*-(1-isobutylpiperidin-4-yl)-3,4-dihydroquinoxaline-1(2H)-carboxamide; |
| 33 | 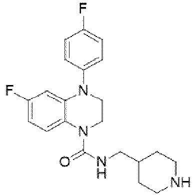 | 6-fluoro-4-(4-fluorophenyl)-*N*-(piperidin-4-ylmethyl)-3,4-dihydroquinoxaline-1(2H)-carboxamide |
| 34 | 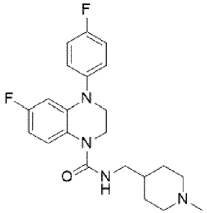 | 6-fluoro-4-(4-fluorophenyl)-*N*-(methylpiperidin-4-yl)methyl)-3,4-dihydroquinoxaline-1(2H)-carboxamide |
| 35 | 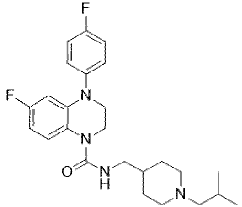 | 6-fluoro-4-(4-fluorophenyl)-*N*-((1-isobutylpiperidin-4-yl)methyl)-3,4-dihydroquinoxaline-1(2H)-carboxamide |
| 36 | 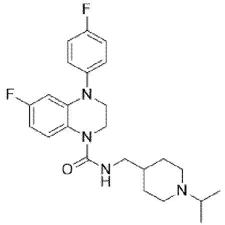 | 6-fluoro-4-(4-fluorophenyl)-*N*-((1-isopropylpiperidin-4-yl)methyl)-3,4-dihydroquinoxaline-1(2H)-carboxamide |
| 37 | 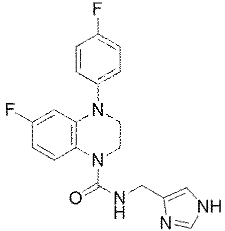 | *N*-((1H-imidazol-4-)methyl)-6-fluoro-4-(4-fluorophenyl)-3,4-dihydroquinoxaline-1(2H)-carboxamide |
| 38 | 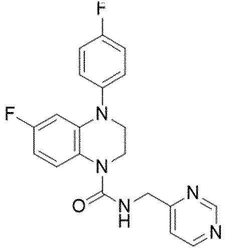 | 6-fluoro-4-(4-fluorophenyl)-*N*-(pyrazin-2-ylmethyl)-3,4-dihydroquinoxaline-1(2H)-carboxamide |
| 39 | 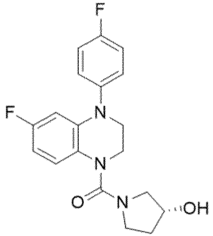 | (*R*)-(6-fluoro-4-(4-fluorophenyl)-3,4-dihydroquinoxalin-1(2H)-yl)(3-hydroxypyrrolidin-1-yl)methanone |
| 40 | 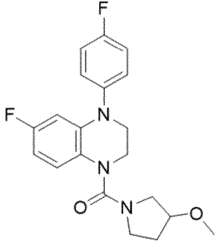 | (6-fluoro-4-(4-fluorophenyl)-3,4-dihydroquinoxalin-1(2H)-yl)(3-methoxypyrrolidin-1-yl)methanone |
| 41 | 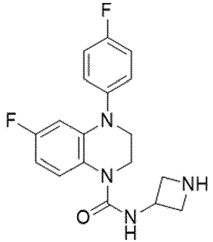 | *N*-(azetidin-3-yl)-6-fluoro-4-(4-fluorophenyl)-3,4-dihydroquinoxaline-1(2H)-carboxamide |
| 42 | 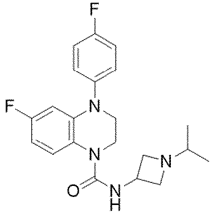 | 6-fluoro-4-(4-fluorophenyl)-*N*-(1-isopropylazetidin-3-yl)-3,4-dihydroquinoxaline-1(2H)-carboxamide |
| 43 | 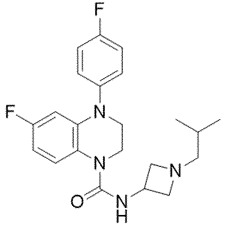 | 6-fluoro-4-(4-fluorophenyl)-*N*-(1-isobutylazetidin-3-yl)-3,4-dihydroquinoxaline-1(2H)-carboxamide |
| 44 | 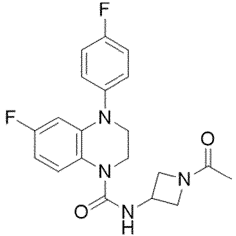 | *N*-(1-acetylazetidin-3-yl)-6-fluoro-4-(4-fluorophenyl)-3,4-dihydroquinoxaline-1(2H)-carboxamide |
| 45 | 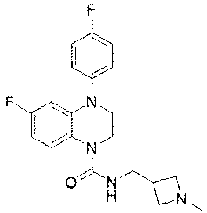 | 6-fluoro-4-(4-fluorophenyl)-*N*-((1-methylazetidin-3-yl)methyl)-3,4-dihydroquinoxaline-1(2H)-carboxamide |
| 46 | 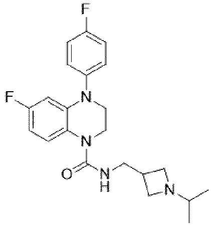 | 6-fluoro-4-(4-fluorophenyl)-*N*-((1-isopropylazetidin-3-yl)methyl)-3,4-dihydroquinoxaline-1(2H)-carboxamide |
| 47 | 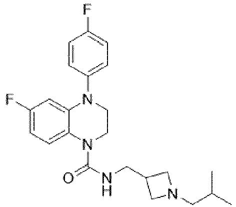 | 6-fluoro-4-(4-fluorophenyl)-*N*-((1-isobutylazetidin-3-yl)methyl)-3,4-dihydroquinoxaline-1(2H)-carboxamide |
| 48 | 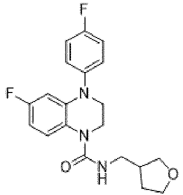 | 6-fluoro-4-(4-fluorophenyl)-*N-*((tetrahydrofuran-3-yl)methyl)-3,4-dihydroquinoxaline-1(2H)-carboxamide |
| 49 | 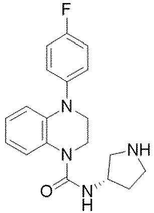 | (S)-4-(4-fluorophenyl)-*N*-(pyrrolidin-3-yl)-3,4-dihydroquinoxaline-1(2H)-carboxamide |
| 50 | 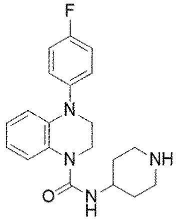 | 4-(4-fluorophenyl)-*N*-(piperidin-4-yl)-3,4-dihydroquinoxaline-1(2H)-carboxamide |
| 51 | 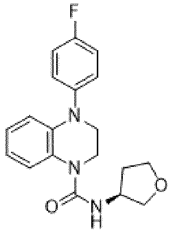 | (*S*)-4-(4-fluorophenyl)-*N*-(tetrahydrofuran-3-yl)-3,4-dihydroquinoxaline-1(2H)-carboxamide |
| 52 | 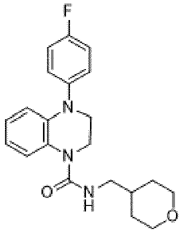 | 4-(4-fluorophenyl)-*N*-((tetrahydro-2H-pyran-4-yl)methyl)-3,4-dihydroquinoxaline-1(2H)-carboxamide |
| 53 | 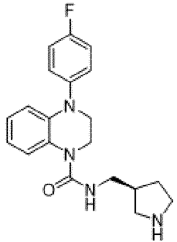 | (*S*)-4-(4-fluorophenyl)-*N*-(pyrrolidin-3-ylmethyl)-3,4-dihydroquinoxaline-1(2H)-carboxamide |
| 54 | 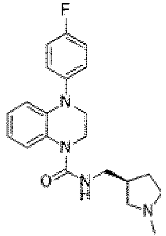 | (*R*)-4-(4-fluorophenyl)-*N*-((1-methylpyrrolidin-3-yl)methyl)-3,4-dihydroquinoxaline-1(2H)-carboxamide |
| 55 | 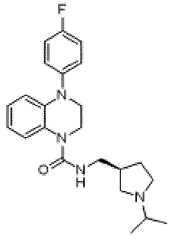 | (*R*)-4-(4-fluorophenyl)-N-((1-isopropylpyrrolidin-3-yl)methyl)-3,4-dihydroquinoxaline-1(2H)-carboxamide |
| 56 | 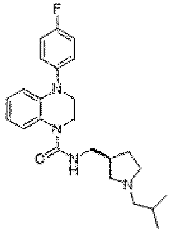 | (*R*)-4-(4-fluorophenyl)-*N*-((1-isobutylpyrrolidin-3-yl)methyl)-3,4-dihydroquinoxaline-1(2H)-carboxamide |
| 57 | 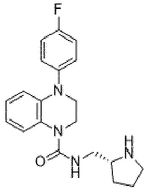 | (*R*)-4-(4-fluorophenyl)-*N*-(pyrrolidin-2-ylmethyl)-3,4-dihydroquinoxaline-1(2H)-carboxamide |
| 58 | 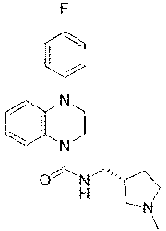 | (*S*)-4-(4-fluorophenyl)-*N*-((1-methylpyrrolidin-3-yl)methyl)-3,4-dihydroquinoxaline-1(2H)-carboxamide |
| 59 | 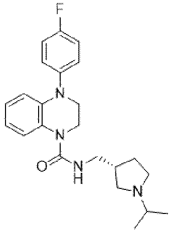 | (*S*)-4-(4-fluorophenyl)-*N*-((1-isopropylpyrrolidin-3-yl)methyl)-3,4-dihydroquinoxaline-1(2H)-carboxamide |
| 60 | 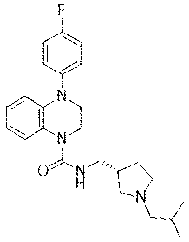 | (*S*)-4-(4-fluorophenyl)-*N*-((1-isobutylpyrrolidin-3-yl)methyl)-3,4-dihydroquinoxaline-1(2H)-carboxamide |
| 61 | 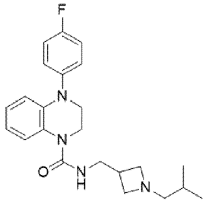 | 4-(4-fluorophenyl)-*N*-((1-isobutylazetidin-3-yl)methyl)-3,4-dihydroquinoxaline-1(2H)-carboxamide |
| 62 | 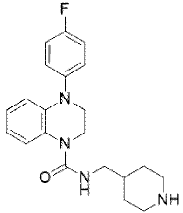 | 4-(4-fluorophenyl)-*N*-(piperidin-4-ylmethyl)-3,4-dihydroquinoxaline-1(2H)-carboxamide |
| 63 | 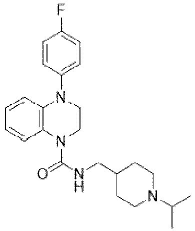 | 4-(4-fluorophenyl)-*N*-((1-isopropylpiperidin-4-yl)methyl)-3,4-dihydroquinoxaline-1(2H)-carboxamide |
| 64 | 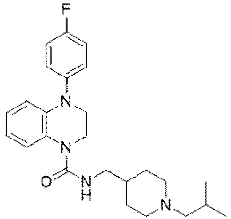 | 4-(4-fluorophenyl)-*N*-((1-isobutylpiperidin-4-yl)methyl)-3,4-dihydroquinoxaline-1(2H)-carboxamide |
| 65 | 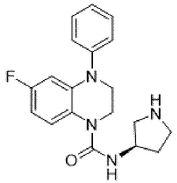 | (*R*)-6-fluoro-4-phenyl-*N*-(pyrrolidin-3-yl)-3,4-dihydroquinoxaline-1(2H)-carboxamide |
| 66 | 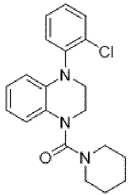 | (4-(2-chlorophenyl)-3,4-dihydroquinoxalin-1 (2H)-yl)(piperidin-1-yl)methanone |
| 67 | 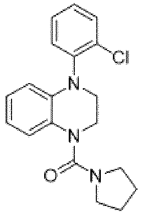 | 4-(2-chlorophenyl)-3,4-dihydroquinoxalin-1(2H)-yl)(pyrrolidin-1-yl)methanone |
| 68 | 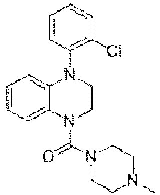 | (4-(2-chlorophenyl)-3,4-dihydroquinoxalin-1(2H)-yl)(4-methylpiperazin-1-yl)methanone |
| 69 | 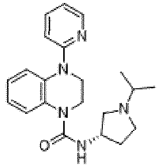 | (*S*)-N-(1-isopropylpyrrolidin-3-yl)-4-(pyridin-2-yl)-3,4-dihydroquinoxaline-1(2H)-carboxamide |
| 70 | 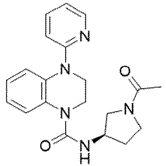 | (*R*)-*N*-(1-acetylpyrrolidin-3-yl)-4-(pyridin-2-yl)-3,4-dihydroquinoxaline-1(2H)-carboxamide |
| 71 | 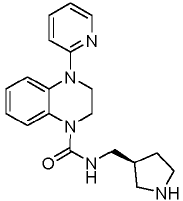 | (*S*)-4-(pyridin-2-yl)-N-(pyrrolidin-3-ylmethyl)-3,4-dihydroquinoxaline-1(2H)-carboxamide |
| 72 | 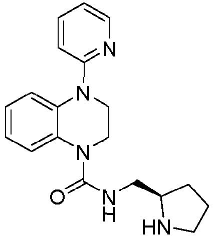 | (*R*)-4-(pyridin-2-yl)-*N*-(pyrrolidin-2-ylmethyl)-3,4-dihydroquinoxaline-1(2H)-carboxamide |
| 73 | 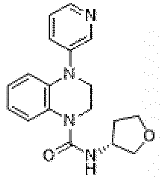 | (*R*)-4-(pyridin-3-yl)-*N-*(tetrahydrofuran-3-yl)-3,4-dihydroquinoxaline-1(2H)-carboxamide |
| 74 | 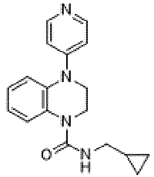 | *N*-(cyclopropylmethyl)-4-(pyridin-4-yl)-3,4-dihydroquinoxaline-1(2H)-carboxamide |
| 75 | 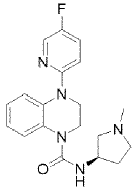 | (*R*)-4-(5-fluoropyridin-2-yl)-*N*-(1-methylpyrrolidin-3-yl)-3,4-dihydroquinoxaline-1(2H)-carboxamide |
| 76 | 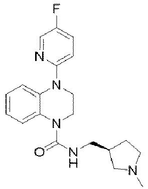 | (*R*)-4-(5-fluoropyridin-2-yl)-*N*-(1-methylpyrrolidin-3-yl)methyl)-3,4-dihydroquinoxaline-1(2H)-carboxamide |
| 77 | 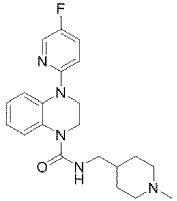 | 4-(5-fluoropyridin-2-yl)-*N*-(1-methylpiperidin-4-yl)methyl)-3,4-dihydroquinoxaline-1(2H)-carboxamide |
| 78 | 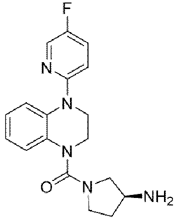 | (*S*)-(3-aminopyrrolidin-1-yl)(4-(5-fluoropyridin-2-yl)-3,4-dihydroquinoxaline-1(2H)-methanone |
| 79 | 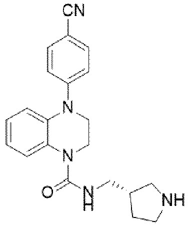 | (*S*)-4-(4-cyanophenyl)-*N*-(pyrrolidin-3-ylmethyl)-3,4-dihydroquinoxaline-1(2H)-carboxamide |
| 80 | 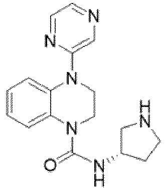 | (*S*)-4-(pyrazin-2-yl)-*N*-(pyrrolidin-3-yl)-3,4-dihydroquinoxaline-1(2H)-carboxamide |
| 81 | 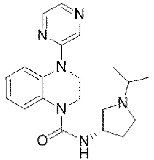 | (*S*)-N-(1-isopropylpyrrolidin-3-yl)-4-(pyrazin-2-yl)-3,4-dihydroquinoxaline-1(2H)-carboxamide |
| 82 | 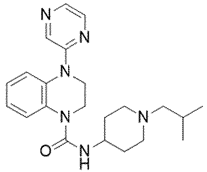 | *N*-(1-isobutylpiperidin-4-yl)-4-(pyrazin-2-yl)-3,4-dihydroquinoxaline-1(2H)-carboxamide |
| 83 | 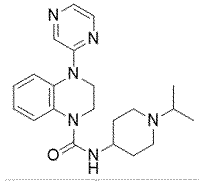 | *N*-(1-isopropylpiperidin-4-yl)-4-(pyrazin-2-yl)-3,4-dihydroquinoxaline-1(2H)-carboxamide |
| 84 | 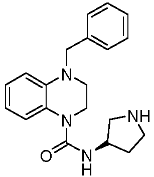 | (*R*)-4-benzyl-*N*-(pyrrolidin-3-yl)-3,4-dihydroquinoxaline-1(2H)-carboxamide |
| 85 | 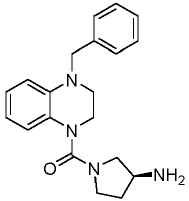 | (*S*)-(3-aminopyrrolidin-1-yl)(4-benzyl-3,4-dihydroquinoxalin-1(2H)-yl)methanone |
| 86 | 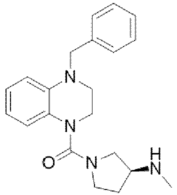 | (*S*)-(4-benzyl-3,4-dihydroquinoxalin-1(2H)-yl)(3-(methylamino)pyrrolidin-1-yl)methanone |
| 87 | 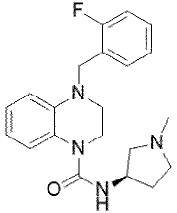 | (*R*)-4-(2-fluorobenzyl)-*N*-(1-methylpyrrolidin-3-yl)-3,4-dihydroquinoxaline-1(2H)-carboxamide |
| 88 | 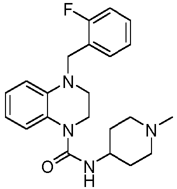 | 4-(2-fluorobenzyl)-*N*-(1-methylpiperidin-4-yl)-3,4-dihydroquinoxaline-1(2H)-carboxamide |
| 89 | 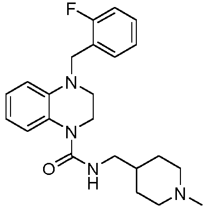 | 4-(2-fluorobenzyl)-*N*-((1-methylpiperidin-4-yl)methyl)-3,4-dihydroquinoxaline-1(2H)-carboxamide |
| 90 | 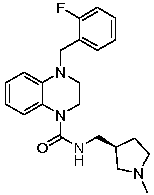 | (*R*)-4-(2-fluorobenzyl)-*N*-((1-methylpyrrolidin-3-yl)methyl)-3,4-dihydroquinoxaline-1(2H)-carboxamide |
| 91 | 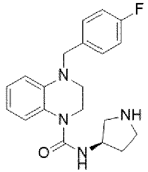 | (*R*)-4-(4-fluorobenzyl)-*N*-(pyrrolidin-3-yl)-3,4-dihydroquinoxaline-1(2H)-carboxamide |
| 92 | 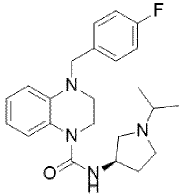 | (*R*)-4-(4-fluorobenzyl)-*N*-(1-isopropylpyrrolidin-3-yl)-3,4-dihydroquinoxaline-1(2H)-carboxamide |
| 93 | 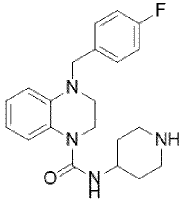 | 4-(4-fluorobenzyl)-*N*-(piperidin-4-yl)-3,4-dihydroquinoxaline-1(2H)-carboxamide |
| 94 | 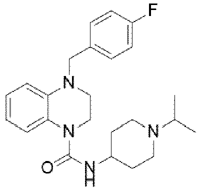 | 4-(4-fluorobenzyl)-*N*-(1-isopropylpiperidin-4-yl)-3,4-dihydroquinoxaline-1(2H)-carboxamide |
| 95 | 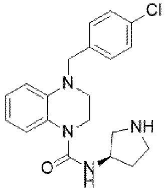 | (*R*)-4-(4-chlorobenzyl)-*N*-(pyrrolidin-3-yl)-3,4-dihydroquinoxaline-1(2H)-carboxamide |
| 96 | 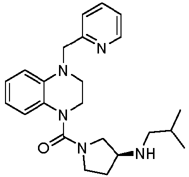 | (*S*)-(3-(isobutylamino)pyrrolidin-1-yl)(4-(pyridin-2-ylmethyl)-3,4-dihydroquinoxalin-1(2H)-yl)methanone |
| 97 | 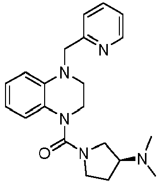 | (*S*)-(3-(dimethylamino)pyrrolidin-1-yl)(4-(pyridin-2-ylmethyl)-3,4-dihydroquinoxalin-1(2H)-yl)methanone |
| 98 | 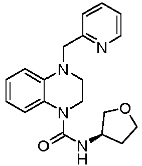 | (*R*)-4-(pyridin-2-ylmethyl)-*N*-(tetrahydrofuran-3-yl)-3,4-dihydroquinoxaline-1(2H)-carboxamide; |
| 99 | 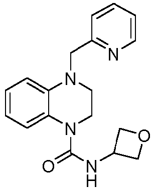 | *N*-(oxetan-3-yl)-4-(pyridin-2-ylmethyl)-3,4-dihydroquinoxaline-1(2H)-carboxamide |
| 100 | 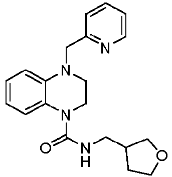 | 4-(pyridin-2-ylmethyl)-*N*-((tetrahydrofuran-3-yl)methyl)-3,4-dihydroquinoxaline-1(2H)-carboxamide |
| 101 | 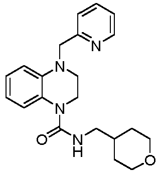 | 4-(pyridin-2-ylmethyl)-*N*-((tetrahydro-2H-pyran-4-yl)methyl)-3,4-dihydroquinoxaline-1(2H)-carboxamide |
| 102 | 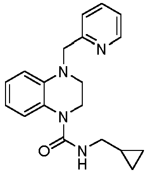 | *N*-(cyclopropylmethyl)-4-(pyridin-2-ylmethyl)-3,4-dihydroquinoxaline-1(2H)-carboxamide |
| 103 | 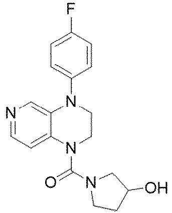 | (4-(4-fluorophenyl)-3,4-dihydropyrido[3,4-*b*]pyrazin-1(2H)-yl)(3-hydroxypyrrolidin-1-yl)methanone |
| 104 | 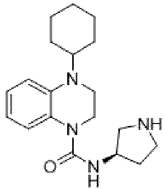 | (*R*)-4-cyclohexyl-*N*-(pyrrolidin-3-yl)-3,4-dihydroquinoxaline-1(2H)-carboxamide |
| 105 | 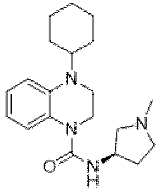 | (*R*)-4-cyclohexyl-*N*-(1-methylpyrrolidin-3-yl)-3,4-dihydroquinoxaline-1(2H)-carboxamide |
| 106 | 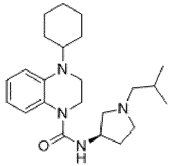 | (*R*)-4-cyclohexyl-*N*-(1-isobutylpyrrolidin-3-yl)-3,4-dihydroquinoxaline-1(2H)-carboxamide |
| 107 | 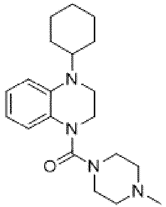 | (4-cyclohexyl-3,4-dihydroquinoxalin-1(2H)-yl)(4-methylpiperazin-1-yl)methanone |
| 108 | 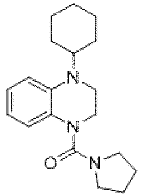 | (4-cyclohexyl-3,4-dihydroquinoxalin-1(2H)-yl)(pyrrolidin-1-yl)methanone |
| 109 | 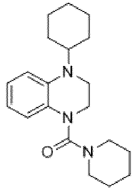 | (4-cyclohexyl-3,4-dihydroquinoxalin-1(2H)-yl)(piperidin-1-yl)methanone |
| 110 | 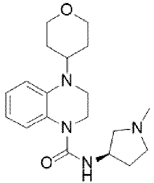 | (*R*)-*N*-(1-methylpyrrolidin-3-yl)-4-(tetrahydro-2H-pyran-4-yl)-3,4-dihydroquinoxaline-1(2H)-carboxamide |
| 111 | 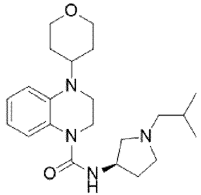 | (*R*)-*N*-(1-isobutylpyrrolidin-3-yl)-4-(tetrahydro-2H-pyran-4-yl)-3,4-dihydroquinoxaline-1(2H)-carboxamide |
| 112 | 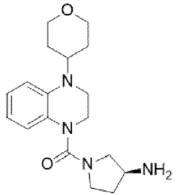 | (*S*)-(3-aminopyrrolidin-1-yl)(4-(tetrahydro-2H-pyran-4-yl)-3,4-dihydroquinoxalin-1(2H)-yl)methanone |
| 113 | 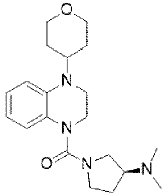 | (*S*)-(3-(dimethylamino)pyrrolidin-1-yl)(4-(tetrahydro-2H-pyran-4-yl)-3,4-dihydroquinoxalin-1(2H)-yl)methanone |
| 114 | 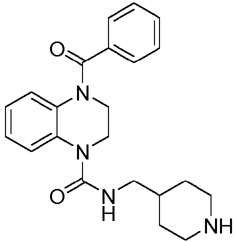 | 4-benzoyl-*N*-(piperidin-4-ylmethyl)-3,4-dihydroquinoxaline-1(2H)-carboxamide |
| 115 | 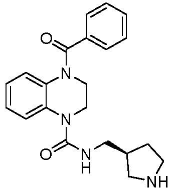 | (*S*)-4-benzoyl-N-(pyrrolidin-3-ylmethyl)-3,4-dihydroquinoxaline-1(2H)-carboxamide |
| 116 | 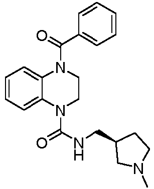 | (*R*)-4-benzoyl-*N*-((1-methylpyrrolidin-3-yl)methyl)-3,4-dihydroquinoxaline-1(2H)-carboxamide |
| 117 | 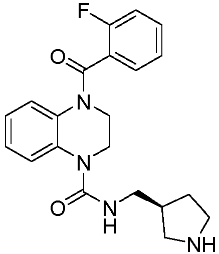 | (*S*)-4-(2-fluorobenzoyl)-*N*-(pyrrolidin-3-ylmethyl)-3,4-dihydroquinoxaline-1(2H)-carboxamide |
| 118 | 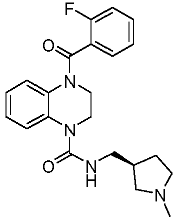 | (*R*)-4-(2-fluorobenzoyl)-*N*-((1-methylpyrrolidin-3-yl)methyl)-3,4-dihydroquinoxaline-1(2H)-carboxamide |
| 119 | 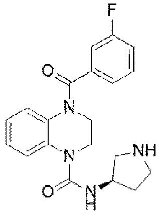 | (*R*)-4-(3-fluorobenzoyl)-*N*-(pyrrolidin-3-yl)-3,4-dihydroquinoxaline-1(2H)-carboxamide |
| 120 | 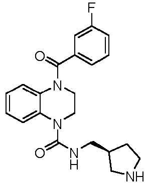 | (*S*)-4-(3-fluorobenzoyl)-*N*-(pyrrolidin-3-yl)methyl)-3,4-dihydroquinoxaline-1(2H)-carboxamide |
| 121 | 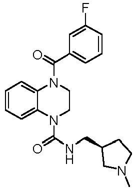 | (*R*)-4-(3-fluorobenzoyl)-*N*-((1-methylpyrrolidin-3-yl)methyl)-3,4-dihydroquinoxaline-1(2H)-carboxamide |
| 122 | 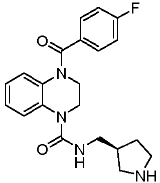 | (*S*)-4-(4-fluorobenzoyl)-*N*-(pyrrolidin-3-ylmethyl)-3,4-dihydroquinoxaline-1(2H)-carboxamide |
| 123 | 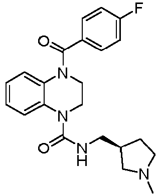 | (*R*)-4-(4-fluorobenzoyl)-*N*-((1-methylpyrrolidin-3-yl)methyl)-3,4-dihydroquinoxaline-1(2H)-carboxamide |
| 124 | 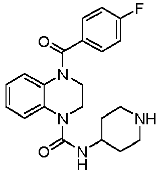 | 4-(4-fluorobenzoyl-*N*-(piperidin-4-yl)-3,4-dihydroquinoxaline-1(2H)-carboxamide |
| 125 | 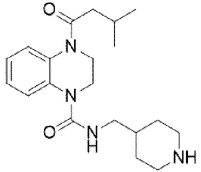 | 4-(3-methylbutanoyl)-*N*-(piperidin-4-ylmethyl)-3,4-dihydroquinoxaline-1(2H)-carboxamide |
| 126 | 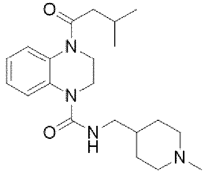 | 4-(3-methylbutanoyl)-*N*-((1-methylpiperidin-4-yl)methyl)-3,4-dihydroquinoxaline-1(2H)-carboxamide |
| 127 | 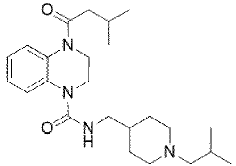 | *N*-((1-isobutylpiperidin-4-yl)methyl)-4-(3-methylbutanoyl)-3,4-dihydroquinoxaline-1(2H)-carboxamide |
| 128 | 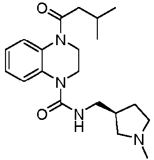 | (*R*)-4-(3-methylbutanoyl)-*N*-((1-methylpyrrolidin-3-yl)methyl)-3,4-dihydroquinoxaline-1(2H)-carboxamide |
| 129 | 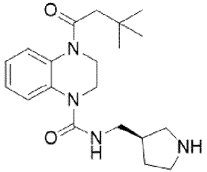 | (*R*)-4-(3,3-dimethylbutanoyl)-*N*-(pyrrolidin-3-ylmethyl)-3,4-dihydroquinoxaline-1(2H)-carboxamide |
| 130 | 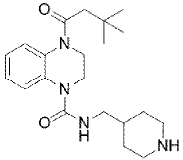 | 4-(3,3-dimethylbutanoyl)-*N*-(piperidin-4-ylmethyl)-3,4-dihydroquinoxaline-1(2H)-carboxamide |

### Experimental Example 2. Evaluation of activity of inhibiting actin polymerization in vitro

Actin polymerization, which is the process of forming an actin filament from actin monomers, is involved in regulating cell movement, but may also promote excessive α-SMA expression, and induce the activation of myofibroblasts, causing fibrosis of lung tissue. Accordingly, in order to confirm the actin polymerization regulating function of the compounds of the present invention, an *in vitro* actin polymerization test was performed. As a representative example, the actin polymerization inhibitory activities of Compounds 32 and 42 were measured.

Specifically, a globular actin (G-actin) stock with pyrene was diluted with a general actin buffer (5 mM Tris-HCl pH 8.0, 0.2 mM CaCl₂) and incubated at 4 °C for 1 hour to produce globular actin monomers. Afterward, 80% of a supernatant was collected by performing centrifugation at 20,000 ×g and 4 °C for 3 hours, and 0.1 mg/mL of G-actin monomers were prepared. An Arp2/3 complex, a VCA domain protein, and Compounds 32 and 42 were added together with the G-actin monomers to an actin polymerization buffer and reacted at room temperature for 1 hour. Increased fluorescence during the reaction of the G-actin monomers to a fibrous actin polymer was measured at 360 nm (excitation)/407 nm (emission) using a microplate reader. The degree of actin polymerization was expressed as area under the curve (AUC).

As a result, as shown in FIG. 1A, it was confirmed that actin polymerization activity in the experimental group treated with the compound of the present invention was reduced compared to an untreated control group, and relative fluorescence units (RFUs), which reflect the rate of actin polymerization over time, were also lower in the experimental group treated with the compound of the present invention compared to the control group (FIG. 1B). This result shows that the compound according to the present invention effectively inhibits the actin polymerization reaction.

### Experimental Example 3. Evaluation of effect of compound according to the present invention in inhibition of F-actin and α-SMA formation in cells

It is known that when the process of pulmonary fibrosis results in structural changes in cells, the level of F-actin increases. Therefore, to confirm the therapeutic efficacy of the compounds of the present invention for pulmonary fibrosis-related diseases, the structural changes of cells after treating fibrosis-induced MRC5 with the compounds of the present invention were measured by the degree of inhibition of F-actin and α-SMA formation.

Specifically, MRC5 cells were treated with Compound 32 (10 µM) or Compound 42 (10 µM) according to the present invention, CK-666 (actin polymerization inhibitor, 10 µM), nintedanib (1 µM), or DMSO, which had been dissolved in a 0.5% fetal bovine serum (FBS)-containing medium, and 2 hours later, the cells were treated with TGF-β1 (5 ng/mL) to cause fibrosis. Due to strong cytotoxicity of the drug of nintedanib, all cells die when treated with nintedanib at the same concentration (10 µM) as that of the compound of the present invention, so it is impossible to conduct a comparative experiment. Therefore, the effect was confirmed by treating the cells with 1 µM nintedanib. 70 hours later, the cells were fixed with a 2% paraformaldehyde solution for 10 minutes, treated with a 0.1% Triton X-100 solution for 10 minutes to provide cell membrane permeability, and treated with a 1% bovine serum albumin (BSA) solution to prevent non-specific binding. Immunostaining was able to confirm an increase or decrease in F-actin by the compounds of the present invention using anti-GAPDH (BIO-RAD) as a primary antibody, Alexa Fluor^{™} Plus 488 for GAPDH labeling as a secondary antibody, and rhodamine phalloidin (cytoskeleton) for staining the cytoskeleton, particularly, F-actin. High-resolution F-actin images acquired with a STELLARIS 5 confocal microscope (Leica) were quantitatively represented by standardizing F-actin stained with rhodamine phalloidin using Alexa Fluor^{™} Plus 488-labeled GAPDH using self-developed analysis software through programming. To measure the degree of inhibition of α-SMA expression, α-SMA was stained with an anti-α-SMA-FITC antibody (Sigma-Aldrich), which was able to confirm an increase or decrease in α-SMA by the compounds. High-resolution α-SMA images acquired with a STELLARIS 5 confocal microscope (Leica) were quantitatively represented by standardizing α-SMA stained with an anti-α-SMA-FITC antibody with Alexa Fluor^{™} Plus 488-labeled GAPDH using self-developed analysis software through programming.

The degree of inhibition of α-SMA and F-actin formation by the compound according to the present invention is shown in FIGS. 2A to 2D. TGF-β1-treated cells showed a significant increase in intracellular F-actin and α-SMA formation compared to a control (control material). On the other hand, in the cells treated with both Compound 32 and Compound 42 of the present invention, intracellular F-actin and α-SMA formation decreased to a level similar to that in the group not treated with TGF-β1, confirming that the compounds of the present invention can significantly inhibit F-actin and α-SMA formation by TGF-β1. In addition, it was seen that the compounds of the present invention more strongly inhibit F-actin and α-SMA formation compared with CK-666 known as an actin polymerization inhibitor. In addition, it was seen that nintedanib, which is used as a therapeutic agent for idiopathic pulmonary fibrosis, did not inhibit F-actin formation, and the effect of inhibiting α-SMA formation was lower than Compounds 32 and 42 of the present invention. These results show that the compounds according to the present invention can effectively inhibit F-actin and α-SMA formation, which is the key factor of myofibroblast activation and pulmonary fibrosis.

### Experimental Example 4. Confirmation of effect of compound according to the present invention on the inhibition of α-SMA expression

The ability of the compounds according to the present invention to inhibit the expression of α-SMA, which is a biomarker for pulmonary fibrosis, was evaluated by an image screening method. MRC5 cells were treated with the compound (10 µM) or DMSO under the condition of TGF-β1 (5 ng/mL). 72 hours later, the cells were fixed with a 4% formaldehyde solution, treated with a 0.1% Triton-x100 solution, and treated with a 1% BSA solution to mask non-specific binding caused by antibodies. Anti-α-SMA-FITC (Sigma Aldrich) and anti-GAPDH (Cell Signaling Technology) were used as primary antibodies, and anti-mouse-TRITC was used as a secondary antibody. Imaging and analysis of the results were performed using Cytation5_Gen5_Version 3.12.08 (Bio Tek). Relative to the α-SMA expression level when TGF-β1 was treated alone, the α-SMA expression level when both the compound and TGF-β1 were treated was calculated as the ability of α-SMA expression inhibition of the corresponding compound. The ability of α-SMA expression inhibition of each compound is summarized in Table 2 below (A: Inhibitory activity≥70%, B: 70%> Inhibitory activity ≥50%, C: 50%>Inhibitory activity≥30%, D: 30%>Inhibitory activity≥10%).

**[Table 2]**

| **Compound** | **Inhibitory activity** | **Compound** | **Inhibitory activity** |
|---|---|---|---|
| 1 | A | 55 | C |
| 2 | A | 56 | C |
| 3 | A | 57 | C |
| 4 | A | 58 | C |
| 5 | D | 59 | C |
| 6 | D | 60 | B |
| 7 | D | 61 | B |
| 8 | A | 62 | C |
| 9 | B | 63 | C |
| 10 | A | 64 | A |
| 11 | C | 65 | C |
| 12 | C | 66 | B |
| 13 | A | 67 | C |
| 14 | C | 68 | C |
| 15 | D | 69 | D |
| 16 | D | 70 | D |
| 17 | C | 71 | C |
| 18 | B | 72 | C |
| 19 | B | 73 | C |
| 20 | A | 74 | C |
| 21 | A | 79 | D |
| 22 | A | 80 | D |
| 23 | A | 81 | D |
| 24 | A | 82 | D |
| 25 | B | 83 | D |
| 26 | B | 84 | C |
| 27 | A | 85 | C |
| 28 | D | 86 | D |
| 29 | B | 87 | B |
| 30 | B | 88 | B |
| 31 | B | 89 | B |
| 32 | A | 90 | C |
| 33 | B | 91 | B |
| 34 | B | 93 | B |
| 35 | A | 95 | A |
| 36 | B | 96 | C |
| 37 | C | 97 | D |
| 38 | D | 98 | C |
| 40 | D | 99 | C |
| 41 | C | 100 | C |
| 42 | B | 101 | C |
| 43 | A | 102 | C |
| 45 | B | 104 | D |
| 46 | C | 106 | C |
| 47 | B | 107 | D |
| 48 | D | 108 | C |
| 49 | D | 109 | C |
| 50 | C | 114 | D |
| 52 | D | 125 | D |
| 53 | D | 129 | D |
| 54 | C | 130 | D |

The above results show that the compounds according to the present invention can exhibit an excellent pulmonary fibrosis inhibitory effect by inhibiting the expression of α-SMA, which is one of the major causes of pulmonary fibrosis. Particularly, it is known that nintedanib, which is used as a therapeutic agent for pulmonary fibrosis, does not suppress α-SMA expression (i.e., α-SMA inhibitory activity of nintedanib = 0%). The compounds of the present invention can not only lower cytotoxicity compared to nintedanib, but also effectively inhibit the expression of α-SMA, which is the main factor of pulmonary fibrosis, indicating that the compounds of the present invention can be used as a novel therapeutic agent for inflammation and/or fibrosis-related pulmonary diseases.

### Experimental Example 5. Evaluation of efficacy of compounds on macrophages

IL-6 is one of the pro-inflammatory cytokines secreted by activated macrophages, M1 macrophages, and is a potent trigger of fibrosis. Therefore, to evaluate the inhibitory activity of the compounds according to the present invention to inhibit pulmonary fibrosis through the inhibition of inflammatory effects, the extent of IL-6 secretion by macrophages after treatment of activated macrophages with the compounds was compared.

First, macrophages were prepared through the following process: 1 mL of a medium containing 5×10⁵ cells/mL of THP-1 cells (Korean Cell Line Bank; KCLB NO.40202) grown in a complete medium (a medium in which Roswell Park Memorial Institute [RPMI] is supplemented with 10% [vol/vol] fetal bovine serum [FBS] and 1% [vol/vol] penicillin/streptomycin [P/S]) was dispensed by 1 mL into each well of a 12-well plate. Here, after adding 200 nM phorbol 12-myristate 13-acetate (PMA), the cells were incubated in an incubator maintained at 37 °C and 5% (vol/vol) CO₂ for 48 hours to induce differentiation into macrophages. The complete medium was removed from the plate, the cells were washed twice with phosphate-buffered saline (PBS) by 1 mL, and a fresh complete medium was added by 1 mL to each well. When differentiation into M1 macrophages was induced, lipopolysaccharide (LPS) extracted from O111:B4, which is the subtype of *Escherichia coli,* was treated at 10 ng/mL for 6 hours. As needed, Compounds 32 and 42 were treated at 1 µM or 10 µM in the same manner as LPS.

To measure a cytokine level in the medium, the medium was harvested from the cultured cells, and the complete medium contained in the 12-well plate on the day of medium harvest was transferred to a 1.5 mL microcentrifuge tube.

Subsequently, the measurement of a cytokine level was performed through the following process: the secured complete medium was centrifuged at 13,000 RPM for 10 minutes in a 4 °C centrifuge, and then a supernatant was transferred to a new 1.5 mL microcentrifuge tube. A portion of the sample was taken and mixed with a cytometric bead array (CBA) human soluble protein master buffer kit (BD Biosciences) and a human IL-6 FlexSet kit (BD Bioscience), and then the resulting mixture was stirred at room temperature for 1 hour while blocking light. To remove cytokines not binding to the beads provided in the kits, the resulting mixture was centrifuged at 200 ×g for 5 minutes and then washed with a 500 µL washing buffer. After centrifugation at 200 ×g for 5 minutes again, a supernatant was removed and then well suspended with 150 µL of washing buffer. The amount of IL-6 binding to the beads was measured using a Novocyte 3000 flow cytometer (Agilent).

Evaluation results are shown in FIG. 3. M1 macrophages derived from THP-1 showed a significant increase in IL-6 secretion ability when treated with LPS for 6 hours, but a decrease in IL-6 production when treated with Compounds 32 and 42 of the present invention in addition to LPS. The above results show that the compounds according to the present invention can inhibit pulmonary fibrosis by inhibiting the IL-6 production ability of activated macrophages.

It should be understood by those of ordinary skill in the art that the above descriptions of the present invention are exemplary, and the embodiments disclosed herein can be easily modified into other specific forms without changing the technical spirit or essential features of the present invention. Therefore, it should be interpreted that the embodiments described above are exemplary in all aspects, and are not limitative.

### Industrial Applicability

The present invention relates to a novel compound useful for the preparation of a medicine capable of treating related diseases by regulating the function and differentiation of the cytoskeleton involved in the process of pulmonary fibrosis. It was confirmed that the novel compound according to the present invention or a pharmaceutically acceptable salt thereof is effective in controlling actin polymerization required for changing the cytoskeleton, and the expression of α-SMA and F-actin, which are the major causative factors of fibrosis in lung tissue-derived fibroblasts, was effectively suppressed. In addition, it was confirmed that when activated macrophages were treated with the compound, the level of inflammatory cytokine generated by macrophages significantly decreases. Accordingly, the novel compound according to the present invention is expected to be useful in the prevention and treatment of various pulmonary fibrosis-related diseases as an agent effective in inhibiting or improving pulmonary fibrosis.

## Claims

1. A compound represented by Chemical Formula 1 below, a stereoisomer thereof, or a pharmaceutically acceptable salt thereof: 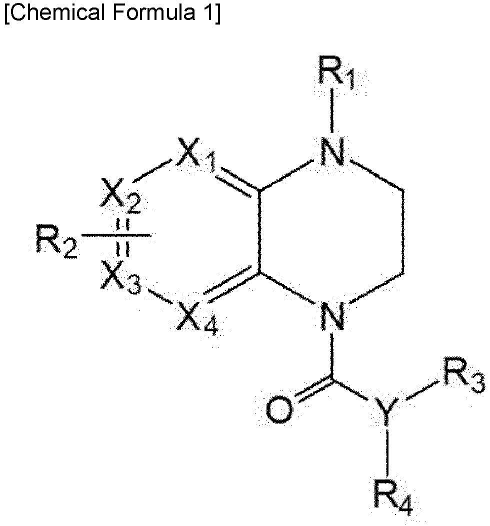 (In Chemical Formula 1,
X₁ to X₄ are each independently N or C;
Y is N, S, or O;
R₁ is C₁-C₁₀ alkyl, C₃-C₂₀ cycloalkyl, C₂-C₂₀ heterocycloalkyl, a 3- to 10-membered cyclic aromatic group, a 3- to 10-membered heterocyclic aromatic group, -CO-(C₁-C₆ alkyl), or -CO-(halogen-substituted or unsubstituted C₆-C₁₂ aryl), and
at least one H of R₁ is substituted with halogen, a cyano group, halogen-substituted or unsubstituted C₆-C₁₂ aryl, or halogen-substituted or unsubstituted C₅-C₁₂ heteroaryl;
when all of X₁ to X₄ are C, R₂ is hydrogen, halogen, or C₁-C₅ alkyl, and when one or more of X₁ to X₄ are N, R₂ is hydrogen;
R₃ and R₄ are linked to each other to form a 5- or 6-membered ring, wherein at least one H in the ring is substituted with -OH, -NH₂, dimethylamine, -NH-(C₁-C₅ alkyl), -NH-(C₁-C₅ alkoxy), -NH-COCH₃, -NH-SO₂CH₃, C₁-C₅ alkyl, or C₁-C₅ alkoxy, and
when R₃ and R₄ do not form a ring, R₄ is hydrogen or C₁-C₅ alkyl, R₃ is C₁-C₁₀ alkyl, C₃-C₁₀ cycloalkyl, C₂-C₁₀ heterocycloalkyl, -CH₂-(C₃-C₆ cycloalkyl), -CH₂-(C₂-C₆ heterocycloalkyl), or -CH₂-(3- to 6-membered heterocyclic aromatic group), at least one H of R₃ is substituted with -OH, C₁-C₅ alkyl, acetyl, C₁-C₅ alkoxy, -COCF₃, or -SO₂CH₃; and
the heterocycloalkyl, heterocyclic group, and heteroaryl each independently include one or more hetero atoms selected from the group consisting of N, O, P, and S.)

2. The compound of claim 1, wherein R₃ and R₄ are linked to each other to form a 5- or 6-membered heterocycloalkyl, which includes one or two N atoms in the ring, wherein Y is N, and
at least one H in the 5- or 6-membered heterocycloalkyl formed by R₃ and R₄ is substituted with -OH, -NH₂, dimethylamine, -NH-(C₁-C₅ alkyl), -NH-(C₁-C₅ alkoxy), -NH-COCH₃, -NH-SO₂CH₃, C₁-C₅ alkyl, or C₁-C₅ alkoxy.

3. The compound of claim 1, wherein R₁ is a 6-membered cyclic aromatic group, 6-membered heterocyclic aromatic group, -CH₂-aryl, -CH₂-heteroaryl, 6-membered cycloalkyl, 6-membered heterocycloalkyl, -CO-aryl, or -CO-(C₄-C₅ alkyl), wherein the 6-membered heterocyclic aromatic group includes at least one N, and the 6-membered heterocycloalkyl includes at least one O, and
at least one H of R₁ is substituted with halogen, a cyano group, halogen-substituted or unsubstituted C₆-C₁₂ aryl, or halogen-substituted or unsubstituted C₅-C₁₂ heteroaryl.

4. The compound of claim 1, wherein the compound represented by Chemical Formula 1 is any one selected from the group consisting of the following compounds:
(1) (R)-6-fluoro-4-(4-fluorophenyl)-N-(pyrrolidin-3-yl)-3,4-dihydroquinoxaline-1 (2H)-carboxamide;
(2) (R)-6-fluoro-4-(4-fluorophenyl)-N-(1-methylpyrrolidin-3-yl)-3,4-dihydroquinoxaline-1(2H)-carboxamide;
(3) (R)-6-fluoro-4-(4-fluorophenyl)-N-(1-isopropylpyrrolidin-3-yl)-3,4-dihydroquinoxaline-1(2H)-carboxamide;
(4) (*R*)-6-fluoro-4-(4-fluorophenyl)-*N*-(1-isobutylpyrrolidin-3-yl)-3,4-dihydroquinoxaline-1(2H)-carboxamide;
(5) (*R*)-6-fluoro-4-(4-fluorophenyl)-N-(1-(2,2,2-trifluoroacetyl)pyrrolidin-3-yl)-3,4-dihydroquinoxaline-1(2H)-carboxamide;
(6) (*R*)-*N*-(1-acetylpyrrolidin-3-yl)-6-fluoro-4-(4-fluorophenyl)-3,4-dihydroquinoxaline-1(2H)-carboxamide;
(7) (*R*)-6-fluoro-4-(4-fluorophenyl)-N-(1-(methylsulfonyl)pyrrolidin-3-yl)-3,4-dihydroquinoxaline-1(2H)-carboxamide;
(8) (*S*)-6-fluoro-4-(4-fluorophenyl)-N-(pyrrolidin-3-yl)-3,4-dihydroquinoxaline-1 (2H)-carboxamide;
(9) (*S*)-6-fluoro-4-(4-fluorophenyl)-N-(1-methylpyrrolidin-3-yl)-3,4-dihydroquinoxaline-1(2H)-carboxamide;
(10) (*S*)-6-fluoro-4-(4-fluorophenyl)-N-(1-isopropylpyrrolidin-3-yl)-3,4-dihydroquinoxaline-1(2H)-carboxamide;
(11) (*S*)-(3-aminopyrrolidin-1-yl)(6-fluoro-4-(4-fluorophenyl)-3,4-dihydroquinoxalin-1(2H)-yl)methanone;
(12) (*S*)-(6-fluoro-4-(4-fluorophenyl)-3,4-dihydroquinoxalin-1(2H)-yl)(3-(methylamino)pyrrolidin-1-yl)methanone;
(13) (*S*)-(6-fluoro-4-(4-fluorophenyl)-3,4-dihydroquinoxalin-1(2H)-yl)(3-(isobutylamino)pyrrolidin-1-yl)methanone;
(14) (*S*)-(6-fluoro-4-(4-fluorophenyl)-3,4-dihydroquinoxalin-1(2H)-yl)(3-(isopropylamino)pyrrolidin-1-yl)methanone;
(15) (*S*)-N-(1-(6-fluoro-4-(4-fluorophenyl)-1,2,3,4-tetrahydroquinoxaline-1-carbonyl)pyrrolidin-3-yl)acetamide;
(16) (*S*)-N-(1-(6-fluoro-4-(4-fluorophenyl)-1,2,3,4-tetrahydroquinoxaline-1-carbonyl)pyrrolidin-3-yl)methanesulfonamide;
(17) (*S*)-6-fluoro-4-(4-fluorophenyl)-N-(pyrrolidin-3-ylmethyl)-3,4-dihydroquinoxaline-1(2H)-carboxamide;
(18) (*R*)-6-fluoro-4-(4-fluorophenyl)-N-((1-methylpyrrolidin-3-yl)methyl)-3,4-dihydroquinoxaline-1(2H)-carboxamide;
(19) (*R*)-6-fluoro-4-(4-fluorophenyl)-N-((1-isopropylpyrrolidin-3-yl)methyl)-3,4-dihydroquinoxaline-1(2H)-carboxamide;
(20) (*R*)-6-fluoro-4-(4-fluorophenyl)-*N*-((1-isobutylpyrrolidin-3-yl)methyl)-3,4-dihydroquinoxaline-1(2H)-carboxamide;
(21) (R)-6-fluoro-4-(4-fluorophenyl)-N-(pyrrolidin-2-ylmethyl)-3,4-dihydroquinoxaline-1(2H)-carboxamide;
(22) (*R*)-6-fluoro-4-(4-fluorophenyl)-N-((1-methylpyrrolidin-2-yl)methyl)-3,4-dihydroquinoxaline-1 (2H)-carboxamide;
(23) (*R*)-6-fluoro-4-(4-fluorophenyl)-N-((1-isopropylpyrrolidin-2-yl)methyl)-3,4-dihydroquinoxaline-1 (2H)-carboxamide;
(24) (*R*)-6-fluoro-4-(4-fluorophenyl)-*N*-((1-isobutylpyrrolidin-2-yl)methyl)-3,4-dihydroquinoxaline-1 (2H)-carboxamide;
(25) (*S*)-6-fluoro-4-(4-fluorophenyl)-N-((1-methylpyrrolidin-3-yl)methyl)-3,4-dihydroquinoxaline-1 (2H)-carboxamide;
(26) (*S*)-6-fluoro-4-(4-fluorophenyl)-N-((1-isopropylpyrrolidin-3-yl)methyl)-3,4-dihydroquinoxaline-1 (2H)-carboxamide;
(27) (*S*)-6-fluoro-4-(4-fluorophenyl)-*N*-((1-isobutylpyrrolidin-3-yl)methyl)-3,4-dihydroquinoxaline-1(2H)-carboxamide;
(28) *N*-(cyclopropylmethyl)-6-fluoro-4-(4-fluorophenyl)-3,4-dihydroquinoxaline-1 (2H)-carboxamide;
(29) 6-fluoro-4-(4-fluorophenyl)-*N*-(piperidin-4-yl)-3,4-dihydroquinoxaline-1(2H)-carboxamide;
(30) 6-fluoro-4-(4-fluorophenyl)-*N*-(1-methylpiperidin-4-yl)-3,4-dihydroquinoxaline-1(2H)-carboxamide;
(31) 6-fluoro-4-(4-fluorophenyl)-*N*-(1-isopropylpiperidin-4-yl)-3,4-dihydroquinoxaline-1(2H)-carboxamide;
(32) 6-fluoro-4-(4-fluorophenyl)-*N*-(1-isobutylpiperidin-4-yl)-3,4-dihydroquinoxaline-1(2H)-carboxamide;
(33) 6-fluoro-4-(4-fluorophenyl)-*N*-(piperidin-4-ylmethyl)-3,4-dihydroquinoxaline-1 (2H)-carboxamide;
(34) 6-fluoro-4-(4-fluorophenyl)-*N*-(methylpiperidin-4-yl)methyl)-3,4-dihydroquinoxaline-1 (2H)-carboxamide;
(35) 6-fluoro-4-(4-fluorophenyl)-*N*-((1-isobutylpiperidin-4-yl)methyl)-3,4-dihydroquinoxaline-1(2H)-carboxamide;
(36) 6-fluoro-4-(4-fluorophenyl)-*N*-((1-isopropylpiperidin-4-yl)methyl)-3,4-dihydroquinoxaline-1(2H)-carboxamide;
(37) *N*-((1H-imidazol-4-)methyl)-6-fluoro-4-(4-fluorophenyl)-3,4-dihydroquinoxaline-1(2H)-carboxamide;
(38) 6-fluoro-4-(4-fluorophenyl)-N-(pyrazin-2-ylmethyl)-3,4-dihydroquinoxaline-1 (2H)-carboxamide;
(39) (*R*)-(6-fluoro-4-(4-fluorophenyl)-3,4-dihydroquinoxalin-1(2H)-yl)(3-hydroxypyrrolidin-1-yl)methanone;
(40) (6-fluoro-4-(4-fluorophenyl)-3,4-dihydroquinoxalin-1(2H)-yl)(3-methoxypyrrolidin-1-yl)methanone;
(41) *N*-(azetidin-3-yl)-6-fluoro-4-(4-fluorophenyl)-3,4-dihydroquinoxaline-1(2H)-carboxamide;
(42) 6-fluoro-4-(4-fluorophenyl)-*N*-(1-isopropylazetidin-3-yl)-3,4-dihydroquinoxaline-1(2H)-carboxamide;
(43) 6-fluoro-4-(4-fluorophenyl)-*N*-(1-isobutylazetidin-3-yl)-3,4-dihydroquinoxaline-1(2H)-carboxamide;
(44) *N*-(1-acetylazetidin-3-yl)-6-fluoro-4-(4-fluorophenyl)-3,4-dihydroquinoxaline-1 (2H)-carboxamide;
(45) 6-fluoro-4-(4-fluorophenyl)-*N*-((1-methylazetidin-3-yl)methyl)-3,4-dihydroquinoxaline-1(2H)-carboxamide;
(46) 6-fluoro-4-(4-fluorophenyl)-*N*-((1-isopropylazetidin-3-yl)methyl)-3,4-dihydroquinoxaline-1(2H)-carboxamide;
(47) 6-fluoro-4-(4-fluorophenyl)-*N*-((1-isobutylazetidin-3-yl)methyl)-3,4-dihydroquinoxaline-1(2H)-carboxamide;
(48) 6-fluoro-4-(4-fluorophenyl)-N-((tetrahydrofuran-3-yl)methyl)-3,4-dihydroquinoxaline-1(2H)-carboxamide;
(49) (*S*)-4-(4-fluorophenyl)-*N*-(pyrrolidin-3-yl)-3,4-dihydroquinoxaline-1(2H)-carboxamide;
(50) 4-(4-fluorophenyl)-*N*-(piperidin-4-yl)-3,4-dihydroquinoxaline-1(2H)-carboxamide;
(51) (*S*)-4-(4-fluorophenyl)-*N*-(tetrahydrofuran-3-yl)-3,4-dihydroquinoxaline-1 (2H)-carboxamide;
(52) 4-(4-fluorophenyl)-*N*-((tetrahydro-2H-pyran-4-yl)methyl)-3,4-dihydroquinoxaline-1(2H)-carboxamide;
(53) (*S*)-4-(4-fluorophenyl)-*N*-(pyrrolidin-3-ylmethyl)-3,4-dihydroquinoxaline-1(2H)-carboxamide;
(54) (*R*)-4-(4-fluorophenyl)-N-((1-methylpyrrolidin-3-yl)methyl)-3,4-dihydroquinoxaline-1(2H)-carboxamide;
(55) (*R*)-4-(4-fluorophenyl)-N-((1-isopropylpyrrolidin-3-yl)methyl)-3,4-dihydroquinoxaline-1(2H)-carboxamide;
(56) (*R*)-4-(4-fluorophenyl)-*N*-((1-isobutylpyrrolidin-3-yl)methyl)-3,4-dihydroquinoxaline-1(2H)-carboxamide;
(57) (*R*)-4-(4-fluorophenyl)-*N*-(pyrrolidin-2-ylmethyl)-3,4-dihydroquinoxaline-1(2H)-carboxamide;
(58) (*S*)-4-(4-fluorophenyl)-*N*-((1-methylpyrrolidin-3-yl)methyl)-3,4-dihydroquinoxaline-1(2H)-carboxamide;
(59) (*S*)-4-(4-fluorophenyl)-*N*-((1-isopropylpyrrolidin-3-yl)methyl)-3,4-dihydroquinoxaline-1(2H)-carboxamide;
(60) (*S*)-4-(4-fluorophenyl)-*N*-((1-isobutylpyrrolidin-3-yl)methyl)-3,4-dihydroquinoxaline-1(2H)-carboxamide;
(61) 4-(4-fluorophenyl)-*N*-((1-isobutylazetidin-3-yl)methyl)-3,4-dihydroquinoxaline-1(2H)-carboxamide;
(62) 4-(4-fluorophenyl)-*N*-(piperidin-4-ylmethyl)-3,4-dihydroquinoxaline-1(2H)-carboxamide;
(63) 4-(4-fluorophenyl)-*N*-((1-isopropylpiperidin-4-yl)methyl)-3,4-dihydroquinoxaline-1(2H)-carboxamide;
(64) 4-(4-fluorophenyl)-*N*-((1-isobutylpiperidin-4-yl)methyl)-3,4-dihydroquinoxaline-1(2H)-carboxamide;
(65) (*R*)-6-fluoro-4-phenyl-*N*-(pyrrolidin-3-yl)-3,4-dihydroquinoxaline-1(2H)-carboxamide;
(66) (4-(2-chlorophenyl)-3,4-dihydroquinoxalin-1(2H)-yl)(piperidin-1-yl)methanone;
(67) 4-(2-chlorophenyl)-3,4-dihydroquinoxalin-1(2H)-yl)(pyrrolidin-1-yl)methanone;
(68) (4-(2-chlorophenyl)-3,4-dihydroquinoxalin-1(2H)-yl)(4-methylpiperazin-1-yl)methanone;
(69) (*S*)-N-(1-isopropylpyrrolidin-3-yl)-4-(pyridin-2-yl)-3,4-dihydroquinoxaline-1(2H)-carboxamide;
(70) (*R*)-*N*-(1-acetylpyrrolidin-3-yl)-4-(pyridin-2-yl)-3,4-dihydroquinoxaline-1(2H)-carboxamide;
(71) (*S*)-4-(pyridin-2-yl)-*N*-(pyrrolidin-3-ylmethyl)-3,4-dihydroquinoxaline-1(2H)-carboxamide;
(72) (*R*)-4-(pyridin-2-yl)-*N*-(pyrrolidin-2-ylmethyl)-3,4-dihydroquinoxaline-1(2H)-carboxamide;
(73) (*R*)-4-(pyridin-3-yl)-*N*-(tetrahydrofuran-3-yl)-3,4-dihydroquinoxaline-1(2H)-carboxamide;
(74) *N*-(cyclopropylmethyl)-4-(pyridin-4-yl)-3,4-dihydroquinoxaline-1(2H)-carboxamide;
(75) (*R*)-4-(5-fluoropyridin-2-yl)-*N*-(1-methylpyrrolidin-3-yl)-3,4-dihydroquinoxaline-1(2H)-carboxamide;
(76) (*R*)-4-(5-fluoropyridin-2-yl)-*N*-(1-methylpyrrolidin-3-yl)methyl)-3,4-dihydroquinoxaline-1(2H)-carboxamide;
(77) 4-(5-fluoropyridin-2-yl)-*N*-(1-methylpiperidin-4-yl)methyl)-3,4-dihydroquinoxaline-1(2H)-carboxamide;
(78) (*S*)-(3-aminopyrrolidin-1-yl)(4-(5-fluoropyridin-2-yl)-3,4-dihydroquinoxaline-1(2H)-methanone;
(79) (*S*)-4-(4-cyanophenyl)-N-(pyrrolidin-3-ylmethyl)-3,4-dihydroquinoxaline-1(2H)-carboxamide;
(80) (*S*)-4-(pyrazin-2-yl)-*N*-(pyrrolidin-3-yl)-3,4-dihydroquinoxaline-1(2H)-carboxamide;
(81) (*S*)-N-(1-isopropylpyrrolidin-3-yl)-4-(pyrazin-2-yl)-3,4-dihydroquinoxaline-1 (2H)-carboxamide;
(82) *N*-(1-isobutylpiperidin-4-yl)-4-(pyrazin-2-yl)-3,4-dihydroquinoxaline-1(2H)-carboxamide;
(83) *N*-(1-isopropylpiperidin-4-yl)-4-(pyrazin-2-yl)-3,4-dihydroquinoxaline-1(2H)-carboxamide;
(84) (*R*)-4-benzyl-*N*-(pyrrolidin-3-yl)-3,4-dihydroquinoxaline-1(2H)-carboxamide;
(85) (S)-(3-aminopyrrolidin-1-yl)(4-benzyl-3,4-dihydroquinoxalin-1(2H)-yl)methanone;
(86) (S)-(4-benzyl-3,4-dihydroquinoxalin-1(2H)-yl)(3-(methylamino)pyrrolidin-1-yl)methanone;
(87) (*R*)-4-(2-fluorobenzyl)-*N*-(1-methylpyrrolidin-3-yl)-3,4-dihydroquinoxaline-1 (2H)-carboxamide;
(88) 4-(2-fluorobenzyl)-*N*-(1-methylpiperidin-4-yl)-3,4-dihydroquinoxaline-1(2H)-carboxamide;
(89) 4-(2-fluorobenzyl)-*N*-((1-methylpiperidin-4-yl)methyl)-3,4-dihydroquinoxaline-1(2H)-carboxamide;
(90) (*R*)-4-(2-fluorobenzyl)-*N*-((1-methylpyrrolidin-3-yl)methyl)-3,4-dihydroquinoxaline-1(2H)-carboxamide;
(91) (*R*)-4-(4-fluorobenzyl)-*N*-(pyrrolidin-3-yl)-3,4-dihydroquinoxaline-1(2H)-carboxamide;
(92) (*R*)-4-(4-fluorobenzyl)-*N*-(1-isopropylpyrrolidin-3-yl)-3,4-dihydroquinoxaline-1 (2H)-carboxamide;
(93) 4-(4-fluorobenzyl)-*N*-(piperidin-4-yl)-3,4-dihydroquinoxaline-1(2H)-carboxamide;
(94) 4-(4-fluorobenzyl)-*N*-(1-isopropylpiperidin-4-yl)-3,4-dihydroquinoxaline-1(2H)-carboxamide;
(95) (*R*)-4-(4-chlorobenzyl)-*N*-(pyrrolidin-3-yl)-3,4-dihydroquinoxaline-1(2H)-carboxamide;
(96) (*S*)-(3-(isobutylamino)pyrrolidin-1-yl)(4-(pyridin-2-ylmethyl)-3,4-dihydroquinoxalin-1(2H)-yl)methanone;
(97) (*S*)-(3-(dimethylamino)pyrrolidin-1-yl)(4-(pyridin-2-ylmethyl)-3,4-dihydroquinoxalin-1(2H)-yl)methanone;
(98) (*R*)-4-(pyridin-2-ylmethyl)-*N*-(tetrahydrofuran-3-yl)-3,4-dihydroquinoxaline-1(2H)-carboxamide;
(99) *N*-(oxetan-3-yl)-4-(pyridin-2-ylmethyl)-3,4-dihydroquinoxaline-1(2H)-carboxamide;
(100) 4-(pyridin-2-ylmethyl)-*N*-((tetrahydrofuran-3-yl)methyl)-3,4-dihydroquinoxaline-1(2H)-carboxamide;
(101) 4-(pyridin-2-ylmethyl)-*N*-((tetrahydro-2H-pyran-4-yl)methyl)-3,4-dihydroquinoxaline-1(2H)-carboxamide;
(102) *N*-(cyclopropylmethyl)-4-(pyridin-2-ylmethyl)-3,4-dihydroquinoxaline-1 (2H)-carboxamide;
(103) (4-(4-fluorophenyl)-3,4-dihydropyrido[3,4-*b*]pyrazin-1(2H)-yl)(3-hydroxypyrrolidin-1-yl)methanone;
(104) (*R*)-4-cyclohexyl-*N*-(pyrrolidin-3-yl)-3,4-dihydroquinoxaline-1(2H)-carboxamide;
(105) (*R*)-4-cyclohexyl-*N*-(1-methylpyrrolidin-3-yl)-3,4-dihydroquinoxaline-1(2H)-carboxamide;
(106) (*R*)-4-cyclohexyl-*N*-(1-isobutylpyrrolidin-3-yl)-3,4-dihydroquinoxaline-1 (2H)-carboxamide;
(107) (4-cyclohexyl-3,4-dihydroquinoxalin-1(2H)-yl)(4-methylpiperazin-1-yl)methanone;
(108) (4-cyclohexyl-3,4-dihydroquinoxalin-1(2H)-yl)(pyrrolidin-1-yl)methanone;
(109) (4-cyclohexyl-3,4-dihydroquinoxalin-1(2H)-yl)(piperidin-1-yl)methanone;
(110) (*R*)-*N*-(1-methylpyrrolidin-3-yl)-4-(tetrahydro-2H-pyran-4-yl)-3,4-dihydroquinoxaline-1(2H)-carboxamide;
(111) (*R*)-*N*-(1-isobutylpyrrolidin-3-yl)-4-(tetrahydro-2H-pyran-4-yl)-3,4-dihydroquinoxaline-1(2H)-carboxamide;
(112) (*S*)-(3-aminopyrrolidin-1-yl)(4-(tetrahydro-2H-pyran-4-yl)-3,4-dihydroquinoxalin-1(2H)-yl)methanone;
(113) (*S*)-(3-(dimethylamino)pyrrolidin-1-yl)(4-(tetrahydro-2H-pyran-4-yl)-3,4-dihydroquinoxalin-1(2H)-yl)methanone;
(114) 4-benzoyl-*N*-(piperidin-4-ylmethyl)-3,4-dihydroquinoxaline-1(2H)-carboxamide;
(115) (*S*)-4-benzoyl-*N*-(pyrrolidin-3-ylmethyl)-3,4-dihydroquinoxaline-1(2H)-carboxamide;
(116) (*R*)-4-benzoyl-*N*-((1-methylpyrrolidin-3-yl)methyl)-3,4-dihydroquinoxaline-1 (2H)-carboxamide;
(117) (*S*)-4-(2-fluorobenzoyl)-*N*-(pyrrolidin-3-ylmethyl)-3,4-dihydroquinoxaline-1 (2H)-carboxamide;
(118) (*R*)-4-(2-fluorobenzoyl)-*N*-((1-methylpyrrolidin-3-yl)methyl)-3,4-dihydroquinoxaline-1(2H)-carboxamide;
(119) (*R*)-4-(3-fluorobenzoyl)-*N*-(pyrrolidin-3-yl)-3,4-dihydroquinoxaline-1(2H)-carboxamide;
(120) (*S*)-4-(3-fluorobenzoyl)-*N*-(pyrrolidin-3-yl)methyl)-3,4-dihydroquinoxaline-1 (2H)-carboxamide;
(121) (*R*)-4-(3-fluorobenzoyl)-*N*-((1-methylpyrrolidin-3-yl)methyl)-3,4-dihydroquinoxaline-1(2H)-carboxamide;
(122) (*S*)-4-(4-fluorobenzoyl)-*N*-(pyrrolidin-3-ylmethyl)-3,4-dihydroquinoxaline-1(2H)-carboxamide;
(123) (*R*)-4-(4-fluorobenzoyl)-*N*-((1-methylpyrrolidin-3-yl)methyl)-3,4-dihydroquinoxaline-1(2H)-carboxamide;
(124) 4-(4-fluorobenzoyl-*N*-(piperidin-4-yl)-3,4-dihydroquinoxaline-1(2H)-carboxamide;
(125) 4-(3-methylbutanoyl)-*N*-(piperidin-4-ylmethyl)-3,4-dihydroquinoxaline-1 (2H)-carboxamide;
(126) 4-(3-methylbutanoyl)-*N*-((1-methylpiperidin-4-yl)methyl)-3,4-dihydroquinoxaline-1(2H)-carboxamide;
(127) *N*-((1-isobutylpiperidin-4-yl)methyl)-4-(3-methylbutanoyl)-3,4-dihydroquinoxaline-1(2H)-carboxamide;
(128) (*R*)-4-(3-methylbutanoyl)-N-((1-methylpyrrolidin-3-yl)methyl)-3,4-dihydroquinoxaline-1(2H)-carboxamide;
(129) (*R*)-4-(3,3-dimethylbutanoyl)-N-(pyrrolidin-3-ylmethyl)-3,4-dihydroquinoxaline-1(2H)-carboxamide; and
(130) 4-(3,3-dimethylbutanoyl)-*N*-(piperidin-4-ylmethyl)-3,4-dihydroquinoxaline-1 (2H)-carboxamide.

5. A pharmaceutical composition for preventing or treating a pulmonary fibrosis-related disease, comprising the compound represented by Chemical Formula 1 of claim 1, a stereoisomer thereof, or a pharmaceutically acceptable salt thereof as an active ingredient.

6. The pharmaceutical composition of claim 5, wherein the composition inhibits actin polymerization.

7. The pharmaceutical composition of claim 5, wherein the composition inhibits the level or activity of an inflammatory cytokine.

8. The pharmaceutical composition of claim 5, wherein the composition reduces the level or activity of one or more proteins selected from the group consisting of α-SMA, F-actin, and IL-6.

9. The pharmaceutical composition of claim 5, wherein the pulmonary fibrosis-related disease is one or more selected from the group consisting of pulmonary fibrosis, idiopathic pulmonary fibrosis (IPF), desquamative interstitial pneumonia, non-specific interstitial pneumonia, cryptogenic organizing pneumonia, respiratory bronchiolitis-associated interstitial lung disease, acute interstitial pneumonia, lymphocytic interstitial pneumonia, idiopathic pleuroparenchymal fibroelastosis, and chronic obstructive pulmonary disease (COPD).

10. The pharmaceutical composition of claim 5, wherein the pulmonary fibrosis-related disease is a pulmonary fibrosis-related disease accompanied by the overactivation of actin polymerization.

11. A kit for preventing or treating a pulmonary fibrosis-related disease, comprising the composition of any one of claims 5 to 10.

12. A method of preventing or treating a pulmonary fibrosis-related disease, comprising:
administering the compound represented by Chemical Formula 1 of claim 1, a stereoisomer thereof, or a pharmaceutically acceptable salt thereof to a subject in need thereof.

13. A use of the compound represented by Chemical Formula 1 of claim 1, a stereoisomer thereof, or a pharmaceutically acceptable salt thereof for preparing a drug for preventing or treating a pulmonary fibrosis-related disease.

14. A use of the compound represented by Chemical Formula 1 of claim 1, a stereoisomer thereof, or a pharmaceutically acceptable salt thereof for preventing or treating a pulmonary fibrosis-related disease.
